(19) Europäisches Patentamt
European Patent Office
Office européen des brevets

(11) EP 3 845 531 A1

(12) EUROPEAN PATENT APPLICATION
published in accordance with Art. 153(4) EPC

(43) Date of publication:
**07.07.2021 Bulletin 2021/27**

(21) Application number: **19865058.2**

(22) Date of filing: **30.09.2019**

(51) Int Cl.:
***C07D 471/04*** (2006.01) ***C07D 487/04*** (2006.01)
***A61K 31/4985*** (2006.01) ***A61K 31/4162*** (2006.01)
***A61P 35/00*** (2006.01) ***A61P 1/00*** (2006.01)

(86) International application number:
**PCT/CN2019/109502**

(87) International publication number:
**WO 2020/064009 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **30.09.2018 CN 201811162497**

(71) Applicant: **Applied Pharmaceutical Science, Inc.**
**Beijing 100176 (CN)**

(72) Inventors:
- **WANG, Zhijian**
  **Beijing 100176 (CN)**
- **ZHONG, Jun**
  **Beijing 100176 (CN)**
- **WANG, Xuebing**
  **Beijing 100176 (CN)**

(74) Representative: **Gille Hrabal Partnerschaftsgesellschaft mbB**
**Patentanwälte**
**Brucknerstraße 20**
**40593 Düsseldorf (DE)**

# (54) SUBSTITUTED PYRAZOLE FUSED RING DERIVATIVE, PREPARATION METHOD THEREFOR, AND APPLICATION THEREOF

(57) The present disclosure relates to the field of pharmaceutical chemistry, and mainly relates to a compound represented by the following formula I, or the stereoisomer, racemate, tautomer, isotope labelled derivative, nitrogen oxide, pharmaceutically acceptable salt or solvate thereof, a preparation method therefor and a use thereof in the preparation of a medicament for treating a RET kinase-mediated disease.

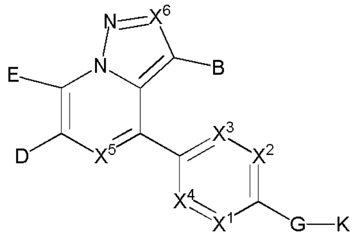

I

EP 3 845 531 A1

## Description

The present application claims the priority right of the prior patent application with the application No. 201811162497.1, entitled "substituted pyrazole fused ring derivative and preparation method and use thereof' filed before the China National Intellectual Property Administration on September 30, 2018. The prior patent application is incorporated herein by reference in its entirety.

## TECHNICAL FIELD

The invention relates to the field of pharmaceutical chemistry, in particular to a substituted pyrazole fused ring derivative, preparation method therefor and use thereof.

## BACKGROUND

The RET (Rearranged during Transfection) proto-oncogene was first confirmed in 1985 by transfection of NIH3T3 (mouse embryo fibroblast cell line) cells with human lymphoma DNA (Cell, 1985, 42(2): 581-588). The RET proto-oncogene is located on chromosome 10q11.2, with a DNA of 60 kb in length, comprises 21 exons, and encodes a RET protein consisting of 1100 amino acids. This RET protein is a tyrosine kinase receptor, comprising an extracellular domain composed of cysteine, a transmembrane domain, and an intracellular domain that can catalyze tyrosine kinase (Mol Cell Endocrinol, 2010, 322(1-2): 2-7). RET is involved in cell proliferation, nerve conduction, cell migration and cell differentiation. Through the signal of ligand/complex receptor/RET multi-protein complex, it activates various downstream pathways, such as RAS/RAF/MEK/ERK, PI3K/AKT and STAT pathway to induce cell proliferation (J. Clin. Oncol., 2012, 30(2): 200-202).

With the gradual progress of research, it has been found that the occurrence of many diseases, such as papillary thyroid carcinoma (PTC) (Cell, 1990, 60(4): 557-563), medullary thyroid carcinoma (MTC) (hyroid, 2009, 19(6): 565-612), multiple endocrineneoplasia type II (MEN2) (Endocr Rev, 2006, 27(5)): 535-560), Hirschsprung's disease (Proc Natl Acad Sci USA, 2000, 97(1): 268-273), lung adenocarcinoma (Nat Med, 2012, 18(3): 375-377), etc., is closely related to the mutation of RET gene. At present, only four RET fusion genes KIF5B-RET, CCDC6-RET, TRIM33-RET and NCOA4-RET have been reported in non-small cell lung cancer, while KIF5B-RET is the most common RET fusion gene in non-small cell lung cancer (Cancer, 2013, 119(8): 1486-1494). KIF5B-RET is a fusion gene formed by the chromosome inversion (p11; q11) of the KIF5B (kinesin family member 5B) gene and the RET gene. Through whole-genome and transcriptome sequencing, it was first confirmed in adenocarcinoma in non-smoking Koreans. KIF5B-RET has a very low proportion in lung cancer, but is more common in non-smokers and adenocarcinoma patients, and is repelled with other mutations such as EGFR, KRAS, BRAF, ErbB2, EML4-ALK (Genome Res, 2012, 22(3): 436-445). The KIF5B-RET fusion protein comprises the motor domain and the coiled-coil domain of KIF5B. Through the dimerization of the coiled-coil domain, the RET tyrosine kinase activity of the fusion protein can be abnormally activated, thereby promoting lung tumorigenesis (Cancer, 2011, 117(12): 2709-2718). In the study of Qian et al. (Mol cancer, 2014, 13: 176), KIF5B-RET fusion kinase was confirmed to have significant oncogenic activity *in vitro* and *in vivo,* and the signal transduction pathway of STAT3 might be the main downstream mediator of tumorigenesis. There is evidence that KIF5B-RET can regulate the continuous activation of STAT3. KIF5B-RET fusion kinase can bind to STAT3 to directly phosphorylate and activate STAT3-Tyr705. It can also mediate the activation of STAT3-Tyr705 through a JAK/STAT3-dependent pathway, trigger the phosphorylation of Ser727 through the RAS/RAF/MEK/ERK1 pathway.

It has been proved that RET fusion is the driving force in some cancers. This has promoted the application of multi-kinase inhibitors having RET inhibitory activity for the treatment of the tumor patients of c-loaded with RET fusion protein. At present, there is no approved medicaments that can be used to target this oncogene in a targeted manner. The current treatments for RET-specific cancers are limited to multi-kinase inhibitors and chemotherapy. However, the clinical manifestations of these nonspecific treatments show poor ORR (Objective Response Rate) and has great off-target toxicity. Moreover, one of the biggest challenges in cancer treatment is that tumor cells become drug-resistant after a certain period of treatment. Once the drug-resistance occurs, the patient's treatment options are usually extremely limited, wherein in most cases, the cancer would be progressing without being suppressed. In order to achieve a breakthrough in this respect, a compound is needed to selectively and specifically target the oncogenic RET fusions and gene mutations.

## *SUMMARY OF THE DISCLOSURE*

In order to improve the above problems, the present disclosure provides a compound represented by the following formula I, or a stereoisomer, racemate, tautomer, isotope labelled derivative, nitrogen oxide, pharmaceutically acceptable salt or solvate thereof:

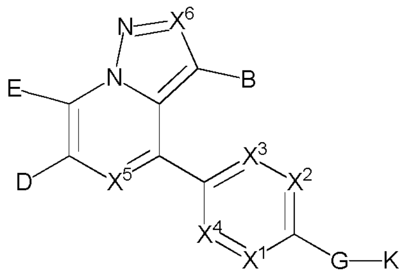

I

wherein, $X^1$, $X^2$, $X^3$, $X^4$, $X^5$ and $X^6$ are the same or different, independently selected from $CR^1$, -C-A or N, wherein each $R^1$ is the same or different, independently selected from H, halogen, CN, OH and the following groups which are unsubstituted or optionally substituted with one, two or more $R^a$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$,$-NHC(O)R^2$, $-C(O)R^4$, $-OCR^5$, $-S(O)_2R^6$ and $OS(O)_2R^7$;

A is selected from H, halogen, CN, OH, the following groups which are unsubstituted or optionally substituted with one, two or more $R^b$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$,$-C(O)R^4$, $-OCR^5$, $-S(O)_2R^6$, $-OS(O)_2R^7$ and $-NHC(O)R^2$;

B is selected from H, halogen, CN, OH, and the following groups which are unsubstituted or optionally substituted by one, two or more $R^c$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, $-C(O)R^4$, $-NHC(O)R^4$, $-OCR^5$,$-S(O)_2R^6$, $OS(O)_2R^7$;

D is selected from the following groups which are unsubstituted or optionally substituted with one, two or more $R^d$: $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{6-20}$ aryl, 5-20 membered heteroaryl and 3-20 membered heterocyclyl;

E is selected from H, halogen, CN, OH and the following groups which are unsubstituted or optionally substituted by one, two or more $R^e$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy or 3-20 membered heterocyclyloxy;

G is selected from the following groups which are unsubstituted or optionally substituted by one, two or more $R^f$: $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy or 3-20 membered heterocyclyloxy;

K is selected from H, halogen, CN, OH, the following groups which are unsubstituted or optionally substituted with one, two or more $R^g$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy , $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy group, $NR^2R^3$, $-C(O)R^4$, $-OCR^5$, $-S(O)_2R^6$, $OS(O)_2R^7$;

each $R^2$ is the same or different and is independently selected H and the following groups which are unsubstituted or optionally substituted by OH or $NH_2$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $-C(O)R^4$ and $-S(O)_2R^6$;

each $R^3$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $-C(O)R^4$ and $-S(O)_2R^6$;

or, $R^2$ and $R^3$ together with the nitrogen atom to which they are attached form a 5-20 membered heteroaryl or a 3-20 membered heterocyclyl;

each $R^4$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy and $NR^2R^3$;

each $R^5$ is the same or different, and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkylcarbonyl, $C_{2-40}$ alkenylcarbonyl, $C_{2-40}$ alkynylcarbonyl, $C_{3-40}$ cycloalkylcarbonyl, $C_{3-40}$ cycloalkenylcarbonyl, $C_{3-40}$ cycloalkynylcarbonyl, $C_{6-20}$ arylcarbonyl, 5-20 membered heteroarylcarbonyl and 3-20 membered heterocyclic carbonyl;

each $R^6$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy and $NR^2R^3$;
each $R^7$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl;
each $R^a$, $R^b$, $R^c$, $R^d$, $R^e$, $R^f$ and $R^g$ is the same or different, and is independently selected from halogen, CN, OH, SH, oxo (=O), $NO_2$, the following groups which are unsubstituted or optionally substituted by one, two or more of $R^h$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl , $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy, $NR^2R^3$,-C(O)$R^4$, -OC$R^5$, -S(O)$_2R^6$ and OS(O)$_2R^7$;
each $R^h$ is the same or different, and is independently selected from halogen, CN, OH, SH, oxo (=O), $NO_2$, and the following groups which are unsubstituted or optionally substituted by one, two or more $R^j$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy, $NR^2R^3$, -C(O)$R^4$, -OC$R^5$, -S(O)$_2R^6$, OS(O)$_2R^7$; or, where the different positions of the cyclic group (including but not limited to $C_{3-40}$ cycloalkyl, $C_{3-40}$ cycloalkenyl, $C_{3-40}$ cycloalkynyl, 3-20 membered heterocyclyl, etc.) are substituted by two or more substituents, two of the substituents may also form a bridged ring together with the cyclic group, wherein a bridge atom other than the bridgehead atoms in the bridged ring may contain 1, 2, 3, 4 or 5 divalent groups selected from $CH_2$, O and NH;
each $R^j$ is the same or different and is independently selected from halogen, CN, OH, SH, oxo (=O), $NO_2$, the following groups which are unsubstituted or optionally substituted with one, two or more $R^h$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl , 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy, $NR^2R^3$, -C(O)$R^4$, -OC$R^5$, -S(O)$_2R^6$ and OS(O)$_2R^7$;
or, where the different positions of the cyclic group (including but not limited to $C_{3-40}$ cycloalkyl, $C_{3-40}$ cycloalkenyl, $C_{3-40}$ cycloalkynyl, 3-20 membered heterocyclyl, etc.) are substituted by two or more substituents, two of the substituents may also form a bridged ring together with the cyclic group, wherein a bridge atom other than the bridgehead atoms in the bridged ring may contain 1, 2, 3, 4 or 5 divalent groups selected from $CH_2$, O and NH;
or, where an atom (such as a carbon atom) is substituted by two or more substituents, two of the substituents may also form a cyclic group (including but not limited to $C_{3-40}$ cycloalkyl, $C_{3-40}$ cycloalkenyl, $C_{3-40}$ cycloalkynyl, 3-20 membered heterocyclyl, etc.) together with the atom to which the two substituents are attached.

According to an embodiment of the present disclosure, the compound represented by formula I is selected from the following compound represented by formula I':

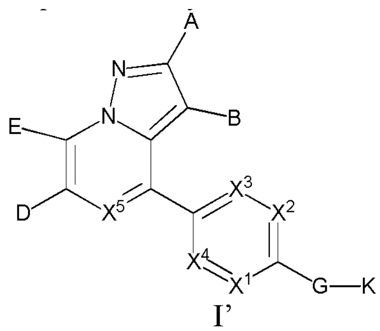

I'

wherein, $X^1$, $X^2$, $X^3$, $X^4$, $X^5$, A, B, D, E, G and K are as defined in the above respectively.
According to an embodiment of the present disclosure, wherein:

$X^1$, $X^2$, $X^3$, $X^4$, $X^5$ and $X^6$ are the same or different, independently selected from $CR^1$, -C-A and N, wherein each $R^1$ is the same or different, independently selected from H, halogen, CN, OH, and the following groups which are unsubstituted or optionally substituted by one, two or more $R^a$: $C_{1-6}$ alkyl, $C_{2-6}$ alkenyl, $C_{2-6}$ alkynyl, $C_{3-8}$ cycloalkyl, $C_{3-8}$ cycloalkenyl , $C_{3-8}$ cycloalkynyl, $C_{1-6}$ alkyloxy, $C_{2-6}$ alkenyloxy, $C_{2-6}$ alkynyloxy, $C_{3-8}$ cycloalkyloxy, $C_{3-8}$ cy-

cloalkenyloxy, $C_{3-8}$ cycloalkynyloxy, $NR^2R^3$, $-NHC(O)R^2$,$-C(O)R^4$, $-OCR^5$, $-S(O)_2R^6$, $OS(O)_2R^7$; A is selected from H, halogen, CN, OH and the following groups which are unsubstituted or optionally substituted with one, two or more $R^b$: $C_{1-6}$ alkyl, $C_{2-6}$ alkenyl, $C_{2-6}$ alkynyl, $C_{3-8}$ cycloalkyl, $C_{3-8}$-cycloalkenyl, $C_{3-8}$ cycloalkynyl, $C_{1-6}$ alkyloxy, $C_{2-6}$ alkenyloxy, $C_{2-6}$ alkynyloxy, $C_{3-8}$ cycloalkyloxy, $C_{3-8}$ cycloalkenyloxy, $C_{3-8}$ cycloalkynyloxy, $NR^2R^3$, $-C(O)R^4$, $-OCR^5$,$-S(O)_2R^6$ and $OS(O)_2R^7$;

B is selected from H, halogen, CN, OH, and the following groups which are unsubstituted or optionally substituted by one, two or more $R^c$: $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{1-6}$ alkyloxy, $C_{2-6}$ alkenyloxy, $C_{2-6}$ alkynyloxy, $C_{3-8}$ cycloalkoxy, $C_{3-8}$ cycloalkenyloxy, $C_{3-8}$ cycloalkynyloxy, $NR^2R^3$, $-C(O)R^4$, $-NHC(O)R^4$, $-OCR^5$, $-S(O)_2R^6$, $OS(O)_2R^7$;

D is selected from the following groups which are unsubstituted or optionally substituted with one, two or more $R^d$: $C_{3-8}$ cycloalkyl, $C_{3-8}$ cycloalkenyl, $C_{3-8}$ cycloalkynyl, $C_{6-10}$ aryl, 5-10 membered heteroaryl and 3-10 membered heterocyclyl;

E is selected from H, halogen, CN, OH, and the following groups which are unsubstituted or optionally substituted by one, two or more $R^e$: $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $C_{1-6}$ alkyloxy, $C_{2-6}$ alkenyloxy, $C_{2-6}$ alkynyloxy, $C_{3-8}$ cycloalkyloxy, $C_{3-8}$ cycloalkenyloxy, $C_{3-8}$ cycloalkynyloxy, $C_{6-10}$ aryloxy, 5-10 membered heteroaryloxy and 3-10 membered heterocyclyloxy;

G is selected from the following groups which are unsubstituted or optionally substituted with one, two or more $R^f$: $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $C_{3-8}$ cycloalkyloxy, $C_{3-8}$ cycloalkenyloxy, $C_{3-8}$ cycloalkynyloxy, $C_{6-10}$ aryloxy, 5-10 membered heteroaryloxy and 3-10 membered heterocyclyloxy;

K is selected from H, halogen, CN, OH, the following groups which are unsubstituted or optionally substituted by one, two or more $R^g$: $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $C_{1-6}$ alkyloxy , $C_{2-6}$ alkenyloxy, $C_{2-6}$ alkynyloxy, $C_{3-8}$ cycloalkyloxy, $C_{3-8}$ cycloalkenyloxy, $C_{3-8}$ cycloalkynyloxy, $C_{6-10}$ aryloxy, 5-10 membered heteroaryloxy, 3-10 membered heterocyclyloxy group, $NR^2R^3$, $-C(O)R^4$, $-OCR^5$, $-S(O)_2R^6$, and $OS(O)_2R^7$;

each $R^2$ is the same or different, independently selected from H and the following groups which are unsubstituted or optionally substituted by OH and/or $NH_2$: $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $-C(O)R^4$, $-S(O)_2R^6$;

each $R^3$ is the same or different, independently selected from H, $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $-C(O)R^4$ and $-S(O)_2R^6$;

or, $R^2$ and $R^3$ together with the nitrogen atom to which they are attached form a 5-10 membered heteroaryl or a 3-10 membered heterocyclyl;

each $R^4$ is the same or different, independently selected from H, $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $C_{1-6}$ alkyloxy, $C_{2-6}$ alkenyloxy, $C_{2-6}$ alkynyloxy, $C_{3-8}$ cycloalkyloxy, $C_{3-8}$ cycloalkenyloxy, $C_{3-8}$ cycloalkynyloxy, $C_{6-10}$ aryloxy, 5-10 membered heteroaryloxy, 3-10 membered heterocyclyloxy, and $NR^2R^3$;

each $R^5$ is the same or different, independently selected from H, $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $C_{1-6}$ alkylcarbonyl, $C_{2-6}$ alkenylcarbonyl, $C_{2-6}$ alkynylcarbonyl, $C_{3-8}$ cycloalkylcarbonyl, $C_{3-8}$ cycloalkenylcarbonyl, $C_{3-8}$ cycloalkynylcarbonyl, $C_{6-10}$ arylcarbonyl, 5-10 membered heteroarylcarbonyl, and 3-10 membered heterocyclylcarbonyl;

each $R^6$ is the same or different, independently selected from H, $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $C_{1-6}$ alkyloxy, $C_{2-6}$ alkenyloxy, $C_{2-6}$ alkynyloxy, $C_{3-8}$ cycloalkyloxy, $C_{3-8}$ cycloalkenyloxy, $C_{3-8}$ cycloalkynyloxy, $C_{6-10}$ aryloxy, 5-10 membered heteroaryloxy, 3-10 membered heterocyclyloxy, and $NR^2R^3$;

each $R^7$ is the same or different, independently selected from H, $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, and 3-10 membered heterocyclyl;

each $R^a$, $R^b$, $R^c$, $R^d$, $R^e$, $R^f$ and $R^g$ is the same or different, independently selected from halogen, CN, OH, SH, oxo (=O), $NO_2$, and the following groups which are unsubstituted or optionally substituted by one, two or more $R^h$: $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl , $C_{6-10}$ aryl, 5-10 membered heteroaryl, 3-10 membered heterocyclyl, $C_{1-6}$ alkyloxy, $C_{2-6}$ alkenyloxy, $C_{2-6}$ alkynyloxy, $C_{3-8}$ cycloalkyloxy, $C_{3-8}$ cycloalkenyloxy, $C_{3-8}$ cycloalkynyloxy, $C_{6-10}$ aryloxy, 5-10 membered heteroaryloxy, 3-10 membered heterocyclyloxy, $NR^2R^3$, $-C(O)R^4$, $-OCR^5$, $-S(O)_2R^6$, and$-OS(O)_2R^7$;

each $R^h$ is the same or different, independently selected from halogen, CN, OH, SH, oxo (=O), $NO_2$, and the following groups which are unsubstituted or optionally substituted by one, two or more $R^j$: $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl , 3-10 membered hete-

rocyclyl, $C_{1-6}$ alkyloxy, $C_{2-6}$ alkenyloxy, $C_{2-6}$ alkynyloxy, $C_{3-8}$ cycloalkyloxy, $C_{3-8}$ cycloalkenyloxy, $C_{3-8}$ cycloalkynyloxy, $C_{6-10}$ aryloxy, 5-10 membered heteroaryloxy, 3-10 membered heterocyclyloxy, $NR^2R^3$, $-C(O)R^4$, $-OCR^5$, $-S(O)_2R^6$, $OS(O)_2R^7$; or, where the different positions of the cyclic group (including but not limited to $C_{3-8}$ cycloalkyl, $C_{3-8}$ cycloalkenyl, $C_{3-8}$ cycloalkynyl, 3-10 membered heterocyclyl, etc.) are substituted by two or more substituents, two of the substituents may also form a bridged ring together with the cyclic group, wherein a bridge atoms other than the bridgehead atoms in the bridged ring may contain 1, 2, 3, 4 or 5 divalent groups selected from $CH_2$, O, NH; each $R^j$ is the same or different, independently selected from halogen, CN, OH, SH, oxo (=O), $NO_2$, and the following groups which are unsubstituted or optionally substituted with one, two or more $R^h$: $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{6-10}$ aryl, 5-10 membered heteroaryl, 3-10 membered heterocyclyl, $C_{1-6}$ alkyloxy, $C_{2-6}$ alkenyloxy, $C_{2-6}$ alkynyloxy, $C_{3-8}$ cycloalkyloxy, $C_{3-8}$ cycloalkenyl oxy, $C_{3-8}$ cycloalkynyloxy, $C_{6-20}$ aryloxy, 5-10 membered heteroaryloxy, 3-10 membered heterocyclyloxy, $NR^2R^3$, $-C(O)R^4$, $-OCR^5$, $-S(O)_2R^6$, and $OS(O)_2R^7$;

or, where the different positions of the cyclic group (including but not limited to $C_{3-8}$ cycloalkyl, $C_{3-8}$ cycloalkenyl, $C_{3-8}$ cycloalkynyl, 3-10 membered heterocyclyl, etc.) are substituted by two or more substituents, two of the substituents may also form a bridged ring together with the cyclic group, wherein a bridge atom other than the bridgehead atoms in the bridged ring may contain 1, 2, 3, 4 or 5 divalent groups selected from $CH_2$, O, and NH;

or, where an atom (such as a carbon atom) is substituted by two or more substituents, two of the substituents may also form a cyclic group (including but not limited to $C_{3-8}$ cycloalkyl, $C_{3-8}$ cycloalkenyl, $C_{3-8}$ cycloalkynyl, 3-10 membered heterocyclyl, etc.) together with the atom to which the two substituents are attached.

According to an embodiment of the present disclosure, wherein $X^1$, $X^2$, $X^3$, $X^4$, $X^5$ and $X^6$ are the same or different and are independently selected from $CR^1$, -C-A and N, wherein $R^1$ is selected from $NH_2$, $CH_3$, $N(CH_3)_2$, OH, H, $NHC(O)CH_3$, $NHCH_3$, F, $OCH_3$, and $NHC_2H_4OH$; for example, at least one of $X^1$, $X^2$, $X^3$, $X^4$ and $X^5$, such as 1, 2, 3, 4 or 5 of $X^1$, $X^2$, $X^3$, $X^4$ and $X^5$, is N;

A can be selected from $NH_2$, OH, H, halogen, cyano, and the following groups which are unsubstituted or optionally substituted with one, two or more groups selected from halogens, OH and $NH_2$: $C_{1-6}$ alkyl, $-NR^2R^3$,$-NH-C_{1-6}$ alkyl-$NR^2R^3$, -NHCO-$C_{1-6}$ alkyl-$NR^2R^3$, -NH-$C_{1-6}$ alkyl-CO-$NR^2R^3$,-NHCO-$C_{1-6}$ alkyl-COO-$C_{1-6}$ alkyl, -NH-$C_{3-8}$ cycloalkyl-CO-$NR^2R^3$, -NH-$C_{2-8}$ alkenyl-CON$R^2R^3$, -NH-$C_{1-6}$ alkyl-CN, -NHCO-NH-$R^2$, -CON$R^2R^3$ or -CONH-$C_{1-6}$ alkyl-$NR^2R^3$, -NHCO-$R^2$, -NH-$C_{1-6}$ alkyl, $C_{1-6}$ alkyloxy, -NH-$C_{1-6}$ alkyl-OH.

According to an embodiment of the present disclosure, wherein each of $R^2$ and $R^3$ is the same or different and is independently selected from H, OH, $C_{1-6}$ alkyl, 5-6 membered heterocyclyl, $C_{1-6}$ alkyl acyl or $C_{1-6}$ alkyl sulfonyl; or $R^2$ and $R^3$ together with the nitrogen atom to which they are attached form a 5-6 membered heterocyclyl which is unsubstituted or optionally substituted by an oxo group.

According to an embodiment of the present disclosure, wherein B can be selected from H, Cl, CN, Br, -COOH, and the following groups which are unsubstituted or optionally substituted with one, two or more $R^c$: $-CH_3$, $-CH_2CH_3$, -cyclopropyl, $-COOCH_3$, $-COOCH_2CH_3$, $-CONH_2$ and $-CONHCH_3$.

according to an embodiment of the present disclosure, wherein D can be selected from the following groups which are unsubstituted or optionally substituted with one, two or more $R^d$: $C_{1-6}$ alkyloxy, 5-10 membered heteroaryl or 3-10 membered heterocyclyl.

According to an embodiment of the present disclosure, each of $R^c$ and $R^d$ is the same or different, and is independently selected from OH, halogen, $C_{1-6}$ alkyl, $C_{1-6}$ alkyloxy, $C_{1-6}$ cycloalkyloxy, hydroxy $C_{1-6}$ alkyl-, monofluoro $C_{1-6}$ alkyl, difluoro $C_{1-6}$ alkyl, trifluoro $C_{1-6}$ alkyl, cyano $C_{1-6}$ alkyl, ($C_{1-6}$ alkoxy) $C_{1-6}$ alkyl-, ($C_{1-6}$ alkoxy) $CH_2C(=O)$-, ($C_{1-6}$ alkoxy)C(=O)$C_{1-6}$ alkyl-, $C_{3-8}$ cycloalkyl-, ($R^2R^3$N)$C_{1-6}$ alkyl-, ($R^2R^3$N)C(=O)$C_{1-6}$ alkyl-, $C_{1-6}$ alkyl $S(O)_2$-$C_{1-6}$ alkyl-, $C_{1-6}$ alkyloxybenzyl, and 3-10 membered heterocyclyl unsubstituted or optionally substituted with a group selected from the following groups: halogen, $C_{1-6}$ alkyl, fluoro $C_{1-6}$ alkyl, difluoro $C_{1-6}$ alkane group, trifluoro $C_{1-6}$ alkyl, ($C_{1-6}$ alkoxy) $C_{1-6}$ alkyl, ($C_{1-6}$ alkyl)$_2NCH_2C(O)$-, ($C_{1-6}$ alkoxy)C(O)- or ($C_{1-6}$ alkoxy) $CH_2C(O)$-, ($C_{1-6}$ alkyl)$_2$N$C_{1-6}$ alkyl-, ($C_{1-6}$ alkyl)$_2$NC(=O)$C_{1-6}$ alkyl-.

According to an embodiment of the present disclosure, wherein E is selected from H, halogen, CN, and the following groups which are unsubstituted or optionally substituted with one, two or more $R^e$: $C_{1-6}$ alkyl, $C_{1-6}$ alkoxy, $C_{1-6}$ cycloalkyl, and 5-membered or 6-membered heteroaryl ring containing 1 to 3 ring heteroatoms selected from N, S and O, wherein the 5-membered and 6-membered heteroaryl ring are optionally substituted by an oxo group.

According to an embodiment of the present disclosure, wherein G can be selected from the following groups which are unsubstituted or optionally substituted with one, two or more $R^f$: 5-10 membered heteroaryl, 3-10 membered heterocyclyl, wherein the heterocyclyl is selected from, for example, pyrrolidinyl, piperidinyl, piperazinyl, morpholinyl or azetidinyl; or, where different positions of the heterocyclyl are substituted by two or more substituents, the two of the substituents may also form a bridge ring together with the heterocyclyl, wherein a bridge atom other than the bridgehead atoms in the bridged ring may contain 1, 2, 3, 4, or 5 divalent groups selected from $CH_2$, O and NH.

According to an embodiment of the present disclosure, wherein $R^f$ can be selected from halogen, CN, OH, SH,

oxo (=O), $NO_2$, and the following groups which are unsubstituted or optionally substituted with one, two or more $R^h$: $C_{1-6}$ alkyl and $C_{3-8}$ cycloalkyl.

According to an embodiment of the present disclosure, wherein K can be selected from H, OH, and the following groups which are unsubstituted or optionally substituted with one or more $R^g$: $C_{1-6}$ alkyl, $C_{3-8}$ cycloalkyl, $C_{6-10}$ aryl, 5-10 membered heteroaryl, 3-10 membered heterocyclyl, $C_{1-6}$ alkyloxy, $C_{3-6}$ cycloalkyloxy, $C_{6-10}$ aryloxy, 5-10 membered heteroaryloxy, 3-10 membered heterocyclyloxy, $N(C_{1-6}\ alkyl)_2$, $-C(O)R^4$, $-OCR^5$, $-S(O)_2R^6$, $-OS(O)_2R^7$.

According to an embodiment of the present disclosure, wherein K can be selected from the following groups which are unsubstituted or optionally substituted with one, two or more $R^g$: $C_{1-6}$ alkyl, $C_{3-10}$ cycloalkyl, $-C(O)R^4$, $-OCR^5$, $-S(O)_2R^6$, $-OS(O)_2R^7$.

According to an embodiment of the present disclosure, $R^4$, $R^5$, $R^6$ and $R^7$ are the same or different and are independently selected from $C_{1-6}$ alkyl, $C_{2-6}$ alkenyl, $C_{2-6}$ alkynyl, $C_{3-8}$ cycloalkyl, $C_{3-8}$ cycloalkenyl, $C_{3-8}$ cycloalkynyl, $C_{1-6}$ alkyloxy.

According to an embodiment of the present disclosure, wherein K may be selected from the following groups which are unsubstituted or optionally substituted with one, two or more $R^h$: $C_{6-10}$ aryl $C_{1-6}$ alkyl- or 3-10 membered heterocyclyl $C_{1-6}$ alkyl-, for example phenyl $C_{1-6}$ alkyl- or pyridyl $C_{1-6}$ alkyl-.

According to an embodiment of the present disclosure, wherein $R^g$ can be selected from halogen, CN, OH, SH, oxo (=O), $NO_2$, and the following groups which are unsubstituted or optionally substituted by one, two or more $R^h$: $C_{1-6}$ alkyl, $C_{3-10}$ cycloalkyl, $C_{6-10}$ aryl, 5-10 membered heteroaryl, 3-10 membered heterocyclyl;

or, where an atom (such as a carbon atom) in K is substituted by two or more $R^g$, the two $R^g$ can also form a cyclic group (including but not limited to $C_{3-6}$ cycloalkyl, $C_{3-6}$ cycloalkenyl, $C_{3-6}$ cycloalkynyl, 4-6 membered heterocyclyl, etc.) together with the atom to which the two substituents are attached.

According to an exemplary embodiment of the present disclosure, for example, $X^6$ is selected from - C-A or N, wherein A can be selected from H, and the following groups which are unsubstituted or optionally substituted with one, two or more $R^b$: $NH_2$, $C_{1-6}$ alkyl, $-NH(C_{1-6}\ alkyl)_2$, OH, F, $-NHC(O)C_{1-6}$ alkyl, $-NHC_{1-6}$ alkyl, $C_{1-6}$ alkyloxy, $-NHC_{1-6}$ alkyl-OH;

each $R^b$ is the same or different, and is independently selected from $C_{1-6}$ alkyl and $C_{1-6}$ alkoxy;

B can be selected from H, CN, $-CONH_2$, and $C_{1-6}$ alkyl which is unsubstituted or optionally substituted with one, two or more $R^c$;

each $R^c$ is the same or different, and is independently selected from halogen and $C_{1-6}$ alkyl;

D can be selected from the following groups which are unsubstituted or optionally substituted with one, two or more $R^d$: $C_{1-6}$ alkyloxy or 5-14 membered heteroaryl, wherein said $R^d$ is selected from $C_{1-6}$ alkyl group and 3-10 membered heterocyclyl which are unsubstituted or optionally substituted with one or more groups selected from the following groups: oxo, halogen, OH, $-N(C_{1-6}\ alkyl)_2$ or $-S(O)_2-C_{1-6}$ alkyl;

E is selected from H, and the following groups which are unsubstituted or optionally substituted by one, two or more $R^e$: $C_{1-6}$ alkyl and $C_{1-6}$ alkoxy;

each $R^e$ is the same or different, and is independently selected from OH, F, and $C_{1-6}$ alkyl groups;

G is selected from 3-10 membered heterocyclyl which is unsubstituted or optionally substituted by an oxo group, such as piperazinyl and piperidinyl which are unsubstituted or optionally substituted by an oxo group or $C_{1-6}$ alkyl; or, where the meta position of the heterocyclyl is substituted by two substituents, the substituents may form a bridged ring together with the heterocyclyl, wherein a bridge atom in the bridged ring other than the bridgehead atoms may contain 1, 2, 3, 4 or 5 divalent groups selected from $CH_2$;

K is selected from the following groups which are unsubstituted or optionally substituted with one, two or more $R^g$: $C_{1-6}$ alkyl and $-C(O)R^4$, wherein $R^g$ is selected from oxo, OH, and the following groups which are unsubstituted or optionally substituted by one, two or more $R^h$: $C_{1-6}$ alkyl, $C_{1-6}$ alkoxy, $C_{3-10}$ cycloalkyl, $C_{6-14}$ aryl, 5-14 membered heteroaryl, $-SO_2-C_{6-14}$ aryl; wherein each $R^4$ is the same or different, independently selected from H, $C_{1-6}$ alkyl, $C_{2-6}$ alkenyl, $C_{2-6}$ alkynyl, $C_{3-10}$ cycloalkyl, $C_{3-10}$ cycloalkenyl, $C_{3-10}$ cycloalkynyl, $C_{6-14}$ aryl, 5-14 membered heteroaryl, 3-10 membered heterocyclyl, $C_{1-6}$ alkyloxy, $C_{2-6}$ alkenyloxy, $C_{2-6}$ alkynyloxy, $C_{3-10}$ cycloalkyloxy, $C_{3-10}$ cycloalkenyloxy, $C_{3-10}$ cycloalkynyloxy, $C_{6-14}$ aryloxy, 5-14 membered heteroaryloxy, 3-10 membered heterocyclyloxy, $-N(C_{1-6}\ alkyl)_2$; wherein $R^h$ is selected from halogen, $C_{1-6}$ alkoxy, $NH_2$, $-N(C_{1-6}\ alkyl)_2$,$-NHC_{6-14}$ aryl, $-NHC_{1-6}$ alkyl; or, where an atom (such as a carbon atom) in K is substituted by two or more $R^g$, two $R^g$ with the atom to which they are attached can also form a ring (including but not limited to $C_{3-10}$ cycloalkyl, $C_{3-10}$ cycloalkenyl, $C_{3-10}$ cycloalkynyl, 3-10 membered heterocyclyl, etc.).

According to an exemplary embodiment of the present disclosure, for example:

$X^6$ is selected from -C-A or N, wherein A can be selected from $NH_2$, methyl, ethyl, propyl, - $NH(CH_3)_2$, OH, F, $-NH(O)CH_3$, $-NHCH_3$, methoxy, $-NHC_2H_4-OH$;

B can be selected from H, CN or $-COCH_3$;

D can be selected from the following groups which are unsubstituted or optionally substituted with one, two or more $R^d$: pyrazolyl, methoxy or ethoxy, such as pyrazol-1-yl, pyrazol-3-yl, pyrazol-4-yl, pyrazol-5-yl;

each $R^d$ is the same or different, independently selected from OH, F, $C_{1-6}$ alkyl, hydroxy $C_{1-6}$ alkyl-, difluoro $C_{1-6}$

alkyl-, $C_{1-6}$ alkyl-$S(O)_2$-$C_{1-6}$ alkyl-, ($C_{1-6}$ alkyl)$_2$N-C(O)$C_{1-6}$ alkyl-, $C_{1-6}$ alkyloxy-, $C_{3-6}$ cycloalkyloxy, 5-6 membered heterocyclyl which is unsubstituted or optional substituted by $C_{1-6}$ alkyl;

G is selected from 5-6 membered heterocyclyl which is unsubstituted or optionally substituted by an oxo group, such as piperazinyl and piperidinyl which are unsubstituted or optionally substituted by an oxo group or methyl; or, where the meta positions of the heterocyclyl are substituted by two substituents, the substituents may form a bridged ring together with the heterocyclyl, wherein a bridge atom other than the bridgehead atoms in the bridged ring may contain 1, 2 or 3 divalent groups selected from $CH_2$;

K is selected from $C_{1-6}$ alkyl and -C(O)$C_{1-6}$ alkyl which are unsubstituted or optionally substituted with one, two or more $R^g$, wherein $R^g$ is selected from oxo, OH, and the following groups which are unsubstituted or optionally substituted by one, two or more $R^h$: $C_{1-6}$ alkyl, $C_{1-6}$ alkoxy, $C_{3-10}$ cycloalkyl, $C_{6-14}$ aryl, 5-14 membered heteroaryl, -$S(O)_2$-$C_{6-14}$ aryl; wherein $R^h$ is selected from halogen, $C_{1-6}$ alkoxy, $NH_2$, -N($C_{1-6}$ alkyl)$_2$, -NH$C_{6-14}$ aryl, -NH$C_{1-6}$ alkyl; or, where an atom (such as a carbon atom) in K is substituted by two or more $R^g$, two $R^g$ may also form a $C_{3-10}$ cycloalkyl together with the atom to which they are attached.

As an example, D can be selected from the following groups:

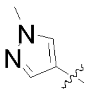 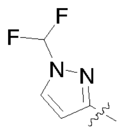 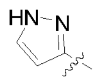 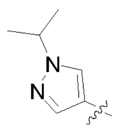 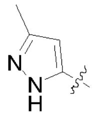 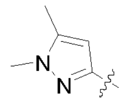

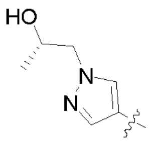 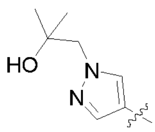 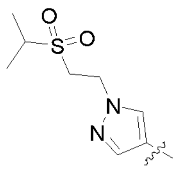 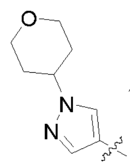 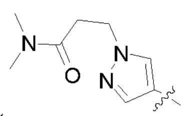 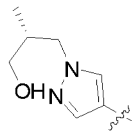

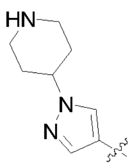 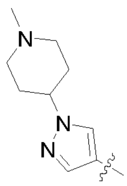 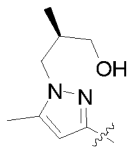 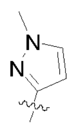 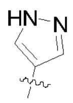 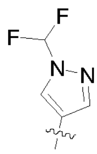

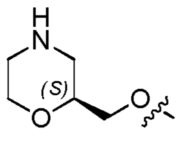 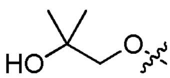 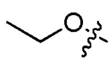 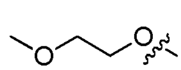 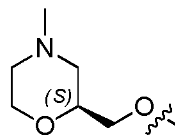

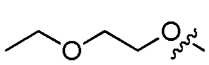 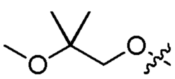 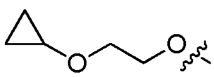 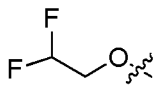 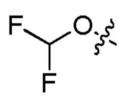

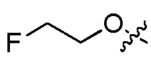 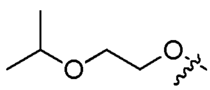 ,

wherein "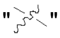" represents the attached position;

As an example, G can be selected from the following groups:

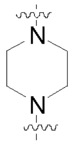 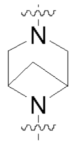 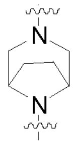 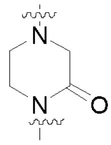 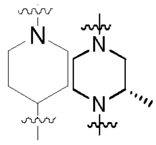 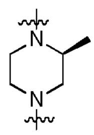 ,

wherein "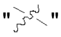" represents the attached position;

Preferably, G is selected from

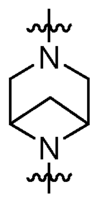 , 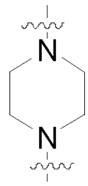

and

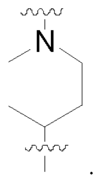 .

According to an embodiment of the present disclosure, K is selected from the following groups:

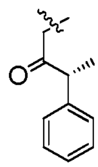 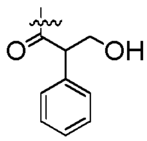 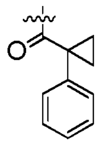 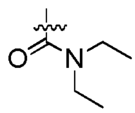 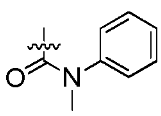 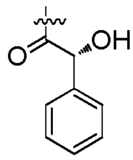

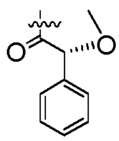 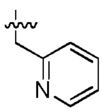 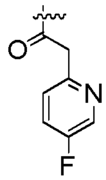 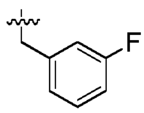 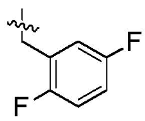 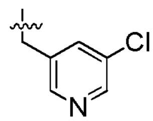

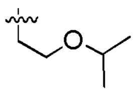 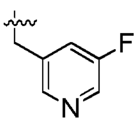 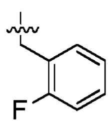 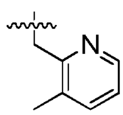 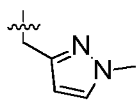 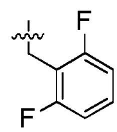

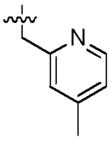 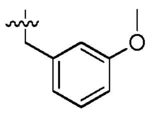 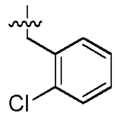 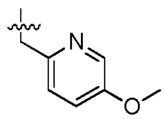 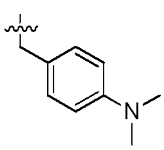 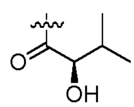

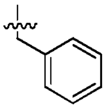 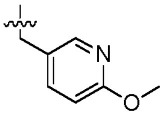 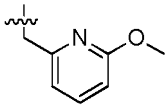 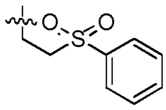 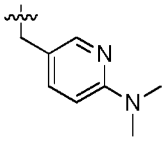 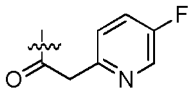

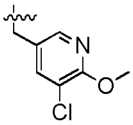 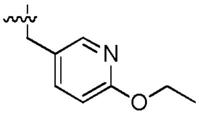 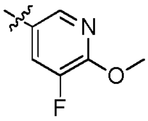 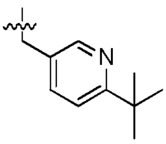 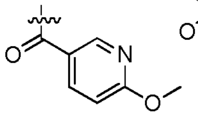 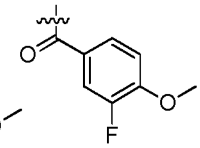

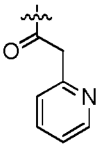 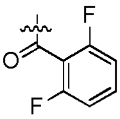 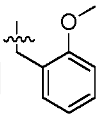 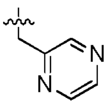 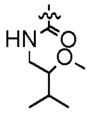 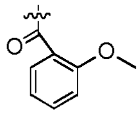 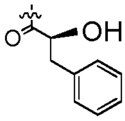 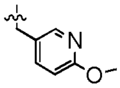

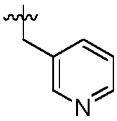 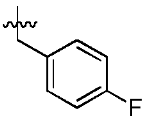 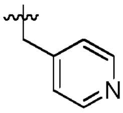 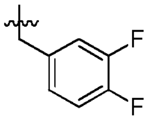 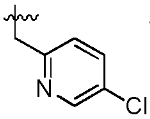 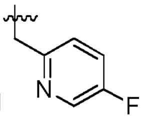 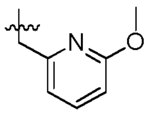 ,

wherein "" represents the attached position;

Preferably, K is selected from

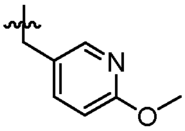 , 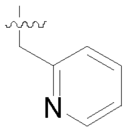

and

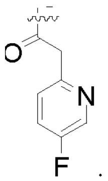 .

As an example, the compound of formula I can be selected from the following compounds:

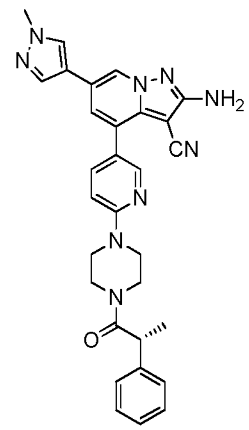

APS007

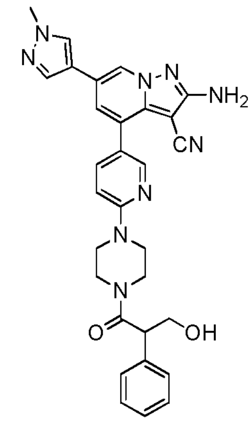

APS008

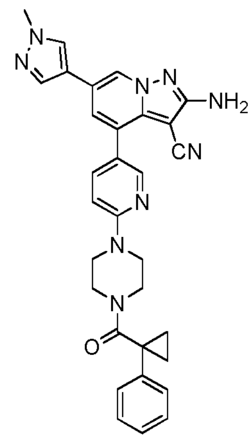

APS009

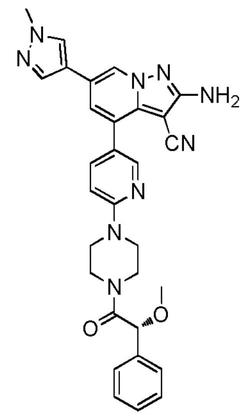

APS010

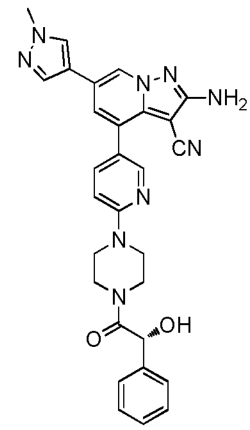

APS011

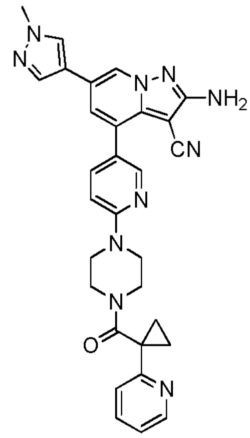

APS012

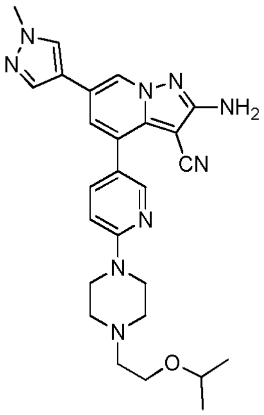

APS013

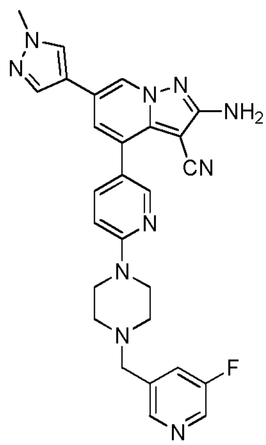

APS014

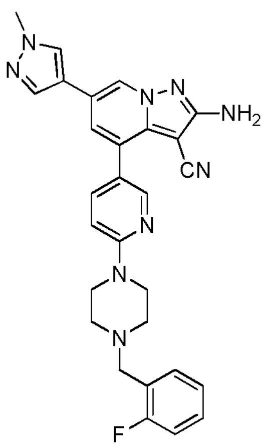

APS015

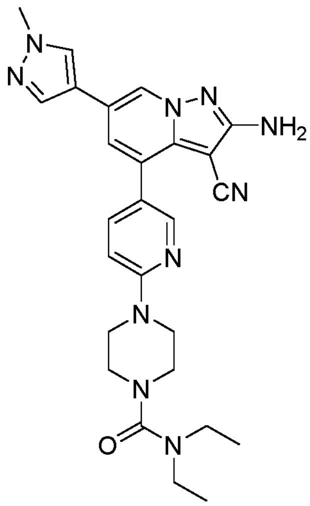

APS016

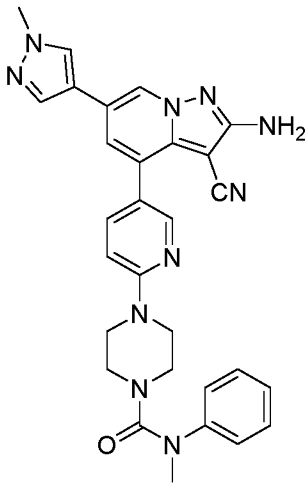

APS017

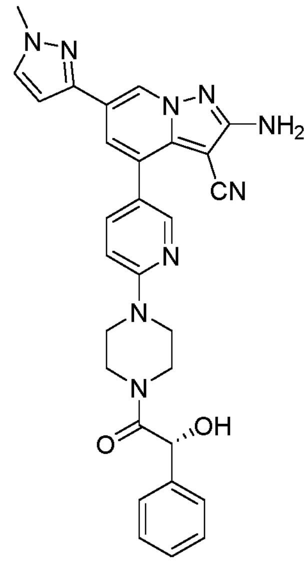

APS018

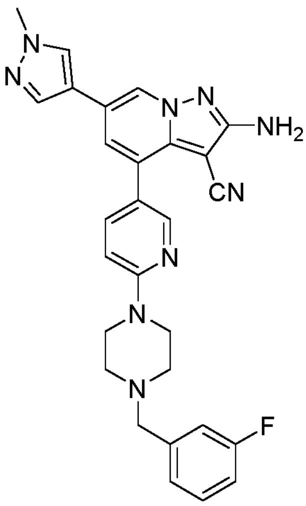

APS019

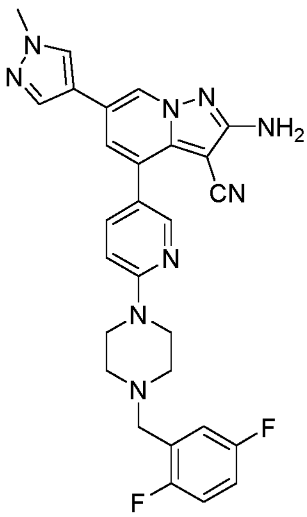

APS020

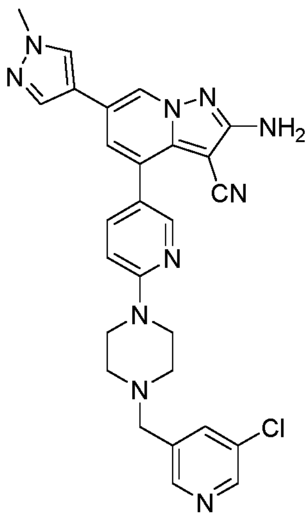

APS021

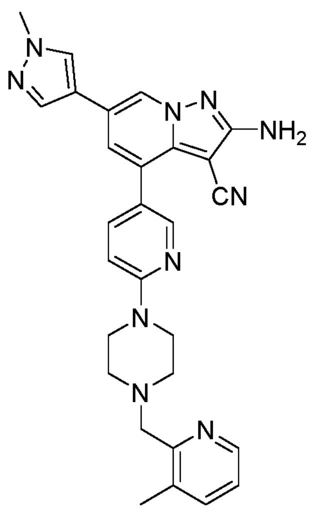

APS022

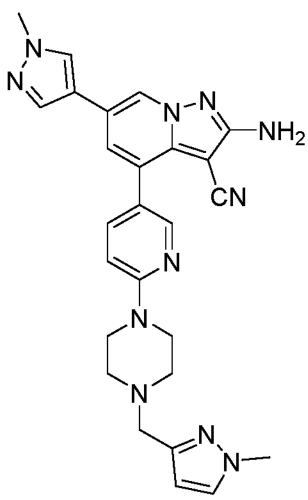

APS023

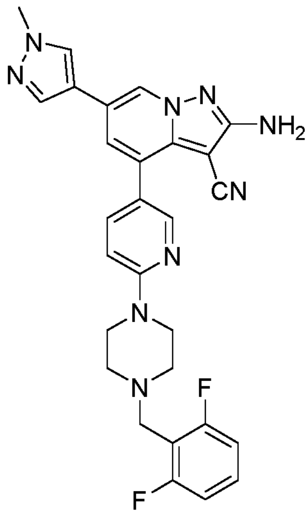

APS024

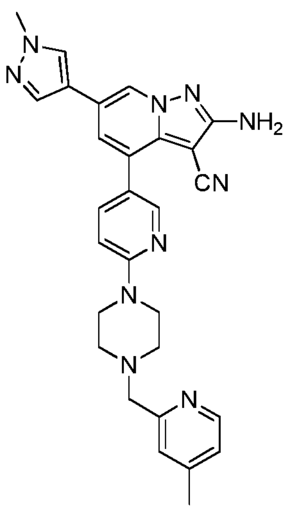

APS025

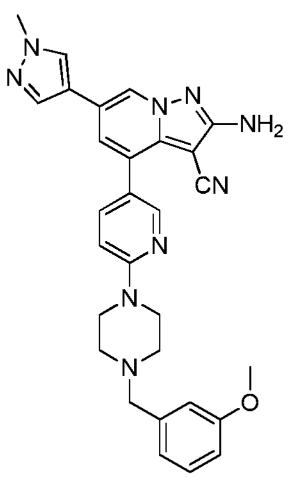

APS026

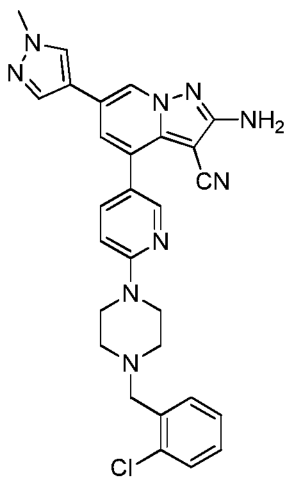

APS027

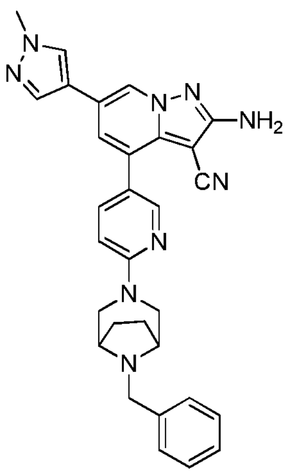

APS028

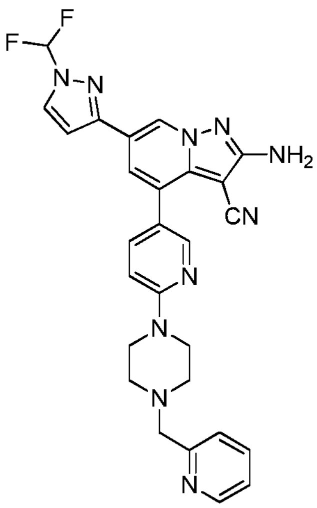

APS029

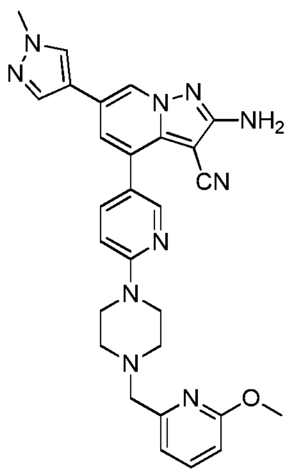

APS030

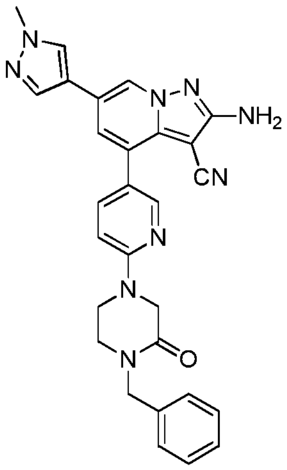

APS031

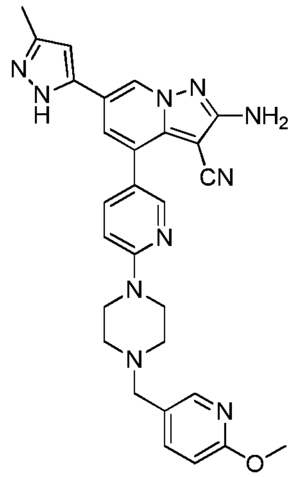

APS032

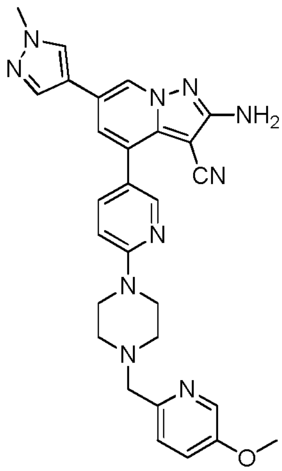

APS033

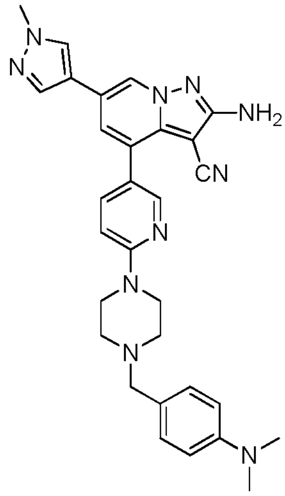

APS034

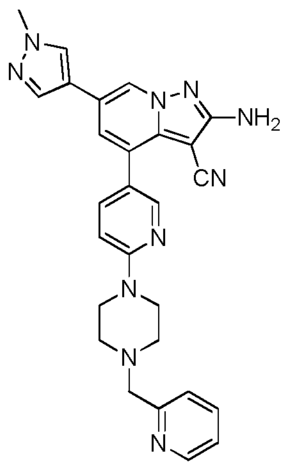

APS035

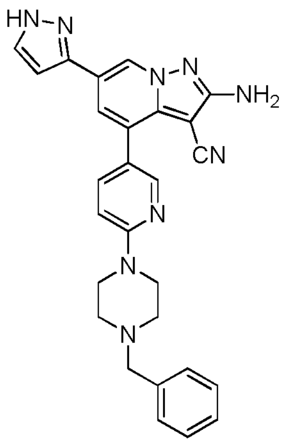

APS036

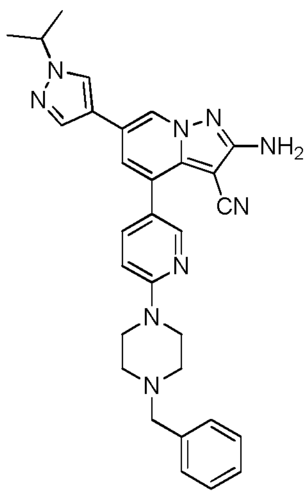

APS037

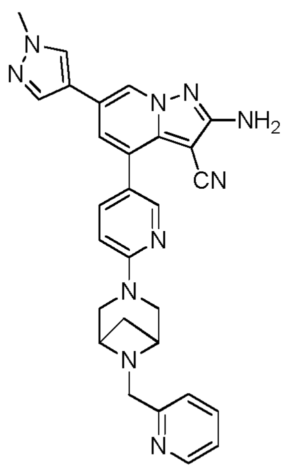

APS038

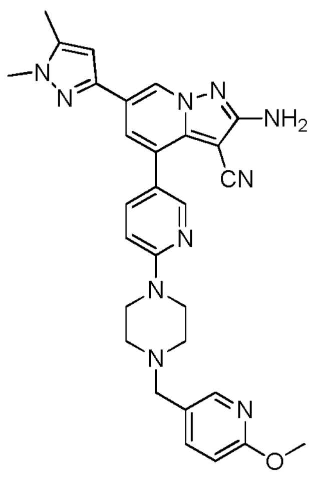

APS039

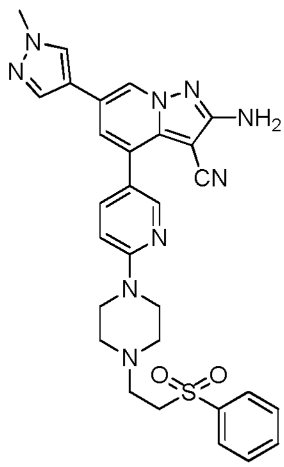

APS040

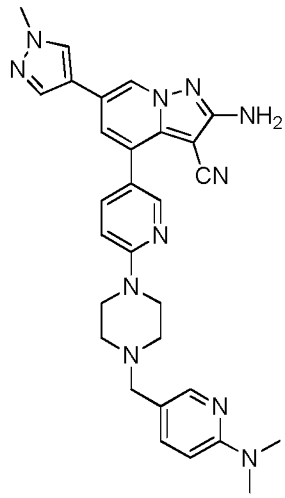

APS041

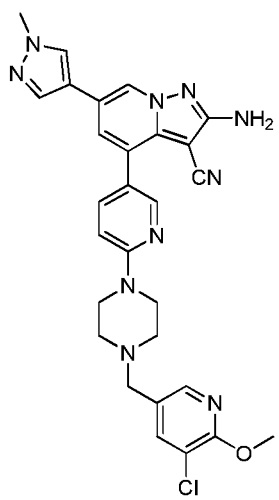

APS042

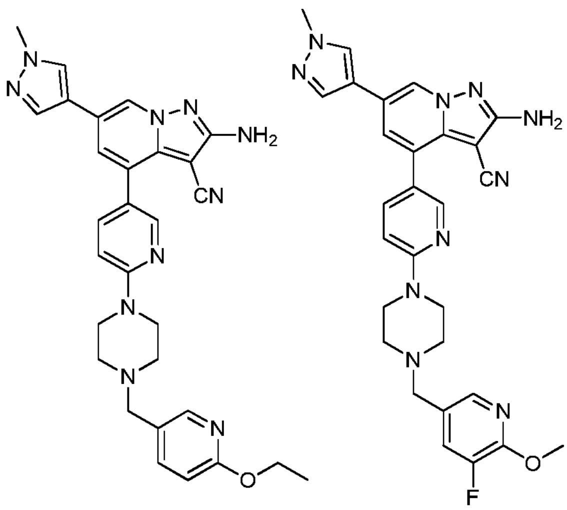

APS043

APS044

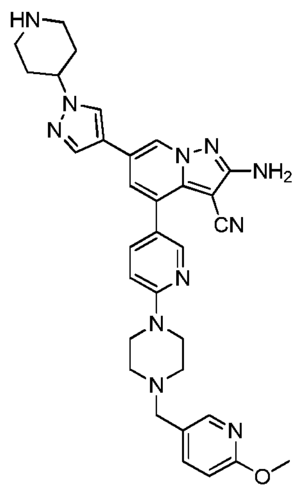

APS045

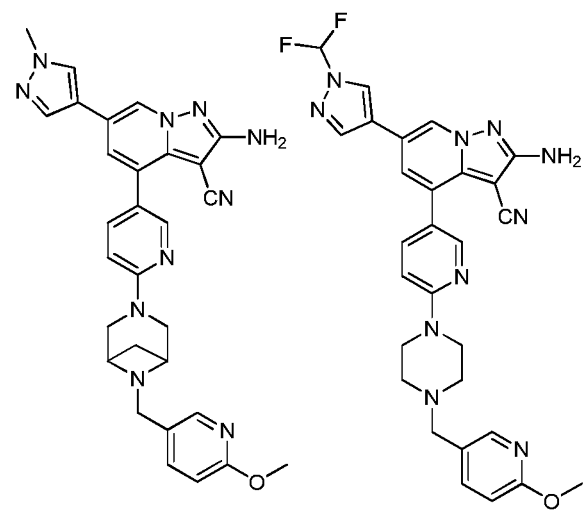

APS046

APS047

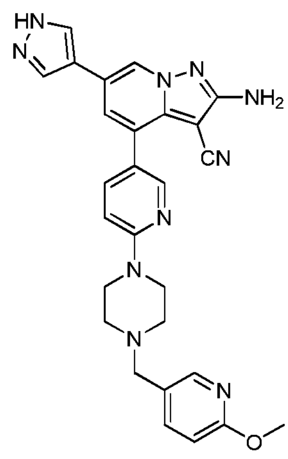

APS048

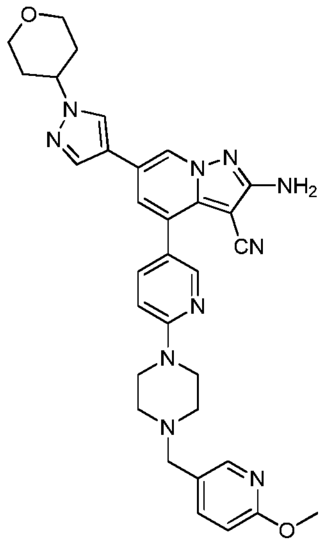

APS049

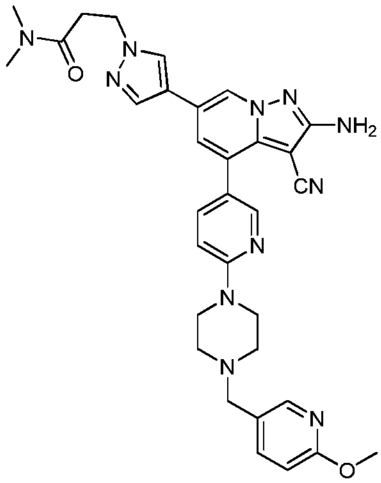

APS050

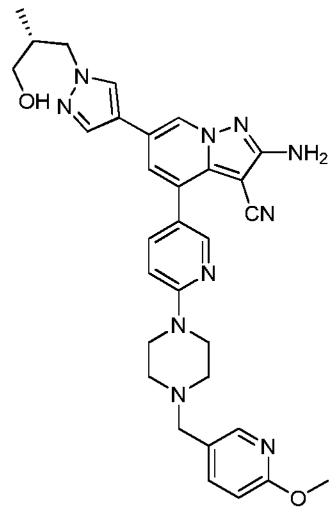

APS051

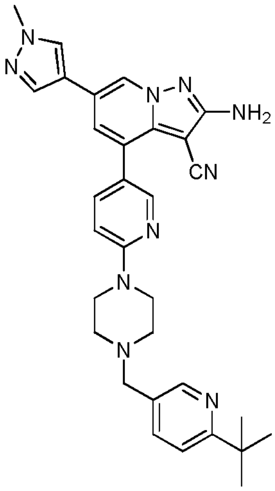

APS052

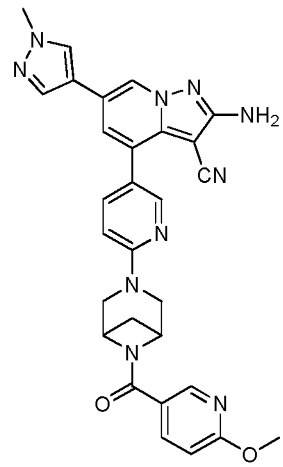

APS053

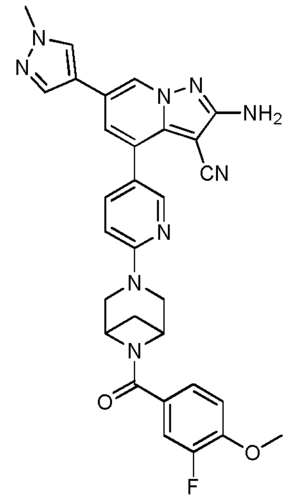

APS054

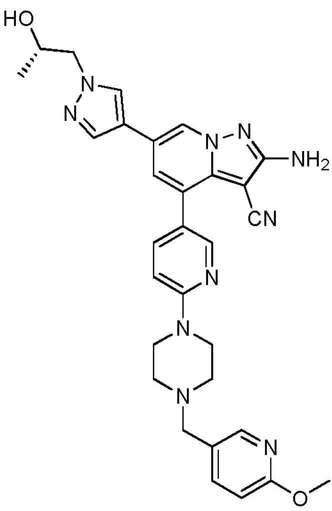

APS055

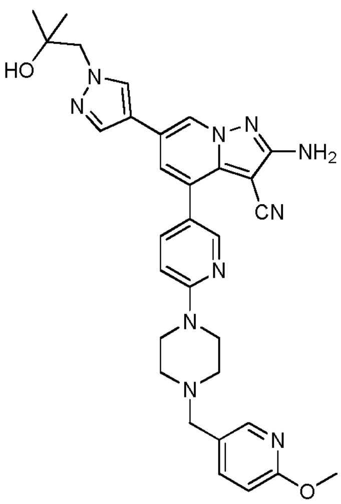

APS056

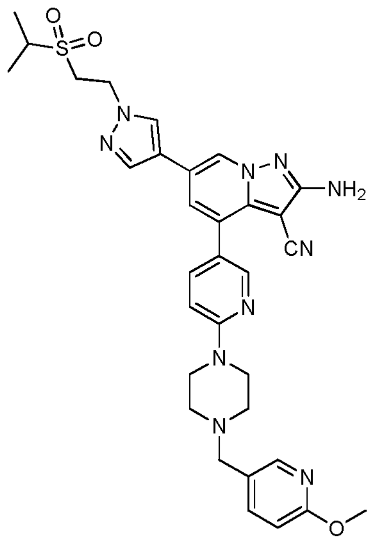

APS057

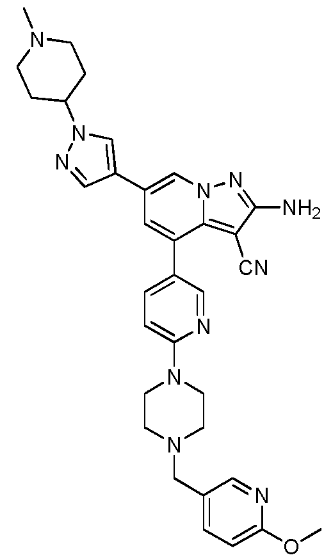

APS058

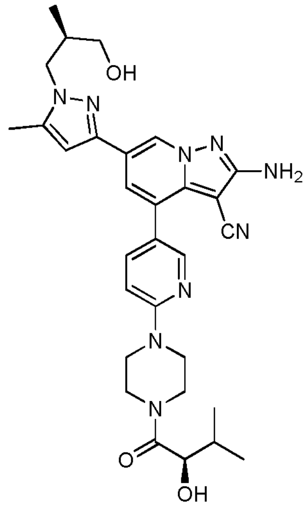

APS059

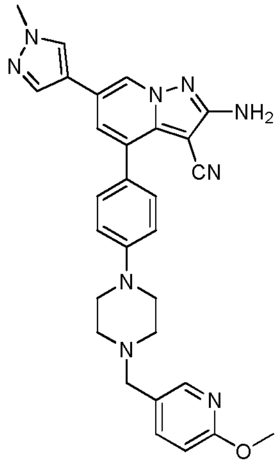

APS060

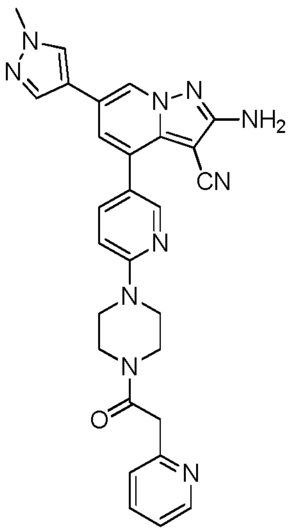

APS061

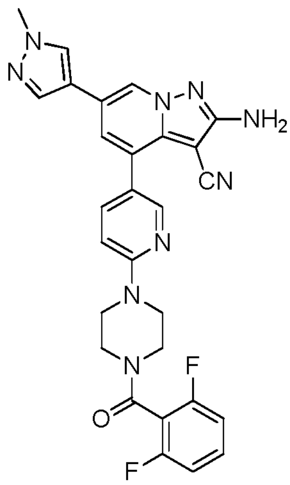

APS062

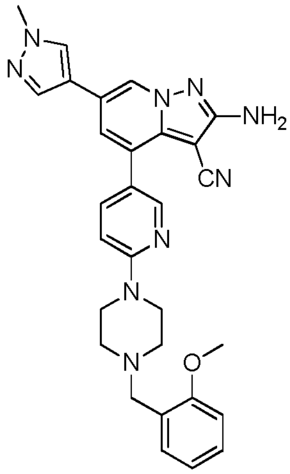

APS063

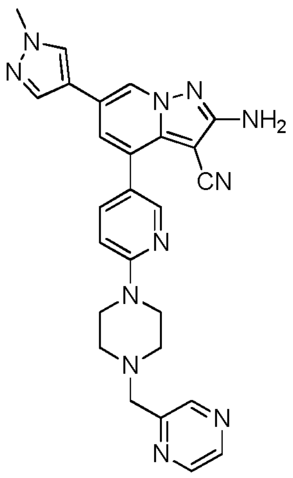

APS064

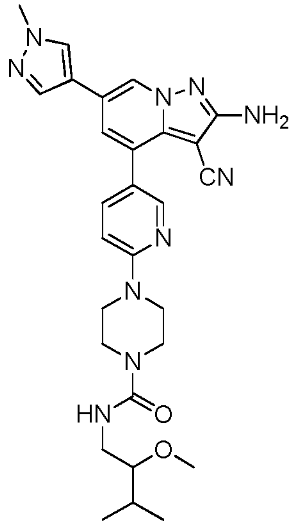

APS065

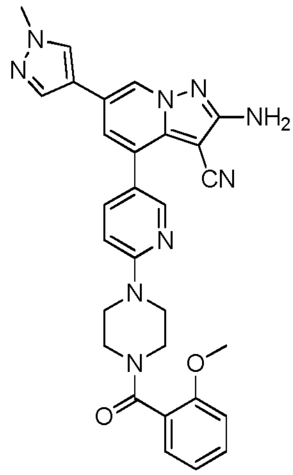

APS066

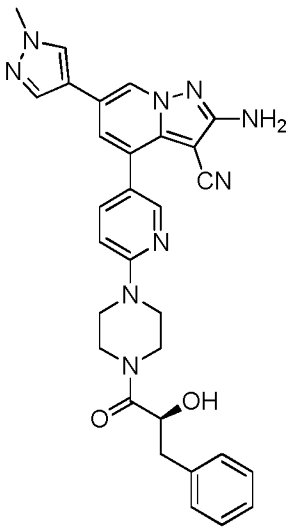

APS067

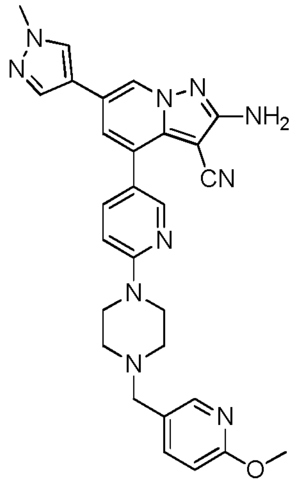

APS068

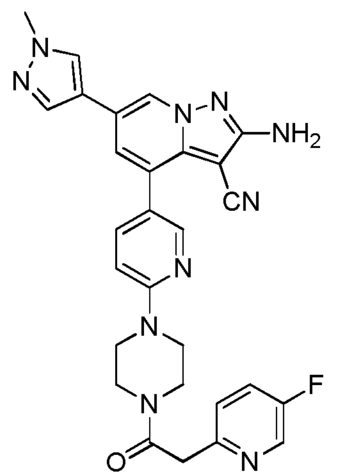

APS069

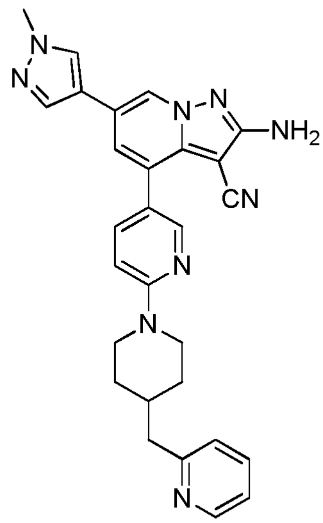

APS070

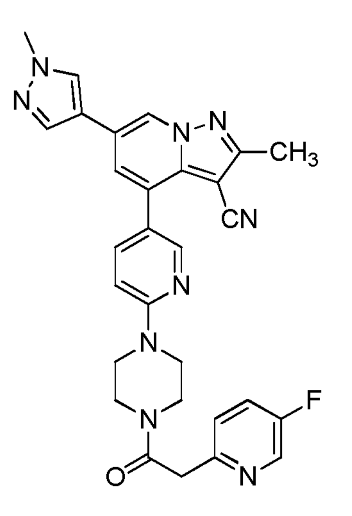

APS071

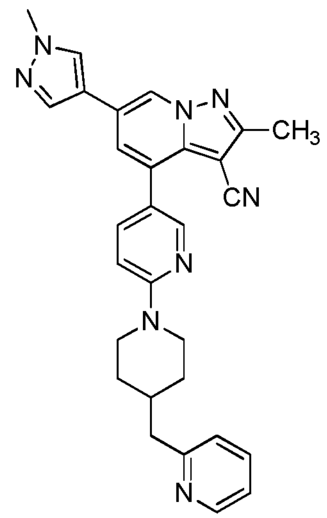

APS072

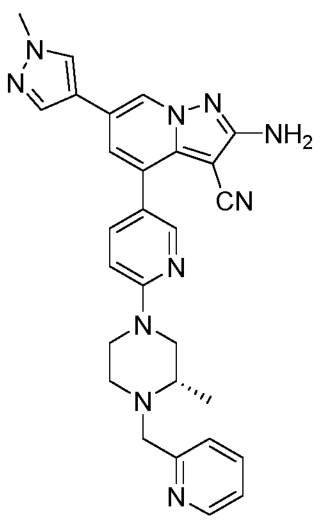

APS073

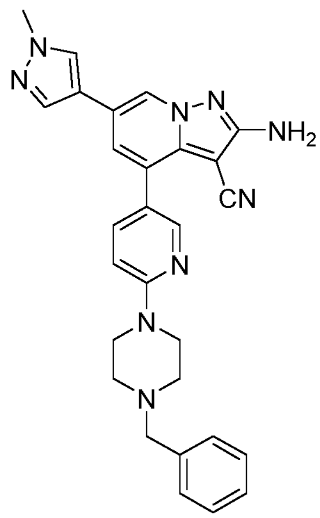

APS074

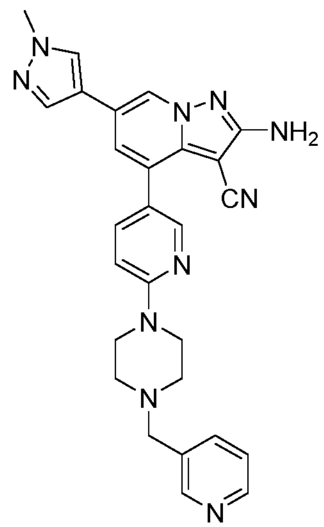

APS075

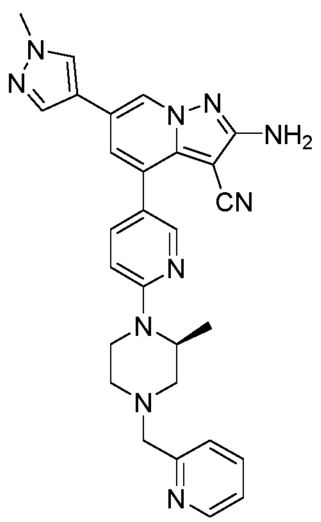

APS076

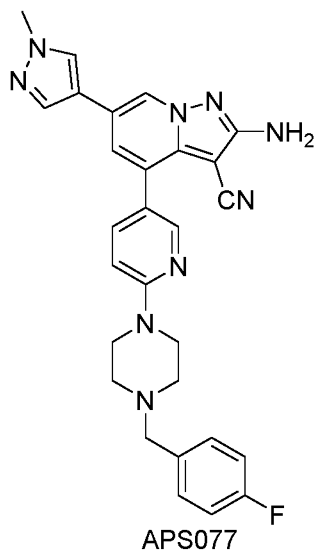

APS077

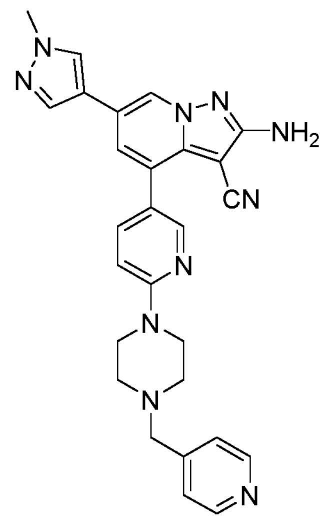

APS078

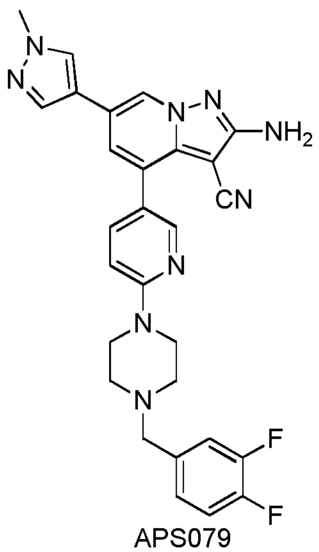

APS079

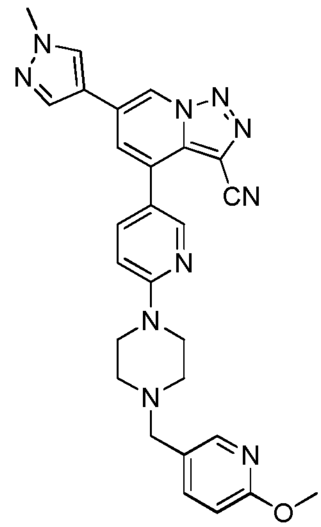

APS081

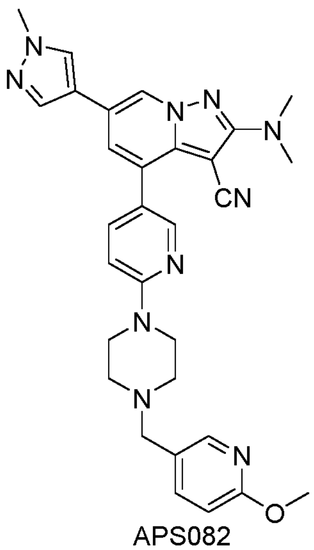

APS082

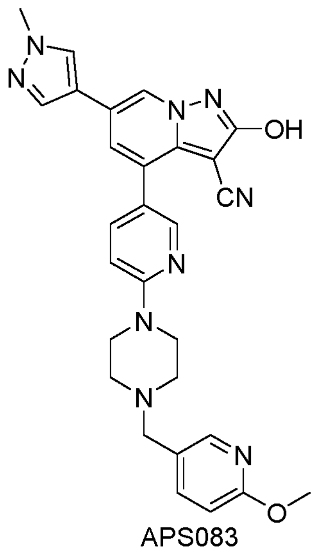

APS083

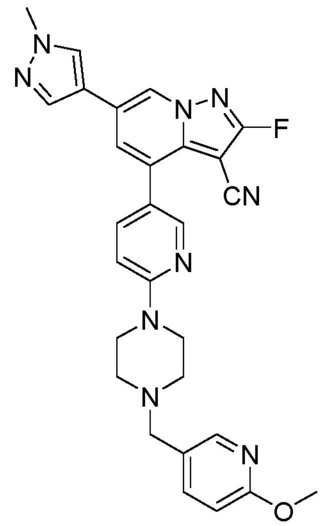

APS084

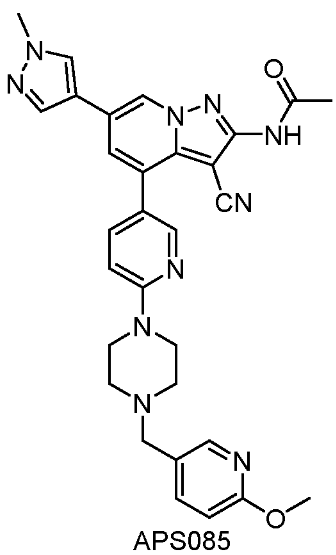

APS085

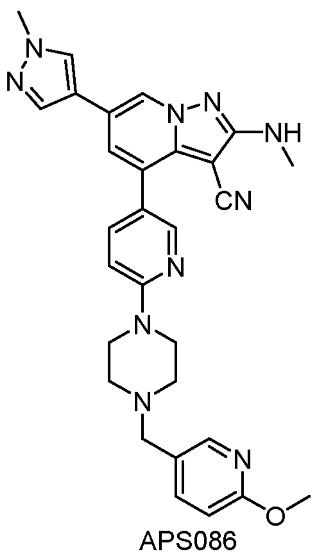

APS086

TFA

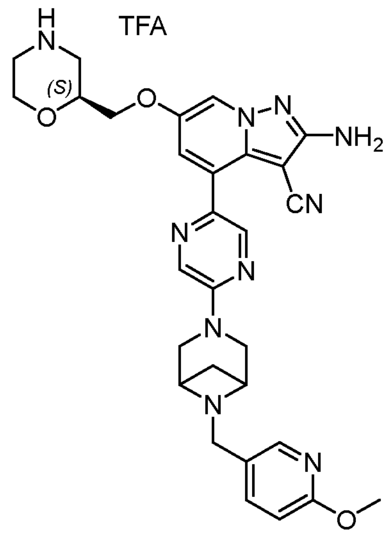

APS087

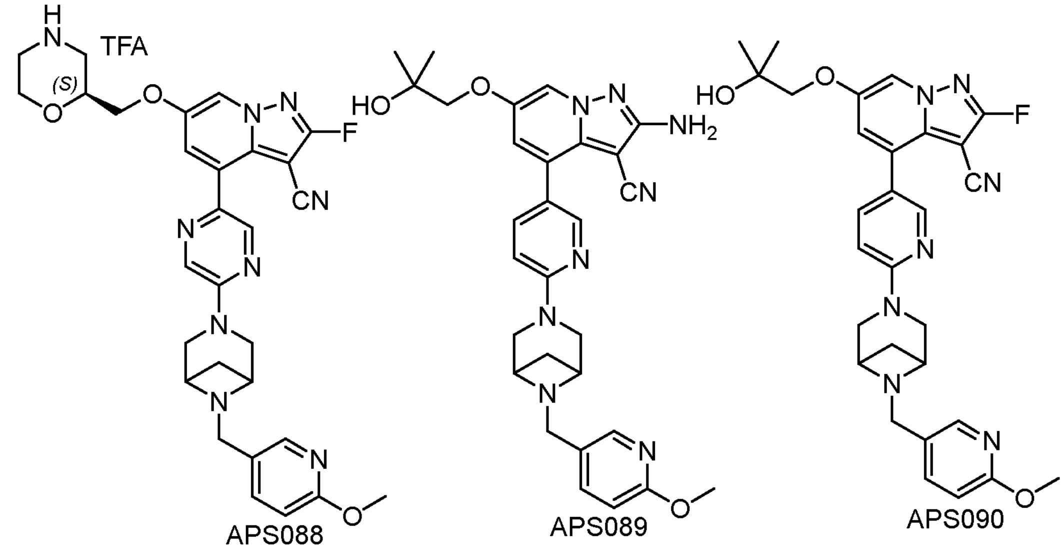

APS088 APS089 APS090

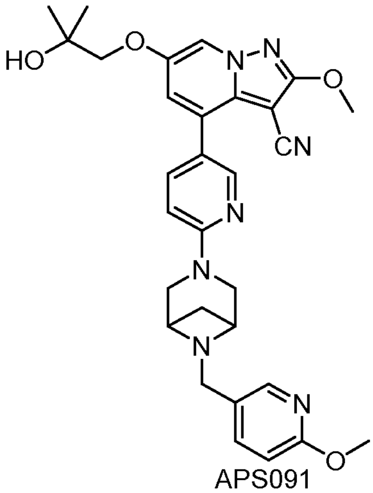

APS091

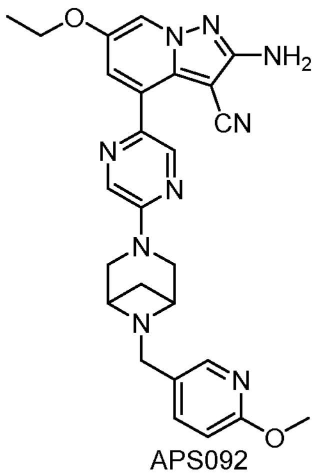

APS092

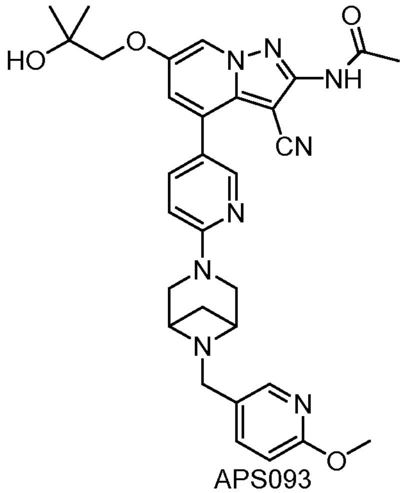

APS093

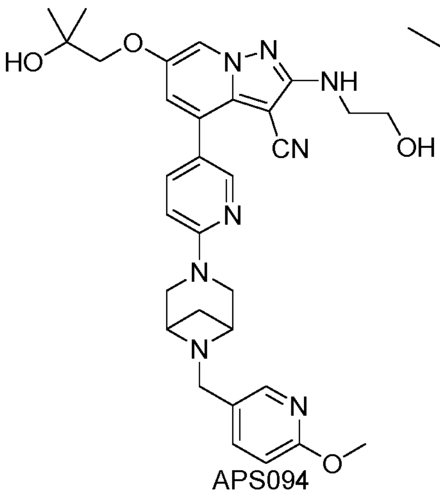

APS094

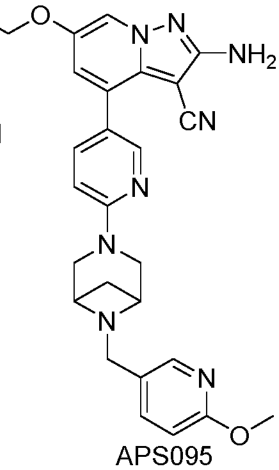

APS095

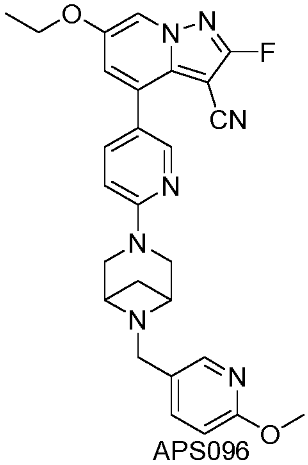

APS096

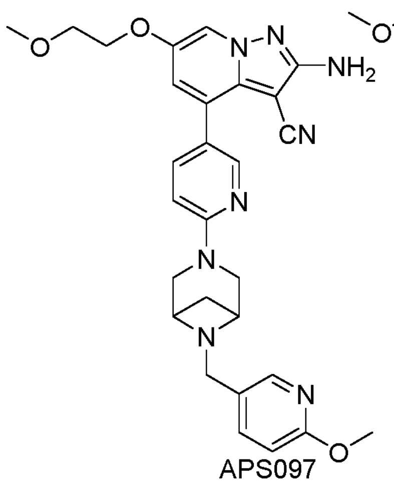

APS097

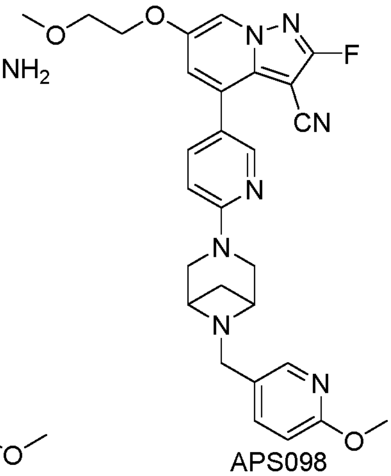

APS098

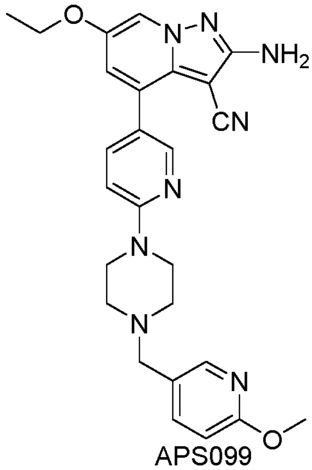

APS099

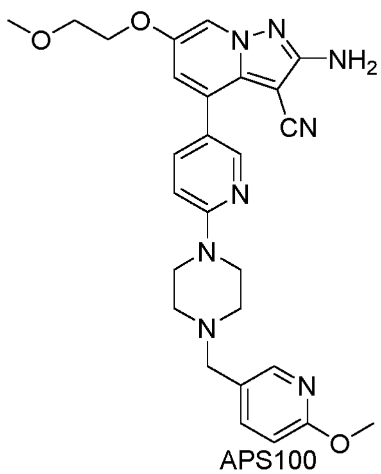

APS100

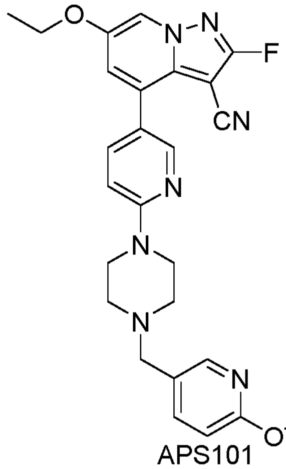

APS101

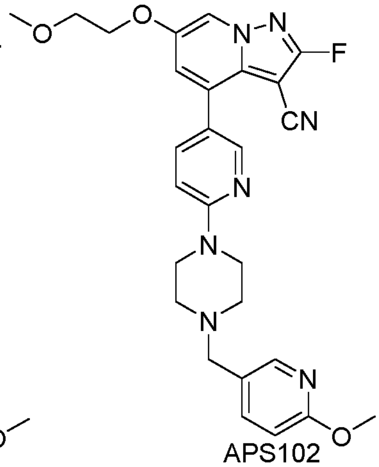

APS102

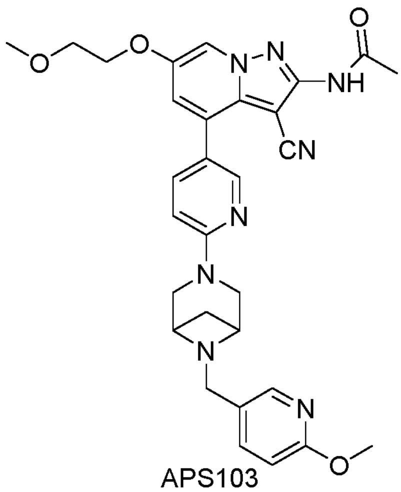

APS103

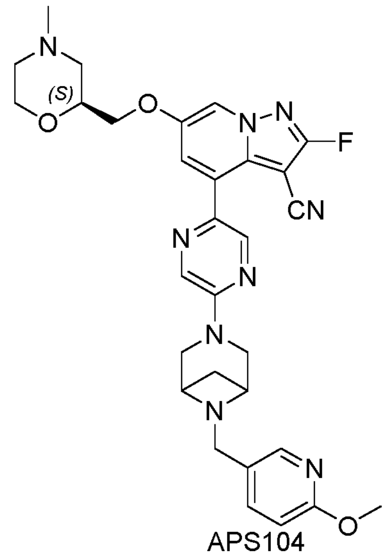

APS104

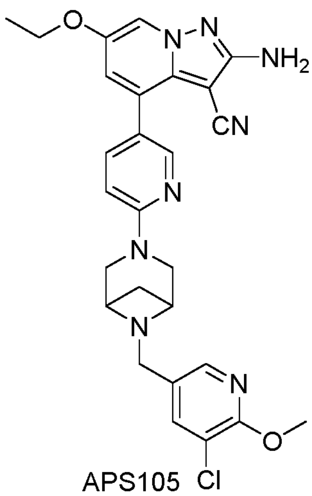

APS105

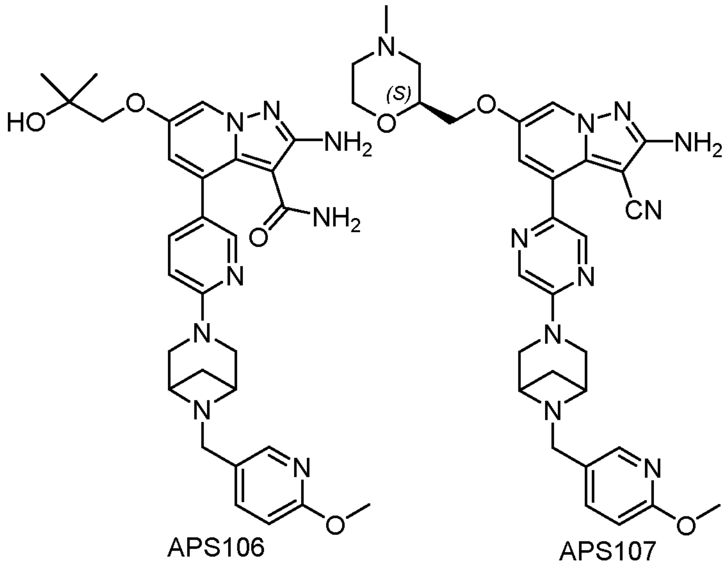

APS106 APS107

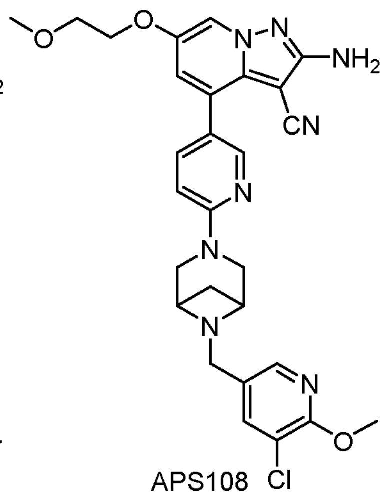

APS108

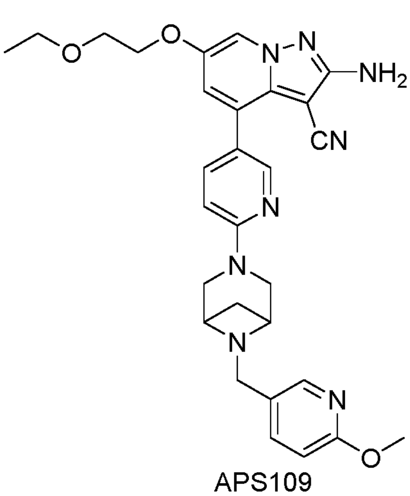

APS109

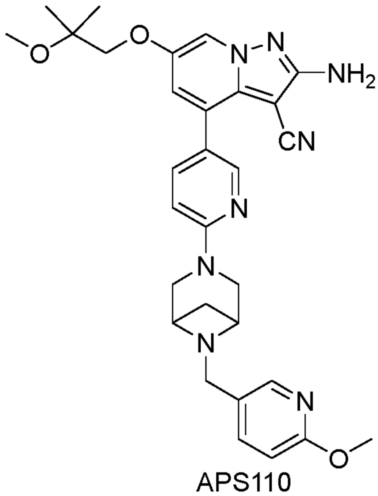

APS110

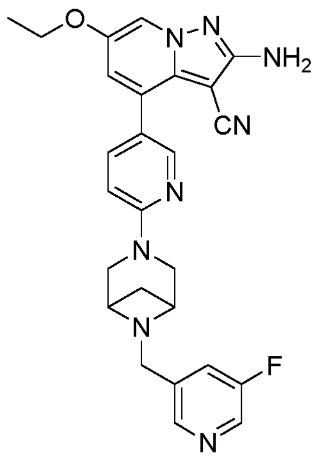

APS111

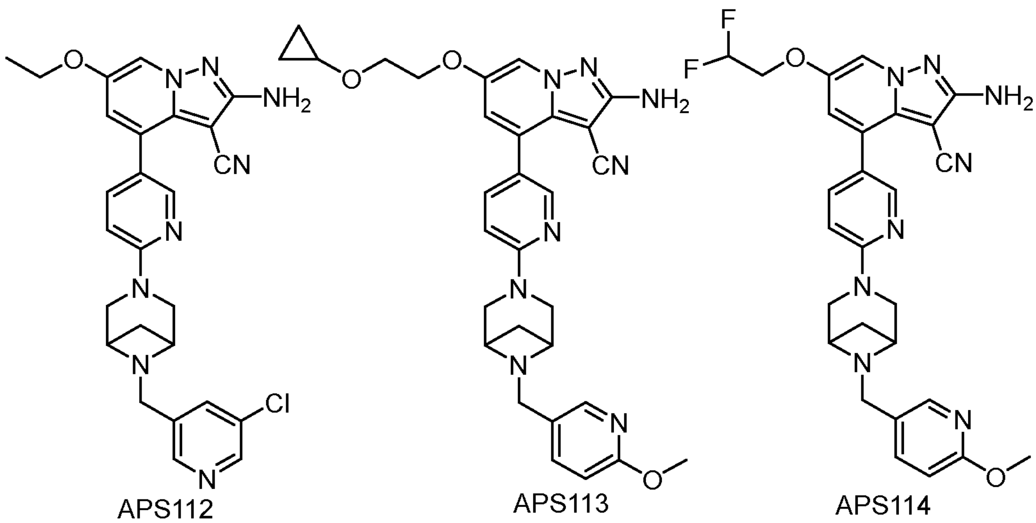

APS112 APS113 APS114

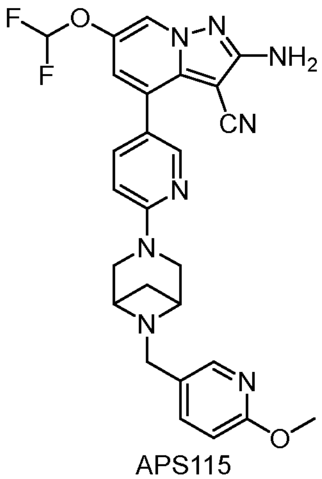

APS115

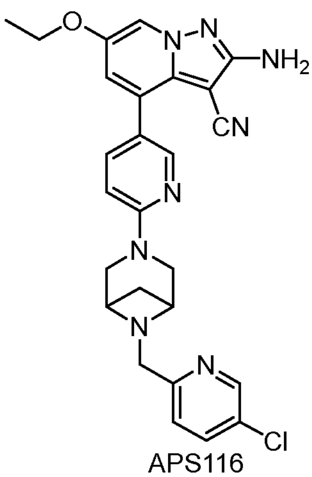

APS116

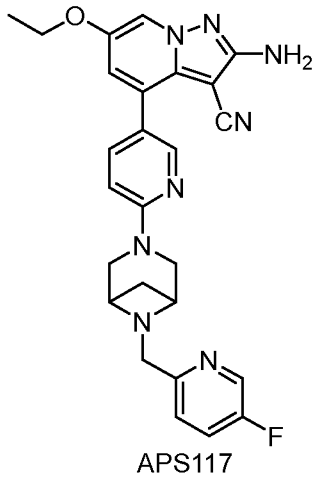

APS117

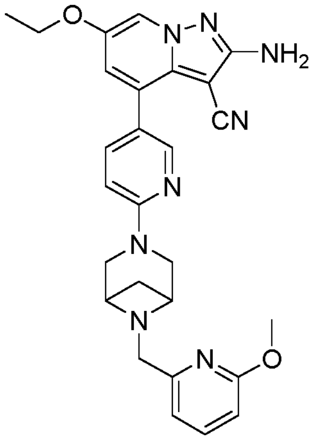

APS118

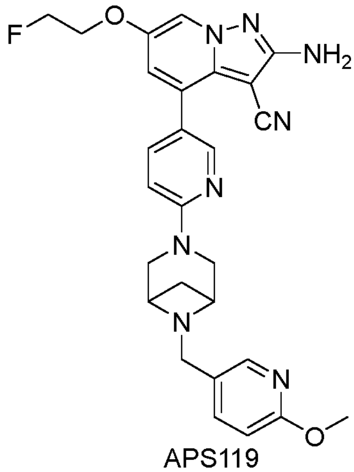

APS119

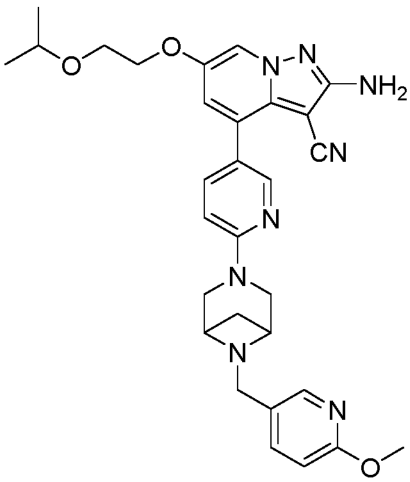

APS120

The present disclosure also provides a preparation method of the compound of formula I, comprising:

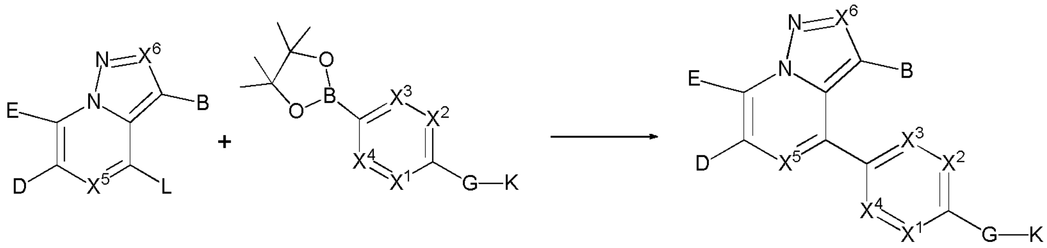

II III I

the compound II undergoes Suzuki reaction with the compound III to obtain the compound I; wherein, B, D, E, G, K, $X^1$, $X^2$, $X^3$, $X^4$, $X^5$ and $X^6$ in the formula II and III are as defined in the above respectively; B in the formula III represents boron element; L is selected from leaving groups such as halogen and OTf.

Those skilled in the art should understand that, unless specifically defined as in the formula III, or its meaning can be determined as boron based on common knowledge of organic chemistry reactions, the "B" as defined in the compound of other general formulae should be understood as the symbol of a substituent as defined in the description.

The present disclosure also provides a pharmaceutical composition, comprising a therapeutically effective amount of at least one of the compounds represented by formula I, and the stereoisomer, racemate, tautomer, isotope labelled derivative, nitrogen oxide, pharmaceutically acceptable salt and solvate thereof.

According to the present disclosure, the pharmaceutical composition may further comprise one or more pharmaceutically acceptable carriers or excipients.

According to the present disclosure, the pharmaceutical composition may further comprise one or more additional therapeutic agents.

The present disclosure also provides a method for inhibiting cell proliferation *in vitro* or *in vivo,* comprising allowing a cell to contact with an effective amount of the compound represented by formula I, or the stereoisomer, racemate, tautomer, isotope labelled derivative, nitrogen oxide, pharmaceutically acceptable salt or solvate thereof, or the pharmaceutical composition thereof.

The present disclosure also provides a method for treating a disease mediated by RET kinase, comprising administering to a patient a therapeutically effective amount of at least one of the compounds represented by formula I, and the stereoisomer, racemate, tautomer, isotope labelled derivative, nitrogen oxide, pharmaceutically acceptable salt and solvate thereof.

Also provided herein is a method of treating a RET-related disease or disorder in a patient in need of treatment, comprising administering to the patient a therapeutically effective amount of a compound of formula I as defined herein, its stereoisomers, racemates, tautomers, isotope labelled derivative, nitrogen oxides, pharmaceutically acceptable salts or solvates or pharmaceutical compositions thereof.

Also provided herein is a method of treating cancer and/or inhibiting metastasis associated with a specific cancer in a patient in need of treatment, wherein the method comprises administering to the patient a therapeutically effective amount of a compound of formula I as defined herein, or the stereoisomer, racemate, tautomer, isotope labelled derivative, nitrogen oxide, pharmaceutically acceptable salt or solvate thereof, or the pharmaceutical composition thereof.

Also provided herein is a method of treating irritable bowel syndrome (IBS) and/or a pain associated with IBS in a patient in need of treatment, comprising administering to the patient a therapeutically effective amount of the compound represented by formula I as defined herein, or the stereoisomer, racemate, tautomer, isotope labelled derivative, nitrogen oxide, pharmaceutically acceptable salt or solvate thereof, or the pharmaceutical composition thereof.

Also provided herein is a method of providing supportive care for a cancer patient, comprising preventing or minimizing a gastrointestinal disease (such as diarrhea) associated with treatment (comprising chemotherapy treatment), wherein the method comprises administering to the patient a therapeutically effective amount of a compound as defined herein or a pharmaceutical composition thereof.

Also provided herein is the compound represented by formula I as defined herein, or the stereoisomer, racemate, tautomer, isotope labelled derivative, nitrogen oxide, pharmaceutically acceptable salt or solvate thereof, or the pharmaceutical composition thereof for use in a treatment.

Also provided herein is the compound represented by formula I as defined herein, or the stereoisomer, racemate, tautomer, isotope labelled derivative, nitrogen oxide, pharmaceutically acceptable salt or solvate thereof, or the pharmaceutical composition thereof use for use in treating cancer and/or inhibiting metastasis associated with a specific cancer.

Also provided herein is the compound represented by formula I as defined herein, or the stereoisomer, racemate, tautomer, isotope labelled derivative, nitrogen oxide, pharmaceutically acceptable salt or solvate thereof, or the pharmaceutical composition thereof for use in the treatment of irritable bowel syndrome (IBS) or pain associated with IBS.

The present disclosure also provides the compound represented by formula I as defined herein, or the stereoisomer, racemate, tautomer, isotope labelled derivative, nitrogen oxide, pharmaceutically acceptable salt or solvate thereof, or the pharmaceutical composition thereof for use in providing a supportive care for cancer patients, including preventing or minimizing a gastrointestinal disorder related to treatment (including chemotherapy treatment), such as diarrhea.

Also provided herein is the compound represented by formula I as defined herein, or the stereoisomer, racemate, tautomer, isotope labelled derivative, nitrogen oxide, pharmaceutically acceptable salt or solvate thereof, or the pharmaceutical composition thereof for use in inhibiting the activity of RET kinase.

Also provided herein is the compound represented by formula I as defined herein, or the stereoisomer, racemate, tautomer, isotope labelled derivative, nitrogen oxide, pharmaceutically acceptable salt or solvate thereof, or the pharmaceutical composition thereof for use in the treatment of a RET-related disease or disorder.

The present disclosure also provides a use of at least one of the compound represented by formula I as defined herein, and the stereoisomer, racemate, tautomer, isotope labelled derivative, nitrogen oxide, pharmaceutically acceptable salt and solvate thereof in the preparation of a medicament useful for treating a disease mediated by RET kinase.

Also provided herein is a use of the compound represented by formula I as defined herein, or the stereoisomer, racemate, tautomer, isotope labelled derivative, nitrogen oxide, pharmaceutically acceptable salt or solvate thereof in the preparation of a medicament useful for treating cancer and/or inhibiting metastasis related to a specific cancer.

Also provided herein is a use of the compound represented by formula I as defined herein, or the stereoisomer, racemate, tautomer, isotope labelled derivative, nitrogen oxide, pharmaceutically acceptable salt or solvate thereof in the preparation of a medicament useful for treating irritable bowel syndrome (IBS) or pain associated with IBS.

Also provided herein is a use of the compound represented by formula I as defined herein, or the stereoisomer,

racemate, tautomer, isotope labelled derivative, nitrogen oxide, pharmaceutically acceptable salt or solvate thereof in the preparation of a medicament useful for providing a supportive care to a cancer patient, wherein the supportive care comprising preventing or minimizing a gastrointestinal disorder associated with treatment (including chemotherapy treatment), such as diarrhea.

Also provided herein is a use of the compound represented by formula I as defined herein, or the stereoisomer, racemate, tautomer, isotope labelled derivative, nitrogen oxide, pharmaceutically acceptable salt or solvate thereof in the preparation of a medicament useful for inhibiting the activity of RET kinase.

Also provided herein is a use of the compound represented by formula I as defined herein, or the stereoisomer, racemate, tautomer, isotope labelled derivative, nitrogen oxide, pharmaceutically acceptable salt or solvate thereof in the preparation of a medicament useful for treating a RET-related disease or disorder.

Also provided herein is a method for treating a cancer in a patient in need of treatment, comprising: (a) determining whether the cancer is related to a disorder of the expression or activity level of RET gene, RET kinase, or any one of them (for example, RET-related cancer); (b) if it is determined that the cancer is related to a disorder of the expression or activity level of RET gene, RET kinase, or any one of them (for example, RET-related cancer), administer to the patient a therapeutically effective amount of the compound represented by formula I as defined herein, or the pharmaceutically acceptable salt or solvate thereof, or the pharmaceutical composition thereof.

Also provided herein is a pharmaceutical combination for use in the treatment of a cancer (for example, RET-related cancer, such as RET-related cancer with one or more RET inhibitor resistance mutations) in patients in need of treatment, comprising: (a) the compound represented by formula I as defined herein, or the stereoisomer, racemate, tautomer, isotope labelled derivative, nitrogen oxide, pharmaceutically acceptable salt or solvate thereof; (b) another therapeutic agent; and (c) optionally, at least one pharmaceutically acceptable carrier, wherein the compound represented by formula I as defined herein, or the stereoisomer, racemate, tautomer, isotope labelled derivative, nitrogen oxide, pharmaceutically acceptable salt or solvate thereof and the another therapeutic agent are formulated into a single composition or dose for simultaneous, separate or sequential use in the treatment of cancer, and the amount of the compound represented by formula I as defined herein, or the stereoisomer, racemate, tautomer, isotope labelled derivative, nitrogen oxide, pharmaceutically acceptable salt or solvate thereof and the another therapeutic agent together are effective for the treatment of cancer. Also provided herein is a pharmaceutical composition comprising the combination. Also provided herein is the use of the combination in the preparation of a medicament useful for the treatment of cancer. Also provided herein is a commercial package or product, which comprises the combination as a combined preparation for simultaneous, separate or sequential use; and relates to a method of treating cancer in a patient in need of treatment.

Also provided herein is a method for reversing or preventing the acquired resistance to an anticancer medicament, comprising administrating a therapeutically effective amount of the compound represented by formula I as defined herein, or the stereoisomer, racemate, tautomer, isotope labelled derivative, nitrogen oxide, pharmaceutically acceptable salt or solvate thereof to a patient being at the risk of developing or having acquired resistance to anticancer medicament.

Also provided herein is a method for delaying and/or preventing the development of an anticancer medicament resistance in an individual, comprising administering to the individual an effective amount of the compound represented by formula I as defined herein, or the stereoisomer, racemate, tautomer, isotope labelled derivative, nitrogen oxide, pharmaceutically acceptable salt or solvate thereof, before, during or after administering an effective amount of the anticancer medicament.

Also provided herein is a method for treating an individual suffering from cancer and having an increased possibility of developing resistance to anticancer medicament, comprising concomitantly administering to the individual (a) an effective amount of the compound represented by formula I as defined herein, or the stereoisomer, racemate, tautomer, isotope labelled derivative, nitrogen oxide, pharmaceutically acceptable salt or solvate thereof; and (b) an effective amount of an anticancer medicament.

Also provided herein is a method for treating an individual suffering from a RET-related cancer having one or more RET inhibitor resistance mutations, wherein the RET inhibitor resistance mutation increasing the resistance to a compound other than the compound represented by formula I as defined herein, and the stereoisomer, racemate, tautomer, isotope labelled derivative, nitrogen oxide, pharmaceutically acceptable salt and solvate thereof (for example, a substitution at amino acid position 804, such as V804M, V804L or V804E). The method comprises administering the compound represented by formula I as defined herein, or the stereoisomer, racemate, tautomer, isotope labelled derivative, nitrogen oxide, pharmaceutically acceptable salt or solvate thereof before, during or after the administration of another anticancer medicament.

Also provided herein is a method for treating an individual suffering from RET-related cancer, the method comprising administering the compound represented by formula I as defined herein, or the stereoisomer, racemate, tautomer, isotope labelled derivative, nitrogen oxide, pharmaceutically acceptable salt or solvate thereof before, during, or after administering another anti-cancer medicament.

Also provided herein is a method for treating cancer (such as RET-related cancer) in a patient in need of

treatment, comprising administering to the patient a therapeutically effective amount of the compound represented by formula I as defined herein, or the pharmaceutically acceptable salt, solvate, or pharmaceutical composition thereof.

According to some embodiments of any of the methods or uses described herein, the cancer (e.g., RET-related cancer) is a hematological cancer. According to some embodiments of any of the methods or uses described herein, the cancer (e.g., RET-related cancer) is a solid tumor. According to some embodiments of any of the methods or uses described herein, the cancer (eg, RET-related cancer) is lung cancer (eg, small cell lung cancer or non-small cell lung cancer), papillary thyroid cancer, medullary thyroid cancer, differentiated thyroid Carcinoma, recurrent thyroid cancer, refractory differentiated thyroid cancer, lung adenocarcinoma, bronchiolar carcinoma, multiple endocrine tumors of type 2A or 2B (MEN2A or MEN2B, respectively), pheochromocytoma, parathyroid hyperplasia, breast cancer, colorectal cancer (e.g. metastatic colorectal cancer), papillary renal cell carcinoma, gangliocytomatosis of the gastrointestinal mucosa, inflammatory myofibroblastoma or cervical cancer. In some embodiments of any of the methods or uses described herein, the cancer (eg, RET-related cancer) is selected from: acute lymphoblastic leukemia (ALL), acute myeloid leukemia (AML), juvenile cancer, adrenocortical cancer, anal cancer , appendix cancer, astrocytoma, atypical teratoma/rhabdoid tumor, basal cell carcinoma, cholangiocarcinoma, bladder cancer, bone cancer, brain stem glioma, brain tumor, breast cancer, bronchial tumor, burkitt lymphoma, carcinoid tumor, unknown primary cancer, heart tumor, cervical cancer, childhood cancer, chordoma, chronic lymphocytic leukemia (CLL), chronic myelogenous leukemia (CML), chronic myeloproliferative tumor, colon cancer, colorectal cancer, craniopharyngioma, skin T-cell lymphoma, cholangiocarcinoma, ductal carcinoma in situ, embryonal tumor, endometrial carcinoma, ependymoma, esophageal carcinoma, sensory neuroblastoma, ewing sarcoma, extracranial germ cell tumor, extragonadal germ cell tumor, extrahepatic cholangiocarcinoma, eye cancer, fallopian tube cancer, bone fibrous histiocytoma, gallbladder cancer, gastric cancer, gastrointestinal carcinoid tumor, gastrointestinal stromal tumor (GIST), germ cell tumor, gestational trophoblastic disease, glioma, hairy cell tumor, hairy cell leukemia, head and neck cancer, heart cancer, hepatocellular carcinoma, histiocytosis, Hodgkin's lymphoma, hypopharyngeal cancer, intraocular melanin tumor, islet cell tumor, pancreatic neuroendocrine tumor, Kaposi's sarcoma, kidney cancer, langerhans cell histiocytosis, laryngeal cancer, leukemia, lip and oral cavity cancer, liver cancer, lung cancer, lymphoma, macroglobulinemia syndrome, bone malignant fibrous histiocytoma, bone cancer, melanoma, merkel cell carcinoma, mesothelioma, metastatic squamous neck cancer, midline carcinoma, oral cancer, multiple endocrine tumor syndrome, multiple myeloma, mycosis mycosis fungoides, myelodysplastic syndrome, myelodysplastic/myeloproliferative tumor, myelogenous leukemia, myelogenous leukemia, multiple myeloma, myeloproliferative tumor, nasal cavity and sinus cancer, nasopharyngeal carcinoma, neuroblastoma, non-Hodgkin's lymphoma, non-small cell lung cancer, oral cancer, oral cancer, lip cancer, oropharyngeal cancer, osteosarcoma, ovarian cancer, pancreatic cancer, papillomatosis, paraganglioma, paranasal sinus and nasal cavity cancer, parathyroid cancer, penile cancer, pharyngeal cancer, pheochromocytoma, pituitary cancer, plasmacytoma, pleuropulmonary blastoma, pregnancy and breast cancer, primary central nervous system lymphoma, primary peritoneal cancer, prostate cancer, rectal cancer, renal cell carcinoma, retinoblastoma, rhabdomyosarcoma, salivary gland cancer, sarcoma, sezari syndrome, skin cancer, small cell lung cancer, small bowel cancer, soft tissue sarcoma, squamous cell Carcinoma, squamous neck cancer, gastric cancer, T-cell lymphoma, testicular cancer, throat cancer, thymoma and thymic cancer, thyroid cancer, transitional cell carcinoma of the renal pelvis and ureter, unknown primary cancer, urethral cancer, uterine cancer, uterine sarcoma , vaginal cancer, vulvar cancer and Wilm's tumor.

According to some embodiments, the hematological cancer (eg, hematological cancer of the cancer associated with RET) is selected from leukemia, lymphoma (non-Hodgkin's lymphoma), Hodgkin's disease (also called Hodgkin's lymphoma) dnd myeloma, for example, acute lymphocytic leukemia (ALL), acute myelogenous leukemia (AML), acute promyelocytic leukemia (APL), chronic lymphocytic leukemia (CLL), chronic myelogenous leukemia (CML), chronic myeloid leukemia (CML), chronic myelomonocytic leukemia (CMML), chronic neutrophil leukemia (CNL), acute undifferentiated leukemia (AUL), anaplastic large cell lymphoma (ALCL), young lymphocytic leukemia (PML), juvenile monocytic leukemia (JMML), adult T-cell ALL, triple myelodysplastic AML (AML/TMDS), mixed lineage leukemia (MLL), myelodysplastic syndrome (MDS), myeloproliferative disease (MPD) and multiple myeloma (MM). Other examples of blood cancers include myeloproliferative diseases (MPD), such as polycythemia vera (PV), essential thrombocytopenia (ET) and idiopathic primary myelofibrosis (IMF/IPF/PMF)). In one embodiment, the hematological cancer (e.g., hematological cancer that is a cancer associated with RET) is AML or CMML.

According to some embodiments, the cancer (e.g., RET-related cancer) is a solid tumor. Examples of solid tumors (e.g., solid tumors that are cancers associated with RET) comprise, e.g., thyroid cancer (e.g., papillary thyroid cancer, medullary thyroid carcinoma), lung cancer (e.g., lung adenocarcinoma, small cell lung cancer), pancreatic cancer, pancreatic duct cancer, breast cancer, colon cancer, colorectal cancer, prostate cancer, renal cell carcinoma, head and neck tumors, neuroblastoma and melanoma. See, for example, Nature Reviews cancer, 2014, 14, 173-186.

According to some embodiments, the cancer is selected from lung cancer, papillary thyroid cancer, medullary thyroid cancer, differentiated thyroid cancer, recurrent thyroid cancer, refractory differentiated thyroid cancer, type 2A or 2B multiple endocrine tumors (respectively MEN2A or MEN2B), pheochromocytoma, parathyroid hyperplasia, breast cancer, colorectal cancer, papillary renal cell carcinoma, gastrointestinal mucosal gangliocytoma and cervical cancer.

According to some embodiments, the patient is a human.

The compound of formula I or the pharmaceutically acceptable salt or solvate thereof can also be used to treat a RET-related cancer.

Also provided herein is a method for the treatment of irritable bowel syndrome (IBS) in a patient in need of treatment, comprising: (a) determining whether the cancer is related to a disorder of the expression or activity level of RET gene, RET kinase, or any one of them (for example, RET-related cancer); (b) if it is determined that the cancer is related to a disorder of the expression or activity level of RET gene, RET kinase, or any one of them (for example, RET-related cancer), administer to the patient a therapeutically effective amount of the compound represented by formula I as defined herein, or the pharmaceutically acceptable salt or solvate thereof, or the pharmaceutical composition thereof.

Also provided herein is a pharmaceutical combination for use in the treatment of irritable bowel syndrome (IBS) in patients in need of treatment, comprising adminstering: (a) the compound represented by formula I as defined herein, or the stereoisomer, racemate, tautomer, isotope labelled derivative, nitrogen oxide, pharmaceutically acceptable salt or solvate thereof; (b) another therapeutic agent; and (c) optionally, at least one pharmaceutically acceptable carrier, for simultaneous, separate or sequential use in the treatment of IBS, wherein the amount of the compound represented by formula I as defined herein, or the stereoisomer, racemate, tautomer, isotope labelled derivative, nitrogen oxide, pharmaceutically acceptable salt or solvate thereof and the another therapeutic agent together are jointly effective for treating IBS. Also provided herein is a pharmaceutical composition comprising the combination. Also provided herein is the use of the combination in the preparation of a medicament useful for the treatment of cancer. Also provided herein is a commercial package or product, which comprises the combination as a combined preparation for simultaneous, separate or sequential use; and relates to a method of treating IBS in a patient in need of treatment.

Where the compound according to the present disclosure is used as a medicine, it can be administered in the form of a pharmaceutical composition. These compositions can be prepared in a manner well known in the pharmaceutical art, and can be administered in a variety of ways, depending on whether local or systemic treatment is needed and the area to be treated. For example, the administration can be a topical administration (for example, transdermal, percutaneous, transdermic and mucous membrane administration comprising intranasal, vaginal and rectal delivery), pulmonary administration (for example, by inhalation or insufflation of powder or aerosol, including through a sprayer; intratracheal, intranasal), oral administration or parenteral administration. Parenteral administration comprises intravenous, intraarterial, subcutaneous, intraperitoneal or intramuscular injection or infusion; or intracranial, such as intrathecal or intracerebroventricular administration. Moreover, they can be administered parenterally in the form of a single bolus dose from, or can be administered by, for example, a continuous infusion pump. Pharmaceutical composition and preparation for topical administration may comprise transdermal patch, ointment, lotion, cream, gel, drop, suppositorie, spray, liquid, and powder. Conventional pharmaceutical carrier, water, powder or oily base, thickener, etc. might be necessary or required. Coated condom, glove, etc. can also be useful.

During the preparation of the composition according to the present disclosure, the active ingredient can be usually mixed with excipients, diluted with excipients or filled into such carriers in the form of such as capsule, sachet, paper or another container. Where the excipient is used as a diluent, it can be a solid, semi-solid, or liquid substance that serves as a vehicle, carrier, or medium for the active ingredient. Therefore, the composition may be in the form of tablet, pill, powder, lozenge, sachet, cachet, elixir, suspension, emulsion, solution, syrup, aerosol (solid or dissolved in a liquid solvent); ointment, soft and hard gelatin capsule, suppository, sterile injectable solution and sterile packaged powder containing, for example, up to 10% by weight of the active compound.

Examples of suitable excipient comprise lactose, glucose, sucrose, sorbitol, mannitol, starch, acacia, calcium phosphate, alginate, tragacanth, gelatin, calcium silicate, microcrystalline cellulose, poly vinylpyrrolidone, cellulose, water, syrup and methylcellulose. The preparation may also comprise: lubricants such as talc, magnesium stearate and mineral oil; wetting agents; emulsifiers and suspending agents; preservatives such as methyl benzoate and hydroxypropyl benzoate; sweeteners and flavoring agents. The composition of the present disclosure can be formulated by using methods known in the art to provide immediate release, sustained release, or delayed release of the active ingredient after the administration to a patient.

The composition can be formulated in a unit dosage form, wherein each dosage may comprise about 5 to about 1000 mg, more usually about 100 to about 500 mg, of the active ingredient. The term "unit dosage form" refers to physically separated single dosage unit suitable for use in human patients and other mammals, wherein each unit comprises a predetermined amount of activity substance which is mixed with a suitable pharmaceutical excipient and calculated to produce the desired therapeutic effect.

The effective dose of the active compound can be used in a wide range, and is usually administered in a pharmaceutically effective amount. However, it can be understood that the amount of the compound actually administered is usually determined by the physician according to the relevant circumstances, which comprise the condition to be treated, the route of administration selected, the actual compound administered; the age, weight and response of the individual patient; the patient's symptoms severity etc.

For the preparation of solid compositions such as tablet, the major active ingredient is mixed with pharmaceutical

excipients to form a solid pre-formulation composition comprising a homogeneous mixture of the compound of the present disclosure. Where these pre-formulation compositions are referred to as homogeneous, it means that the active ingredients are usually evenly distributed throughout the composition, so that the composition can be easily divided into equally effective unit dosage forms such as tablet, pill, and capsule. The solid pre-formulation can be then divided into the aforementioned types of unit dosage forms comprising, for example, about 0.1 to about 1000 mg of the active ingredient of the present disclosure.

The tablet or pill of the present disclosure can be coated or compounded to obtain a dosage form that provides the advantage of long-acting action. For example, a tablet or pill comprises an inner dose component and an outer dose component, wherein the outer dose component constitutes the coating film of the inner dose. The two components can be separated by an enterosoluble layer which is used to prevent the disintegration in the stomach, so that the inner component can pass through the duodenum intact or delay its release. A variety of substances, comprising a variety of polymer acids and the mixture of the polymer acid with substances such as shellac, cetyl alcohol and cellulose acetate, can be used for such enterosoluble layer or coating agent.

The compounds and compositions of the present disclosure can be incorporated into a liquid form for oral or injection administration comprising an aqueous solution, appropriately flavored syrup, water or oil suspension; and edible oils such as cottonseed oil, sesame oil, and coconut oil or peanut oil flavored emulsions; and elixir and similar pharmaceutical solvents.

The composition for inhalation or insufflation comprises solution, suspension, and powder of the compound according to the disclosure dissolved in pharmaceutically acceptable water or organic solvents or mixtures thereof. The liquid or solid composition may comprise a suitable pharmaceutically acceptable excipient as described above. In some embodiments, the composition is administered via oral or nasal respiratory routes to achieve local or systemic effects. The composition can be atomized by using an inert gas. The atomized solution can be directly inhaled by the atomizing device, or the atomizing device can be connected with the mask drape or intermittent positive pressure breathing machine. The solution, suspension, or powder composition can be administered orally or nasally from a device that delivers the formulation in an appropriate manner.

The amount of the compound or composition administered to the patient is not invariable, and depends on the medicament to be administered, the purpose of administration, such as prevention or treatment; the state of the patient, the way of administration, and the like. In therapeutic applications, the composition can be administered to patients who have already suffered from the disease in an amount sufficient to cure or at least partially inhibit the symptoms of the disease and its complications. The effective dose should depend on the disease state to be treated and the judgment of the attending clinician, which depends on factors such as the severity of the disease, the age, weight and general condition of the patient.

The composition administered to the patient may be in the form of the above-mentioned pharmaceutical composition. These compositions can be sterilized by conventional sterilization techniques or filter sterilization. The aqueous solution can be used as it is packaged or lyophilized, wherein the lyophilized preparation can be mixed with a sterile aqueous carrier before administration. The pH of the compound preparation is usually 3 to 11, more preferably 5 to 9, most preferably 7 to 8. It is understood that the use of some aforementioned excipients, carriers or stabilizers can result in the formation of a pharmaceutical salt.

The therapeutic dose of the compound of the present disclosure may be determined based on, for example, the following factors: the specific use of the treatment, the manner of administration of the compound, the health and condition of the patient, and the judgment of the prescribing physician. The ratio or concentration of the compound of the present disclosure in the pharmaceutical composition may not be fixed, depending on a variety of factors, including dosage, chemical properties (such as hydrophobicity), and route of administration. For example, the compound of the present disclosure can be provided by a physiological buffer aqueous solution containing about 0.1-10% w/v of the compound for parenteral administration. Some typical dosage ranges are about 1 μg/kg to about 1 g/kg body weight/day. In certain embodiments, the dosage range is from about 0.01 mg/kg to about 100 mg/kg body weight/day. The dosage is likely to depend on such variables, such as the type and degree of development of the disease or condition, the general health status of the specific patient, the relative biological efficacy of the selected compound, the excipient formulation and its route of administration. The effective dose can be obtained by extrapolating the dose-response curve derived from the in vitro or animal model test system.

## Terms and definitions

Unless otherwise stated, the definitions of groups and terms described in the description and claims of this application comprise the definitions as described in examples, exemplary definitions, preferred definitions, definitions described in tables, and definitions of specific compounds in the examples. These definitions can be combined with each other optionally. Such combined group definition and compound structure should be regarded as described within the scope of the present description.

Unless otherwise specified, the numerical ranges described in this specification and claims are equivalent to at least describing each specific integer value therein. For example, the numerical range "1-40" is equivalent to describing each integer value within the numerical range "1-10", namely 1, 2, 3, 4, 5, 6, 7, 8, 9, 10, and the numerical range each integer value in "11-40" is 11, 12, 13, 14, 15, ..., 35, 36, 37, 38, 39, 40. It should also be understood that where the number of the groups or substituents is described as "one, two or more" herein, the term "more" shall refer to an integer $\geq$ 3, such as 3, 4, 5, 6, 7, 8, 9 or 10. In addition, where a certain numerical range is defined as a "number", it should be understood as describing the two endpoints of the range, each integer within the range, and each decimal within the range. For example, "a number from 0 to 10" should be understood as not only describing each integer of 0, 1, 2, 3, 4, 5, 6, 7, 8, 9 and 10, but also describing at least each the sum of each integer with 0.1, 0.2, 0.3, 0.4, 0.5, 0.6, 0.7, 0.8 or 0.9.

The term "halogen" represents fluorine, chlorine, bromine and iodine.

The term "$C_{1-40}$ alkyl" should be understood to preferably represent a linear or branched saturated monovalent hydrocarbon group having 1 to 40 carbon atoms. For example, "$C_{1-6}$ alkyl" represents a linear or branched alkyl group having 1, 2, 3, 4, 5, or 6 carbon atoms. The alkyl group can be, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl group, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc. or their isomers.

The term "$C_{2-40}$ alkenyl" should be understood to preferably represent a linear or branched monovalent hydrocarbon group, which comprises one or more double bonds and has 2-40 carbon atoms, preferably "$C_{2-6}$ alkenyl". "$C_{2-6}$ alkenyl" should be understood to preferably represent a straight-chain or branched monovalent hydrocarbon group, which comprises one or more double bonds and has 2, 3, 4, 5 or 6 carbon atoms, especially 2 or 3 carbon atoms ("$C_{2-3}$ alkenyl"), it should be understood that where the alkenyl group comprises more than one double bond, the double bonds may be separated from each other or conjugated. The alkenyl group is, for example, vinyl, allyl, (E)-2-methylvinyl, (Z)-2-methylvinyl, (E)-but-2-enyl, (Z)-but-2-enyl, (E)-but-1-enyl, (Z)-but-1-enyl, pent-4-enyl, (E)-pent-3-enyl, (Z) -pent-3-enyl, (E)-pent-2-enyl, (Z)-pent-2-enyl, (E)-pent-1-enyl, (Z)-pent-1-ene group, hex-5-enyl, (E)-hex-4-enyl, (Z)-hex-4-enyl, (E)-hex-3-enyl, (Z)-hex-3-alkenyl, (E)-hex-2-enyl, (Z)-hex-2-enyl, (E)-hex-1-enyl, (Z)-hex-1-enyl, isopropenyl , 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-alkenyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propyl vinyl, 1-isopropyl vinyl.

The term "$C_{2-40}$ alkynyl" should be understood to represent a linear or branched monovalent hydrocarbon group, which comprises one or more triple bonds and has 2-40 carbon atoms, preferably "$C_2$-$C_6$-alkynyl". The term "$C_2$-$C_6$-alkynyl" should be understood to preferably represent a straight-chain or branched monovalent hydrocarbon group, which comprises one or more triple bonds and has 2, 3, 4, 5 or 6 carbon atoms, especially 2 or 3 carbon atoms ("$C_2$-$C_3$-alkynyl"). The $C_2$-$C_6$-alkynyl group is, for example, ethynyl, prop-1-ynyl, prop-2-ynyl, but-1-ynyl, but-2-ynyl, but-3-ynyl, pent-1-alkynyl, pent-2-ynyl, pent-3-ynyl, pent-4-ynyl, hex-1-ynyl, hex-2-ynyl, hex-3-ynyl, hex-4 -alkynyl, hex-5-ynyl, 1-methylprop-2-ynyl, 2-methylbut-3-ynyl, 1-methylbut-3-ynyl, 1-methylbut-2-alkynyl, 3-methylbut-1-ynyl, 1-ethylprop-2-ynyl, 3-methylpent-4-ynyl, 2-methylpent-4-ynyl, 1 -methylpent-4-ynyl, 2-methylpent-3-ynyl, 1-methylpent-3-ynyl, 4-methylpent-2-ynyl, 1-methylpent-2 -alkynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl group is ethynyl, prop-1-ynyl or prop-2-ynyl.

The term "$C_{3-40}$ cycloalkyl" should be understood to represent a saturated monovalent monocyclic, bicyclic hydrocarbon ring or bridged cycloalkyl having 3-40 carbon atoms, preferably "$C_{3-10}$ cycloalkyl". The term "$C_{3-10}$ cycloalkyl" should be understood to represent a saturated monovalent monocyclic, bicyclic hydrocarbon ring or bridged cycloalkyl having 3, 4, 5, 6, 7, 8, 9 or 10 carbon atoms. The $C_{3-10}$ cycloalkyl group may be a monocyclic hydrocarbon group, such as cyclopropyl, cyclobutyl, cyclopentyl, cyclohexyl, cycloheptyl, cyclooctyl, cyclononyl or cyclodecyl, or a bicyclic hydrocarbon group such as decalin ring.

The term "3-20 membered heterocyclyl" represents a saturated monovalent monocyclic, bicyclic hydrocarbon ring or bridged cycloalkane, which is a non-aromatic cyclic group comprising 3-20 (such as 3, 4, 5, 6, 7, 8, 9, 10, etc.) of ring atoms in total and 1-5 heteroatoms independently selected from N, O and S, and is preferably a "3-10 membered heterocyclyl". The term "3-10 membered heterocyclyl" represents a saturated monovalent monocyclic, bicyclic hydrocarbon ring or bridged cycloalkane, comprising 1-5, preferably 1-3 heteroatoms selected from N, O and S. The heterocyclyl may be attached to the rest of the molecule through any one of the carbon atoms or a nitrogen atom (if present). In particular, the heterocyclyl includes but is not limited to: 4-membered ring, such as azetidinyl, oxetanyl; 5-membered ring, such as tetrahydrofuranyl, dioxolyl, pyrrole alkyl, imidazolidinyl, pyrazolidinyl, pyrrolinyl; or 6-membered ring, such as tetrahydropyranyl, piperidinyl, morpholinyl, dithiaalkyl, thiomorpholinyl, piperazinyl or trithiaalkyl; or a 7-membered ring, such as diazacycloheptanyl. Optionally, the heterocyclyl may be benzo-fused. The heterocyclyl may be bicyclic,

such as but not limited to a 5,5-membered ring, such as hexahydrocyclopenta[c]pyrrole-2(1H)-yl ring, or a 5,6-membered bicyclic ring, such as hexahydropyrrole and [1,2-a]pyrazine-2(1H)-yl ring. The ring containing nitrogen atoms may be partially unsaturated, that is, it may contain one or more double bonds, such as but not limited to 2,5-dihydro-1H-pyrrolyl, 4H-[1,3,4]thiadiazinyl, 4,5-dihydrooxazolyl or 4H-[1,4]thiazinyl, or it may be benzo-fused, such as but not limited to dihydroisoquinolinyl. According to the present disclosure, the heterocyclyl is non-aromatic. Where the 3-20 membered heterocyclyl is attached to another group to form the compound of the present disclosure, the carbon atom or hetero atom of the 3-20 membered heterocyclyl may be attached to said group. For example, where the 3-20 membered heterocyclyl is selected from the piperazinyl group, the nitrogen atom in the piperazinyl group may be attached to another group. Alternatively, where the 3-20 membered heterocyclyl is selected from piperidinyl, the nitrogen atom in the piperidinyl ring or the carbon atom in the para position can be attached to another groups respectively.

The term "$C_{6-20}$ aryl" should be understood to preferably represent a monovalent aromatic or partially aromatic monocyclic, bicyclic or tricyclic hydrocarbon ring having 6 to 20 carbon atoms, preferably "$C_{6-14}$ aryl". The term "$C_{6-14}$ aryl" should be understood to preferably represent a monocyclic, bicyclic or tricyclic monocyclic, bicyclic or tricyclic aryl having 6, 7, 8, 9, 10, 11, 12, 13 or 14 carbon atoms ("$C_{6-14}$ aryl"), especially a ring having 6 carbon atoms ("$C_6$ aryl"), such as phenyl or biphenyl; or a ring having 9 carbon atoms (" $C_9$ aryl"), such as indanyl or indenyl; or a ring having 10 carbon atoms ("$C_{10}$ aryl"), such as tetrahydronaphthyl, dihydronaphthyl or naphthyl; or a ring having 13 carbon atoms ("$C_{13}$ aryl"), such as fluorenyl; or a ring having 14 carbon atoms ("$C_{14}$ aryl"), such as anthryl. Where the $C_{6-20}$ aryl group is substituted, it may be mono-substituted or multi-substituted. In addition, there is no restriction on the substitution site, for example, ortho, para, or meta substitution can be adopted.

The term "5-20 membered heteroaryl" should be understood to comprise such a monovalent monocyclic, bicyclic or tricyclic aromatic ring system: it has 5-20 ring atoms and comprises 1-5 heteroatoms independently selected from N, O and S, such as "5-14 membered heteroaryl". The term "5-14 membered heteroaryl" should be understood to comprise a monovalent monocyclic, bicyclic or tricyclic aromatic ring system having 5, 6, 7, 8, 9, 10, 11, 12, 13 or 14 ring atoms, especially 5, 6, 9 or 10 carbon atoms, and comprise 1-5, preferably 1-3 heteroatoms each independently selected from N, O and S. Additionally, in each case the ring can be benzo-fused. In particular, the heteroaryl is selected from thienyl, furyl, pyrrolyl, oxazolyl, thiazolyl, imidazolyl, pyrazolyl, isoxazolyl, isothiazolyl, oxadiazolyl, triazolyl, thiol diazolyl, thio-4H-pyrazolyl, etc. and their benzo derivatives, such as benzofuranyl, benzothienyl, benzoxazolyl, benzisoxazolyl, benzimidazolyl, benzotriazolyl, indazolyl, indolyl, isoindolyl, etc.; or pyridyl, pyridazinyl, pyrimidinyl, pyrazinyl, triazinyl, etc., and their benzo derivatives, such as quinoline group, quinazolinyl, isoquinolinyl, etc.; or azepinyl, indazinyl, purinyl, etc. and their benzo derivatives; or cinolinyl, phthalazinyl, quinazolinyl, quinoxalinyl, naphthyridinyl, pterridinyl, carbazolyl, acridinyl, phenazinyl, phenothiazinyl, phenoxazinyl, etc. Where the 5-20 membered heteroaryl is attached to another group to form the compound of the present disclosure, the carbon atom or the hetero atom in the 5-20 membered heteroaryl ring may be attached to another group. Where the 5-20 membered heteroaryl is substituted, it may be mono-substituted or multi-substituted. In addition, there is no restriction on the substitution position. For example, the hydrogen attached to the carbon atom in the heteroaryl ring or the hydrogen attached to the heteroatom in the heteroaryl ring may be substituted respectively.

Unless otherwise specified, heterocyclyl, heteroaryl and heteroarylene group comprises all possible isomeric forms thereof, such as positional isomers thereof. Therefore, as to some illustrative and nonlimiting examples, these groups may comprise the forms chemically bonded to other groups at one, two or more positions selected from 1-, 2-, 3-, 4-, 5-, 6-, 7-, 8-, 9-, 10-, 11- and 12-position, etc. (if present), comprising pyridin-2-yl, pyridin-2-ylene, pyridin-3-yl, pyridin-3-ylene, pyridin-4-yl and pyridin-4-ylene; thienyl and thienylene comprise thiophen-2-yl, thiophen-2-ylene, thiophen-3-yl and thiophene-3-ylene; pyrazol-1-yl, pyrazol-3-ylene, pyrazol-4-yl and pyrazol-5-ylene.

The term "oxo" means that the carbon atom, nitrogen atom or sulfur atom in a substituent is oxidated to form an oxo substituent (=O).

Unless otherwise specified, the definitions of the terms in this article are also suitable for the group comprising the terms. For example, the definition of $C_{1-6}$ alkyl is also applicable to $C_{1-6}$ alkyloxy, -N($C_{1-6}$ alkyl)$_2$, -NH$C_{1-6}$ alkyl or -S(O)$_2$-$C_{1-6}$ alkyl, etc.

Those skilled in the art can understand that the compound represented by formula I may exist in the form of various pharmaceutically acceptable salts. If these compounds have basic centers, they can form acid addition salts; while if these compounds have acidic centers, they can form basic addition salts; if these compounds contain both acidic centers (such as carboxyl groups) and basic centers (for example, amino), they can also form internal salts. In the present application, the number of salts formed by the compound can be determined by the basic center or the acidic center. For example, where the compound comprises multiple salt-forming sites, the number of salt-forming sites is equal to the number of salt-forming sites. Acid addition salts include but are not limited to hydrochloride, hydrofluoride, hydrobromide, hydroiodide, sulfate, pyrosulfate, phosphate, nitrate, methanesulfonate, ethanesulfonate, 2-hydroxyethanesulfonate, benzenesulfonate, toluenesulfonate, sulfamate, 2-naphthalenesulfonate, formate, acetoacetic acid, pyruvic acid, lauric acid, cinnamate, benzoate, acetate, glycolate, trifluoroacetate, trimethylacetate, propionate, butyrate, caproate, heptanoate, undecanoate, hard fatty acid salt, ascorbate, camphorate, camphorsulfonate, citrate, fumarate,

malate, maleate, hydroxymaleate, oxalate, salicylate, succinate acid salt, gluconate, quinate, pamoate, glycolate, tartrate, lactate, 2-(4-hydroxybenzoyl)benzoate, cyclopentane propionic acid Salt, digluconate, 3-hydroxy-2-naphthoate, nicotinate, pamoate, pectinate, 3-phenylpropionate, picrate, pivalate, itaconic acid salt, trifluoromethane sulfonate, lauryl sulfate, p-toluene sulfonate, naphthalene disulfonate, malonate, adipate, alginate, mandelate, gluopentate, glycerophosphate, sulfosalicylate, hemisulfuric acid or thiocyanate, aspartate, etc.; alkali addition salts such as alkali metal salts, alkaline earth metal salts and ammonium salts, etc., specifically including but not limited to: sodium salt, lithium salt, potassium salt, ammonium salt, aluminum salt, magnesium salt, calcium salt, barium salt, iron salt, ferrous salt, manganese salt, manganous salt, zinc salt, ammonium salt (including with $NH_3$ Salts formed with organic amines ($NH_4$ salt), methylammonium salt, trimethylammonium salt, diethylammonium salt, triethylammonium salt, propylammonium salt, tripropylammonium salt, isopropylammonium salt, tert-butylammonium salt, N,N'-dibenzylethylenediammonium salt, dicyclohexylammonium salt, 1,6-hexamethylenediammonium salt, benzylammonium salt, ethanolammonium salt, N,N-dimethylethanolammonium salt, N,N-diethylethanolammonium salt, triethanolammonium salt, tromethamine salt, lysine salt, arginine salt, histidine salt, glucosamine salt, N-methyl glucosamine salt, dimethylglucose ammonium salt, ethyl glucosamine salt, meglumine salt, betaine salt, caffeine salt, chloroprocaine salt, procaine salt, lidocaine salt, pyridine salt, picoline salt, piperidine salt, morpholine salt, piperazine salt, purine salt, theobromine salt, choline salt), etc.

The compound of the present disclosure may exist in the form of a solvate (such as a hydrate), wherein the compound of the present disclosure comprises a polar solvent as a structural element of the crystal lattice of the compound, especially such as water, methanol or ethanol. The amount of polar solvent, especially water, can be present in a stoichiometric or non-stoichiometric ratio.

According to its molecular structure, the compound of the present disclosure may be chiral, and therefore may exist in various enantiomeric forms. Therefore, these compounds may exist in racemate form or optically active form. The compounds of the present disclosure or intermediates thereof can be separated into enantiomeric compounds by chemical or physical methods known to those skilled in the art, or used in synthesis in this form. In the case of racemic amines, diastereomers are prepared from the mixture by reaction with optically active resolving reagents. Examples of suitable resolution reagents are optically active acids, such as R and S forms of tartaric acid, diacetyltartaric acid, dibenzoyltartaric acid, mandelic acid, malic acid, lactic acid, appropriate N-protected amino acids (e.g., N-Benzoyl proline or N-benzenesulfonyl proline) or various optically active camphor sulfonic acids. With the aid of optically active resolving reagents (such as dinitrobenzoylphenylglycine, cellulose triacetate or other carbohydrate derivatives or chiral derivatized methacrylate polymers) immobilized on silica gel, chromatographic enantiomeric resolution is advantageously performed. Suitable eluents for this purpose are aqueous or alcohol-containing solvent mixtures, for example, hexane/isopropanol/acetonitrile.

The term "tautomer" refers to an isomer of a functional group resulting from the rapid movement of an atom in two positions in a molecule. The compounds of the present disclosure may exhibit tautomerism. Tautomeric compounds can exist in two or more mutually convertible species. Proton shift tautomers result from the migration of covalently bonded hydrogen atoms between two atoms. Tautomers generally exist in an equilibrium form. An attempt to separate a single tautomer usually produces a mixture having physical and chemical properties consistent with a mixture of compounds. The position of equilibrium depends on the chemical properties of the molecule. For example, in many aliphatic aldehydes and ketones such as acetaldehyde, the ketone type is dominant; in phenol, the enol type is dominant. The present disclosure encompasses all tautomeric forms of the compound.

The corresponding stable isomers can be separated according to known methods, such as extraction, filtration or column chromatography.

The term "patient" refers to any animal comprising mammals, preferably mouse, rat and other rodents, rabbit, dog, cat, pig, cattle, caprid, horse or primate, and most preferably human.

The phrase "therapeutically effective amount" used herein refers to the amount of the active compound or medicament that causes a biological or medical response that researchers, veterinarians, physicians, or other clinicians are looking for in tissues, systems, animals, individuals, or humans. Including one or more of the following: (1) Prevention of disease: For example, prevention of disease, disorder or disease in individuals who are susceptible to disease, disorder, or disease but who have not experienced or exhibited disease pathology or symptoms. (2) Inhibiting disease: for example, inhibiting the disease, disorder or condition in an individual who is experiencing or experiencing the pathology or symptoms of the disease, disorder or condition (ie, preventing the further development of the pathology and/or symptoms). (3) Alleviation of disease: for example, alleviation of the disease, disorder or condition (ie reversing the pathology and/or symptoms) in an individual who is experiencing or experiencing the pathology or symptoms of the disease, disorder, or condition.

Advantageous effect of the present disclosure

The inventors surprisingly found that the compound prepared by the present disclosure have significant inhibitory activity against RET wild type, mutant type and fusion type. As compared with the existing compounds, the activity of

the compound according to the present disclosure has significant improvement. Furthermore, as compared with other RET inhibitors, the representative example compounds of the present disclosure further have particularly excellent pharmacokinetic properties so that they can be administered to patients in smaller dose upon being used as an active ingredient, thereby reducing the cost of treatment for patient. In addition, the preparation method of the compound of the present application is simple and suitable for large-scale production.

## DETAILED DESCRIPTION

The technical solutions according to the disclosure will be further described in detail in combination with specific examples. It should be understood that the following examples are merely illustrative of the present disclosure and are not to be construed as limiting the scope of the present disclosure. The technology that is implemented based on the above-described contents of the present disclosure is encompassed within the protection scope of the present disclosure.

Unless otherwise specified, all the raw materials and reagents used in the following examples are commercially available or can be prepared by a known method.

Reference Example 1

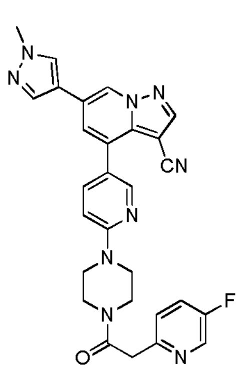

APS001

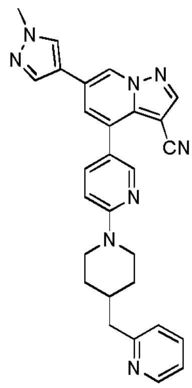

APS002

The compound APS001 shown in the above formula can be prepared with reference to the method disclosed in Example 342 of the patent literature CN108349969A. After its trifluoroacetate is prepared, the target compound APS001 can be isolated and obtained under an alkaline condition (sodium bicarbonate).

The compound APS002 can be prepared with reference to the method disclosed in Example 570 of the patent literature CN108349969A.

Example 1

2-amino-4-(6-(4-(2-(5-fluoropyridin-2-yl)acetyl)piperazin-1-yl)pyridin-3-yl)-6-(1-methyl-1H-pyrazol-4-yl)pyrazolo[1,5-a]pyridine-3-carbonitrile (APS069)

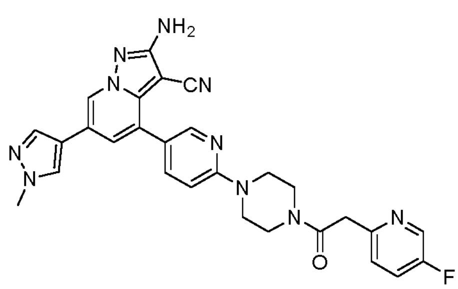

Step A: tert-butyl((methylsulfonyl)oxy)carbamate

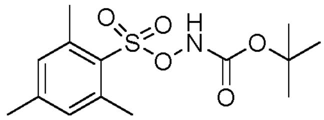

Under ice bath stirring, to the solution of 2,4,6-trimethylbenzenesulfonyl chloride (20.0 g, 91.5 mmol) and tert-butyl N-hydroxycarbamate (12.2 g, 91.5 mmol) in methyl tert-butyl ether (500 mL), triethylamine (13.0 mL, 93.3 mmol) was added slowly with a constant pressure dropping funnel, the temperature of the reaction system was kept below 5°C during the addition. Under the ice bath, the reaction system was stirred for 4.0 hours, filtered under reduced pressure to remove triethylamine hydrochloride, and rinsed with methyl tert-butyl ether three times. All the filtrate was concentrated under reduced pressure at a water bath temperature of less than 15°C to remove most of the methyl tert-butyl ether; under ice bath, n-hexane was added to the residue after concentration, stirred vigorously for 10 minutes, a large amount of white solid was precipitated out, filtered under reduced pressure, the filter cake was washed twice with n-hexane, vacuum dried to obtain tert-butyl ((methanesulfonyl)oxy)carbamate (26.1 g, 90% yield). m/z=316[M+1]$^+$.

Step B: 2-[(Aminooxy)sulfonyl]-1,3,5-trimethylbenzene

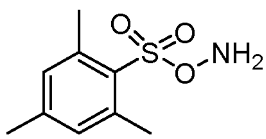

At 0 °C, tert-butyl((methylsulfonyl)oxy)carbamate (10.0 g, 31.7 mmol) was added to trifluoroacetic acid (80 mL) in batches. After completion, the reaction system continues to stir at 0 °C for 3 hours; TLC was used to confirm that the reaction was completed, poured the reaction system into a large amount of ice water and stirred for 15 minutes, a large amount of white solid was precipitated, filtered under reduced pressure, a large amount of water was used to wash the filter cake until the pH of the solid is neutral, which was then vacuum filtrated until the solid water content was about 20%. The product was directly used in the next reaction without further purification.

Step C: 2,4,6-trimethylbenzenesulfonic acid 1-amino-3-bromo-5-methoxypyridine-1-ium

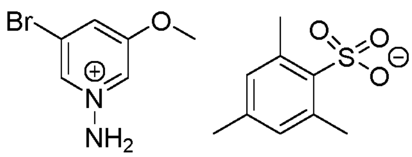

At 0 °C, 3-bromo-5-methoxypyridine (6.0 g, 32.0 mmol) was added to 2-[(aminooxy)sulfonyl]-1,3,5-trimethylbenzene (6.8 g, 31.7 mmol) in dichloromethane (50 mL) and stirred at 0 °C for 3 hours, a large amount of white solid was precipitated out. After the reaction was completed, ethyl ether (50 mL) was added to the reaction system at 0 °C and stirred for 10 minutes, filtered under reduced pressure, rinsed with ethyl ether, dried in vacuum to obtain 2,4,6-trimethylbenzenesulfonate 1-amino-3-bromo-5-methoxypyridine-1-ium (12.8 g, 100% yield), the product was used directly in the next reaction without further purification.
m/z=204[M+1]$^+$.

Step D: ethyl 2-cyanoiminoacetate hydrochloride

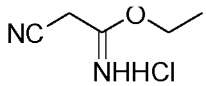

Malononitrile (4.9 g, 74.2 mmol), absolute ethanol (3.4 g, 74.2 mmol) and ethyl acetate (6 mL) were added to the sealed tube, cool the reaction system to 0° C, and added 2 M HCl ether solution (41 mL, 82 mmol) dropwise to the reaction system. After the addition, the temperature was raised to room temperature, and a large amount of white solid precipitated out. The reaction was carried out overnight at room temperature, TLC was used to monitor the completion of the reaction, filtered under reduced pressure, washed with ethyl ether, and dried in vacuo to obtain ethyl 2-cyanoiminoacetate hydrochloride (9.0 g, 82% yield). The product was used directly in the next reaction without further purification.

Step E: 2-amino-6-bromo-4-methoxrazolo[1,5-a]pyridine-3-carbonitrile

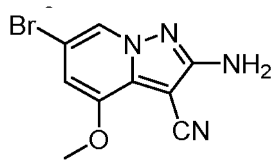

At room temperature, potassium carbonate (4.2 g, 30.0 mmol) was added to the solution of 2,4,6-trimethylbenzenesulfonic acid-1-amino-3-bromo-5-methoxypyridine-1-ium (4.0 g, 10.0 mmol) in DMF (30 mL). The reaction system was cooled to 0 °C, ethyl 2-cyanoiminoacetate hydrochloride (3.0 g, 20.0 mmol) was added in batches, and the temperature was raised to room temperature and further stirred for 1 hour, and then stirred at 90°C for 1 hour. After the reaction was completed, cooled to room temperature, quenched with water, extracted with ethyl acetate, and the organic phases were combined, washed with water, concentrated under reduced pressure, and separated by column chromatography to obtain the product 2-amino-6-bromo-4-methoxypyrazolo[1,5-a]pyridine-3-carbonitrile (420 mg, 16% yield).

$^1$HNMR (400MHz, DMSO-d6) δ 8.48 (d, *J* = 0.8 Hz, 1H), 7.08 (s, 1H), 6.34 (brs, 2H), 3.95 (s, 3H).m/z=267[M+1]$^+$.

Step F: 2-amino-4-methoxy-6-(1-methyl-1H-pyrazol-4-yl)pyrazolo[1,5-a]pyridine-3-carbonitrile

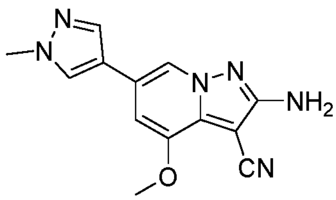

At room temperature, to a solution of 2-amino-6-bromo-4-methoxypyrazolo[1,5-a]pyridine-3-carbonitrile (220 mg, 0.824 mmol) and 1-methyl-4-(4,4,5,5-tetramethyl-1,3,2-dioxaborolan-2-yl)-1H-pyrazole (206 mg, 0.989 mmol) in 1,4-dioxane (10 mL), a sodium carbonate (262 mg, 2.472 mmol, 2M) aqueous solution was added, purged with nitrogen gas for 2 minutes, then tetrakistriphenylphosphine palladium (190 mg, 0.165 mmol) was added, purged with nitrogen gas for 2 minutes, stirred at 80 °C for 3 hours, After the reaction was completed as shown by TLC, water was added and extracted with ethyl acetate, and the organic phases were combined, washed with water, concentrated under reduced pressure, and separated by column chromatography to obtain the product 2-amino-4-methoxy-6-(1-methyl-1H-pyrazol-4-yl)pyrazolo[1,5-a]pyridine-3-carbonitrile (220 mg, 100% yield). $^1$HNMR (400MHz, DMSO-d6) δ 8.42 (s, 1H), 8.27 (s, 1H), 8.00 (s, 1H), 7.12 (s, 1H), 6.22 (s, 2H), 4.05 (s, 3H), 3.89 (d, 3H).m/z=269[M+1]$^+$.

Step G: 2-amino-4-hydroxy-6-(1-methyl-1H-pyrazol-4-yl)pyrazolo[1,5-a]pyridine-3-carbonitrile

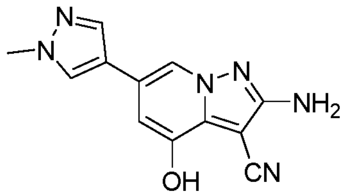

At room temperature, anhydrous aluminum trichloride (550 mg, 4.12 mmol) was added to a suspension of 2-amino-4-methoxy-6-(1-methyl-1H-pyrazol-4-yl)pyrazolo[1,5-a]pyridine-3-carbonitrile (220 mg, 0.824 mmol) in 1,2-dichloroethane (15 mL), raised to 80 °C and stirred for 3 hours. After the reaction was completed as shown by TLC, water was added, extracted with ethyl acetate, and the organic phases were combined, washed with water, concentrated under reduced pressure, and separated by column chromatography to obtain the product 2-amino-4-hydroxy-6-(1-methyl-1H-pyrazole-4-yl)pyrazolo[1,5-a]pyridine-3-carbonitrile (70 mg, 33% yield). m/z=255[M+1]$^+$.

Step H: 2-amino-3-cyano-6-(1-methyl-1H-pyrazol-4-yl)pyrazolo[1,5-a]pyridin-4-yl triflate

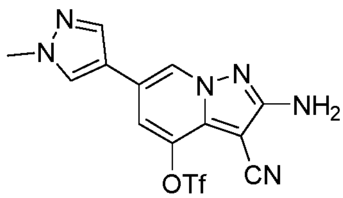

At room temperature, to a solution of 2-amino-4-hydroxy-6-(1-methyl-1H-pyrazol-4-yl)pyrazolo[1,5-a]pyridine-3-carbonitrile (70 mg, 0.275 mmol) in DMA (5 mL) were added DIEA (71 mg, 0.55 mmol) and N-phenylbis(trifluoromethane)sulfonimide (148 mg, 0.413 mmol), stirred and reacted for 3 hours. After the reaction was completed as shown by TLC, water was added, extracted with ethyl acetate, the organic phases were combined, washed with water, concentrated under reduced pressure, and separated by column chromatography to obtain the product 2-amino-3-cyano-6-(1-methyl-1H-pyrazol-4-yl)pyrazolo[1,5-a]pyridin-4-yl triflate (26 mg, 25% yield). m/z=387[M+1]$^+$.

Step I: diethyl 2-(5-fluoropyridin-2-yl)malonate

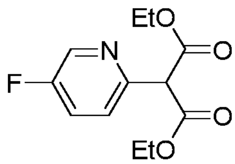

2-bromo-5-fluoropyridine (20 g, 0.114mol), dipicolinic acid (11.22 g, 0.091mol), diethyl malonate (73 g, 0.457mol), cuprous iodide (8.66 g, 0.046mol), cesium carbonate (111.49 g, 0.342mol), and 1,4-dioxane (400 mL) were added to a 1000 mL single-mouth flask, reacted at 100°C for 24 hours. After the reaction was completed as shown by TLC, water was added, extracted with ethyl acetate, the organic phases were combined, washed with water, concentrated under reduced pressure, and separated by column chromatography to obtain the product diethyl 2-(5-fluoropyridin-2-yl)malonate esters (16.6 g, 57% yield). m/z=256[M+1]$^+$.

Step J: ethyl 2-(5-fluoropyridin-2-yl)acetate

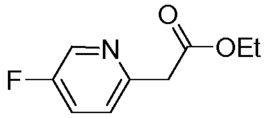

2-(5-fluoropyridin-2-yl) diethyl malonate (16.6 g, 0.065 mol), sodium chloride (15.1 g, 0.26 mol), water (4.68 g, 0.26 mol), DMSO (50 mL) were added to a 250 mL single mouth bottle, reacted at 150°C for 48 hours. After the reaction was completed as shown by TLC, water was added, extracted with ethyl acetate, the organic phases were combined, washed with water, dried with anhydrous sodium sulfate, filtered, concentrated under reduced pressure, and separated by column chromatography to obtain the product 2-(5-fluoropyridine-2-yl)ethyl acetate (9.1 g, 76% yield). m/z=184[M+1]$^+$.

Step K: 2-(5-fluoropyridin-2-yl)acetic acid

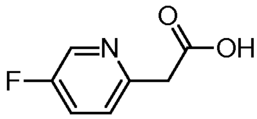

At room temperature, 2-(5-fluoropyridin-2-yl)ethyl acetate (5.5 g, 0.03 mol), tetrahydrofuran (50 mL), water (10 mL) amd lithium hydroxide (1.44 g, 0.06 mol) were added to a 250 mL single-mouth bottle, reacted at 60°C for 24 hours. After the raw materials were reacted completely, the reaction solution was concentrated to one third of the volume of the original reaction solution, and extracted with ethyl acetate. The layers are separated to obtain an aqueous phase, which was then adjusted to a pH of about 3 with 1M hydrochloric acid, then extracted with ethyl acetate, the organic phases were combined, dried with anhydrous sodium sulfate, filtered, and concentrated under reduced pressure to obtain the product 2-(5-fluoropyridin-2-yl)acetic acid (2.04 g, 44% yield).

Step L: 1-5-bromoridine-2-ierazine

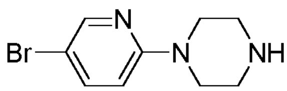

At room temperature, 2,5-dibromopyridine (12.2 g, 0.052 mol) and piperazine (5.0 g, 0.058 mol) were added to a 250 mL single-neck flask, then added DMSO (20 mL), and reacted overnight at 120°C. It was confirmed by LCMS that the reaction was completed, the DMSO was removed under reduced pressure, methanol was added to dissolve the residue, and saturated sodium hydroxide solution was slowly added under an ice-water bath, the pH was adjusted to about 13, and the mixture was further stirred for 2 hours. The mixture was concentrated under reduced pressure to remove methanol, extracted with dichloromethane, dried over anhydrous sodium sulfate, concentrated under reduced pressure, and separated by column chromatography to obtain the product (4.6 g, 37% yield). m/z=242[M+1]$^+$.

Step M: 1-(4-(5-bromopyridin-2-yl)piperazin-1-yl)-2-(5-fluoropyridin-2-yl)ethanone

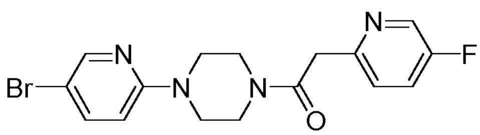

2-(5-fluoropyridin-2-yl)acetic acid (500 mg, 3.22 mmol), HATU (1.34 g, 3.54 mmol), DIEA (830 mg, 6.44 mmol), and DMF (20 mL) were added to a 100 mL single-neck flask, stirred at room temperature for 15 minutes, 1-(5-bromopyridine)-2-piperazine (780 mg, 3.22 mmol) was added, and stirred for 2 hours. After the reaction was completed as shown by TLC, water was added, extracted with ethyl acetate, the organic phases were combined, washed with water, dried

with anhydrous sodium sulfate, filtered, concentrated under reduced pressure, and separated by column chromatography to obtain the product 1-(4-(5-bromopyridine-2-yl)piperazin-1-yl)-2-(5-fluoropyridin-2-yl)ethanone (350 mg, 29% yield). m/z=379[M+1]$^+$.

Step N: 2-(5-fluoropyridin-2-yl)-1-(4-(5-(4,4,5,5-tetramethyl-1,3,2-dioxboronic acid-2-yl)pyridin-2-yl)piperazin-1-yl)ethanone

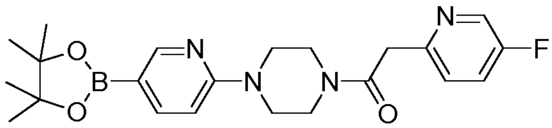

1-(4-(5-bromopyridin-2-yl)piperazin-1-yl)-2-(5-fluoropyridin-2-yl)ethan-1-one (50 mg, 0.132 mmol), duplex pinacol borate (50 mg, 0.198 mmol), [1,1'-bis(diphenylphosphino)ferrocene]palladium dichloride (10 mg, 0.013 mmol), potassium acetate (39 mg, 0.396 mmol) and 1,4-dioxane (20 mL) were added to a 10 mL sealed tube, purged with nitrogen for three times, reacted at 100°C for 3 hours, LCMS confirmed that the raw materials were reacted completely to give the product. The product was used for the next reaction directly without post-processing. m/z=427[M+l]$^+$.

Step O: 2-amino-4-(6-(4-(2-(5-fluoropyridin-2-yl)acetyl)piperazin-1-yl)pyridin-3 -yl)-6-(1-methyl-1H-pyrazol-4-yl)pyrazolo[1,5-a]pyridine-3-carbonitrile

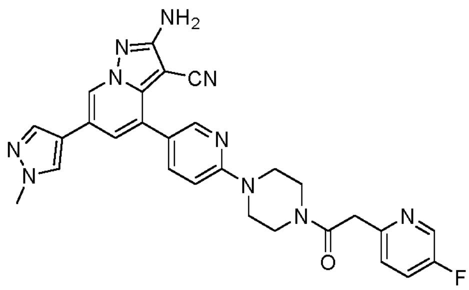

2-amino-3-cyano-6-(1-methyl-1H-pyrazol-4-yl)pyrazolo[1,5-a]pyridin-4-yl triflate (26 mg, 0.067 mol), palladium tetrakistriphenylphosphorus (15 mg, 0.013 mol), potassium carbonate (36 mg, 0.264 mol), 1,4-dioxane (2 mL) and $H_2O$ (1 mL) were added to the reaction solution of step N, purged with nitrogen for three times, reacted at 90°C for 2 hours. LCMS confirmed that the reaction of the raw materials was completed to give the product. Water was added, extracted with ethyl acetate, the organic phases were combined, washed with water, dried over anhydrous sodium sulfate, filtered, concentrated under reduced pressure, and separated by column chromatography to obtain the product 2-amino-4-(6-(4-(2-(5-fluoropyridin-2-yl)acetyl)piperazin-1-yl)pyridin-3 -yl)-6-(1-methyl-1H-pyrazol-4-yl)pyrazolo[1,5-a] pyridine-3-carbonitrile (5 mg, 13% yield).
$^1$HNMR (400MHz, DMSO-$d_6$) δ 8.79 (s, 1H), 8.49 (d, *J* = 3.0 Hz, 1H), 8.35 (d, *J* = 2.5 Hz, 1H), 8.28 (s, 1H), 8.02 (s, 1H), 7.80-7.78 (m, 1H), 7.71-7.66 (m, 1H), 7.57-7.54 (m, 1H), 7.42-7.38 (m, 1H), 6.98 (d, *J* = 8.8 Hz, 1H), 6.29 (s, 2H), 3.98 (s, 2H), 3.86 (s, 3H), 3.70-3.69 (d, *J* = 4.4 Hz, 2H), 3.613-3.604 (d, *J* = 3.6 Hz, 6H).m/z=537[M+l]$^+$.

Example 2

2-amino-6-(1-methyl-1H-pyrazol-4-yl)-4-(6-(4-(pyridin-2-yl-methyl)piperidin-1-yl)pyridin-3-yl)pyrazolo[1,5-a]pyridine-3-carbonitrile (APS070)

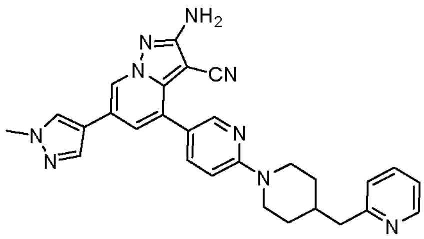

Step A: 1-tert-butoxcarbonl-4-ridin-2-1-methlieridine

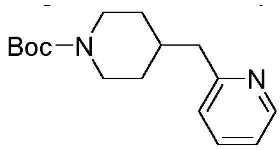

The mixture of 1-tert-butoxycarbonyl-4-methylenepiperidine (6.0 g, 30.4 mmol) and 9-borabicyclo[3,3,2]nonane (60.8 mL, 30.4 mmol, a solution in THF with a concentration of 2M) were heated to reflux for 3 hours under the protection of nitrogen, cooled to room temperature, 2-bromopyridine (5.28 g, 33.44 mmol), 1,1'-bis(diphenylphosphino)ferrocene palladium(II) chloride (666 mg, 0.91 mmol), potassium carbonate (5.04 g, 36.48 mmol), DMF (80 mL), and $H_2O$ (12 mL) were added to the reaction mixture. The reaction mixture was stirred at 60°C overnight, and the reaction was completed as shown by TLC, water was added, the pH of the reaction mixture was adjusted to 11 with 10% sodium hydroxide aqueous solution, extracted with ethyl acetate, and the organic phases were combined, washed with water, concentrated under reduced pressure, and separated by column chromatography to obtain the product 1-tert-butoxycarbonyl-4-(pyridin-2-yl-methyl)piperidine (5.8 g, 69% yield). m/z=277$[M+1]^+$.

Step B: 2-(piperidin-4-ylmethyl)pyridine hydrochloride

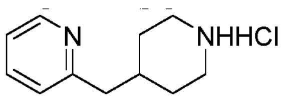

At room temperature, to a solution of 1-tert-butyloxycarbonyl-4-(pyridin-2-yl-methyl)piperidine (5.66 g, 20.507 mmol) in 1,4-dioxane (21 mL) was dropwised a solution of hydrogen chloride in 1,4-dioxane (21 mL, 84.000 mmol, 4M), stirred at room temperature for 1 hour, the reaction was completed as shown by TLC, concentrated under reduced pressure, slurried with ethyl acetate (100 mL) to obtain product 2-(piperidin-4-yl-methyl)pyridine hydrochloride (6.10 g, 140% yield), which was used directly in the next reaction. m/z=177$[M+I]^+$.

Step C: 5-bromo-2-(4-(pyridin-2-yl-methyl)piperidin-1-yl)pyridine

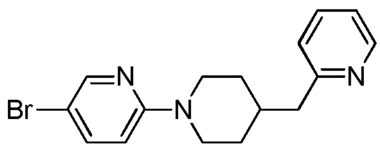

At room temperature, to a solution of 2-(piperidin-4-yl-methyl)pyridine hydrochloride (7.60 g, 35.681 mmol) in dimethyl sulfoxide (80 mL) was added 2,5-dibromopyridine (10.00 g, 42.195 mmol), anhydrous potassium phosphate (38.00 g, 179.245 mmol), stirred at 120°C for 12 hours, the reaction was completed as shown by TLC, cooled to room temperature, filtered out the excess potassium phosphate solid, the filter cake was washed with ethyl acetate, the filtrate was washed with water, the organic phase was collected, washed with saturated brine, concentrated under reduced

pressure, and separated by column chromatography to obtain the product 5-bromo-2-(4-(pyridin-2-yl-methyl)piperidine-1-yl)pyridine (7.00 g, 59% yield). m/z=332[M+1]$^+$.

Step D: 2-(4-(pyridin-2-yl-methyl)piperidin-1-yl)-5-(4,4,5,5-tetramethyl-1,3,2-dioxaboran-2-yl)pyridine

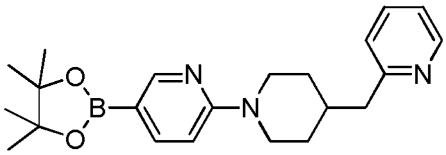

At room temperature, to a solution of 5-bromo-2-(4-(pyridin-2-yl-methyl)piperidin-1-yl)pyridine (500 mg, 1.505 mmol) and pinacol diborate (421 mg, 1.656 mmol) in 1,4-dioxane (4.5 mL) were added potassium acetate (443 mg, 4.515 mmol), purged with nitrogen gas for 2 minutes, and then [1,1'-bis(diphenylphosphino)ferrocene]palladium dichloride (110 mg, 0.151 mmol), purged with nitrogen gas for 2 minutes, stirred at 100°C for 1 hour, the reaction was completed as shown by TLC, water was added, extracted with ethyl acetate, the organic phases were combined, washed with saturated brine, concentrated under reduced pressure, and slurried to obtain the product 2-(4-(pyridin-2-yl-methyl)piperidin-1-yl)-5-(4,4,5,5-tetramethyl-1,3,2-dioxaborolan-2-yl)pyridine (480 mg, 84% yield). m/z=380[M+1]$^+$.

Step E: 2-amino-6-(1-methyl-1-hydro-pyrazol-4-yl)-4-(6-(4-pyridin-2-yl-methyl)piperidin-1-yl) piperidin-3-yl)pyrazolo[1,5-a]pyridine-3-carbonitrile

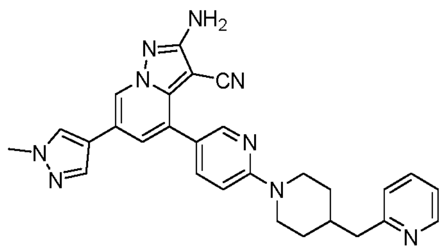

At room temperature, to a solution of 2-(4-(pyridin-2-yl-methyl)piperidin-1-yl)-5-(4,4,5,5-tetramethyl-1,3,2-dioxaborolan-2-yl)pyridine (33 mg, 0.087 mmol) and 2-amino-3-cyano-6-(1-methyl-1H-pyrazol-4-yl)pyrazolo[1,5-a]pyridin-4-yl-trifluoromethanesulfonate (28 mg, 0.073 mmol) in 1,4-dioxane (2.5 mL) were added sodium carbonate (38 mg, 0.358 mmol, 2M) aqueous solution, purged with nitrogen gas for 2 minutes, then tetratriphenylphosphine palladium (4 mg, 0.003 mmol) was added, purged with nitrogen gas for 2 minutes, stirring at 90 °C for 1 hour, the reaction was completed as shown by TLC, water was added, extracted with ethyl acetate ester, organic phases were combined, washed with saturated brine, concentrated under reduced pressure, to give 2-amino-6-(1-methyl-1hydro-pyrazol-4-yl)-4-(6-(4-pyridine-2-yl-methyl)piperidin-1-yl)piperidin-3-yl)pyrazolo[1,5-a]pyridine-3-carbonitrile (10 mg, 28% yield). $^1$HNMR (400MHz, DMSO-$d_6$) δ 8.77 (s, 1H), 8.50 (s, 1H), 8.30-8.27 (t, *J* = 2.4 Hz, 2H), 8.01 (s, 1H), 7.77-7.68 (m, 2H), 7.53 (s, 1H), 7.30-7.19 (m, 2H), 6.96-6.89 (m, 1H), 6.26 (brs, 2H), 4.42-4.31 (m, 2H), 3.86 (s, 3H), 2.90-2.80 (m, 2H), 2.70-2.68 (m, 2H), 2.12-1.99 (m, 1H), 1.70-1.61 (m, 2H), 1.31-1.18 (m, 2H).m/z=490[M+1]$^+$.

Example 3

2-methyl-4-(6-(4-(2-(5-fluoropyridin-2-yl)acetyl)piperazin-1-yl)pyridin-3-yl)-6-(1-methyl-1H-pyrazol-4-yl)pyrazolo[1,5-a]pyridine-3-carbonitrile (APS071)

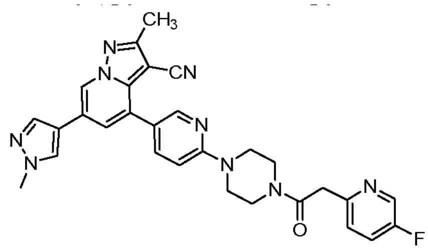

Step A: ethyl 2-methyl-6-bromo-4-methoxypyrazolo[1,5-a]pyridine-3-carboxylate

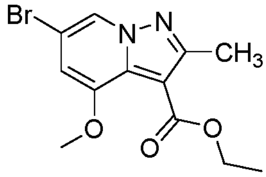

At room temperature, to a solution of 2,4,6-trimethylbenzenesulfonic acid 1-amino-3-bromo-5-methoxypyridine-1-ium (2.79 g, 6.91 mmol) in DMF (25 mL) was added triethylamine (1.93 mL, 13.82 mmol); the reaction system was cooled to 0°C, ethyl butynoate (1.62 mL, 13.82 mmol) was added in batches, and the mixture was heat to room temperature and stirred overnight; the reaction was completed and quenched by adding water , extracted with ethyl acetate, the organic phases were combined, washed with water, concentrated under reduced pressure, and separated by column chromatography to obtain the product ethyl 2-methyl-6-bromo-4-methoxypyrazolo[1,5-a]pyridine-3-carboxylate (510 mg, 24% yield).

$^1$HNMR (400MHz, $CDCl_3$) δ 8.13 (d, *J* = 1.2 Hz, 1H), 6.58 (d, *J* = 13.2 Hz, 1H), 4.31-4.17 (m, 2H), 3.85 (t, *J* = 20.8 Hz, 3H), 2.51 (s, 3H), 1.39-1.31 (m, 3H).m/z=313[M+l]$^+$.

Step B: 2-methyl-6-bromo-4-methoxypyrazolo[1,5-a]pyridine

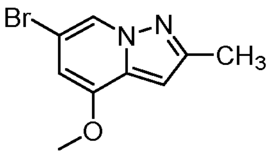

At room temperature, 48% hydrobromic acid (10 mL) was added to a sealed tube containing ethyl 2-methyl-6-bromo-4-methoxypyrazolo[1,5-a]pyridine-3-carboxylate (510 mg, 1.63 mmol), sealed, heat to 80°C, and stirred at 80°C for 2.0 hours, cooled to room temperature, the solvent was removed in vacuo, and separated by dry column chromatography to give the product 2-methyl-6-bromo-4-methoxypyrazolo[1,5-a]pyridine (392 mg, 100% yield), which was used directly for the next reaction. m/z=241[M+1]$^+$.

Step C: 2-methyl-6-bromo-4-methoxypyrazolo[1,5-a]pyridine-3-carbaldehyde

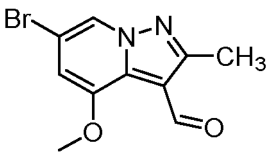

At 0°C , phosphorus oxychloride (0.45 mL, 4.89 mmol) was added to a solution of 2-methyl-6-bromo-4-methoxypyrazolo[1,5-a]pyridine (392 mg, 1.63 mmol) in DMF (10 mL), raised to room temperature and stirred for 4.0 hours,

the reaction was completed as shown by TLC, water was added to dilute the mixture, which was then adjusted to about pH 10 with aqueous sodium hydroxide (1 M) and stirred for 1.0 hour, vacuum filtered, the filter cake was washed with water, and then with methyl tert-butyl ether, dried under vacuum to obtain the product 2-methyl-6-bromo-4-methoxypyrazolo[1,5-a]pyridine-3-carbaldehyde (414 mg , 100% yield), which was used directly for the next reaction. m/z=269[M+1]$^+$.

Step D: (E)-2-methyl-6-bromo-4-methoxypyrazolo[1,5-a]pyridine-3-carbaldehyde oxime

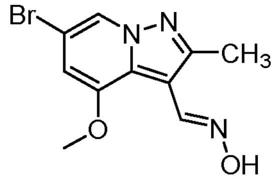

2-methyl-6-bromo-4-methoxypyrazolo[1,5-a]pyridine-3-carbaldehyde (414 mg, 1.63 mmol), hydroxylamine hydrochloride (170 mg, 2.45 mmol), water (The mixture of 6 mL) and ethanol (3 mL) were mixed and stirred at 50°C for 4.0 hours. After the the reaction was completed as shown by TLC, the reaction system was cooled to room temperature, the ethanol was removed under reduced pressure, and the reaction system was neutralized with saturated aqueous sodium bicarbonate solution. After extraction with ethyl acetate, the organic phases were combined, washed with water, concentrated under reduced pressure, and separated by column chromatography to obtain the product (E)-2-methyl-6-bromo-4-methoxypyrazolo[1,5-a]pyridine-3-formaldehyde oxime (463 mg, 100% yield), which was used directly for the next reaction. m/z=284[M+1]$^+$.

Step E: 2-methyl-6-bromo-4-methoxypyrazolo[1,5-a]pyridine-3-carbonitrile

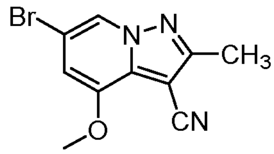

A mixture of 2-methyl-6-bromo-4-methoxypyrazolo[1,5-a]pyridine-3-carbonitrile (463 mg, 1.63 mmol) and acetic anhydride (10 mL) was heated to 120°C and stirred overnight. After the the reaction was completed as shown by TLC, the reaction system was cooled to room temperature, the acetic anhydride was removed under reduced pressure, diluted with water, the reaction system was neutralized with saturated aqueous sodium bicarbonate, extracted with ethyl acetate, the organic phases were combined, washed with water, concentrated and separated by column chromatography to obtain the product 2-methyl-6-bromo-4-methoxypyrazolo[1,5-a]pyridine-3-carbonitrile (300 mg, 69% yield). m/z=266[M+1]$^+$.

Step F: 2-methyl-4-methoxy-6-(1-methyl-1H-pyrazol-4-yl)pyrazolo[1,5-a]pyridine-3-carbonitrile

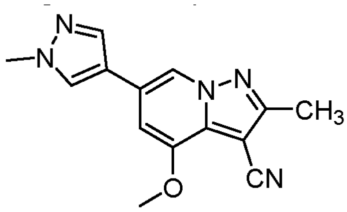

2-methyl-6-bromo-4-methoxypyrazolo[1,5-a]pyridine-3-carbonitrile (300 mg, 1.13 mmol), 1-methyl-4-(4,4,5,5-tetramethyl-1,3,2-dioxaborolan-2-yl)-1H-pyrazole (282 mg, 1.35 mmol), palladium tetrakistriphenylphosphine (26 mg, 0.02 mmol)), potassium carbonate (467 mg, 3.39 mmol), 1,4-dioxane (5 mL) and $H_2O$ (1 mL) were added into a 20 mL

sealed tube, purged with nitrogen three times, reacted at 80°C for 3 hours. After the the reaction was completed as shown by TLC, the reaction solution was concentrated, diluted with ethyl acetate, washed with water, the organic phase was dried with anhydrous sodium sulfate, concentrated under reduced pressure, and separated by column chromatography to obtain the product 2-amino-4-methoxy-6-(1-methyl-1H-pyrazol-4-yl)pyrazolo[1,5-a]pyridine-3-carbonitrile (300 mg, 99% yield). m/z=268[M+1]$^+$.

Step G: 2-methyl-4-hydroxy-6-(1-methyl-1H-pyrazol-4-yl)pyrazolo[1,5-a]pyridine-3-carbonitrile

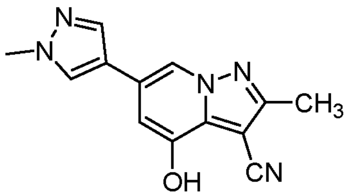

2-amino-4-methoxy-6-(1-methyl-1H-pyrazol-4-yl)pyrazolo[1,5-a]pyridine-3-carbonitrile (300 mg, 1.12 mmol), DCE (20 mL), aluminum chloride (446 mg, 3.36 mmol) were added in a 100 mL single-neck flask, protected by nitrogen, heated and stirred at 80°C so that the color of the mixture gradually turned dark brown, reacted for 3 hours. After the the reaction was completed as shown by TLC, water was added under stirring for quench, extracted with ethyl acetate, the organic phase was dried with anhydrous sodium sulfate, concentrated under reduced pressure, and separated by column chromatography to obtain the product 2-methyl-4-hydroxy-6-(1-methyl-1H-pyrazole-4)-yl)pyrazolo[1,5-a]pyridine-3-carbonitrile (150 mg, 53% yield). m/z=254[M+1]$^+$.

Step H: 2-methyl-3-cyano-6-(1-methyl-1H-pyrazol-4-yl)pyrazolo[1,5-a]pyridin-4-yltrifluoromethanesulfonic acid ester

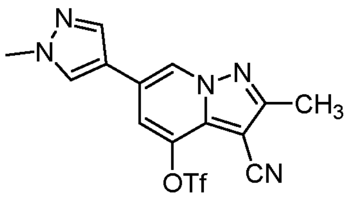

2-methyl-4-hydroxy-6-(1-methyl-1H-pyrazol-4-yl)pyrazolo[1,5-a]pyridine-3-carbonitrile (150 mg, 0.59 mmol), DMF (15 mL), DIEA(152 mg, 1.17 mmol), N-phenylbis(trifluoromethane)sulfonimide (317 mg, 0.89 mmol) were placed in a 100 mL single-necked flask, stirred at room temperature for 2 hours. After the the reaction was completed as shown by TLC, the mixuter was diluted with water, extracted with ethyl acetate, the organic layers were combined, , washed with water, and washed with saturated brine. The organic phase was dried over anhydrous sodium sulfate, concentrated under reduced pressure, and separated by column chromatography to obtain 2-methyl-3-cyano-6-(1-methyl-1H-pyrazol-4-yl)pyrazolo[1,5-a]pyridin-4-yl-triflate (180 mg, 79% yield). m/z=386[M+1]$^+$.

Step I: 2-(5-fluoropyridin-2-yl)-1-(4-(5-(4,4,5,5-tetramethyl-1,3,2-dioxboronic acid-2-yl)pyridin-2-yl)piperazin-1-yl)ethanone

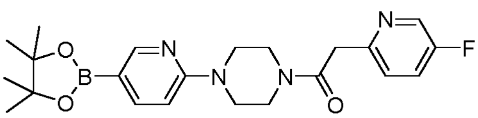

To a 10 mL sealed tube, 1-(4-(5-bromopyridin-2)piperazin-1-yl)-2-(5-fluoropyridin-2-yl)ethan-1-one (50 mg, 0.132 mmol), duplex pinacol borate (50 mg, 0.198 mmol), [1,1'-bis(diphenylphosphino)ferrocene]palladium dichloride

(10 mg, 0.013 mmol), potassium acetate (39 mg, 0.396 mmol), and 1,4-dioxane (20 mL) were added, purged with nitrogen three times, reacted at 100°C for 3 hours, LCMS confirmed that the raw materials were reacted completely to give the product, which was used directly to the next step without post-processing. m/z=427[M+1]$^+$.

Step J: 2-methyl-4-(6-(4-(2-(5-fluoropyridin-2-yl)acetyl)piperazin-1-yl)pyridin-3-yl)-6-(1-methyl-1H-pyrazol-4-yl)pyrazolo[1,5-a]pyridine-3-carbonitrile

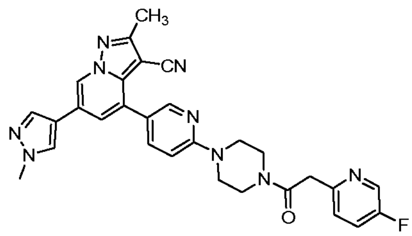

2-methyl-3-cyano-6-(1-methyl-1H-pyrazol-4-yl)pyrazolo[1,5-a]pyridin-4-yl-triflate (50 mg, 0.13 mol), palladium tetrakistriphenylphosphorus (15 mg, 0.01 mol), potassium carbonate (36 mg, 0.26 mol), 1,4-dioxane (4 mL), and $H_2O$ (1 mL) were added to the reaction solution of step I, purged with nitrogen three times, reacted at 90°C for 2 hours, LCMS confirmed that the reaction of the raw materials was completed, and products were formed. Water was added, extracted with ethyl acetate, the organic phases were combined, washed with water, dried with anhydrous sodium sulfate, filtered, concentrated under reduced pressure, and separated by column chromatography to obtain the product 2-methyl-4-(6-(4-(2-(5-fluoropyridin-2-yl)acetyl)piperazin-1-yl)pyridin-3-yl)-6-(1-methyl-1H-pyrazol-4-yl)pyrazolo[1,5-a]pyridine-3-carbonitrile (14 mg, 20% yield).
$^1$HNMR (400MHz, DMSO-$d_6$) δ 9.12 (s, 1H), 8.49 (d, *J* = 1.2 Hz, 1H), 8.39-8.36 (m, 2H), 8.09 (s, 1H), 7.84 (brs, 1H), 7.81-7.65 (m, 2H), 7.48-7.36 (m, 1H), 7.01 (d, *J*= 9.2 Hz, 1H), 3.98 (s, 3H), 3.88 (s, 3H), 3.80-3.56 (m, 8H), 3.89 (t, *J*= 2.8 Hz, 2H).m/z=536[M+1]$^+$.

Example 4

2-methyl-6-(1-methyl-1H-pyrazol-4-yl)-4-(6-(4-(pyridin-2-yl-methyl)piperidin-1-yl)pyridine-3-yl)pyrazolo[1,5-a]pyridine-3-carbonitrile (APS072)

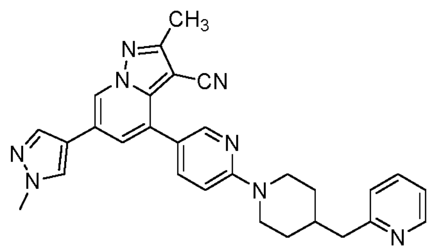

At room temperature, to a solution of 2-(4-(pyridin-2-yl-methyl)piperidin-1-yl)-5-(4,4,5,5-tetramethyl-1,3,2-dioxy heteropentaborin-2-yl)pyridine (45 mg, 0.12 mmol) and 2-methyl-3-cyano-6-(1-methyl-1-hydro-pyrazol-4-yl)pyrazolo[1,5-a]pyridin-4-yl triflate (38 mg, 0.10 mmol) in 1,4-dioxane (3 mL) were added sodium carbonate (53 mg, 0.50 mmol, 2M) aqueous solution, purged with nitrogen for 2 minutes, then tetrakistriphenylphosphine palladium (12 mg, 0.01 mmol) was added, purged with nitrogen for 2 minutes, stirred at 80 °C for 3 hours, the reaction was completed as shown by TLC, water was added, extracted with ethyl acetate, the organic phases were combined, washed with saturated brine, concentrated under reduced pressure, and separated by column chromatography to obtain the product 2-methyl-6-(1-methyl-1-hydro-pyrazol-4-yl)-4-(6-(4-pyridin-2-yl-methyl)piperidin-1-yl)piperidin-3-yl)pyrazolo[1,5-a]pyridine-3-carbonitrile (12 mg, 25% yield).
$^1$HNMR (400MHz, DMSO-$d_6$) δ 9.1 (s, 1H), 8.55-8.50 (m, 1H), 8.36-8.34 (m, 2H), 8.09 (s, 1H), 7.79-7.76 (m, 1H), 7.73-7.68 (m, 2H), 7.26-7.19 (m, 2H), 6.95-6.93 (d, *J* = 9.2 Hz, 1H), 4.40-4.37 (d, *J* = 12.8 Hz, 2H), 3.93 (s, 3H), 2.92-2.79 (m, 2H), 2.75-2.66 (m, 3H), 2.33 (s, 2H), 2.07 (brs, 1H), 1.67-1.64 (d, *J*= 12.8 Hz, 2H), 1.29-1.19 (m, 2H).m/z=489[M+1]$^+$.

Example 5

2-amino-4-(6-(4-((5-fluoropyridin-3-yl)methyl)piperazine-6-(1-methyl-1H-pyrazol-4-yl)pyrazolo[1,5-a]pyridine-3-carbonitrile (APS014)

Step A: 1-(5-bromopyridin-2-yl)-4-((5-fluoropyridin-3-yl)methyl)piperazine

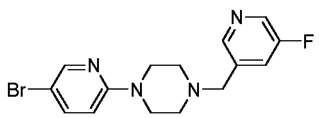

To a 100 mL single-neck flask, 5-fluoronicotinaldehyde (2.4 g, 19.2 mmol), 1-(5-bromopyridine)-2-piperazine (4.6g, 19.2 mmol), sodium acetate borohydride (12.2 g, 57.6 mmol), and 200 mL of dichloromethane were added, and stirred at room temperature for 12 hours. After the the reaction was completed as shown by TLC, water was added, extracted with ethyl acetate, the organic phases were combined, washed with water, dried with anhydrous sodium sulfate, filtered, concentrated under reduced pressure, and separated by column chromatography to obtain the product 1-(5-bromopyridine-2-yl)-4-((5-fluoropyridin-3-yl)methyl)piperazine (1.7 g, yield 25%). m/z=352[M+1]$^+$.

Step B: 1-((4-fluoropyridin-3-yl)methyl)-4-(5-(4,4,5,5-tetramethyl-1,3,2-dioxapentaborane-2-yl)pyridin-2-yl)piperazine

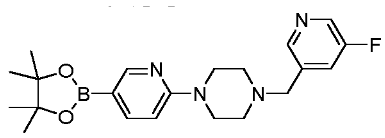

To a 10 mL sealed tube, 1-(5-bromopyridin-2-yl)-4-((5-fluoropyridin-3-yl)methyl)piperazine (47 mg, 0.132 mmol), duplex pinacol borate (50 mg, 0.198 mmol), [1,1'-bis(diphenylphosphino)ferrocene] palladium dichloride (10 mg, 0.013 mmol), potassium acetate (39 mg, 0.396 mmol), and 1,4-dioxane (20 mL) were added, purged with nitrogen three times, and reacted at 100°C for 3 hours. LCMS confirmed that the raw materials were reacted completely to give the product, which was used directly for the next step without post-treatment. m/z=399[M+1]$^+$.

Step C: 2-amino-4-(6-(4-((5-fluoropyridin-3-yl)methyl)piperazine-6-(1-methyl-1H-pyrazol-4-yl)pyridine azolo[1,5-a]pyridine-3-carbonitrile

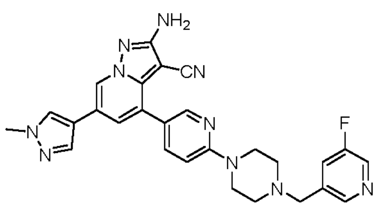

At room temperature, to a solution of 1-((4-fluoropyridin-3-yl)methyl)-4-(5-(4,4,5,5-tetramethyl-1,3,2-dioxa penboran-2-yl)pyridin-2-yl)piperazine (35 mg, 0.087 mmol) and 2-amino-3-cyano-6-(1-methyl-1H-pyrazol-4-yl) pyrazol[1,5-a]pyridin-4-yl-trifluoromethanesulfonate (28 mg, 0.073 mmol) in 1,4-dioxane (2.5 mL) was added sodium carbonate (38 mg, 0.358 mmol, 2M) aqueous solution, puorged with nitrogen gas for 2 minutes, then tetratriphenylphosphine palladium (4 mg, 0.003 mmol) was added, purged with nitrogen gas for 2 minutes, stirred at 90°C for 1 hour. After the reaction was completed as shown by TLC, water was added, extracted with ethyl acetate, the organic phases were combined, washed with saturated brine, concentrated under reduced pressure, to give 2-amino-4-(6-(4-((5-fluoropyridin-3-yl)methyl)piperazine-6-(1-methyl-1H-pyrazol-4-yl)pyrazolo[1,5-a]pyridine-3-carbonitrile (17 mg, 46% yield).

$^1$HNMR (400MHz, DMSO-$d_6$) δ 8.79 (d, 1H), 8.51 (d, 1H), 8.50 (d, 1H), 8.33 (d, 1H), 8.28 (s, 1H), 8.02 (s, 1H), 7.75-7.78 (m, 1H), 7.72 (d, 1H), 7.55 (d, 1H), 6.95 (d, 1H), 6.29 (s, 2H), 4.08-4.12 (m, 1H), 3.86 (s, 3H), 3.59-3.64 (m, 6H), 3.17 (d, 2H), 2.50 (s, 3H). m/z=509[M+1]$^+$.

Example 6

2-amino-4-(6-(4-((2-fluorobenzyl)piperazine-6-(1-methyl-1H-pyrazol-4-yl)pyrazolo[1,5-a]pyridine-3-carbonitrile (APS015)

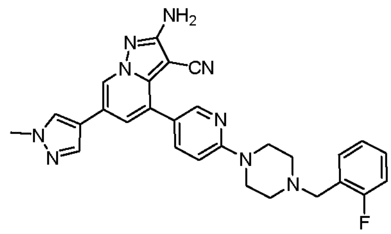

The preparation method according to Example 5 was perfomed, with the difference that 5-fluoronicotinaldehyde was replaced with 2-fluorobenzaldehyde, to obtain 2-amino-4-(6-(4-((2-fluorobenzyl)piperazine-6-(1-methyl-1H-pyrazol-4-yl)pyrazolo[1,5-a]pyridine-3-carbonitrile (12 mg, 31% yield). $^1$HNMR (400MHz, DMSO-$d_6$) δ 8.79 (d, 1H), 8.33 (d, 1H), 8.28 (s, 1H), 8.02 (s, 1H), 7.75 (d, 1H), 7.55 (s, 1H), 7.42-7.48 (m, 1H), 7.35 (s, 1H), 7.20-7.21 (m, 2H), 6.94 (d, 1H), 6.29 (s, 2H), 3.86 (s, 3H), 3.61 (d, 6H), 3.49 (s, 1H), 1.24 (s, 3H).m/z=508[M+1]$^+$.

Example 7

4-(5-(2-amino-3-cyano-6-(1-methyl-1H-pyrazol-4-yl)pyrazolo[1,5-a]pyridin-4-yl)pyridine-2-yl)-N,N-diethylpiperazine-1-carboxamide (APS016)

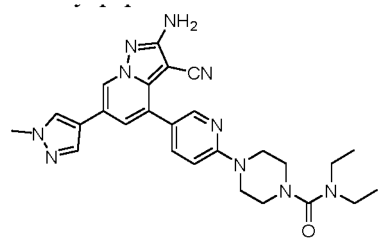

The preparation method according to Example 1 was perfomed, with the difference that 2-(5-fluoropyridin-2-yl)acetic acid was replaced with diethylcarbamoyl chloride, to obtain 4-(5-(2-amino-3-cyano-6-(1-methyl-1H-pyrazol-4-yl)pyrazolo[1,5-a]pyridin-4-yl)pyridin-2-yl)-N,N-diethylpiperazine-1-carboxamide (5.1 mg, yield 14%), $^1$HNMR (400MHz, $CDCl_3$) δ8.79 (s, 1H), 8.39 (s, 1H), 8.30 (s, 1H), 7.74-7.83 (s, 2H), 7.70 (s, 1H), 7.52 (s, 1H), 6.83 (d, 1H), 4.00 (d, 3H), 3.70 (s, 4H), 3.39 (d, 4H), 3.24-3.30 (m, 4H), 1.16 (t, 6H).m/z=489[M+1]$^+$.

Example 8

2-amino-4-(6-(4-((3-fluorobenzyl)piperazine-6-(1-methyl-1H-pyrazol-4-yl)pyrazolo[1,5-a] pyridine-3-carbonitrile (APS019)

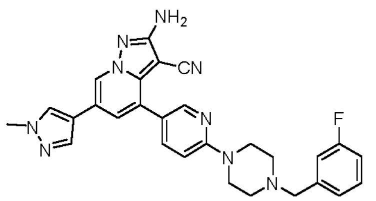

The preparation method according to Example 5 was perfomed, with the difference that 5-fluoronicotinaldehyde was replaced with 3-fluorobenzaldehyde to obtain 2-amino-4-(6-(4-((3-fluorobenzyl)piperazine-6-(1-methyl-1H-pyrazol-4-yl)pyrazolo[1,5-a]pyridine-3-carbonitrile (26.9 mg, yield 73%). $^1$HNMR (400MHz, DMSO-$d_6$) δ8.79 (s, 1H), 8.78 (d, 1H), 8.32 (s, 1H), 8.02 (s, 1H), 7.75 (d, 2H), 7.55 (d, 1H), 7.37-7.42 (m, 1H), 7.17-7.21 (m, 2H), 7.08-7.13 (m, 1H), 6.92-6.95 (d, 1H), 6.29 (s, 2H), 4.08-4.12 (m, 1H), 3.86 (s, 3H), 3.57-3.61 (m, 6H), 3.18 (d, 2H). m/z=508[M+1]$^+$.

Example 9

2-amino-4-(6-(4-((2,5-difluorobenzyl)piperazine-6-(1-methyl-1H-pyrazol-4-yl)pyrazolo[1,5-a] pyridine-3-carbonitrile (APS020)

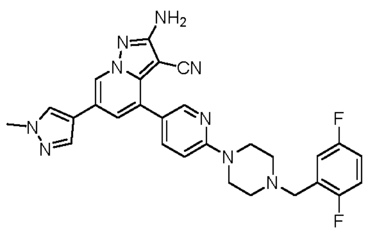

The preparation method according to Example 5 was perfomed, with the difference that 5-fluoronicotinic aldehyde was replaced with 2,5-difluorobenzaldehyde to obtain 2-amino-4-(6-(4-((2,5-difluorobenzyl)piperazine-6-(1-methyl-1H-pyrazol-4-yl)pyrazolo[1,5-a]pyridine-3-carbonitrile (9.9 mg, yield 26%). $^1$HNMR (400MHz, DMSO-$d_6$) δ8.79 (d, 1H), 8.33 (d, 1H), 8.28 (s, 1H), 8.02 (s, 1H), 7.75-7.78 (dd, 1H), 7.55 (d, 1H), 6.95-7.33 (m, 3H), 6.92 (d, 1H), 6.28 (s, 2H), 3.86 (s, 3H), 3.60 (s, 6H).m/z=526[M+1]$^+$.

Example 10

2-amino-4-(6-(4-((5-chloropyridin-3-yl)methyl)piperazine-6-(1-methyl-1H-pyrazol-4-yl)pyrazolo[1,5-a]pyridine-3-carbonitrile (APS021)

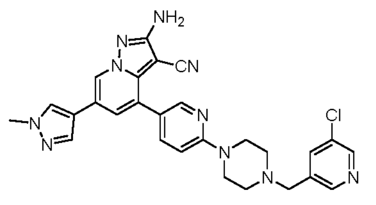

The preparation method according to Example 5 was perfomed, with the difference that 5-fluoronicotinaldehyde was replaced with 5-chloro-nicotinaldehyde to obtain 2-amino-4-(6-(4-((5-chloropyridin-3-yl)methyl)piper azin-6-(1-methyl-1H-pyrazol-4-yl)pyrazolo[1,5-a]pyridine-3-carbonitrile (21 mg, yield 55%). $^1$HNMR (400MHz, $CDCl_3$) δ8.39-8.44 (m, 2H), 8.25-8.29 (m, 2H), 7.65-7.71 (m, 2H), 7.54-7.59 (m, 1H), 7.19-7.21 (m, 4H), 6.63-6.71 (m, 1H), 4.45 (s, 1H), 3.90 (s, 2H), 3.53-3.72 (d, 7H), 2.54 (s, 4H), m/z=525[M+1]$^+$.

Example 11

2-amino-6-(1-methyl-1H-pyrazol-4-yl)-4-(6-((3-methylpyridin-2-yl)methyl)piperazin-1-yl)pyridine-3-yl)pyrazolo[1,5-a]pyridine-3-carbonitrile (APS022)

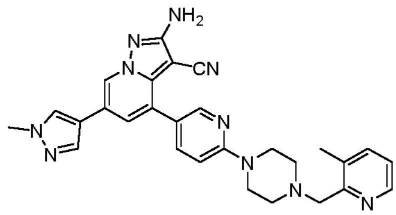

The preparation method according to Example 5 was perfomed, with the difference that 5-fluoronicotinaldehyde was replaced with 3-methyl-2-pyridinecarbaldehyde to obtain 2-amino-6-(1-methyl-1H-pyrazol-4-yl)-4-(6-((3-methylpyridin-2-yl)methyl)piperazin-1-yl)pyridin-3-yl)pyrazolo[1,5-a]pyridine-3-carbonitrile (5mg, yield 14%). $^1$HNMR (400MHz, DMSO-$d_6$) δ 8.8.79 (s, 1H), 8.28-8.78 (m, 3H), 8.02 (s, 1H), 7.74-7.77 (m, 1H), 7.54-7.60 (m, 2H), 7.21-7.24 (m, 1H), 6.93 (d, 1H), 6.28 (d, 2H), 4.08-4.11 (m, 1H), 3.86 (s, 3H), 3.65 (s, 2H), 3.58 (s, 4H), 3.3 (s, 1H), 3.17 (d, 2H), 2.43 (s, 3H).m/z=505[M+1]$^+$.

Example 12

2-amino-4-(6-(4-((2,6-difluorobenzyl)piperazine-6-(1-methyl-1H-pyrazol-4-yl)pyrazolo[1,5-a] pyridine-3-carbonitrile (APS024)

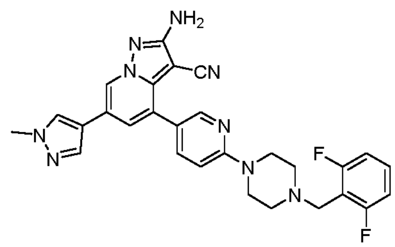

The preparation method according to Example 5 was perfomed, with the difference that 5-fluoronicotinaldehyde was replaced with 2,6-difluorobenzaldehyde to obtain 2-amino-4-(6-(4-((2,6-difluorobenzyl)piperazine-6-(1-methyl-1H-pyrazol-4-yl)pyrazolo[1,5-a]pyridine-3-carbonitrile (3.5 mg, yield 9%). $^1$HNMR (400MHz, DMSO-$d_6$) δ 8.78 (s, 1H), 8.31 (d, 2H), 8.27-8.31(m, 2H), 8.01 (s, 1H), 7.73 (d, 1H), 7.54 (s, 1H), 7.42-7.48 (m, 1H), 7.13 (t, 2H), 6.28 (s, 2H), 3.86 (s, 3H), 3.56-3.65 (s, 7H), 3.27 (s, 1H).m/z=526[M+1]$^+$.

Example 13

2-amino-6-(1-methyl-1H-pyrazol-4-yl)-4-(6-((4-methylpyridin-2-yl)methyl)piperazin-1-yl)pyridine-3-yl)pyrazolo[1,5-a]pyridine-3-carbonitrile (APS025)

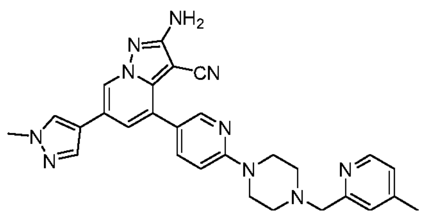

The preparation method according to Example 5 was perfomed, with the difference that 5-fluoronicotinaldehyde was replaced with 4-methyl-2-pyridinecarbaldehyde to obtain 2-amino-6-(1-methyl-1H-pyrazol-4-yl)-4-(6-((4-methylpyridin-2-yl)methyl)piperazin-1-yl)pyridin-3-yl)pyrazolo[1,5-a]pyridine-3-carbonitrile (3.9 mg, yield 11%). $^1$HNMR (400MHz, DMSO-$d_6$) δ 8.79 (d, 1H), 8.29-8.33 (m, 3H), 8.02 (s, 1H), 8.75 (d, 1H), 7.55 (d, 1H), 7.33 (s, 1H), 7.23 (d, 1H), 6.95 (d, 2H), 6.30 (s, 2H), 3.86 (s, 3H), 3.62 (d, 6H), 3.32 (s, 2H)2.55 (s, 4H), 2.34 (s, 3H). 1.23 (s, 1H).m/z=505[M+1]$^+$.

Example 14

2-amino-4-(6-(4-(3-methoxybenzyl)piperazin-1-yl)pyridin-3-yl)-6-(1-methyl-1H-pyrazole-4-yl)pyrazolo[1,5-a]pyridine-3-carbonitrile (APS026)

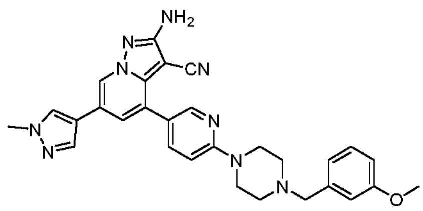

The preparation method according to Example 5 was perfomed, with the difference that 5-fluoronicotinaldehyde was replaced with 3-methoxybenzaldehyde to obtain 2-amino-4-(6-(4-(3-methoxybenzyl)piperazine-1-yl)pyridin-3-yl)-6-(1-methyl-1H-pyrazol-4-yl)pyrazolo[1,5-a]pyridine-3-carbonitrile (14.2 mg, yield 37%) . $^1$HNMR (400MHz, DMSO-$d_6$) δ 8.79 (d, 1H), 8.51 (d, 1H), 8.45 (s, 1H), 8.33 (d, 1H), 8.28 (s, 1H), 8.02 (s, 1H), 7.75-7.78 (d, 1H), 7.72 (d, 1H), 7.55 (d, 1H), 6.95 (d, 1H), 6.29 (s, 2H), 4.11 (t, 1H), 3.86 (s, 3H), 3.59-3.64 (m, 6H), 3.17 (d, 2H), 2.53 (s, 3H). m/z=520[M+1]$^+$.

Example 15

2-amino-4-(6-(4-(2-chlorobenzyl)piperazin-1-yl)pyridin-3-yl)-6-(1-methyl-1H-pyrazol-4-yl) pyrazolo[1,5-a]pyridine-3-carbonitrile (APS027)

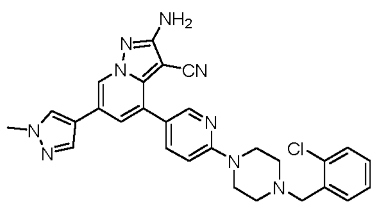

The preparation method according to Example 5 was perfomed, with the difference that 5-fluoronicotinaldehyde was replaced with 2-chlorobenzaldehyde to obtain 2-amino-4-(6-(4-(2-chlorobenzyl)piperazin-1-yl)pyridine-3-yl)-6-(1-methyl-1H-pyrazol-4-yl)pyrazolo[1,5-a]pyridine-3-carbonitrile (9.4 mg, yield 25%). $^1$HNMR (400MHz, DMSO-$d_6$) δ8.79 (s, 1H), 8.80 (d, 1H), 8.28 (s, 1H), 8.02 (s, 1H), 7.78 (d, 1H), 7.56-7.58 (m, 3H), 7.45-7.47 (m, 2m/z=524[M+1]$^+$.

Example 16

2-amino-4-(6-(4-((6-methoxypyridin-2-yl)methyl)piperazin-1-yl)pyridin-3-yl)-6-(1-methyl-1H-pyrazol-4-yl)pyrazolo[1,5-a]pyridine-3-carbonitrile (APS030)

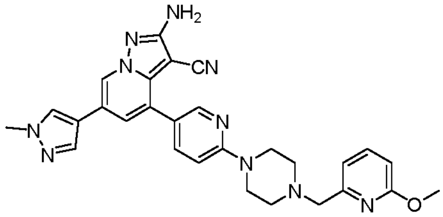

The preparation method according to Example 5 was perfomed, with the difference that 5-fluoronicotinaldehyde was replaced with 6-methoxy-2-pyridinecarbaldehyde to obtain 2-amino-4-(6-(4-((6-methoxypyridine-2-yl)methyl)piperazin-1-yl)pyridin-3-yl)-6-(1-methyl-1H-pyrazol-4-yl)pyrazolo[1,5-a]pyridine-3-nitrile (8.6 mg, yield 23%). $^1$HNMR (400MHz, $CDCl_3$) δ8.27 (d, 2H), 7.65-7.68 (m, 2H), 7.45-7.54 (m, 2H), 7.21 (s, 1H), 7.01 (s, 1H), 6.71 (d, 1H), 6.59 (d, 1H), 4.45 (s, 2H), 3.86-3.90 (d, 6H), 3.66 (s, 6H), 2.69(brs, 4H), 1.54 (brs, 4H), m/z=521$[M+1]^+$.

Example 17

2-amino-4-(6-(4-((5-methoxypyridin-2-yl)methyl)piperazin-1-yl)pyridin-3-yl)-6-(1-methyl-1H-pyrazol-4-yl)pyrazolo[1,5-a]pyridine-3-carbonitrile (APS033)

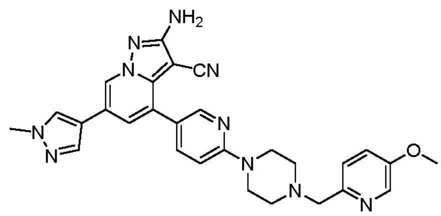

The preparation method according to Example 5 was perfomed, with the difference that 5-fluoronicotinaldehyde was replaced with 5-methoxynicotinaldehyde to obtain 2-amino-4-(6-(4-((5-methoxypyridin-2-yl)methyl)piperazin-1-yl)pyridin-3-yl)-6-(1-methyl-1H-pyrazol-4-yl)pyrazolo[1,5-a]pyridine-3-carbonitrile (21.9 mg, yield 58%). $^1$HNMR (400MHz, DMSO-$d_6$) δ 8.77 (s, 1H), 8.32 (d, 1H), 8.27 (d, 1H), 8.22 (d, 1H), 8.01 (s, 1H), 7.77 (dd, 1H), 7.54 (s, 1H), 7.37-7.43 (m, 2H), 6.94 (d, 1H), 6.26 (s, 2H), 4.08 (s, 1H), 3.83-3.86 (m, 6H), 3.60 (s, 6H), 3.28 (s, 2H), 3.18 (d, 2H).m/z=521$[M+1]^+$.

Example 18

2-amino-4-(6-(4-(4-(dimethylamino)benzyl)piperazin-1-yl)pyridin-3-yl)-6-(1-methyl-1H-pyrazole-4-yl)pyrazolo[1,5-a]pyridine-3-carbonitrile (APS034)

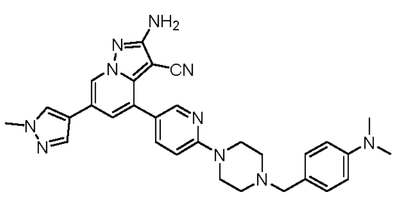

The preparation method according to Example 5 was perfomed, with the difference that 5-fluoronicotinaldehyde was replaced with 4-dimethylaminobenzaldehyde to obtain 2-amino-4-(6-(4-(4-(dimethylamino)benzyl)piperazine-1-yl)pyridin-3-yl)-6-(1-methyl-1H-pyrazol-4-yl)pyrazolo[1,5-a]pyridine-3-carbonitrile (7.0 mg, yield 18 %), $^1$HNMR (400MHz, DMSO-$d_6$) δ8.78 (d, 1H), 8.28-8.32 (m, 2H), 8.02 (s, 1H), 7.74-7.76 (d, 1H), 7.54 (d, 1H), 7.15 (d, 2H), 6.93 (d, 1H), 6.71 (d, 1H), 6.28 (s, 2H), 4.08-4.11 (m, 1H), 3.86 (s, 3H), 3.56 (s, 4H), 3.41 (s, 2H), 3.17 (s, 2H), 2.88 (s, 6H), 2.44-2.50 (m, 4H).m/z=533$[M+1]^+$.

Example 19

2-amino-6-(1-methyl-1H-pyrazol-4-yl)-4-(6-(4-(4-(pyridin-2-yl)methyl)piperazin-1-yl) pyridin-3-yl)pyrazolo[1,5-a]pyridine-3-carbonitrile (APS035)

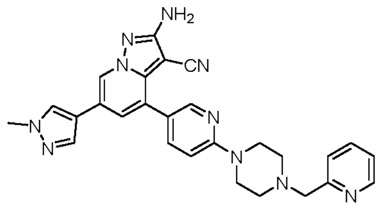

The preparation method according to Example 5 was perfomed, with the difference that 5-fluoronicotinaldehyde was replaced with 2-pyridinecarboxaldehyde to obtain 2-amino-6-(1-methyl-1H-pyrazol-4-yl)-4-(6-(4-(4-(pyridin-2-yl)methyl)piperazin-1-yl)pyridin-3-yl)pyrazolo[1,5-a]pyridine-3-carbonitrile (12.7 mg, yield 35%), $^1$HNMR (400MHz, DMSO-$d_6$) δ8.79 (d, 1H), 8.52 (d, 1H), 8.33 (d, 1H), 8.28 (s, 1H), 8.02 (s, 1H), 7.72-8.80 (m, 2H), 7.48-7.51 (m, 2H), 7.24-7.31 (m, 1H), 6.91-6.96 (d, 1H), 6.29 (s, 2H), 3.86 (s, 3H), 3.59-3.67 (m, 8H), 2.56 (s, 2H).m/z=491$[M+1]^+$.

Example 20

2-amino-6-(1-methyl-1H-pyrazol-4-yl)-4-(6-(6-(pyridin-2-yl-methyl)-3,6-diazabicyclo[3.1.1]heptan-3-yl)pyridin-3-yl)pyrazolo[1,5-a]pyridine-3-carbonitrile (APS038)

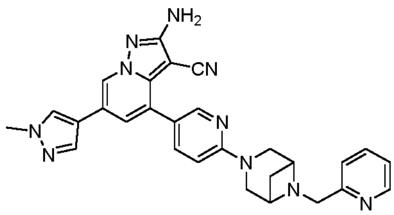

Step A: tert-butyl 3-(5-bromopyridin-2-yl)-3,6-diazabicyclo[3.1.1]heptane-6-carbonate

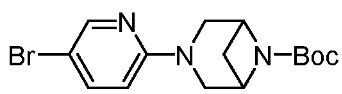

At room temperature, tert-butyl 2,5-dibromopyridine (12.2 g, 0.052 mol), 3,6-diazabicyclo[3.1.1]heptane-6-carbonate (11.5 g, 0.058 mol) were added to DMSO (20 mL) in a 250 mL single mouth bottle, and reacted overnight at 120°C. It was confirmed by LCMS that the reaction was completed, DMSO was removed under reduced pressure, methanol was added to dissolve the mixture, saturated sodium hydroxide solution was slowly added under an ice-water bath, the pH was adjusted to about 13, and the mixture was further stirred for 2 hours. The methanol was concentrated under reduced pressure, the residue was extracted with dichloromethane, dried over anhydrous sodium sulfate, concentrated under reduced pressure, and separated by column chromatography to obtain the product (5.2 g, 28% yield). m/z=355$[M+1]^+$.

Step B: 3-(5-bromopyridin-2-yl)-3,6-diazabicyclo[3.1.1]heptane dihydrochloride

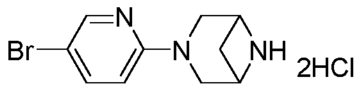

To a 100 mL single-neck flask, tert-butyl 3-(5-bromopyridin-2-yl)-3,6-diazabicyclo[3.1.1]heptane-6-carbonate (5.0g, 14 mmol) and a solution of hydrogen chloride in dioxane (15 mL) were added, stirred at room temperature for 2h, directly concentrated and used in the next reaction. m/z=255$[M+1]^+$.

Step C: 3-(5-bromopyridin-2-yl)-6-(pyridin-2-yl-methyl)-3,6-diazabicyclo[3.1.1]heptane

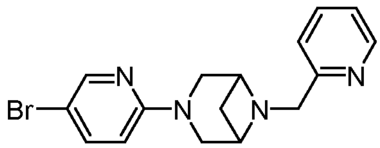

To a 100 mL single-neck flask, 2-pyridinecarboxaldehyde (0.21g, 1.9 mmol), 3-(5-bromopyridin-2-yl)-3,6-diazabicyclo[3.1.1]heptane dihydrochloride (0.6 g, 1.9 mmol), sodium acetate borohydride (1.2 g, 5.8 mmol), and 20 mL of dichloromethane were added, and stirred at room temperature for 12 hours. After the the reaction was completed as shown by TLC, water was added, extracted with ethyl acetate, the organic phases were combined, washed with water, dried with anhydrous sodium sulfate, filtered, concentrated under reduced pressure, and separated by column chromatography to obtain the product 3-(5-bromopyridine-2-yl)-6-(pyridin-2-yl-methyl)-3,6-diazabicyclo[3.1.1]heptane (0.6 g, yield 85%). m/z=346$[M+1]^+$.

Step D: 6-(pyridin-2-yl-methyl)-3-(5-(4,4,5,5-tetramethyl-1,3,2-dioxaborolan-2-yl)(pyridin-2-yl)-3,6-diazabicyclo[3.1.1]heptane

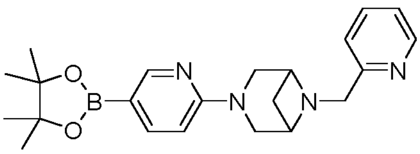

3-(5-bromopyridin-2-yl)-6-(pyridin-2-yl-methyl)-3,6-diazabicyclo[3.1.1]heptane (46 mg, 0.132 mmol), duplex pinacol borate (50 mg, 0.198 mmol), [1,1'-bis(diphenylphosphino)ferrocene]palladium dichloride (10 mg, 0.013 mmol)), potassium acetate (39 mg, 0.396 mmol), and 1,4-dioxane (20 mL) were added to a 10 mL sealed tube, purged with nitrogen three times, reacted at 100°C for 3 hours, LCMS confirmed that the raw materials were reacted completely to give the product, which was used directly without post-processing.

Step E: At room temperature, to a solution of 6-(pyridin-2-yl-methyl)-3-(5-(4,4,5,5-tetramethyl-1,3,2-dioxaborole Alk-2-yl)pyridin-2-yl)-3,6-diazabicyclo[3.1.1]heptane (34 mg, 0.087 mmol) and 2-amino-3-cyano-6-(1-methyl-1H-pyrazol-4-yl)pyrazolo[1,5-a]pyridin-4-yl-trifluoromethanesulfonate (28 mg, 0.073 mmol) in 1,4-dioxane (2.5 mL) were added sodium carbonate (38 mg, 0.358 mmol, 2M) aqueous solution, purged with nitrogen for 2 minutes, then tetrakistriphenylphosphine palladium (4 mg, 0.003 mmol) was added, purged with nitrogen for 2 minutes, stirred at 90 °C for 1 hour, the TLC showed the reaction was completed, water was added, extracted with ethyl acetate, the organic phases were combined, washed with saturated brine, and concentrated under reduced pressure. 2-amino-6-(1-methyl-1H-pyrazole)-4-yl)-4-(6-(6-(pyridin-2-yl-methyl)-3,6-diazabicyclo[3.1.1]heptan-3-yl)pyridin-3-yl) pyrazolo[1,5-a]pyridine-3-carbonitrile (10 mg, 28% yield).

$^1$HNMR (400MHz, DMSO-$d_6$) δ8.79 (d, 1H), 8.46 (d, 1H), 8.39 (d, 1H), 8.29 (s, 1H), 8.03 (s, 1H), 7.76-7.83 (m, 2H), 7.57 (d, 1H), 7.49 (d, 1H), 6.79 (d, 1H), 6.29 (s, 2H), 3.86 (s, 2H), 3.73 (d, 2H), 3.57 (s, 2H), 3.52-3.55 (m, 2H), 3.31 (s, 1H), 1.98-2.00 (m, 1H), 1.74 (brs,1H).m/z=503$[M+1]^+$.

Example 21

2-amino-4-(6-(4-((6-(dimethylamino)pyridin-3-yl)methyl)piperazin-1-yl)pyridin-3-yl)-6-(1-methyl-1H-pyrazol-4-yl)pyrazolo[1,5-a]pyridine-3-carbonitrile (APS041)

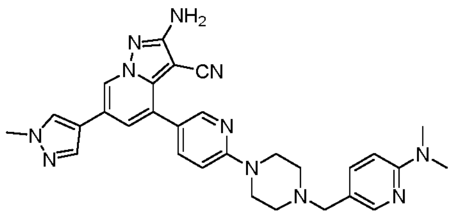

The preparation method according to Example 5 was perfomed, with the difference that 5-fluoronicotinaldehyde was replaced with 6-dimethylaminonicotinaldehyde to obtain 2-amino-4-(6-(4-((6-(dimethylamino)pyridin-3-yl))Methyl)piperazin-1-yl)pyridin-3-yl)-6-(1-methyl-1H-pyrazol-4-yl)pyrazolo[1,5-a]pyridine-3-carbonitrile (22.2 mg, yield 56%), $^1$HNMR (400MHz, DMSO-$d_6$) δ8.79 (d, 1H), 8.28-8.33 (m, 2H), 8.02 (d, 2H), 7.74-7.77 (m, 1H), 7.55 (d, 1H), 7.46-7.49 (m, 1H), 6.94 (d, 1H), 6.64 (d, 1H), 6.28 (s, 2H), 3.86 (s, 3H), 3.57 (s, 4H), 3.40 (s, 2H), 3.30 (s, 1H), 3.02 (s, 6H), 2.50 (s, 4H).m/z=534$[M+1]^+$.

Example 22

2-amino-4-(6-(4-(2,6-difluorobenzoyl)piperazin-1-yl)pyridin-3-yl)-6-(1-methyl-1H-pyrazole-4-yl)pyrazolo[1,5-a]pyridine-3-carbonitrile (APS062)

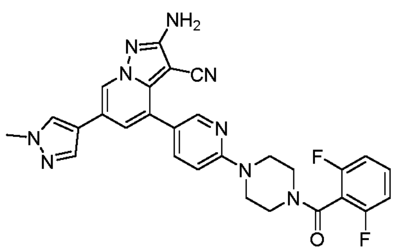

The preparation method according to Example 1 was perfomed, with the difference that 2-(5-fluoropyridin-2-yl)acetic acid was replaced with 2,6-difluorobenzoyl chloride to obtain 2-amino-4-(6-(4-(2,6-Difluorobenzoyl)piperazin-1-yl)pyridin-3-yl)-6-(1-methyl-1H-pyrazol-4-yl)pyrazolo[1,5-a]pyridine-3-carbonitrile (28.7 mg, yield 73%). $^1$HNMR (400MHz, DMSO-$d_6$) δ9.25 (d, 1H), 8.66 (s, 1H), 8.40-8.43 (m, 2H), 8.13 (s, 1H), 7.90 (d, 1H), 7.80 (d, 1H), 7.5-7.63 (m, 1H), 7.25-7.29 (m, 2H), 7.04 (d, 1H), 4.08-4.12 (m, 1H), 3.89 (s, 3H), 3.82-3.85 (m, 2H), 3.72-3.75 (d, 2H), 3.61-3.63 (m, 2H), 3.42 (d, 2H), 3.18 (d, 3H).m/z=539$[M+1]^+$.

Example 23

2-amino-6-(1-methyl-1H-pyrazol-4-yl)-4-(6-(4-(pyrazin-2-yl-methyl)piperazin-1-yl)pyridine-3-yl)pyrazolo[1,5-a]pyridine-3-carbonitrile (APS064)

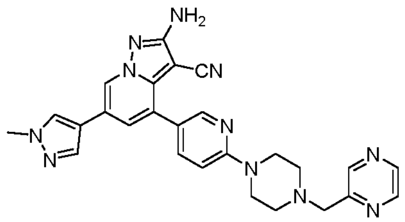

The preparation method according to Example 5 was perfomed, with the difference that 5-fluoronicotinaldehyde was replaced with pyrazine-2-carbaldehyde to obtain 2-amino-6-(1-methyl-1H-pyrazol-4-yl)-4-(6-(4-(pyrazin-2-yl-methyl)piperazin-1-yl)pyridin-3-yl)pyrazolo[1,5-a]pyridine-3-carbonitrile (9.5 mg, yield 26%) . $^1$HNMR (400MHz, $CDCl_3$) δ8.82 (s, 1H), 8.60 (s, 1H), 8.56 (s, 1H), 8.34-8.37 (m, 2H), 7.75-7.78 (m, 2H), 7.63 (s, 1H), 7.30 (s, 1H), 6.81 (d, 1H), 4.54 (s, 2H), 3.99 (s, 3H), 3.78-3.88 (brs, 5H), 2.76 (brs, 3H).m/z=492[M+1]$^+$.

Example 24

2-amino-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS068)

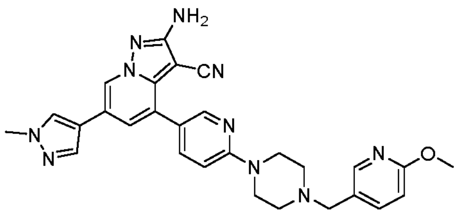

The preparation method according to Example 5 was perfomed, with the difference that 5-fluoronicotinaldehyde was replaced with 6-methoxy-3-pyridinecarbaldehyde to obtain 2-amino-4-(6-(4-((6-methoxypyridine-3-yl)methyl)piperazin-1-yl)pyridin-3-yl)-6-(1-methyl-1H-pyrazol-4-yl)pyrazolo[1,5-a]pyridine-3-carbonitrile (20mg, yield 53%). $^1$HNMR (400MHz, DMSO-$d_6$) δ8.79 (s, 1H), 8.34 (d, 2H), 8.20 (d, 2H), 7.68-7.77 (m, 2H), 7.55 (s, 1H), 6.81-6.94 (dd, 2H), 6.29 (s, 2H), 3.86 (d, 6H), 3.49-3.58 (d, 6H).m/z=521[M+1]$^+$.

Example 25

(R)-2-amino-6-(1-methyl-1H-pyrazol-4-yl)-4-(6-(3-methyl-4-(pyridin-2-yl-methyl)piperazine-1-yl)pyridin-3-yl)pyrazolo[1,5-a]pyridine-3-carbonitrile (APS073)

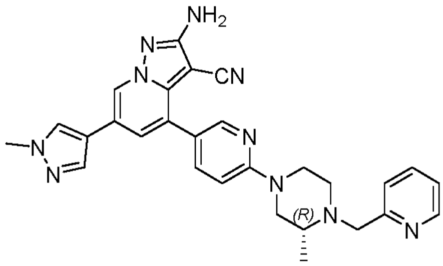

The preparation method according to Example 20 was perfomed, with the difference that 3,6-diazabicyclo[3.1.1]heptane-6-tert-butyl carbonate was replaced with (R)-4-N-tert-butoxycarbonyl-2-methylpiper azine to obtain (R)-2-amino-6-(1-methyl-1H-pyrazol-4-yl)-4-(6-(3-methyl-4-(pyridin-2-yl-methyl)piperazin-1-yl)pyridin-3-yl)pyrazolo [1,5-a]pyridine-3-carbonitrile (23 mg, yield 62%). $^1$HNMR (400MHz, DMSO-$d_6$) δ 8.79 (d, 1H), 8.52 (d, 1H), 8.32 (d, 1H), 8.28 (s, 1H), 8.02 (s, 1H), 7.74-7.81 (m, 2H), 7.50-7.55 (m, 2H), 7.26-7.29 (m, 1m/z=505[M+l]$^+$.

Example 26

2-amino-4-(6-(4-benzylpiperazin-1-yl)pyridin-3-yl)-6-(1-methyl-1H-pyrazol-4-yl)pyrazolo[1,5-a] ridine-3-carbonitrile (APS074)

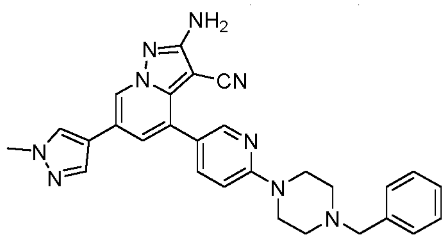

The preparation method according to Example 5 was perfomed, with the difference that 5-fluoronicotinaldehyde was replaced with benzaldehyde to obtain 2-amino-4-(6-(4-benzylpiperazin-1-yl)pyridin-3-yl)-6-(1-methyl-1H-pyrazol-4-yl)pyrazolo[1,5-a]pyridine-3-carbonitrile (27mg, yield 75%). $^1$HNMR (400MHz, DMSO-$d_6$) δ 8.79 (s, 1H), 8.33 (d, 1H), 8.28 (s, 1H), 8.02 (s, 1H), 7.75-7.77 (m, 1H), 7.55 (d, 1H), 7.37 (d, 4H), 7.29 (s, 1H), 6.95 (d, 1H), 6.28 (s, 2H). 3.86 (s, 3H), 3.55-3.59 (m, 7H), 1.24 (s, 3H).m/z=490[M+1]$^+$.

Example 27

2-amino-6-(1-methyl-1H-pyrazol-4-yl)-4-(6-(4-(pyridin-3-yl-methyl)piperazin-1-yl)pyridine-3-yl)pyrazolo[1,5-a]pyridine-3-carbonitrile (APS075)

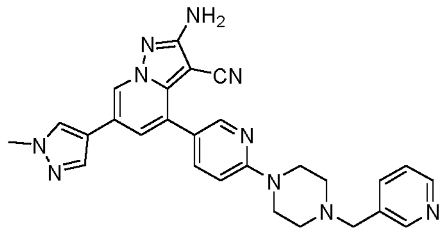

The preparation method according to Example 5 was perfomed, with the difference that 5-fluoronicotinaldehyde was replaced with nicotinaldehyde to obtain 2-amino-6-(1-methyl-1H-pyrazol-4-yl)-4-(6-(4-(pyridin-3-yl-methyl)piperazin-1-yl)pyridin-3-yl)pyrazolo[1,5-a]pyridine-3-carbonitrile (7 mg, 19%). $^1$HNMR (400MHz, $CDCl_3$) δ 8.62 (d, 2H), 8.36 (dd, 2H), 7.74-7.77 (m, 2H), 7.63 (s, 1H), 7.36 (s, 1H), 7.28 (s, 1H), 6.81 (d, 1H), 4.53 (s, 2H), 3.99 (s, 3H), 3.65 (s, 6H), 2.70 (s, 4H).m/z=491[M+1]$^+$.

Example 28

(R)-2-amino-6-(1-methyl-1H-pyrazol-4-yl)-4-(6-(2-methyl-4-(pyridin-2-yl-methyl)piperazine-1-yl)pyridin-3-yl)pyrazolo[1,5-a]pyridine-3-carbonitrile(APS076)

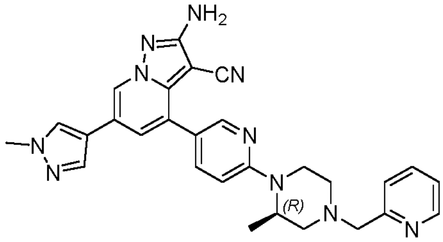

The preparation method according to Example 20 was perfomed, with the difference that 3,6-diazabicyclo[3.1.1]heptane-6-tert-butyl carbonate was replaced with (R)-4-N-tert-butoxycarbonyl-3-methylpiper to obtain (R)-2-amino-6-(1-methyl-1H-pyrazol-4-yl)-4-(6-(2-methyl-4-(pyridin-2-yl-methyl))piperazin-1-yl)pyridin-3-yl)pyrazolo [1,5-a]pyridine-3-carbonitrile (12 mg, 33%). $^1$HNMR (400MHz, DMSO-$d_6$) δ 8.94 (s, 1H), 8.78 (s, 1H), 8.52 (d, 2H), 8.05 (s, 1H), 7.74-7.84 (m, 2H), 7.56 9d, 2H), 7.31 (t, 1H), 6.89 (d, 1H), 6.27 (s, 1H), 3.70 (s, 3H), 3.62-3.86 (m, 1H), 3.30 (s, 5H), 3.12 (t, 1H), 2.95 (d, 1H), 2.74 (d, 1H), 1.23 (t, 3H). m/z=505[M+1]$^+$.

Example 29

2-amino-4-(6-(4-(4-fluorobenzyl)piperazin-1-yl)pyridin-3-yl)-6-(1-methyl-1H-pyrazol-4-yl) pyrazolo[1,5-a]pyridine-3-carbonitrile (APS077)

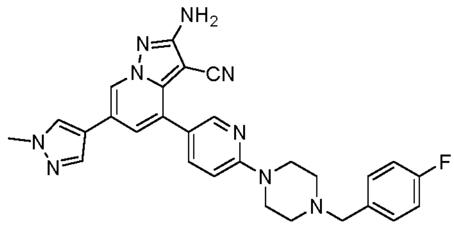

The preparation method according to Example 5 was perfomed, with the difference that 5-fluoronicotinaldehyde was replaced with 4-fluorobenzaldehyde to obtain 2-amino-4-(6-(4-(4-fluorobenzyl)piperazin-1-yl)pyridine-3-yl)-6-(1-methyl-1H-pyrazol-4-yl)pyrazolo[1,5-a]pyridine-3-carbonitrile (10 mg, yield 27%). $^1$HNMR (400MHz, DMSO-$d_6$) δ 8.79 (d, 1H), 8.33 (d, 1H), 8.28 (s, 1H), 8.02 (s, 1H), 7.75 (d, 1H), 7.55 (d, 1H), 7.38-7.40 (m, 1H), 7.17-7.20 (m, 2H), 6.95 (d, 1H), 6.28 (s, 2H), 3.86 (s, 3H), 3.53-3.59 (m, 6H).m/z=508[M+1]$^+$.

Example 30

2-amino-6-(1-methyl-1H-pyrazol-4-yl)-4-(6-(4-(pyridin-4-yl-methyl)piperazin-1-yl)pyridine-3-yl)pyrazolo[1,5-a]pyridine-3-carbonitrile (APS078)

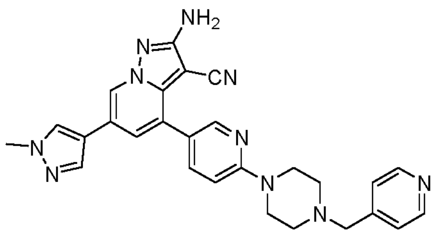

The preparation method according to Example 5 was perfomed, with the difference that 5-fluoronicotinaldehyde was replaced with isonicotinaldehyde to obtain 2-amino-6-(1-methyl-1H-pyrazol-4-yl)-4-(6-(4-(pyridin-4-yl-methyl)piperazin-1-yl)pyridin-3-yl)pyrazolo[1,5-a]pyridine-3-carbonitrile (8.4 mg, yield 23%). $^1$HNMR (400MHz, DMSO-$d_6$) δ 8.79 (s, 1H), 8.53 (d, 2H), 8.29-8.32 (m, 2H), 8.02 (s, 1H), 7.78 (d, 1H), 7.52 (s, 1H), 7.40 (d, 2H), 6.98 (d, 1H), 6.29 (s, 2H),

3.86 (s, 3H), 3.62)d, 7H).m/z=491[M+1]+.

Example 31

2-amino-4-(6-(4-(3,4-difluorobenzyl)piperazin-1-yl)pyridin-3-yl)-6-(1-methyl-1H-pyrazole-4-yl)pyrazolo[1,5-a]pyridine-3-carbonitrile (APS079)

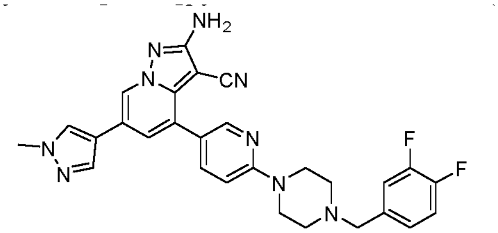

The preparation method according to Example 5 was perfomed, with the difference that 5-fluoronicotinaldehyde was replaced with 3,4-difluorobenzaldehyde to obtain 2-amino-4-(6-(4-(3,4-difluorobenzyl)piperazine-1-yl)pyridin-3-yl)-6-(1-methyl-1H-pyrazol-4-yl)pyrazolo[1,5-a]pyridine-3-carbonitrile (12.7 mg, yield 33 %). $^1$HNMR (400MHz, $CDCl_3$) δ 8.27 (d, 1H), 7.65 (s, 1H), 7.41 (s, 1H), 7.20 (s, 4H), 7.02-7.08 (m, 2H), 6.70 (d, 1H), 4.45 (s, 1H), 4.07 (s, 2H), 3.59 (s, 3H), 3.45 (s, 1H), 2.50 (s, 2H), 2.01 (s, 1H).m/z=526[M+1]+.

Example 32

2-amino-4-(6-(4-(2,6-difluorobenzyl)piperazin-1-yl)pyridin-3-yl)-6-(1-methyl-1H-pyrazole-4-yl)pyrazolo[1,5-a]pyridine-3-carbonitrile (APS024)

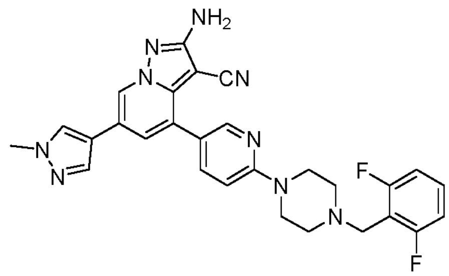

The preparation method according to Example 5 was perfomed, with the difference that 5-fluoronicotinaldehyde was replaced with 2,6-difluorobenzaldehyde to obtain 2-amino-4-(6-(4-(2,6-difluorobenzyl)piperazine-1-yl)pyridin-3-yl)-6-(1-methyl-1H-pyrazol-4-yl)pyrazolo[1,5-a]pyridine-3-carbonitrile (27 mg, yield 70 %). $^1$HNMR (400MHz, DMSO-$d_6$) δ10.9 (brs, 1H), 9.27 (d, 1H), 8.67 (s, 1H), 8.46 (d, 1H), 8.40 (s, 1H), 8.13 (s, 1H), 7.94-7.97 (m, 1H), 7.80 (d, 1H), 7.75-7.68 (m, 1H), 7.28-7.32 (m, 2H), 7.14 (d, 1H), 7.48-7.56 (m, 4H), 3.89 (s, 4H), 3.25-3.45 (m, 5H).m/z=526[M+1]+.

Example 33

4-(6-(4-((6-methoxypyridin-3-yl)methyl)piperazin-1-yl)pyridin-3-yl)-6-(1-methyl-1H-pyrazole-4-yl)pyrazolo[1,2,3]triazole[1,5-a]pyridine-3-carbonitrile (APS081)

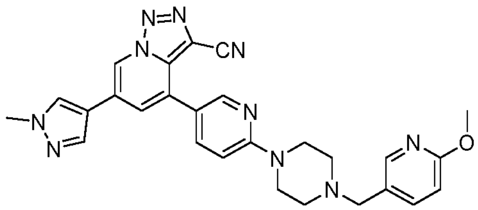

Step A: 6-bromo-4-chloro-[1,2,3]triazolo[1,5-a]pyridine-3-carbonitrile

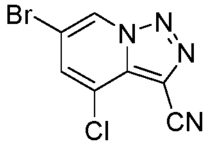

2-(5-bromo-3-chloropyridin-2-yl)acetonitrile (1.2g, 5mmol), p-toluenesulfonyl azide (1.2g, 6mmol) and cesium carbonate (3.3g, 10mmol) were added to DMF respectively (50 mL), heated to 100°C and reacted for 12h. After the the reaction was completed as shown by TLC, water was added, extracted with ethyl acetate, the organic phases were combined, washed with water, dried over anhydrous sodium sulfate, filtered, concentrated under reduced pressure, and separated by column chromatography to obtain the product 6-bromo-4-chloro-[1,2,3]triazole[1,5-a]pyridine-3-carbonitrile (450mg, yield 35%). m/z=258$[M+1]^+$.

Step B: 4-chloro-6-(1-methyl-1H-pyrazol-4-yl)-[1,2,3]triazolo[1,5-a]pyridine-3-carbonitrile

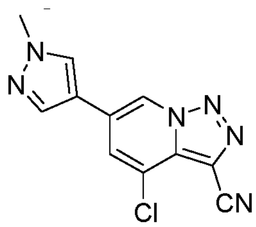

Step F according to Example 1 was perfomed, with the difference that 2-amino-6-bromo-4-methoxypyrazolo[1,5-a]pyridine-3-carbonitrile was replaced with 6-bromo-4-chloro-[1,2,3]triazolo[1,5-a]pyridine-3-carbonitrile to obtain 4-chloro-6-(1-methyl-1H-pyrazol-4-yl)-[1,2,3]triazolo[1,5-a]pyridine-3-carbonitrile (20mg, yield 40%). m/z=259$[M+1]^+$.

Step C: The preparation method according to Example 5 was perfomed, with the difference that 5-fluoronicotinaldehyde was replaced with 6-methoxynicotinaldehyde to obtain 4-(6-(4-((6-methoxypyridin-3-yl)methyl))piperazin-1-yl)pyridin-3-yl)-6-(1-methyl-1H-pyrazol-4-yl)pyrazolo[1,2,3]triazolo[1,5-a]pyridine-3-carbonitrile (10 mg, yield 27%). $^1$HNMR (400MHz, DMSO-$d_6$) δ 9.64 (d, 1H), 8.46-8.51 (m, 2H), 8.23 (s, 1H), 8.01 (d, 1H), 7.91 (d, 1H), 7.69 (d, 2H), 7.02 (d, 1H), 6.83 (d, 1H), 3.91 (s, 3H), 3.85 (s, 3H), 3.62 (s, 4H), 3.49 (s, 2H), 3.32 (s, 1H), 2.50 (s, 3H).m/z=507$[M+1]^+$.

Example 34

2-(dimethylamino)-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS082)

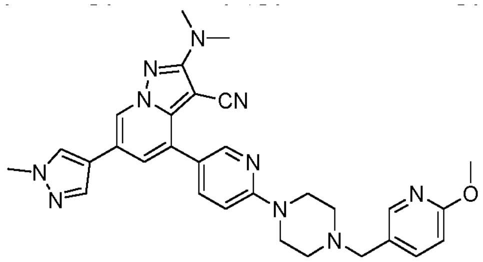

2-amino-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS068) (50mg, 0.1 mmol) was dissolved in DMF (5.0 mL) and cooled to 0 °C in ice water. 60% mineral oil NaH (8mg, 0.2 mmol) was added and stirred for 30 minutes, methyl iodide (28 mg, 0.2 mmol) was added, and naturally warmed to room temperature to react for 12 hours. After the the reaction was completed as shown by TLC, water was added, extracted with ethyl acetate, the organic phases were combined, washed with water, dried with anhydrous sodium sulfate, filtered, concentrated under reduced pressure, and separated by column chromatography to obtain the product 2-(dimethylamino)-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (9.1 mg, yield 16%). $^1$HNMR (400MHz, $CDCl_3$) δ8.31-8.37 (m, 2H), 8.09 (s, 1H), 7.68-7.72 (m, 2H), 7.61-7.63 (m, 2H), 7.22 (s, 1H), 6.76 (d, 2H), 3.97 (d, 6H), 3.64 (s, 3H), 3.49 (s, 2H), 3.19 (s, 6H), 2.56 (s, 3H). m/z=549[M+1]$^+$.

Example 35

2-fluoro-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS084)

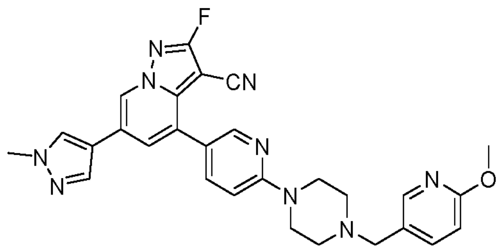

The preparation method according to Example 40 was perfomed, with the difference that 2-amino-4-(5-(6-((6-methoxypyridin-3-yl)methyl)-3,6-diazabicyclo[3.1.1]heptan-3-yl)pyridine(azin-2-yl)-6-(((S)-morpholin-2-yl)methoxy)pyrazolo[1,5-a]pyridine-3-carbonitrile trifluoroacetate was replaced with 2-amino-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile to obtain 7-fluoro-4-(6-(4-((6-methoxypyridin-3-yl)methyl)piperazin-1-yl)pyridin-3 -yl)-6-(1-methyl-1H-pyrazol-4-yl)pyrazolo [1,5-a]pyridine-3-carbonitrile (20 mg, yield 47%). $^1$HNMR (400MHz, DMSO-$d_6$) δ 8.79 (s, 1H), 8.37 (s, 2H), 8.10 (d, 2H), 7.94 (d, 1H), 7.70 (d, 1H), 7.55 (d, 1H), 6.98 (d, 1H), 6.81 (d, 1H), 3.93 (s, 3H), 3.85 (s, 3H), 3.60 (s, 4H), 3.49 (s, 2H), 2.51 (s, 4H). m/z=524[M+1]$^+$.

Example 36

2-hydroxy-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS083)

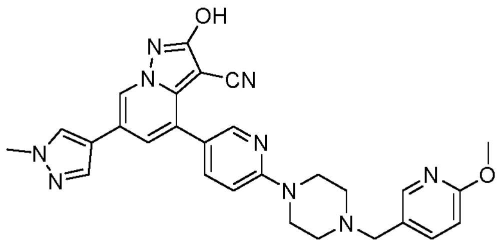

2-fluoro-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS084) (50 mg, 0.1 mmol) and potassium carbonate (41 mg, 0.3 mmol) were added to DMSO (2.0 mL), water (0.5 mL) was added and heated to 100°C to react for 6h. After the the reaction was completed as shown by TLC, water was added, extracted with ethyl acetate, the organic phases were combined, washed with water, dried with anhydrous sodium sulfate, filtered, concentrated under reduced pressure, and separated by column chromatography to obtain the product 2-hydroxy-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (7.3 mg, yield 14%). m/z=522[M+1]+.

Example 37

N-(3-cyano-4-(6-(4-((6-methoxypyridin-3-yl)methyl)piperazin-1-yl)pyridin-3-yl)-6-(1-methyl-1H-pyrazol-4-yl)pyrazolo[1,5-a]pyridin-2-yl)acetamide (APS085)

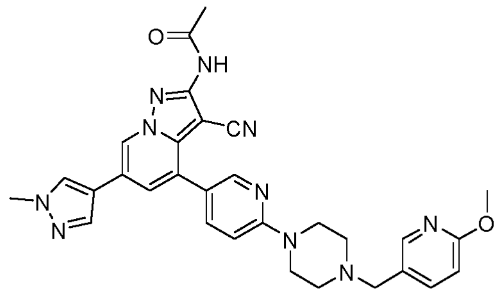

2-amino-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS068) (20 mg, 0.038 mmol) was dissolved in acetic anhydride (2.0 mL) and heated to 80°C to react for 4h. Water was added to the reaction solution, extracted with ethyl acetate, the organic phases were combined, washed with water, dried over anhydrous sodium sulfate, filtered, concentrated under reduced pressure, and separated by column chromatography to obtain the product N-(3-cyano-4-(6-(4-((6-methoxypyridin-3-yl)methyl)piperazin-1-yl)pyridin-3-yl)-6-(1-methyl-1H-pyrazol-4-yl) pyrazolo[1,5-a]pyridin-2-yl)acetamide (14 mg, yield 65%). m/z=563[M+1]+.

Example 38

2-methylamino-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (APS086)

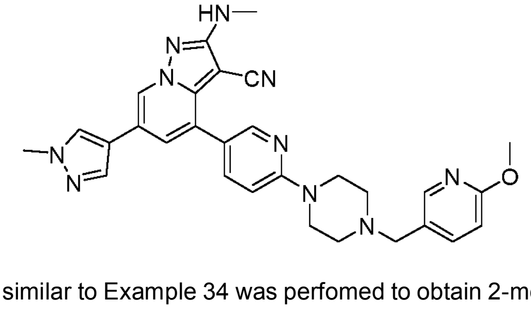

The preparation method similar to Example 34 was perfomed to obtain 2-methylamino-4-(6-(4-((6-methoxypyridin-3-yl)methyl)piperazin-1-yl)pyridin-3-yl)-6-(1-methyl-1H-pyrazol-4-yl)pyrazolo[1,5-a]pyridine-3-carbonitrile (8 mg, yield 15%). m/z=535[M+1]$^+$.

Example 39

2-amino-4-(5-(6-((6-methoxypyridin-3-yl)methyl)-3,6-diazabicyclo[3.1.1]heptan-3-yl)pyrazin-2-yl)-6-(((S)-morpholin-2-yl)methoxy)pyrazolo[1,5-a]pyridine-3-carbonitrile trifluoroacetate (APS087)

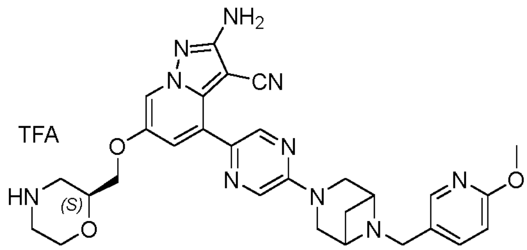

Step A: tert-butyl 3-(5-chloropyrazin-2-yl)-3,6-diazabicyclo[3.1.1]heptane-6-carbonate

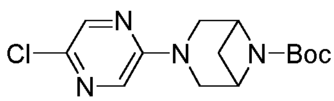

At room temperature, 2-chloro-5-fluoropyrazine (6.9 g, 0.052 mol), and tert-butyl 3,6-diazabicyclo[3.1.1]heptane-6-carbonate (11.5 g, 0.058 mol) were added to DMSO (20 mL) in a 250 mL single mouth bottle, and reacted overnight at 120°C. It was confirmed by LCMS that the reaction was completed, the DMSO was removed under reduced pressure, methanol was added to dissolve it, saturated sodium hydroxide solution was slowly added under an ice-water bath, the pH was adjusted to about 13, and the mixture was stirred for 2 hours. The methanol was concentrated under reduced pressure, extracted with dichloromethane, dried over anhydrous sodium sulfate, concentrated under reduced pressure, and separated by column chromatography to obtain the product (12 g, 74% yield). m/z=311[M+1]$^+$.

Step B: 3-(5-chloropyrazine-2-yl)-3,6-diazabicyclo[3.1.1]heptane dihydrochloride

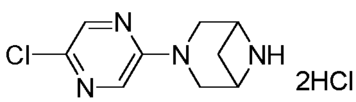

To a 100 mL single-neck flask, tert-butyl 3-(5-chloropyrazin-2-yl)-3,6-diazabicyclo[3.1.1]heptane-6-carbonate (5.0 g, 14 mmol), and a solution of hydrogen chloride in dioxane (15 mL) were added, stirred at room temperature for 2h, directly concentrated and the product was used for the next reaction. m/z=211[M+1]$^+$.

Step C: 3-(5-chloropyrazine-2-yl)-6-((6-methoxypyridin-3-yl)methyl)-3,6-diazabicyclo[3.1.1]heptan alkyl

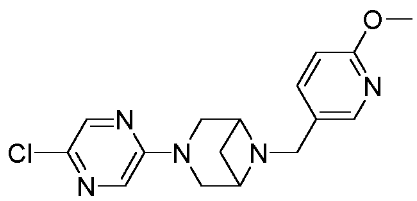

To a 100 mL single-neck flask, 6-methoxynicotinaldehyde (0.21 g, 1.9 mmol), 3-(5-chloropyrazin-2-yl)-3,6-diazabicyclo[3.1.1]heptane dihydrochloride (0.4 g, 1.9 mmol), sodium acetate borohydride (1.2 g, 5.8 mmol), and dichloromethane 20 mL were added, and stirred at room temperature for 12 hours. After the reaction was completed as shown by TLC, water was added, extracted with ethyl acetate, the organic phases were combined, washed with water, dried over anhydrous sodium sulfate, filtered, concentrated under reduced pressure, and separated by column chromatography to obtain the product 3-(5-chloropyrazine-2-yl)-6-((6-methoxypyridin-3-yl)methyl)-3,6-diazabicyclo[3.1.1]heptane (0.5g, yield 80%). m/z=332[M+1]$^+$.

Step D: 6-((6-methoxypyridin-3-yl)methyl)-3-(5-(4,4,5,5-tetramethyl-1,3,2-dioxolane boran-2-yl)pyrazin-2-yl)-3,6-diazabicyclo[3 .1.1]heptane

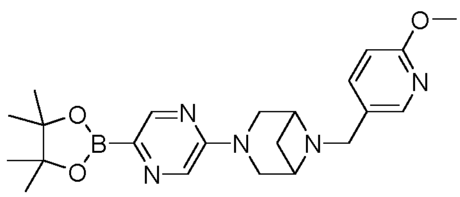

To a 10 mL sealed tube, 3-(5-chloropyrazin-2-yl)-6-((6-methoxypyridin-3-yl)methyl)-3,6-diazabicyclo[3.1.1]heptane (44 mg, 0.132 mmol), duplex pinacol borate (50 mg, 0.198 mmol), [1,1'-bis(diphenylphosphino)ferrocene] dichloride palladium (10 mg, 0.013 mmol), potassium acetate (39 mg, 0.396 mmol), and 1,4-dioxane (20 mL) were added, purged with nitrogen three times, reacted at 100°C for 3 hours, LCMS confirmed that the raw materials have been reacted. The product is formed, which was used directly for the next step reaction without post-treatment.

Step E: 2-amino-4-chloro-6-methoxypyrazolo[1,5-a]pyridine-3-carbonitrile

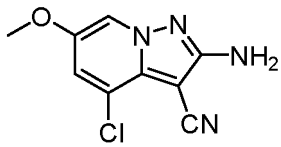

At room temperature, to a solution of 2,4,6-trimethylbenzenesulfonic acid 1-amino-3-chloro-5-methoxypyridine-1-ium (4.0 g, 10.0 mmol) in DMF (30 mL) was added potassium carbonate (4.2 g, 30.0 mmol), the reaction system was cooled to 0°C, ethyl 2-cyanoiminoacetate hydrochloride (3.0 g, 20.0 mmol) was added in batches, and the temperature was raised to room temperature, stirred for 1 hour and then at 90°C for 1 hour; after the reaction was completed, the mixture was cooled to room temperature, quenched with water, extracted with ethyl acetate, the organic phases were combined, washed with water, concentrated under reduced pressure, and separated by column chromatography to obtain the product 2-amino-4-chloro-6-methoxypyrazolo[1,5-a]pyridine-3-carbonitrile (89 mg, 4% yield). m/z=223[M+1]$^+$.

Step F: 4-chloro-2-(1,3-phthalimide-2-yl)-6-methoxypyrazolo[1,5-a]pyridine-3-carbonitrile

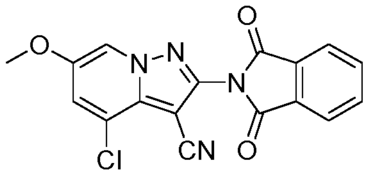

2-amino-4-chloro-6-methoxypyrazolo[1,5-a]pyridine-3-carbonitrile (80 mg, 0.35 mmol) and phthalic anhydride (78 mg, 0.53 mmol) were added to glacial acetic acid (5.0 mL), heated to 100°C to react for 5 hours, concentrated under reduced pressure, and separated by column chromatography to obtain the product 4-chloro-2-(1,3-phthalimide-2-yl)-6-methoxypyrazolo[1,5-a]pyridine-3-carbonitrile (80 mg, yield 65%). m/z=353[M+1]$^+$.

Step G: 4-chloro-2-(1,3-phthalimid-2-yl)-6-hydroxypyrazolo[1,5-a]pyridine-3-carbonitrile

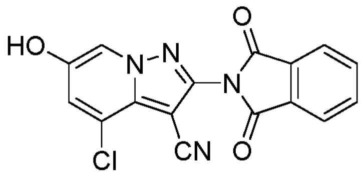

4-chloro-2-(1,3-phthalimide-2-yl)-6-methoxypyrazolo[1,5-a]pyridine-3-carbonitrile (80 mg, 0.22 mmol) and anhydrous aluminum trichloride (146 mg, 1.1 mmol) were added to acetonitrile (5.0 mL) and heated to 80°C to react for 2h. After the reaction was completed and cooled to room temperature, quenched with water, extracted with ethyl acetate, the organic phases were combined, washed with water, concentrated under reduced pressure, and separated by column chromatography to obtain the product 4-chloro-2-(1,3-1,3-phthalimid-2-yl)-6-hydroxypyrazolo[1,5-a]pyridine-3-carbonitrile (62 mg, yield 84%). m/z=339[M+1]$^+$.

Step H: (S)-2-(((4-chloro-3-cyano-2-(1,3-phthalimide-2-yl)pyrazolo[1,5-a](pyridin-6-yl)oxy)methyl)morpholine-4-tert-butyl carbonate

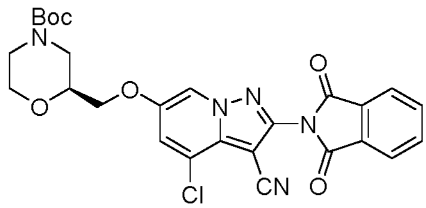

4-chloro-2-(1,3-phthalimid-2-yl)-6-hydroxypyrazolo[1,5-a]pyridine-3-carbonitrile (60 mg, 0.17 mmol), (2S)-2-(bromomethyl)-4-morpholinecarboxylic acid tert-butyl ester (52 mg, 0.19 mmol), and cesium carbonate (166 mg, 0.51 mmol) were added to DMF (10mL) and heated at 60 °C to react for 12h. After the reaction was completed and cooled to room temperature, quenched with water, extracted with ethyl acetate, the organic phases were combined, washed with water, concentrated under reduced pressure, and separated by column chromatography to obtain the product tert-butyl (S)-2-(((4-chloro-3-cyano-2-(1,3-phthalimid-2-yl)pyrazolo[1,5-a]pyridin-6-yl)oxy)methyl)morpholine-4-carbonate (77 mg, 85% yield).m/z=538[M+1]$^+$.

Step I: At room temperature, to a solution of 6-((6-methoxypyridin-3-yl)methyl)-3-(5-(4,4,5,5-tetramethyl-1,3,2-dioxaborolan-2-yl)pyrazin-2-yl)-3,6-diazabicyclo[3.1.1]heptane (37mg, 0.087 mmol) and (S)-2-(((4-Chloro-3-cyano-2-(1,3-phthalimide-2-yl)pyrazolo[1,5-a]pyridine-6-yl)oxy)methyl)morpholine-4-tert-butyl carbonate (39 mg, 0.073 mmol) in 1,4-dioxane (2.5 mL), sodium carbonate (38 mg, 0.358 mmol, 2M) aqueous solution was added, purged with nitrogen gas for 2 minutes, tetrakistriphenylphosphine palladium (4 mg, 0.003 mmol) was added, purged with nitrogen gas for 2 minutes, and stirred at 90° C for 1 hour. The reaction was completed as shown by TLC. A solution of hydrogen chloride in dioxane (0.5 mL, 4.0M) was added to continue the reaction for 2h, raised to room temperature, and then 50% ammonia waterwas added to further react at room temperature for 2 hours. After the the reaction was completed as shown by

TLC, water was added, extracted with ethyl acetate, the organic phases were combined, washed with saturated brine, concentrated under reduced pressure, and separated by reversed-phase column chromatography to obtain the product 2-amino-4-(5-(6-((6-methoxypyridin-3-yl)methyl)-3,6-diazabicyclo[3.1.1]heptan-3-yl)pyrazin-2-yl)-6-(((S)-morpholin-2-yl)methoxy)pyrazolo[1,5-a]pyridine-3-carbonitrile trifluoroacetate (12 mg, 25% yield). $^{1}$HNMR (400MHz, DMSO-$d_6$) δ9.34 (s, 1H), 8.99 (s, 2H), 8.55-8.65 (m, 1H), 8.25-8.38 (m, 3H), 7.83-7.90 (m, 1H), 7.38-7.44 (m, 1H), 6.91-6.95 (m, 1H), 6.18 (s, 1H), 4.65 (d, 2H), 4.49 (d, 1H), 4.26 (s, 1H), 4.11-4.20 (m, 5H), 4.00-4.11 (m, 3H), 3.93-4.00 (m, 1H), 3.89 (s, 3H), 3.67-3.74 (m, 1H), 3.5-3.26 (m, 1H), 2.97-3.04 (m, 1H).m/z=569[M+1]$^+$.

Example 40

2-fluoro-4-(5-(6-((6-methoxypyridin-3-yl)methyl)-3,6-diazabicyclo[3.1.1]heptan-3-yl)pyrazin-2-yl)-6-(((S)-morpholin-2-yl)methoxy)pyrazolo[1,5-a]pyridine-3-carbonitrile trifluoroacetate (APS088)

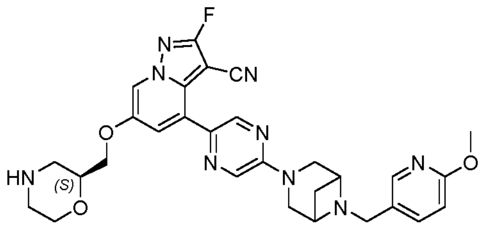

2-amino-4-(5-(6-((6-methoxypyridin-3-yl)methyl)-3,6-diazabicyclo[3.1.1]heptan-3-yl) pyrazin-2-yl)-6-(((S)-morpholin-2-yl)methoxy)pyrazolo[1,5-a]pyridine-3-carbonitrile trifluoroacetate (APS087) (50 mg, 0.08 mmol) was dissolved in tetrafluoroboric acid (5.0 mL), cooled to 0°C in an ice-water bath, added sodium nitrite (71 mg, 1.0 mmol), raised naturally to room temperature and reacted for 12 hours, water was added, and extracted with ethyl acetate, the organic phases were combined, washed with saturated brine, concentrated under reduced pressure, and separated by column chromatography to obtain the product 2-fluoro-4-(5-(6-((6-methoxypyridin-3-yl)methyl))-3,6-diazabicyclo[3.1.1]heptan-3-yl)pyrazin-2-yl)-6-(((S)-morpholin-2-yl)methoxy)pyridine azolo[1,5-a]pyridine-3-carbonitrile (3.4 mg, yield 7.4%). m/z=572[M+1]$^+$.

Example 41

2-amino-6-(2-hydroxy-2-methylpropoxy)-4-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS089)

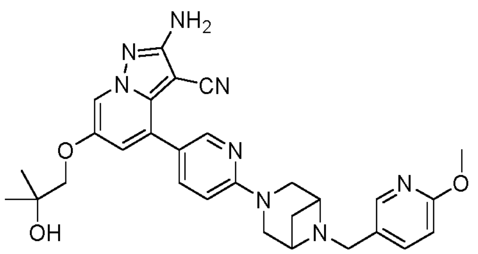

Step A: 4-chloro-2-(1,3-phthalimide-2-yl)-6-(2-hydroxy-2-methylpropoxy)pyrazolo[1,5-a]pyridine-3-nitrile

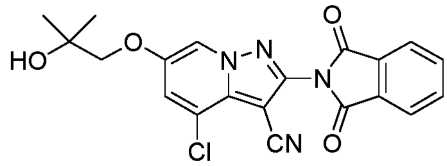

4-chloro-2-(1,3-phthalimide-2-yl)-6-hydroxypyrazolo[1,5-a]pyridine-3-carbonitrile (250 mg, 0.71 mmol), isobutylene oxide (500 mg, 7.1 mmol), potassium carbonate (300 mg, 2.13 mmol) were added to DMF (5.0 mL), and the reaction was carried out at 60°C for 16 hours. After the the reaction was completed as shown by TLC, water is added, extracted with ethyl acetate, the organic phases are combined, washed with saturated brine, concentrated under reduced pressure, and separated by column chromatography to obtain the product 4-chloro-2-(1,3-phthalimide-2-yl)-6-(2-hydroxy-2-methylpropoxy)pyrazolo[1,5-a] pyridine-3-carbonitrile (30 mg, yield 10%). m/z=411[M+1]$^+$.

Step B: 2-amino-4-chloro-6-2-hdrox-2-methlrooxrazolo1,5-aridine-3-carbonitrile

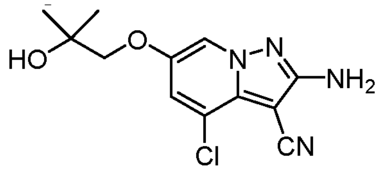

4-chloro-2-(1,3-phthalimide-2-yl)-6-(2-hydroxy-2-methylpropoxy)pyrazolo[1,5-a]pyridine-3-carbonitrile (30 mg, 0.073 mmol) was dissolved in THF (4.0 mL), 0.5 mL of 50% ammonia was added, and stirred at room temperature for 1 h. After the the reaction was completed as shown by TLC, water was added, extracted with ethyl acetate, the organic phases were combined, washed with saturated brine, concentrated under reduced pressure, and separated by column chromatography to obtain the product 2-amino-4-chloro-6-(2-hydroxy-2-methylpropoxy)pyrazolo[1,5-a]pyridine-3-carbonitrile (18mg, yield 87%). m/z=281[M+1]$^+$.

Step C: 3-(5-bromopyridin-2-yl)-6-((6-methoxypyridin-3-yl)methyl)-3,6-diazabicyclo[3.1.1]heptane

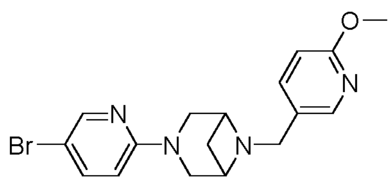

To a 100 mL single-neck flask, 6-methoxynicotinaldehyde (0.26g, 1.9 mmol), 3-(5-bromopyridin-2-yl)-3,6-diazabicyclo[3.1.1]heptane dihydrochloride (0.6 g, 1.9 mmol), sodium acetate borohydride (1.2 g, 5.8 mmol), dichloromethane 20 mL were added, and stirred at room temperature for 12 hours. After the the reaction was completed as shown by TLC, water was added, extracted with ethyl acetate, the organic phases were combined, washed with water, dried with anhydrous sodium sulfate, filtered, concentrated under reduced pressure, and separated by column chromatography to obtain the product 3-(5-bromopyridin-2-yl)-6-(pyridin-2-ylmethyl)-3,6-diazabicyclo[3.1.1]heptane (356 mg, yield 50%). m/z=375[M+1]$^+$.

Step D: 6-((6-methoxypyridin-3-yl)methyl)-3-(5-(4,4,5,5-tetramethyl-1,3,2-dioxolane boran-2-yl)pyridin-2-yl)-3,6-diazabicyclo[3 .1.1]heptane

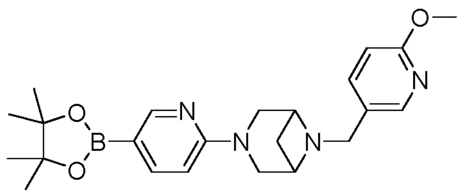

3-(5-bromopyridin-2-yl)-6-(pyridin-2-yl-methyl)-3,6-diazabicyclo[3.1.1]heptane (49 mg, 0.132 mmol), duplex pinacol borate (50 mg, 0.198 mmol), [1,1'-bis(diphenylphosphino)ferrocene]palladium dichloride (10 mg, 0.013 mmol)), potassium acetate (39 mg, 0.396 mmol), and 1,4-dioxane (20 mL) were added into a 10 mL sealed tube, purged with nitrogen three times, reacted at 100°C for 3 hours, LCMS confirmed that the raw materials were reacted, and products were formed, no post-processing is required, which was used directly for the next reaction.

Step E: At room temperature, to a solution of 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane (37mg, 0.087 mmol) and 2-amino-4-chloro-6-(2-Hydroxy-2-methylpropoxy)pyrazolo[1,5-a]pyridine-3-carbonitrile (21 mg, 0.073 mmol) in 1,4-dioxane (2.5 mL) was added sodium carbonate (38 mg, 0.358 mmol, 2M) aqueous solution, purged with nitrogen for 2 minutes, then added tetratriphenylphosphine palladium (4 mg, 0.003 mmol), purged with nitrogen for 2 minutes, and stirred at 110°C for 4 hours, TLC plate showed the reaction was completed, water was added, extracted with ethyl acetate, the organic phases were combined, washed with saturated brine, concentrated under reduced pressure, and separated by column chromatography to obtain 2-amino-6-(1-methyl-1H-pyrazol-4-yl)-4-(6-(6-(pyridin-2-yl-methyl)-3,6-diazabicyclo[3.1.1]heptan-3-yl)pyridine-3-yl)pyrazolo[1,5-a]pyridine-3-carbonitrile (11.3 mg, 28% yield). $^1$HNMR (400MHz, DMSO-$d_6$) δ 8.35 (d, 1H), 8.27 (d, 1H), 8.07 (s, 1H), 7.81 (d, 1H), 7.67 (d, 1H), 7.10 (d, 1H), 6.78 (d, 2H), 6.15 (s, 2H), 4.67 (s, 1H), 3.82 (d, 5H), 3.62-3.68 (m, 4H), 3.48-3.55 (m, 4H), 1.58 (s, 1H), 1.21 (s, 6H).m/z=541[M+1]$^+$.

Example 42

2-fluoro-6-(2-hydroxy-2-methylpropoxy)-4-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS090)

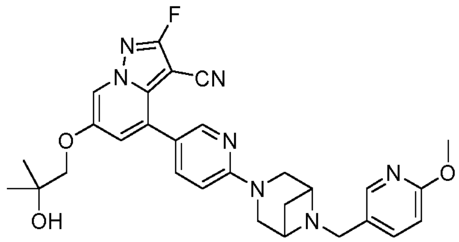

The preparation method according to Example 40 was perfomed, with the difference that 2-amino-4-(5-(6-((6-methoxypyridin-3-yl)methyl)-3,6-diazabicyclo[3.1.1]heptan-3-yl)pyridine(azin-2-yl)-6-(((S)-morpholin-2-yl)methoxy) pyrazolo[1,5-a]pyridine-3-carbonitrile trifluoroacetate (APS087) was replaced with 2-amino-6-(2-hydroxy-2-methylpropoxy)-4-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS089) to obtain the product (2.8 mg , Yield 6.8%). $^1$HNMR (400MHz, DMSO-$d_6$) δ 8.63 (s, 1H), 8.42 (s, 1H), 8.07 (s, 1H), 7.87 (s, 1H), 7.69 (s, 1H), 7.44 (s, 1H), 6.79 (s, 2H), 4.74 (s, 1H), 3.82-3.88 (m, 5H), 3.68-3.72 (m 4H), 3.51 (brs, 5H), 1.59 (s, 1H), 1.23 (s, 6H).m/z=544[M+1]$^+$.

Example 43

6-(2-hydroxy-2-methylpropoxy)-2-methoxy-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS091)

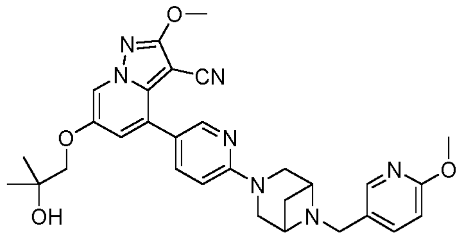

2-fluoro-6-(2-hydroxy-2-methylpropoxy)-4-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS090) (15 mg, 0.028 mmol), potassium carbonate (11 mg, 0.08 mmol) were added to methanol (2.0 mL), and the tube was sealed and the mixture was heated to 60°C to react for 12h. Water was added, extracted with ethyl acetate, the organic phases were combined, washed with saturated brine, concentrated under reduced pressure, and separated by column chromatography to obtain 6-(2-hydroxy-2-2methylpropoxy)-2-methoxy-4-(6-(6-((6-methoxypyridin-3-yl)methyl)-3,6-diazabicyclo [3.1.1]heptan-3-yl)pyridine-3-yl)pyrazolo[1,5-a]pyridine-3-carbonitrile (14 mg, yield 89%). $^1$HNMR (400MHz, DMSO-$d_6$) δ8.55 (d, 1H), 8.39 (d, 1H), 8.07 (d, 1H), 7.83 (dd, 1H), 7.69 (dd, 1H), 7.29 (d, 1H), 6.79 (dd, 2H), 4.70 (s, 1H), 4.05 (s, 3H), 3.85 (s, 2H), 3.82 (s, 3H), 3.62-3.78 (m, 4H), 3.50-3.55 (m, 4H), 1.59 (d, 1H), 1.22 (s, 6H).m/z=556[M+1]$^+$.

Example 44

2-amino-6-ethoxy-4-(5-(6-((6-methoxypyridin-3-yl)methyl)-3,6-diazabicyclo[3.1.1]heptane-3-yl) pyrazin-2-yl)pyrazolo[1,5-a]pyridine-3-carbonitrile (APS092)

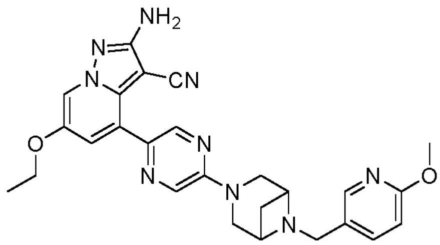

The preparation method according to Example 39 was perfomed, with the difference that tert-butyl (2S)-2-(bromomethyl)-4-morpholinecarboxylate was replaced with iodoethane to obtain 2-amino-6-ethoxy-4-(5-(6-((6-methoxypyridin-3-yl)methyl)-3,6-diazabicyclo[3.1.1]heptan-3-yl)pyrazin-2-yl)pyrazolo [1,5-a]pyridine-3-carbonitrile (2.9 mg, yield 8%). $^1$HNMR (400MHz, DMSO-$d_6$) δ 8.56 (s, 1H), 8.30 (d, 2H), 8.10 (s, 1H), 7.83-8.04 (m, 1H), 7.71 (d, 1H), 7.35 (s, 1H), 6.78 (d, 1H), 6.11 (s, 2H), 4.12 (d, 2H), 3.55-3.82 (m, 12H), 1.63 (s, 1H), 1.36 (brs, 3H).m/z=498[M+1]$^+$.

Example 45

N-(3-cyano-6-(2-hydroxy-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS093)

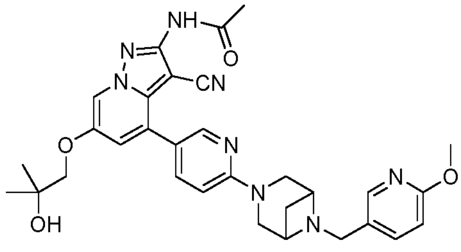

2-amino-6-(2-hydroxy-2-methylpropoxy)-4-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS089) (50 mg, 0.093 mmol) was dissolved in DCM (2.0 mL), triethylamine (28 mg, 0.3 mmol) was added, cooled to 0°C in an ice-water bath, acetyl chloride (8 mg, 0.1 mmol) was added and the temperature was raised naturally to room temperature to react for 12h. Water was added, extracted with ethyl acetate, the organic phases were combined, washed with saturated brine, concentrated under reduced pressure, and separated by column chromatography to obtain N-(3-cyano-6-(2-hydroxy-2-methylpropoxy))-4-(6-(6-((6-methoxypyridin-3-yl)methyl)-3,6-diazabicyclo[3.1.1]heptan-3-yl)pyridine-3-yl)pyrazolo[1,5-a]pyridine-3-carbonitrile (2.7 mg, yield 5%). $^1$HNMR (400MHz, DMSO-$d_6$) δ 10.67 (s, 1H), 8.55 (d, 1H), 8.38 (d, 1H), 8.07 (s, 1H), 7.82 (dd, 1H), 7.69 (dd, 1H), 7.27 (d, 1H), 6.80 (t, 2H), 4.72 (s, 1H), 3.87 (s, 2H), 3.82 (s, 3H), 3.67-3.75 (m, 4H), 3.50-3.59 (m, 5H), 2.10 (s, 3H), 1.60 (d, 1H), 1.23 (s, 6H). m/z=583[M+1]$^+$.

Example 46

6-(2-Hydroxy-2-methylpropoxy)-2-((2-2-hydroxyethyl)amino)-4-(6-(6-((6-methoxypyridine-3-yl) methyl)-3,6-diazabicyclo[3.1.1]heptan-3-yl)pyridin-3-yl)pyrazolo[1,5-a]pyridine-3-carbonitrile (APS094)

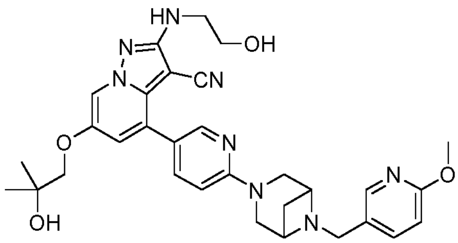

2-amino-6-(2-hydroxy-2-methylpropoxy)-4-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS089) (100 mg, 0.17 mmol) and 2-bromoethanol (21 mg, 0.17 mmol) were added to DMF (2.0 mL) and heated to 120°C to react for 12h. Water was added, extracted with ethyl acetate, the organic phases were combined, washed with saturated brine, concentrated under reduced pressure, and separated by column chromatography to obtain 6-(2-hydroxy-2-methylpropoxy)-2-((2-2-hydroxyethyl)amino)-4-(6-(6-((6-methoxypyridin-3-yl)methyl)-3,6-diazabicyclo[3.1.1]heptane-3-yl)pyridin-3-yl)pyrazolo[1,5-a]pyridine-3-carbonitrile (5.0 mg, yield 5%). $^1$HNMR (400MHz, DMSO-$d_6$) δ 8.38 (dd, 2H), 8.07 (s, 1H), 7.79 (dd, 1H), 7.69 (dd, 1H), 7.12 (d, 1H), 6.78 (d, 2H), 6.36 (t, 1H), 4.66-4.72 (m, 2H), 3.82 (d, 5H), 3.62-3.78 (m, 4H), 3.45-3.61 (m, 6H), 1.60 (d, 1H), 1.21 (s, 6H).m/z=585[M+1]$^+$.

Example 47

2-amino-6-ethoxy-4-(6-((6-methoxypyridin-3-yl)methyl)-3,6-diazabicyclo[3.1.1]heptane-3-yl) pyridin-3-yl)pyrazolo[1,5-a]pyridine-3-carbonitrile (APS095)

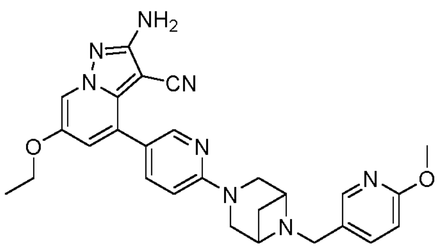

The preparation method according to Example 44 was perfomed, with the difference that the 6-((6-methoxypyridin-3-yl)methyl)-3-(5-(4,4,5,5-tetramethyl-1,3,2-dioxapentaborane-2-yl)pyrazin-2-yl)-3,6-diazabicyclo[3.1.1]heptane was replaced with 6-((6-methoxypyridin-3-yl)methyl)-3-(5-(4,4,5,5-tetramethyl-1,3,2-dioxapentaborane-2-yl)pyridin-2-yl)-3,6-diazabicyclo[3.1.1]heptane to obtain 2-amino-6-ethoxy-4-(6-((6-methoxypyridin-3-yl)methyl)-3,6-diazabicyclo[3.1.1]heptane-3-yl)pyridin-3-yl)pyrazolo[1,5-a]pyridine-3-carbonitrile (9.2 mg, yield 25%). $^1$HNMR (400MHz, DMSO-$d_6$) δ8.35 (d, 1H), 8.26 (d, 1H), 8.08 (d, 1H), 7.79 (dd, 1H), 7.70 (dd, 1H), 7.08 (d, 1H), 6.75-6.78 (m, 2H), 6.15 (s, 2H),

4.06-4.12 (m, 2H), 3.82 (s, 3H), 3.67-3.74 (m, 4H), 3.51-3.55 (m, 4H), 2.08 (s, 1H), 1.59 (d, 1H), 1.35 (t, 3H).m/z=497[M+1]+.

Example 48

6-ethoxy-2-fluoro-4-(6-((6-methoxypyridin-3-yl)methyl)-3,6-diazabicyclo[3.1.1]heptane-3-yl) pyridin-3-yl)pyrazolo[1,5-a]pyridine-3-carbonitrile (APS096)

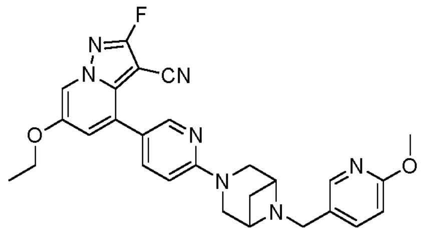

The preparation method according to Example 40 was perfomed, with the difference that the 2-amino-4-(5-(6-((6-methoxypyridin-3-yl)methyl)-3,6-diazabicyclo[3.1.1]heptan-3-yl)pyridine(azin-2-yl)-6-(((S)-morpholin-2-yl)methoxy) pyrazolo[1,5-a]pyridine-3-carbonitrile trifluoroacetate (APS087) was replaced with 2-Amino-6-ethoxy-4-(6-((6-methoxypyridin-3-yl)methyl)-3,6-diazabicyclo[3.1.1]heptane-3-yl)pyridin-3-yl)pyrazolo[1,5-] pyridine-3-carbonitrile (APS095) to obtain 6-ethoxy-2-fluoro-4-(6-((6-methoxypyridin-3-yl)methyl)-3,6-diazabicyclo[3.1.1]heptane-3-yl)pyridin-3-yl)pyrazolo[1,5-a]pyridine-3-carbonitrile (7.1 mg, yield 19%). m/z=500[M+1]+.

Example 49

2-amino-6-(2-methoxyethoxy)-4-(6-(6-((6-methoxypyridin-3-yl)methyl)-3,6-diazabicyclo[3.1.1] heptan-3-yl)pyridin-3-yl)pyrazolo[1,5-a]pyridine-3-carbonitrile (APS097)

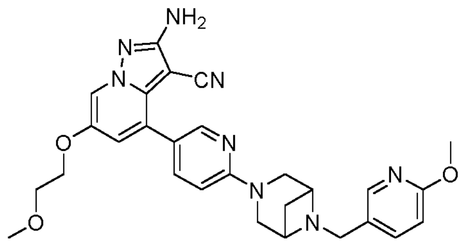

The preparation method according to Example 47 was perfomed, with the difference that iodoethane was replaced with 2-bromoethyl methyl ether to obtain 2-amino-6-(2-methoxyethoxy)-4-(6-(6-((6-methoxypyridin-3-yl)methyl)-3,6-diazabicyclo[3.1.1]heptan-3-yl)pyridin-3-yl)pyrazolo[1,5-a] pyridine-3-carbonitrile (20 mg, yield 52%). $^1$HNMR (400MHz, DMSO-$d_6$) δ 8.35 (d, 1H), 8.30 (d, 1H), 8.08 (d, 1H), 7.79 (dd, 1H), 7.70 (dd, 1H), 7.10 (d, 1H), 6.77 (t, 2H), 6.16 (s, 2H), 4.19 (dd, 2H), 3.82 (s, 3H), 3.66-3.74 (m, 6H), 3.50 (s, 4H), 1.60 (d, 1H).m/z=527[M+1]+.

Example 50

2-fluoro-6-(2-methoxyethoxy)-4-(6-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS098)

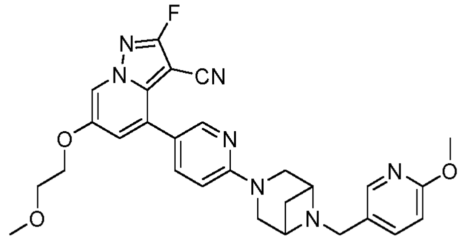

The preparation method according to Example 40 was perfomed, with the difference that 2-amino-4-(5-(6-((6-methoxypyridin-3-yl)methyl)-3,6-diazabicyclo[3.1.1]heptan-3-yl)pyridine(azin-2-yl)-6-(((S)-morpholin-2-yl)methoxy) pyrazolo[1,5-a]pyridine-3-carbonitrile trifluoroacetate (APS087) was replaced with 2-amino-6-(2-methoxyethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS097) to obtain 2-fluoro-6-(2-methoxyethoxy)-4-(6-(6-((6-methoxypyridin-3-yl)methyl)-3,6-diazabicyclo [3.1.1]Heptan-3-yl)pyridin-3-yl)pyrazolo[1,5-a]pyridine-3-carbonitrile (5.2 mg, yield 14%). $^1$HNMR (400MHz, DMSO-$d_6$) δ8.65 (d, 1H), 8.42 (d, 1H), 8.07 (d, 2H), 7.82 (dd, 1H), 7.70 (dd, 1H), 7.45 (d, 1H), 6.81 (t, 2H), 4.25 (t, 2H), 3.82 (s, 3H), 3.68-3.82 (m, 6H), 3.44-3.58 (m, 5H), 1.23 (d, 1H), 1.21 (s, 1H).m/z=530[M+1]$^+$.

Example 51

2-amino-6-ethoxy-4-(6-(6-((6-methoxypyridin-3-yl)methyl)piperazin-1-yl)pyridin-3-yl)pyrazolo [1,5-aridine-3-carbonitrile (APS099)

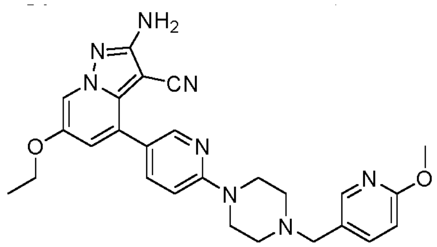

The preparation method according to Example 47 was perfomed, with the difference that 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane was replaced with 1-((6-methoxypyridin-3-yl))methyl)-4-(5-(4,4,5,5-tetramethyl-1,3,2-dioxaborolan-2-yl)pyridin-2-yl)piperazine to obtain 2-amino-6-ethoxy-4-(6-(6-((6-methoxypyridin-3-yl)methyl)piperazin-1-yl)pyridin-3-yl)pyrazolo[1,5-a]pyridine-3-carbonitrile (8.9 mg, yield 25%). $^1$HNMR (400MHz, DMSO-$d_6$) δ 8.27 (dd, 2H), 8.09 (d, 1H), 7.66-7.72 (m, 2H), 7.06 (d, 1H), 6.91 (d, 1H), 6.82 (d, 1H), 6.14 (s, 2H), 4.05-4.10 (m, 2H), 3.84 (s, 3H), 3.55 (brs, 4H), 3.48 (s, 2H), 2.49 (s, 4H), 1.34 (t, 3H).m/z=485[M+1]$^+$.

Example 52

2-amino-6-(2-methoxyethoxy)-4-(6-(4-((6-methoxypyridin-3-yl)methyl)piperazin-1-yl)pyridine-3-yl)pyrazolo[1,5-a]pyridine-3-carbonitrile (APS100)

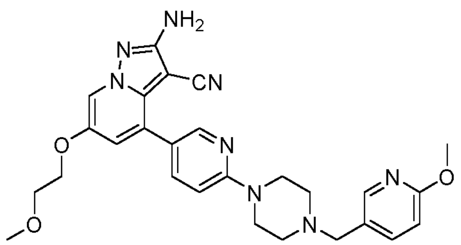

The preparation method according to Example 49 was perfomed, with the difference that 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane was replaced with 1-((6-methoxypyridin-3-yl))Methyl)-4-(5-(4,4,5,5-tetramethyl-1,3,2-dioxaborolan-2-yl) pyridin-2-yl)piperazine to obtain 2-amino-6-(2-methoxyethoxy)-4-(6-(4-((6-methoxypyridin-3-yl)methyl)piperazin-1-yl)pyridine-3-yl)pyrazolo[1,5-a]pyridine-3-carbonitrile (5.1 mg, yield 13%). $^1$HNMR (400MHz, DMSO-$d_6$) δ8.29 (dd, 2H), 8.09 (d, 1H), 7.62-7.71 (m, 2H), 7.90 (d, 1H), 6.89 (d, 1H), 6.82 (d, 1H), 6.16 (s, 2H), 4.16 (d, 2H), 3.84 (s, 3H), 3.66-3.67 (m, 2H), 3.55-3.57 (s, 4H), 3.48 (s, 2H), 3.31 (s, 3H), 2.46 (s, 3H).m/z=515[M+1]$^+$.

Example 53

6-ethoxy-2-fluoro-4-(6-(4-((6-methoxypyridin-3-yl)methyl)piperazin-1-yl)pyridin-3-yl)pyrazolo 15-aridine-3-carbonitrile (APS101)

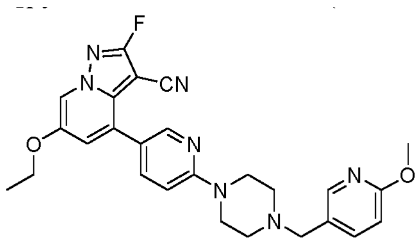

The preparation method according to Example 40 was perfomed, with the difference that 2-amino-4-(5-(6-((6-methoxypyridin-3-yl)methyl)-3,6-diazabicyclo[3.1.1]heptan-3-yl)pyridine(azin-2-yl)-6-(((S)-morpholin-2-yl)methoxy) pyrazolo[1,5-a]pyridine-3-carbonitrile trifluoroacetate (APS087) was replaced with 2-amino-6-ethoxy-4-(6-(4-((6-methoxypyridin-3-yl)methyl)piperazin-1-yl)pyridin-3-yl)pyrazolo [1,5-a]pyridine-3-carbonitrile (APS099) to obtain 6-ethoxy-2-fluoro-4-(6-(4-((6-methoxypyridin-3-yl)methyl)piperazin-1-yl)pyridin-3-yl)pyrazolo [1,5-a]pyridine-3-carbonitrile (3.4 mg, yield 9%). $^1$HNMR (400MHz, DMSO-$d_6$) δ 8.61 (d, 1H), 8.33 (d, 1H), 8.09 (d, 1H), 7.78 (dd, 1H), 7.68 (dd, 1H), 7.41 (d, 1H), 6.91 (d, 1H), 6.82 (d, 1H), 4.15 (d, 2H), 3.84 (s, 3H), 3.57-3.59 (m, 4H), 3.48 (s, 2H), 1.39 (t, 3H).m/z=488[M+1]$^+$.

Example 54

2-fluoro-6-(2-methoxyethoxy)-4-(6-(4-((6-methoxypyridin-3-yl)methyl)piperazin-1-yl)pyridine-3-yl)pyrazolo[1,5-a]pyridine-3-carbonitrile (APS102)

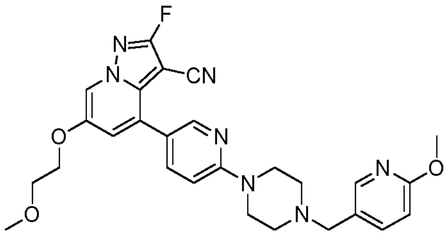

The preparation method according to Example 40 was perfomed, with the difference that 2-amino-4-(5-(6-((6-methoxypyridin-3-yl)methyl)-3,6-diazabicyclo[3.1.1]heptan-3-yl)pyridine(azin-2-yl)-6-(((S)-morpholin-2-yl)methoxy) pyrazolo[1,5-a]pyridine-3-carbonitrile trifluoroacetate (APS087) was replaced with 2-amino-6-(2-methoxyethoxy)-4-(6-(4-((6-methoxypyridin-3-yl)methyl)piperazin-1-yl)pyridine-3-yl)pyrazolo[1,5-a]pyridine-3-carbonitrile (APS100) to obtain 2-fluoro-6-(2-methoxyethoxy)-4-(6-(4-((6-methoxypyridin-3-yl)methyl)piperazin-1-yl)pyridine-3-yl)pyrazolo[1,5-a]pyridine-3-carbonitrile (5.3 mg, 14%). $^1$HNMR (400MHz, DMSO-$d_6$) δ 8.64 (d, 1H), 8.33 (d, 1H), 8.09 (d, 1H), 7.80 (dd, 1H), 7.69 (dd, 1H), 7.44 (d, 1H), 6.95 (d, 1H), 6.82 (d, 1H), 4.24 (t, 2H), 3.84 (s, 3H), 3.68-3.70 (m, 2H), 3.57-3.60 (m, 4H), 3.48 (s, 4H), 2.45 (t, 3H). m/z=518[M+1]$^+$.

Example 55

N-(3-cyano-6-(2-methoxyethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS103)

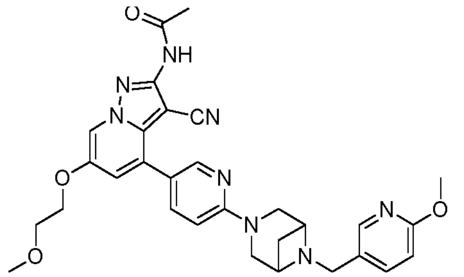

2-amino-6-(2-methoxyethoxy)-4-(6-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS097) (50 mg, 0.09 mmol) was added to acetic anhydride (2.0 mL), heated to 80°C to react for 4h. Water was added, extracted with ethyl acetate, the organic phases were combined, washed with saturated brine, concentrated under reduced pressure, and separated by column chromatography to obtain N-(3-cyano-6-(2-methoxyethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (30 mg, yield 58%). $^1$HNMR (400MHz, DMSO-$d_6$) $\delta$10.65 (s, 1H), 8.57 (d, 1H), 8.37 (d, 1H), 8.07 (d, 1H), 7.79 (d, 1H), 7.67 (d, 1H), 7.28 (d, 1H), 6.76-6.79 (m, 2H), 4.23-4.25 (m, 2H), 3.82 (s, 3H), 3.66-3.71 (m, 6H), 3.50-3.59 (m, 4H), 2.10 (s, 3H), 1.60 (d, 1H).m/z=569[M+1]$^+$.

Example 56

2-fluoro-4-(5-(6-((6-methoxypyridin-3-yl)methyl)-3,6-diazabicyclo[3.1.1]heptan-3-yl)pyrazin-2-yl)-6-(((S)-4-methylmorpholin-2-yl)methoxy)pyrazolo[1,5-a]pyridine-3-carbonitrile (APS104)

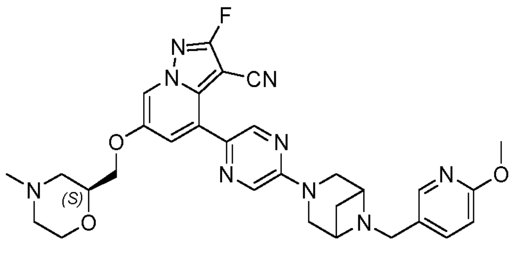

2-fluoro-4-(5-(6-((6-methoxypyridin-3-yl)methyl)-3,6-diazabicyclo[3.1.1]heptan-3-yl) pyrazin-2-yl)-6-(((S)-morpholin-2-yl)methoxy)pyrazolo[1,5-a]pyridine-3-carbonitrile trifluoroacetate (APS088) (120 mg, 0.21 mmol), 37% aqueous formaldehyde solution (120 mg, 1.48 mmol), sodium acetate borohydride (133 mg, 0.63 mmol), and dichloromethane (10 mL) were stirred at room temperature to react for 12 hours. After the the reaction was completed as shown by TLC, water was added, extracted with ethyl acetate, the organic phases were combined, washed with water, dried over anhydrous sodium sulfate, filtered, concentrated under reduced pressure, and separated by column chromatography to obtain the product 2-fluoro-4-(5-(6-((6-methoxypyridin-3-yl)methyl)-3,6-diazabicyclo[3.1.1]heptan-3-yl)pyrazin-2-yl)-6-(((S)-4-methylmorpholin-2-yl)methoxy)pyrazolo[1,5-a]pyridine-3-carbonitrile (8.2 mg, yield 6%). m/z=586[M+1]$^+$.

Example 57

2-amino-4-(6-(6-((5-chloro-6-methoxypyridin-3-yl)methyl)-3,6-diazabicyclo[3.1.1]heptane-3-yl)-6-ethoxypyrazolo[1,5-a]pyridine-3-carbonitrile (APS105)

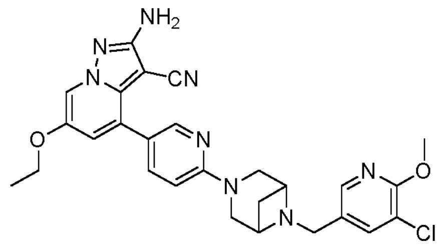

Step A: 3-(5-bromopyridin-2-yl)-6-((5-chloro-6-methoxypyridin-3-yl)methyl)-3,6-diazabicyclo[3.1. 1]heptane

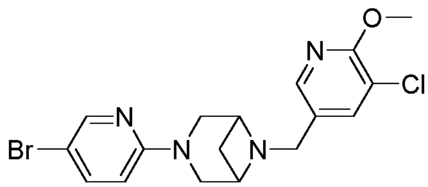

To a 100 mL single-neck flask, 5-chloro-6-methoxynicotinaldehyde (0.32g, 1.9 mmol), 3-(5-bromopyridin-2-yl)-3,6-diazabicyclo[3.1.1] heptane dihydrochloride (0.6 g, 1.9 mmol), sodium acetate borohydride (1.2 g, 5.8 mmol), and dichloromethane 20 mL were added, and stirred at room temperature for 12 hours. After the the reaction was completed as shown by TLC, water was added, extracted with ethyl acetate, the organic phases were combined, washed with water, dried with anhydrous sodium sulfate, filtered, concentrated under reduced pressure, and separated by column chromatography to obtain the product 3-(5-bromopyridine-2-yl)-6-((5-chloro-6-methoxypyridin-3-yl)methyl)-3,6-diazabicyclo[3.1.1]heptane (0.4g, yield 51%). m/z=409[M+1]$^+$.

Step B: 6-((5-chloro-6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane

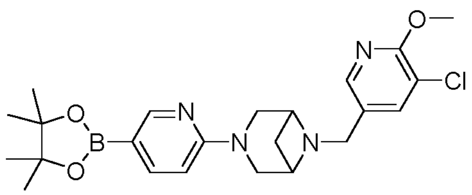

To a 10 mL sealed tube, 3-(5-bromopyridin-2-yl)-6-(pyridin-2-yl-methyl)-3,6-diazabicyclo[3.1.1]heptane (53 mg, 0.132 mmol), duplex pinacol borate (50 mg, 0.198 mmol), [1,1'-bis(diphenylphosphino)ferrocene]palladium dichloride (10 mg, 0.013 mmol)), potassium acetate (39 mg, 0.396 mmol), and 1,4-dioxane (20 mL) were added, purged with nitrogen three times, reacted at 100°C for 3 hours, LCMS confirmed that the raw materials were reacted completely to give a product, which was used directly for the next reaction without post-processing.

Step C: 4-chloro-2-(1,3-phthalimide-2-yl)-6-ethoxypyrazolo[1,5-a]pyridine-3-carbonitrile

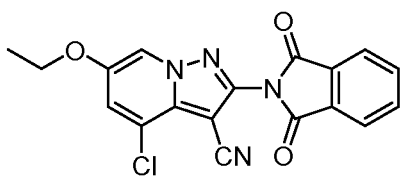

The preparation method according to Step H of Example 39 was performed with the difference that (2S)-2-(bromomethyl)-4-morpholinecarboxylic acid tert-butyl ester was replaced with iodoethane to obtain 4-chloro-2-(1,3-phthalimide-2-yl)-6-ethoxypyrazolo[1,5-a]pyridine-3-carbonitrile (60 mg, yield 86%). m/z=367[M+1]$^+$.

Step D: The preparation method according to Step I of Example 39 was perfomed, with the difference that 6-((6-Methoxypyridin-3-yl)methyl)-3-(5-(4,4,5,5-tetramethyl-1,3,2-dioxapentaborane-2-yl)pyrazin-2-yl)-3,6-diazabicyclo[3.1.1]heptane was replaced with 6-((5-chloro-6-methoxypyridin-3-yl)methyl)-3-(5-(4,4,5,5-tetramethyl-1,3,2-dioxa pentaboran-2-yl)pyridin-2-yl)-3,6-diazabicyclo[3.1.1]heptane to obtain 2-amino-4-(6-(6-((5-chloro-6-methoxypyridin-3-yl)methyl)-3,6-diazabicyclo[3.1.1]heptane-3-yl)-6-ethoxypyrazolo[1,5-a]pyridine-3-carbonitrile (14 mg, yield 36%). $^1$HNMR (400MHz, $CDCl_3$) δ 8.38 (d, 1H), 8.01 (d, 1H), 7.86 (d, 1H), 7.80 (dd, 2H), 7.02 (d, 1H), 6.69 (d, 1H), 4.45 (d, 2H), 4.01-4.42 (m, 5H), 3.82 (brs, 4H), 3.60 (brs, 4H), 2.75 (s, 1H), 1.63-1.67 (m, 1H), 1.47 (t, 3H).m/z=531[M+1]$^+$.

Example 58

2-amino-6-(2-hydroxy-2-methylpropoxy)-4-(6-((6-methoxypyridin-3-yl)methyl)-3,6-diazabicyclo [3.1.1]heptan-3-yl)pyridin-3-yl)pyrazolo[1,5-a]pyridine-3-carboxamide (APS106)

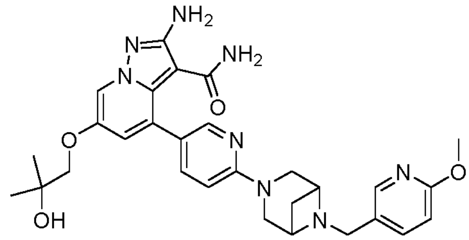

2-amino-6-(2-hydroxy-2-methylpropoxy)-4-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS089) (40 mg, 0.074 mol) was dissolved in DMSO (2.0 mL), to which hydrogen peroxide (0.2 mL) was added and the mixture was heated to 65°C to react for 6 hours. Water was added, extracted with ethyl acetate, the organic phases were combined, washed with saturated brine, concentrated under reduced pressure, and separated by column chromatography to obtain 2-amino-6-(2-hydroxy-2-methylpropoxy)-4-(6-((6-methoxypyridin-3-yl)methyl)-3,6-diazabicyclo[3.1.1]heptan-3-yl)pyridin-3-yl)pyrazolo[1,5-a]pyridine-3-carboxamide (3.5 mg, yield 8%). $^1$HNMR (400MHz, DMSO-$d_6$) δ 8.27 (d, 1H), 8.17 (d, 1H), 8.08 (d, 1H), 7.63-7.69 (m, 2H), 6.96 (d, 1H), 6.78 (d, 1H), 6.72 (d, 1H), 5.76 (s, 2H), 4.66 (s, 1H), 3.79-3.82 (m, 5H), 3.61-3.68 (m, 4H), 3.48 (brs, 4H), 1.55 (d, 1H), 1.21 (s, 6H).m/z=559[M+1]$^+$.

Example 59

2-amino-4-(5-(6-((6-methoxypyridin-3-yl)methyl)-3,6-diazabicyclo[3.1.1]heptan-3-yl)pyrazin-2-yl)-6-(((S)-4-methylmorpholin-2-yl)methoxy)pyrazolo[1,5-a]pyridine-3-carbonitrile (APS107)

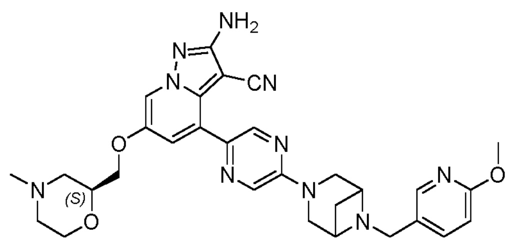

The preparation method according to Example 56 was performed with the difference that 2-fluoro-4-(5-(6-((6-methoxypyridin-3-yl)methyl)-3,6-diazabicyclo[3.1.1]heptan-3-yl)pyrazin-2-yl)-6-(((S)-morpholin-2-yl)methoxy)pyrazolo [1,5-a]pyridine-3-carbonitrile trifluoroacetate (APS088) was replaced with 2-amino-4-(5-(6-((6-methoxypyridin-3-yl)methyl)-3,6-diazabicyclo[3.1.1]heptan-3-yl)pyrazin-2-yl)-6-(((S)-morpholin-2-yl)methoxy)pyrazolo[1,5-a]pyridine-3-carbonitrile trifluoroacetate (APS087) to obtain the product 2-amino-4-(5-(6-((6-methoxypyridin-3-yl)methyl)-3,6-diazabicyclo[3.1.1]heptan-3-yl)pyrazin-2-yl)-6-(((S)-4-methylmorpholin-2-yl)methoxy)pyrazolo[1,5-a] pyridine-3-carbonitrile (30 mg, yield 24%). $^1$HNMR (400MHz, $CDCl_3$) δ 8.48 (d, 1H), 8.25 (d, 1H), 8.11 (d, 1H), 7.94 (d, 1H), 7.64 (dd, 1H), 7.33 (d, 1H), 6.73 (d, 1H), 4.53 (s, 2H), 3.93-4.18 (m, 4H), 3.90-3.92 (m, 3H), 3.81-3.90 (m, 2H), 3.71-3.80 (m, 3H), 3.67-3.75 (m, 2H), 3.59-3.62 (m, 2H), 2.86 (d, 1H), 2.68-2.75 (m, 2H), 2.34 (s, 3H), 2.20 (t, 1H), 2.08 (t, 1H), 1.65 (d, 1H).m/z=583$[M+1]^+$.

Example 60

2-amino-4-(6-(6-((5-chloro-6-methoxypyridin-3-yl)methyl)-3,6-diazabicyclo[3.1.1]heptane-3-yl)pyridin-3-yl)-6-(2-methoxyethoxy)pyrazolo[1,5-a]pyridine-3-carbonitrile (APS108)

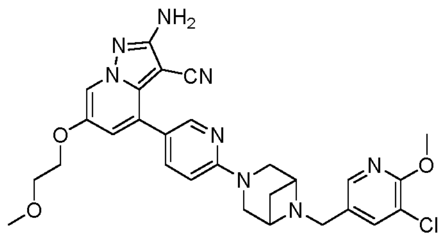

The preparation method according to Example 52 was performed with the difference that 6-((6-methoxypyridin-3-yl)methyl)-3-(5-(4,4,5,5-tetramethyl-1,3,2-dioxaborolan-2-yl)pyridin-2-yl)-3,6-diazabicyclo[3.1.1]heptane was replaced with 6-((5-chloro-6-methoxypyridine-3-yl)methyl)-3-(5-(4,4,5,5-tetramethyl-1,3,2-dioxaborolan-2-yl)pyridin-2-yl)-3,6-diazabicyclo[3.1.1]heptane to obtain the product 2-amino-4-(6-(6-((5-chloro-6-methoxypyridin-3-yl)methyl)-3,6-diazabicyclo[3.1.1]heptan-3-yl)pyridin-3-yl)-6-(2-methoxyethoxy)pyrazolo[1,5-a] pyridine-3-carbonitrile (10.6 mg, yield 26%). $^1$HNMR (400MHz, $CDCl_3$) δ8.38 (d, 1H), 8.02 (s, 1H), 7.92 (d, 1H), 7.79 (dd, 2H), 7.08 (d, 1H), 6.69 (d, 1H), 4.45 (d, 2H), 4.13-4.15 (m, 2H), 4.03 (d, 3H), 3.78-3.84 (m, 6H), 3.61 (brs, 4H), 3.41 (s, 3H), 2.76 (s, 1H), 1.70(d, 1H).m/z=561$[M+1]^+$.

Example 61

2-amino-6-(2-ethoxyethoxy)-4-(6-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS109)

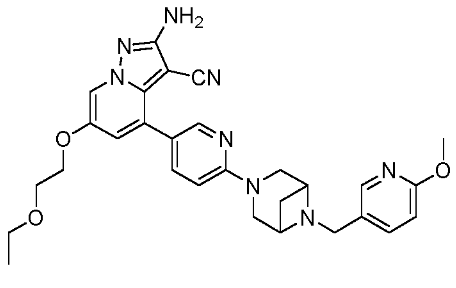

The preparation method according to Example 47 was performed with the difference that iodoethane was replaced with 2-bromoethyl ethyl ether to obtain the product 2-amino-6-(2-ethoxyethoxy)-4-(6-(6-((6-methoxypyridin-3-yl)methyl)-3,6-diazabicyclo [3.1.1]Heptan-3-yl)pyridin-3-yl)pyrazolo[1,5-a]pyridine-3-carbonitrile (16.2 mg, yield 41%). $^1$HNMR (400MHz, DMSO-$d_6$) δ 8.30-8.36 (m, 2H), 8.08 (s, 1H), 7.77-7.80 (m, 1H), 7.70 (d, 1H), 7.11 (d, 1H), 6.75-6.77 (m, 2H), 6.17 (s, 2H), 4.16-4.18 (m, 2H), 3.82 (s, 3H), 3.70-3.74 (m, 6H), 3.48-3.53 (m, 6H), 1.60 (d, 1H), 1.15 (t, 3H). m/z=541$[M+1]^+$.

Example 62

2-amino-6-(2-methoxy-2-methylpropoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS110)

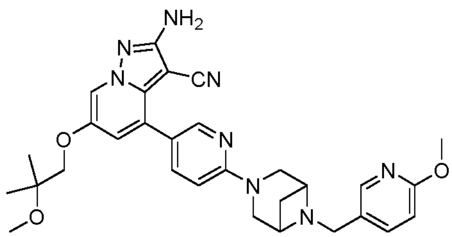

The preparation method according to Example 47 was performed with the difference that iodoethane was replaced with 1-bromo-2-methoxy-2-methylpropane to obtain the product 2-amino-6-(2-methoxy-2-methylpropoxy)-4-(6-(6-((6-methoxypyridin-3-yl)methyl)-3,6-diazabicyclo[3.1.1]heptan-3-yl)pyridine-3-yl)pyrazolo[1,5-a]pyridine-3-carbonitrile (2.8 mg, yield 7%). m/z=555[M+1]$^+$.

Example 63

2-amino-6-ethoxy-4-(6-(6-((5-fluoropyridin-3-yl)methyl)-3,6-diazabicyclo[3.1.1]heptane-3-yl)pyridin-3-yl)pyrazolo[1,5-a]pyridine-3-carbonitrile (APS111)

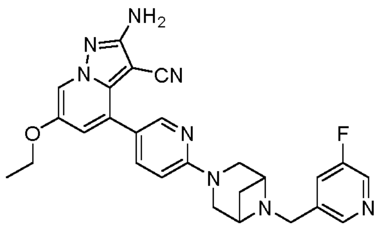

The preparation method according to Example 57 was performed with the difference that 5-chloro-6-methoxynicotinaldehyde was replaced with 5-fluoronicotinic aldehyde to obtain the product 2-amino-6-ethoxy-4-(6-(6-((5-(Fluoropyridin-3-yl)methyl)-3,6-diazabicyclo[3.1.1]heptan-3-yl)pyridin-3-yl)pyrazolo[1,5-a]pyridine-3-nitriles (3.6 mg, yield 10%). $^1$HNMR (400MHz, DMSO-$d_6$) δ 8.44 (d, 2H), 8.35 (d, 1H), 8.26 (d, 1H), 7.79 (dd, 1H), 7.71 (d, 1H), 7.07 (d, 1H), 6.77 (d, 1H), 6.16 (s, 1H), 4.10 (d, 2H), 3.72-3.74 (m, 5H), 3.65 (s, 2H), 2.51 (brs, 1H), 2.01 (d, 1H), 1.62 (d, 1H), 1.23 (s, 3H).m/z=485[M+1]$^+$.

Example 64

2-amino-4-(6-(6-((5-chloropyridin-3-yl)methyl)-3,6-diazabicyclo[3.1.1]heptan-3-yl)pyridine-3-yl)-6-ethoxypyrazolo[1,5-a]pyridine-3-carbonitrile (APS112)

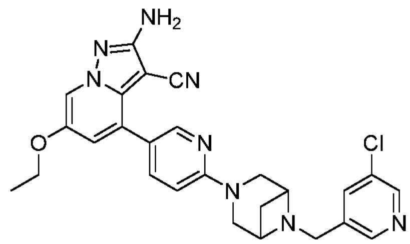

The preparation method according to Example 57 was performed with the difference that 5-chloro-6-methoxynicotinaldehyde was replaced with with 5-chloronicotinaldehyde to obtain 2-amino-4-(6-(6-((5-chloropyridin-3-yl)methyl)-3,6-diazabicyclo[3.1.1]heptan-3-yl)pyridine-3-yl)-6-ethoxypyrazolo[1,5-a]pyridine-3-carbonitrile (13.6 mg, yield 37%). $^1$HNMR (400MHz, DMSO-$d_6$) δ 8.40-8.51 (m, 2H), 8.35 (d, 1H), 8.26 (d, 1H), 7.90 (s, 1H), 7.79 (dd, 1H), 7.07 (d, 1H), 6.77 (d, 1H), 6.16 (s, 2H), 4.90 (q, 2H), 3.72-3.74 (d, 4H), 3.63 (s, 2H), 3.52 (brs, 2H), 2.00 (s, 1H), 1.61 (d, 1H), 1.37 (t, 3H).m/z=501[M+1]$^+$.

Example 65

2-amino-6-(2-cyclopropoxyethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS113)

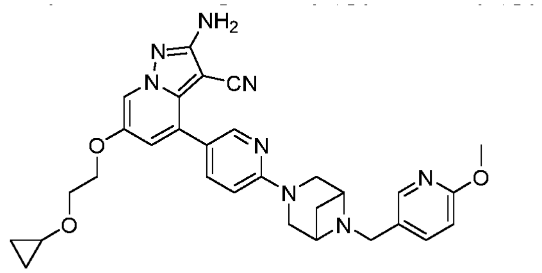

The preparation method according to Example 47 was performed with the difference that iodoethane was replaced with 2-cyclopropoxyethyl p-toluenesulfonate to obtain the product 2-amino-6-(2-cyclopropoxyethoxy)-4-(6-(6-((6-methoxypyridin-3-yl)methyl)-3,6-diazabicyclo[3.1.1]heptan-3-yl)pyridin-3-yl)pyrazolo[1,5-a] pyridine-3-carbonitrile (31.5 mg, yield 78%). $^1$HNMR (400MHz, DMSO-$d_6$) δ8.36 (d, 1H), 8.30 (d, 1H), 8.08 (d, 1H), 7.80 (dd, 1H), 7.70 (dd, 1H), 7.10 (d, 1H), 6.78 (dd, 2H), 6.18 (s, 2H), 4.18 (t, 2H), 3.82 (s, 3H), 3.64-3.78 (m, 6H), 3.50-3.55 (m, 4H), 3.35-3.40 (m, 1H), 1.60 (d, 1H), 0.43-0.52 (m, 4H). m/z=553[M+1]$^+$.

Example 66

2-amino-6-(2,2-difluoroethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS114)

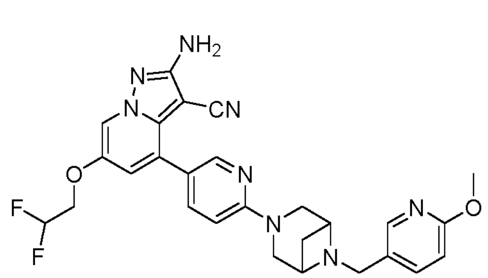

The preparation method according to Example 47 was performed with the difference that iodoethane was replaced with 1,1-difluoro-2-iodoethane to obtain the product 2-amino-6-(2,2-difluoroethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (3.8 mg, yield 9%). $^1$HNMR (400MHz, DMSO-$d_6$) δ8.43 (d, 1H), 8.37 (d, 1H), 8.08 (s, 1H), 7.81 (dd, 1H), 7.70 (dd, 1H), 7.18 (d, 1H), 6.78 (d, 2H), 6.23 (s, 2H), 4.39-4.47 (m, 2H), 3.82 (s, 3H), 3.67-3.74 (m, 4H), 3.50-3.55 (m, 4H), 1.60 (d, 1H).m/z=533[M+1]$^+$.

Example 67

2-amino-6-(difluoromethoxy)-4-(6-(6-((6-methoxypyridin-3-yl)methyl)-3,6-diazabicyclo[3.1. 1]heptan-3-yl)pyridin-3-yl)pyrazolo[1,5-a]pyridine-3-carbonitrile (APS115)

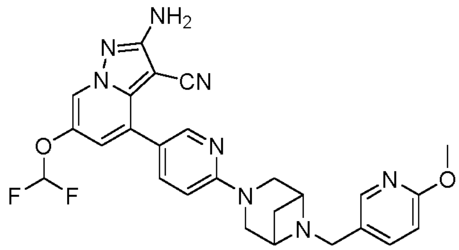

The preparation method according to Example 47 was performed with the difference that iodoethane was replaced with difluoromethyl iodide to obtain the product 2-amino-6-(difluoromethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (2.2 mg, yield 5.8%). m/z=519[M+1]$^+$.

Example 68

2-amino-4-(6-(6-((5-chloropyridin-2-yl)methyl)-3,6-diazabicyclo[3.1.1]heptan-3-yl)pyridine-3-yl)-6-ethoxrazolo[1,5-a]ridine-3-carbonitrile (APS116)

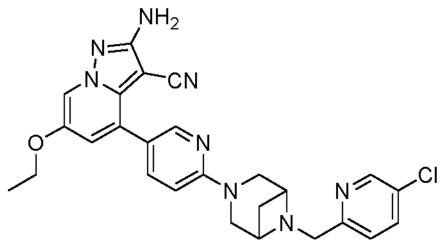

The preparation method according to Example 57 was performed with the difference that 5-chloro-6-methoxynicotinaldehyde was replaced with 5-chloropyridinecarbaldehyde to obtain the product 2-amino-4-(6-(6-((5-chloropyridin-2-yl)methyl)-3, 6-Diazabicyclo[3.1.1]heptan-3-yl)pyridin-3-yl)-6-ethoxypyrazolo[1,5-a]pyridine-3-carbonitrile (7.0 mg, yield 19%). $^1$HNMR (400MHz, DMSO-$d_6$) δ8.51 (d, 1H), 8.34 (d, 1H), 8.26 (d, 1H), 7.88 (dd, 1H), 7.78 (dd, 1H), 7.53 (d, 1H), 7.08 (d, 1H), 6.75 (d, 1H), 6.17 (s, 2H), 4.10 (d, 2H), 3.72-3.74 (m, 4H), 3.65 (s, 2H), 3.55 (brs, 2H), 2.52 (s, 1H), 2.08 (s, 3H), 2.60 (d, 1H).m/z=501[M+1]$^+$.

Example 69

2-amino-4-(6-(6-((5-fluoropyridin-2-yl)methyl)-3,6-diazabicyclo[3.1.1]heptan-3-yl)pyridine-3-yl)-6-ethoxypyrazolo[1,5-a]pyridine-3-carbonitrile (APS117)

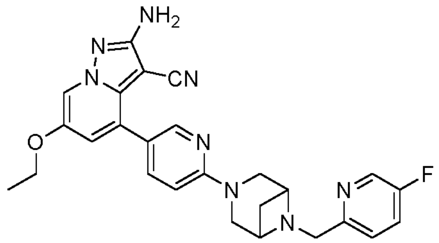

The preparation method according to Example 57 was performed with the difference that 5-chloro-6-methoxynicotinaldehyde was replaced with 5-fluoropyridinecarbaldehyde to obtain the product 2-amino-4-(6-(6-((5-fluoropyridin-2-yl)methyl)-3, 6-Diazabicyclo[3.1.1]heptan-3-yl)pyridin-3-yl)-6-ethoxypyrazolo[1,5-a]pyridine-3-carbonitrile (10.8 mg, yield 30%). $^1$HNMR (400MHz, DMSO-$d_6$) $\delta$ 8.46 (d, 1H), 8.34 (d, 1H), 8.26 (d, 1H), 7.78 (t, 1H), 7.67-7.72 (m, 1H), 7.53-7.56 (m, 1H), 7.08 (d, 1H), 6.76 (d, 1H), 6.17 (s, 2H), 4.06-4.11 (t, 2H), 3.71-3.79 (m, 4H), 3.64 (s, 2H), 3.55 (d, 2H), 2.08 (s, 1H), 1.62 (d, 1H), 1.37 (t, 3H).m/z=485[M+1]$^+$.

Example 70

2-amino-6-ethoxy-4-(6-(6-((6-methoxypyridin-2-yl)methyl)-3,6-diazabicyclo[3.1.1]heptane-3-yl)pyridin-3-yl)pyrazolo[1,5-a]pyridine-3-carbonitrile (APS118)

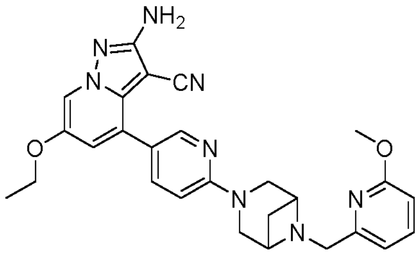

The preparation method according to Example 57 was performed with the difference that 5-chloro-6-methoxynicotinaldehyde was replaced with 6-methoxypyridinecarbaldehyde to obtain the product 2-amino-6-ethoxy-4-(6-(6-((6-methoxypyridin-2-yl)methyl)-3,6-diazabicyclo[3.1.1]heptan-3-yl)pyridin-3-yl)pyrazolo[1,5-a]pyridine-3-carbonitrile (28 mg, yield 77%). $^1$HNMR (400MHz, DMSO-$d_6$) $\delta$8.34 (d, 1H), 8.26 (d, 1H), 7.78 (dd, 1H), 7.65 (t, 1H), 7.02-7.08 (m, 2H), 6.77 (d, 1H), 6.66 (d, 1H), 6.17 (s, 2H), 4.06-4.11 (t, 2H), 3.77-3.83 (m, 7H), 3.60 (brs, 4H), 2.54-2.57 (m, 1H), 1.63 (d, 1H), 1.35 (t, 3H). m/z=497[M+1]$^+$.

Example 71

2-amino-6-(2-fluoroethoxy)-4-(6-(6-((6-methoxypyridin-3-yl)methyl)-3,6-diazabicyclo[3.1.1]heptan-3-yl)pyridin-3-yl)pyrazolo[1,5-a]pyridine-3-carbonitrile (APS119)

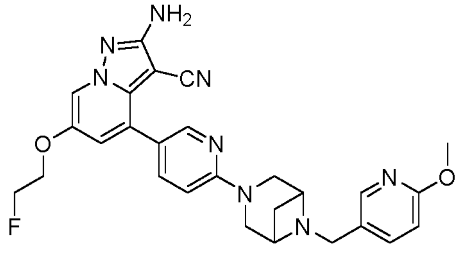

The preparation method according to Example 47 was performed with the difference that iodoethane was replaced with 1-fluoro-2-iodoethane to obtain 2-amino-6-(2-fluoroethoxy)-4-(6-(6-((6-methoxypyridin-3-yl)methyl)-3,6-diazabicyclo[3.1 .1]Heptan-3-yl)pyridin-3-yl)pyrazolo[1,5-a]pyridine-3-carbonitrile (5.0 mg, yield 13%). $^1$HNMR (400MHz, DMSO-$d_6$)8.36 (dd, 2H), 8.08 (d, 1H), 7.80 (dd, 1H), 7.68 (dd, 1H), 7.14 (d, 1H), 6.78 (d, 2H), 6.20 (s, 2H), 4.84 (t, 1H), 4.72 (t, 1H), 4.38 (t, 1H), 4.31 (t, 1H), 3.82 (s, 3H), 3.64-3.74 (m, 4H), 3.50-3.55 (m, 4H), 1.60 (d, 1H).m/z=515$[M+1]^+$.

Example 72

2-amino-6-(2-isopropoxyethoxy)-4-(6-(6-((6-methoxypyridin-3-yl)methyl)-3,6-diazabicyclo [3.1.1]heptan-3-yl)pyridin-3-yl)pyrazolo[1,5-a]pyridine-3-carbonitrile (APS120)

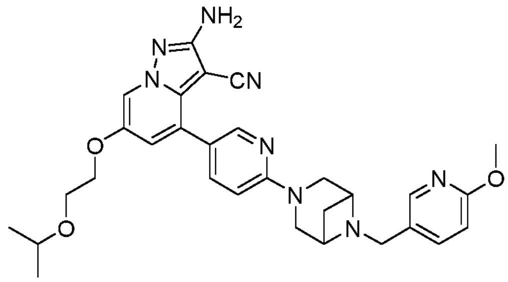

The preparation method according to Example 47 was performed with the difference that iodoethane was replaced with 2-(2-bromoethoxy)propane to obtain 2-amino-6-(2-isopropoxyethoxy)-4-(6-(6-((6-methoxypyridin-3-yl)methyl)-3,6-diazabicyclo [3.1.1]heptan-3-yl)pyridin-3-yl)pyrazolo[1,5-a]pyridine-3-carbonitrile (32.6 mg, yield 80%). $^1$HNMR (400MHz, $CDCl_3$) $\delta$ 8.39 (s, 1H), 8.13 (s, 1H), 7.93 (s, 1H), 7.80 (d, 1H), 7.08 (d, 1H), 6.68-6.76 (m, 2H), 4.44 (d, 1H), 4.14 (d, 2H), 3.86 (s, 4H), 3.80-3.82 (m, 3H), 3.64-3.76 (m, 3H), 1.23-1.25 (m, 10H).m/z=555$[M+1]^+$.

With reference to the methods of the above examples, the following compounds were also prepared. The characterization data of these compounds together with the above-mentioned compounds are summarized in Table 1 below:

Table 1

| Compound No. | MS $[M+1]^+$ | Compound No. | MS $[M+1]^+$ |
|---|---|---|---|
| APS007 | 532 | APS038 | 503 |
| APS008 | 548 | APS039 | 535 |
| APS009 | 544 | APS040 | 568 |
| APS010 | 548 | APS041 | 534 |
| APS011 | 534 | APS042 | 555 |
| APS012 | 545 | APS043 | 535 |
| APS013 | 486 | APS044 | 539 |
| APS014 | 509 | APS045 | 590 |
| APS015 | 508 | APS046 | 533 |
| APS016 | 489 | APS047 | 557 |
| APS017 | 533 | APS048 | 507 |
| APS018 | 534 | APS049 | 591 |
| APS019 | 508 | APS050 | 606 |
| APS020 | 526 | APS051 | 579 |
| APS021 | 525 | APS052 | 547 |
| APS022 | 505 | APS053 | 547 |
| APS023 | 494 | APS054 | 564 |

(continued)

| Compound No. | MS $[M+1]^+$ | Compound No. | MS $[M+1]^+$ |
|---|---|---|---|
| APS024 | 526 | APS055 | 565 |
| APS025 | 505 | APS056 | 579 |
| APS026 | 520 | APS057 | 641 |
| APS027 | 524 | APS058 | 604 |
| APS028 | 516 | APS059 | 572 |
| APS029 | 527 | APS060 | 520 |
| APS030 | 521 | APS061 | 519 |
| APS031 | 504 | APS062 | 540 |
| APS032 | 521 | APS063 | 520 |
| APS033 | 521 | APS064 | 492 |
| APS034 | 533 | APS065 | 543 |
| APS035 | 491 | APS066 | 534 |
| APS036 | 476 | APS067 | 548 |
| APS037 | 518 | APS068 | 521 |

Kinase activity test

Transfection recombinant gene (RET) is a confirmed proto-oncogene. The single transmembrane receptor tyrosine kinase it encodes is necessary for the development, maturation and maintenance of many tissues and cell types. Under normal conditions, the binding of glial cell line-derived neurotrophic factor (GDNF) family ligands to RET on the cell surface results in the dimerization and autophosphorylation of tyrosine residues in the cell. This in turn leads to the activation of downstream RAS-MAPK, PI3K-AKT and phospholipase Cy (PLCy) pathways, and increases cell survival and proliferation. Examples of mutations that activate RET include C634W, M918T and gatekeeper mutations, V804L and V804M.

This test combined peptide substrates and a single proprietary monoclonal antibody with HTRF technology, which was a highly sensitive and stable technology for detecting molecular interactions of proteins. The enzyme phosphorylates the substrate, then the Eu-labeled antibody binds to the phosphorylated substrate, and streptavidin-XL665 binds to all substrates. The TR-FRET signal was generated by the HTRF principle. Once the inhibitor (test compound) was added, a weaker TR-FRET signal is obtained. Based on this, the inhibitory effect was evaluated.

5.1. Reagents and Consumables

| Materials and reagents | Supplier | Model |
|---|---|---|
| HTRF KinEASE-TK Reagent test kit | Cisbio | 62TK0PEC |
| Ret wt | Invitrogen | PV3082 |
| RET (V804L), activated | Signalchem | R02-12BG-10 |
| RET (V804M) | Signalchem | R02-12GG |
| DMSO | Sigma | D8418-1L |
| ATP | Sigma | A7699 |
| DTT | Sigma | D0632 |
| CEP-32496 | MCE | HY-15200 |
| Staurosporine | MCE | HY-15141 |

(continued)

| Materials and reagents | Supplier | Model |
|---|---|---|
| Plate oscillator | Thermo | 4625-1CECN/THZ Q |
| Centrifuge | Eppendorf | 5810R |
| Envision 2104 Multi-marker plate reader | PerkinElmer | 2104-0010 |
| Echo | Labcyte | 550 |

5.2 Preparation of solution

CEP-32496 was diluted 3-fold gradiently with DMSO from 10 mM and 1 mM to a total of 10 concentrations respectively.

The other compounds were diluted 3-fold gradiently with DMSO from the 10 mM stock solution to a total of 10 concentrations respectively.

1000× positive control (1mM CEP-32496 and 0.2mM Staurosporine) and 1000× negative control (100% DMSO) were prepared.

Shaking on the plate oscillator for 5 minutes.

5.3 Preparation of 1x Kinase Buffer

Adding 4 volumes of distilled water to 1 volume of enzyme buffer 5X; 5mM $MgCl_2$; 1mM DTT.

5.4 Screening method

a) Transferring 10nl of the compound dilution (prepared in 5.2) to each well of the test plate (784075, Greiner);
b) centrifuging the compound plate for 1 minute at 1000 g.
c) sealing the test board.
d) preparing 5X Ret wt (0.2ng/μl) and 5X Ret V804L (0.5ng/μl)/5X Ret V804M (0.5ng/μl) in 1x kinase buffer respectively.
e) adding 2μl of 5X Ret wt or 2μl of Ret V804L/RET V804M to a 384-well test plate (784075, Greiner).
f) adding 4μl of 1x kinase buffer to each well of the test plate, centrifuging the sample plate at 1000g for 30 seconds, and placing it at room temperature for 10 minutes.
g) preparing a solution of 5x TK-substrate-biotin (5μM) in kinase buffer and 5x ATP (50μM) in kinase buffer respectively.
h) starting the reaction by adding 2μl STK-substrate-biotin and 2μl ATP (prepared in the step g).
i) centrifuging the sample plate for 30 seconds at 1000 g. Sealing the test board and placing it at room temperature for 30 minutes.
j) preparing 4X Sa-XL 665 (250 nM) in HTRF detection buffer.
k) adding 5 μl of Sa-XL 665 and 5 μl of TK-antibody-Cryptate (prepared in the step i) to each well of the test plate.
l) centrifuging at 1000g for 30 seconds and placing it at room temperature for 1 hour.
m) reading the fluorescence signal values at 615nm (Cryptate) and 665nm (XL665) on the Envision 2104 plate reader.

5.5 Data analysis

Calculating the ratio of each hole (665nm/615nm).

The inhibition rate% was calculated as follows:

Inhibition rate% = [1- (fluorescence signal value of test compound - fluorescence signal value of positive control)/(average ratio of negative control - average ratio of positive control)]*100%

Ratio: generated from the measured fluorescence signal value

The average ratio of the positive control is the average ratio of the positive control (200nM AT13148) in the sample plate;

The average ratio of negative controls is the average ratio of negative controls (0.1% DMSO) in the sample plate.

GraphPad 6.0 was used to fit the logarithm of the% inhibition rate and the compound concentration into a nonlinear regression (dose response - variable slope) to calculate the $IC_{50}$ value.

Table 2

| Compound No. | $IC_{50}$ (nM) | |
|---|---|---|
| | RET wt | RET V804L |
| CEP-32496 | 0.7009 | 110.6 |
| APS001 | 0.1480 | 0.2610 |
| APS002 | 0.4469 | 0.2917 |
| APS069 | 0.3247 | 0.1243 |
| APS070 | 0.4177 | 0.0759 |

Cell inhibitory activity test

1. preparing 90μl of cell suspension in a 96-well plate with 4000 cells/well. Pre-incubating the culture plate in an incubator for 24 hours (37°C, 5% $CO_2$).
2. adding 10 μl of test compounds of different concentrations to each well of cells, and each compound has multiple wells.
3. incubating the culture plate in the incubator for 72 hours.
4. adding 10μl of CCK8 solution to each well (be careful not to generate bubbles in the well, otherwise they would affect the reading of the OD value).
5. incubating the culture plate in the incubator for 1-2 hours.
6. measuring the absorbance at 450nm with a microplate reader.
7. according to the compound concentration and absorbance value, GraphpadPrism software was used to calculate the $IC_{50}$ of each compound.

The calculation results are shown in the following table:

Table 3

| Compound | RET Enzyme (wild type) $IC_{50}$ (nM) | RET Enzyme (V804M) $IC_{50}$ (nM) | TT Cell $IC_{50}$ (nM) |
|---|---|---|---|
| APS014 | 1.0 | N/A | 44.6 |
| APS015 | 1.7 | N/A | 27.7 |
| APS016 | N/A | N/A | 31.1 |
| APS019 | <1.0 | N/A | 7.8 |
| APS020 | 1.1 | N/A | 14.3 |
| APS021 | N/A | N/A | 119.5 |
| APS022 | 2.2 | N/A | 45.5 |
| APS024 | 1.0 | N/A | 7.1 |
| APS025 | 1.4 | N/A | 38.2 |
| APS026 | 1.4 | N/A | 18.1 |
| APS027 | 2.4 | N/A | 85.7 |
| APS030 | N/A | N/A | 57.6 |
| APS034 | N/A | N/A | 32.8 |
| APS035 | 1.7 | N/A | 65.4 |

(continued)

| Compound | RET Enzyme (wild type) $IC_{50}$ (nM) | RET Enzyme (V804M) $IC_{50}$ (nM) | TT Cell $IC_{50}$ (nM) |
|---|---|---|---|
| APS038 | 1.0 | N/A | 37.7 |
| APS041 | N/A | N/A | 32.8 |
| APS062 | N/A | N/A | 34.1 |
| APS064 | N/A | N/A | 150.4 |
| APS068 | N/A | N/A | 11.3 |
| APS069 | <1.0 | N/A | 28.8 |
| APS070 | <1.0 | N/A | 15.6 |
| APS071 | 64.6 | N/A | N/A |
| APS072 | 78.3 | N/A | N/A |
| APS073 | N/A | N/A | 143.5 |
| APS074 | N/A | N/A | 25.2 |
| APS075 | N/A | N/A | 121.7 |
| APS076 | N/A | N/A | 96.4 |
| APS077 | N/A | N/A | 58.3 |
| APS078 | N/A | N/A | 57.1 |
| APS079 | N/A | N/A | 119.6 |
| APS083 | 212 | N/A | N/A |
| APS084 | N/A | N/A | 342 |
| APS085 | 865 | N/A | N/A |
| APS086 | 234 | N/A | N/A |
| APS087 | <1.0 | <1.0 | <1.0 |
| APS088 | N/A | N/A | 264.8 |
| APS089 | 1.8 | 1.6 | 18.6 |
| APS090 | 54.0 | 486.1 | 31.4 |
| APS091 | N/A | 957.1 | N/A |
| APS092 | 4.3 | 2.7 | 60 |
| APS093 | 2891 | 1553 | N/A |
| APS094 | 942 | 492.7 | N/A |
| APS095 | <1.0 | <1.0 | 38.6 |
| APS096 | 122.1 | 186.4 | N/A |
| APS097 | <1.0 | <1.0 | 75.5 |
| APS098 | 143.5 | 85.6 | N/A |
| APS099 | N/A | N/A | 16.2 |
| APS100 | N/A | N/A | 22.4 |
| APS103 | 261.4 | 154.6 | N/A |
| APS104 | 6.9 | 14.0 | N/A |
| APS105 | <1.0 | <1.0 | 28.1 |
| APS106 | 744.7 | 971.1 | N/A |

(continued)

| Compound | RET Enzyme (wild type) $IC_{50}$ (nM) | RET Enzyme (V804M) $IC_{50}$ (nM) | TT Cell $IC_{50}$ (nM) |
|---|---|---|---|
| APS107 | <1.0 | <1.0 | 16.3 |
| APS108 | <1.0 | <1.0 | 10.7 |
| APS109 | <1.0 | <1.0 | 57.9 |
| APS110 | N/A | N/A | 198.1 |
| APS111 | N/A | N/A | 6.4 |
| APS112 | 18.9 | N/A | N/A |
| APS113 | <1.0 | <1.0 | 83.5 |
| APS114 | 2.0 | <1.0 | N/A |
| APS115 | 45.9 | 8.8 | N/A |
| APS116 | N/A | N/A | 54.8 |
| APS119 | <1.0 | <1.0 | 6.6 |
| APS120 | N/A | N/A | 16.9 |

"N/A" in the table above means that the activity was not tested.

As shown in the above table, the example compounds of the present disclosure have improved inhibitory activity against RET wild-type, mutant and/or fusion-type. Especially the compounds such as APS014, APS015, APS019, APS020, APS022, APS024, APS025, APS026, APS027, APS035, APS038, APS068, APS069, APS070, APS087, APS089, APS092, APS095, APS097, APS099, APS100, APS104, APS105, APS107, APS108, APS109, APS111, APS113, APS114, APS119 and APS120, etc. have achieved excellent activity.

In the foregoing, the embodiments of the present disclosure have been illustrated. Nevertheless, the present disclosure should not be confined to the above-mentioned embodiments. Any modifications, equivalent replacements, improvements, etc. made within the spirit and principle of the present disclosure shall be comprised in the protection scope of the present disclosure.

## Claims

1. A compound represented by the following formula I or a stereoisomer, racemate, tautomer, isotope labelled derivative, nitrogen oxide, pharmaceutically acceptable salt or solvate thereof:

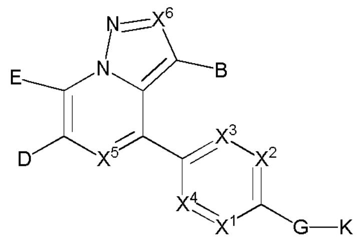

I

wherein, $X^1$, $X^2$, $X^3$, $X^4$, $X^5$ and $X^6$ are the same or different, independently selected from $CR^1$, -C-A or N, wherein each $R^1$ is the same or different, independently selected from H, halogen, CN, OH and the following groups which are unsubstituted or optionally substituted with one, two or more $R^a$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, - $NHC(O)R^2$, $-C(O)R^4$, $-OCR^5$, $-S(O)_2R^6$ and $OS(O)_2R^7$; A is selected from H, halogen, CN, OH and the following groups which are unsubstituted or optionally substituted with one, two or more $R^b$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, - $C(O)R^4$, $-OCR^5$, $-S(O)_2R^6$, $-OS(O)_2R^7$ and

$-NHC(O)R^2$;

B is selected from H, halogen, CN, OH and the following groups which are unsubstituted or optionally substituted by one, two or more $R^c$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $NR^2R^3$, $-C(O)R^4$, $-NHC(O)R^4$, $-OCR^5$, $-S(O)_2R^6$ and $OS(O)_2R^7$;

D is selected from the following groups which are unsubstituted or optionally substituted with one, two or more $R^d$: $C_{3-40}$ cycloalkyl, $C_{3-40}$ cycloalkenyl, $C_{3-40}$ cycloalkynyl, $C_{1-40}$ alkyloxy, $C_{2-40}$ alkenyloxy, $C_{2-40}$ alkynyloxy, $C_{6-20}$ aryl, 5-20 membered heteroaryl and 3-20 membered heterocyclyl;

E is selected from H, halogen, CN, OH and the following groups which are unsubstituted or optionally substituted by one, two or more $R^e$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy or 3-20 membered heterocyclyloxy;

G is selected from the following groups which are unsubstituted or optionally substituted by one, two or more $R^f$: $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy or 3-20 membered heterocyclyloxy;

K is selected from H, halogen, CN, OH, the following groups which are unsubstituted or optionally substituted with one, two or more $R^g$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy , $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy group, $NR^2R^3$, $-C(O)R^4$, $-OCR^5$, $-S(O)_2R^6$, $OS(O)_2R^7$;

each $R^2$ is the same or different and is independently selected H and the following groups which are unsubstituted or optionally substituted by OH and/or $NH_2$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $-C(O)R^4$ and $-S(O)_2R^6$;

each $R^3$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $-C(O)R^4$ and $-S(O)_2R^6$;

or, $R^2$ and $R^3$ together with the nitrogen atom to which they are attached form a 5-20 membered heteroaryl or a 3-20 membered heterocyclyl;

each $R^4$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy and $NR^2R^3$;

each $R^5$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkylcarbonyl, $C_{2-40}$ alkenylcarbonyl, $C_{2-40}$ alkynylcarbonyl, $C_{3-40}$ cycloalkylcarbonyl, $C_{3-40}$ cycloalkenylcarbonyl, $C_{3-40}$ cycloalkynylcarbonyl, $C_{6-20}$ arylcarbonyl, 5-20 membered heteroarylcarbonyl and 3-20 membered heterocyclic carbonyl;

each $R^6$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy and $NR^2R^3$;

each $R^7$ is the same or different and is independently selected from H, $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl and 3-20 membered heterocyclyl;

each $R^a$, $R^b$, $R^c$, $R^d$, $R^e$, $R^f$ and $R^g$ is the same or different, and is independently selected from halogen, CN, OH, SH, oxo (=O), $NO_2$, the following groups which are unsubstituted or optionally substituted by one, two or more of $R^h$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl , $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy, $NR^2R^3$,$-C(O)R^4$, $-OCR^5$, $-S(O)_2R^6$ and $OS(O)_2R^7$;

each $R^h$ is the same or different, and is independently selected from halogen, CN, OH, SH, oxo (=O), $NO_2$, and the following groups which are unsubstituted or optionally substituted by one, two or more $R^j$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl, 3-20 membered heterocyclyl, $C_{1-40}$ alkyloxy, $C_{2-40}$ alkenyloxy, $C_{2-40}$ alkynyloxy, $C_{3-40}$ cycloalkyloxy,

$C_{3-40}$ cycloalkenyloxy, $C_{3-40}$ cycloalkynyloxy, $C_{6-20}$ aryloxy, 5-20 membered heteroaryloxy, 3-20 membered heterocyclyloxy, $NR^2R^3$, $-C(O)R^4$, $-OCR^5$, $-S(O)_2R^6$ and $OS(O)_2R^7$; or, where the different positions of the cyclic group (including but not limited to $C_{3-40}$ cycloalkyl, $C_{3-40}$ cycloalkenyl, $C_{3-40}$ cycloalkynyl, 3-20 membered heterocyclyl, etc.) are substituted by two or more substituents, two of the substituents may also form a bridged ring toghther with the cyclic group, wherein a bridge atom other than the bridgehead atoms in the bridged ring may contain 1, 2, 3, 4 or 5 divalent groups selected from $CH_2$, O and NH;

each $R^1$ is the same or different and is independently selected from halogen, CN, OH, SH, oxo (=O), $NO_2$, the following groups which are unsubstituted or optionally substituted with one, two or more $R^h$: $C_{1-40}$ alkyl, $C_{2-40}$ alkenyl, $C_{2-40}$ alkynyl, $C_{3-40}$ cycloalkyl, $C_{3-40}$ cycloalkenyl, $C_{3-40}$ cycloalkynyl, $C_{6-20}$ aryl, 5-20 membered heteroaryl , 3-20 membered heterocyclyl, $C_{1-40}$ alkyloxy, $C_{2-40}$ alkenyloxy, $C_{2-40}$ alkynyloxy, $C_{3-40}$ cycloalkyloxy, $C_{3-40}$ cycloalkenyloxy, $C_{3-40}$ cycloalkynyloxy, $C_{6-20}$ aryloxy, 5-20 membered heteroaryloxy, 3-20 membered heterocyclyloxy, $NR^2R^3$, $-C(O)R^4$, $-OCR^5$, $-S(O)_2R^6$ and $OS(O)_2R^7$;

or, where the different positions of the cyclic group (including but not limited to $C_{3-40}$ cycloalkyl, $C_{3-40}$ cycloalkenyl, $C_{3-40}$ cycloalkynyl, 3-20 membered heterocyclyl, etc.) are substituted by two or more substituents, two of the substituents may also form a bridged ring together with the cyclic group, wherein a bridge atom in the bridged ring other than the bridgehead atoms may contain 1, 2, 3, 4 or 5 divalent groups selected from $CH_2$, O and NH;

or, where an atom (such as a carbon atom) is substituted by two or more substituents, two of the substituents may also form a cyclic group (including but not limited to $C_{3-40}$ cycloalkyl, $C_{3-40}$ cycloalkenyl, $C_{3-40}$ cycloalkynyl, 3-20 membered heterocyclyl, etc.) together with the atom to which the two

**2.** The compound according to claim 1, wherein the compound represented by the formula I is selected from the following compound represented by formula I',

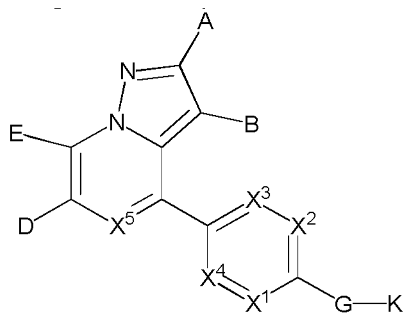

I'

wherein, $X^1$, $X^2$, $X^3$, $X^4$, $X^5$, A, B, D, E, G, and K are as defined in claim 1 respectively.

**3.** The compound according to claim 1 or 2, wherein:

$X^6$ is selected from -C-A or N, wherein A may be selected from H and the following groups which are unsubstituted or optionally substituted with one, two or more $R^b$: $NH_2$, $C_{1-6}$ alkyl, $-NH(C_{1-6}\ alkyl)_2$, OH, F, $-NHC(O)C_{1-6}$ alkyl, $-NHC_{1-6}$ alkyl, $C_{1-6}$ alkyloxy, $-NHC_{1-6}$ alkyl-OH;

each $R^b$ is the same or different, and is independently selected from $C_{1-6}$ alkyl and $C_{1-6}$ alkoxy;

B can be selected from H, CN, $-CONH_2$, and $C_{1-6}$ alkyl which is unsubstituted or optionally substituted with one, two or more $R^c$;

each $R^c$ is the same or different, and is independently selected from halogen and $C_{1-6}$ alkyl;

D can be selected from the following groups which are unsubstituted or optionally substituted with one, two or more $R^d$: $C_{1-6}$ alkyloxy or 5-14 membered heteroaryl, wherein $R^d$ is selected from $C_{1-6}$ alkyl group and 3-10 membered heterocyclyl which are unsubstituted or optionally substituted with one or more groups seleced from the following groups: oxo, halogen, OH, $-N(C_{1-6}\ alkyl)_2$ or $-S(O)_2-C_{1-6}$ alkyl;

E is selected from H and the following groups which are unsubstituted or optionally substituted by one, two or more $R^e$: $C_{1-6}$ alkyl and $C_{1-6}$ alkoxy;

each $R^e$ is the same or different, and is independently selected from OH, F, and $C_{1-6}$ alkyl groups;

G is selected from 3-10 membered heterocyclyl which is unsubstituted or optionally substituted by an oxo group, such as a groupd selected from piperazinyl and piperidinyl which are unsubstituted or optionally substituted by an oxo group or substituted by $C_{1-6}$ alkyl; or, where the meta position of the heterocyclyl is substituted by two substituents, the substituents may form a bridged ring together with the heterocyclyl, wherein a bridge atom other than the bridgehead atoms in the bridged ring may include 1, 2, 3, 4 or 5 divalent groups selected from $CH_2$;

K is selected from the following groups which are unsubstituted or optionally substituted with one, two or more $R^g$: $C_{1-6}$ alkyl and -C(O)$R^4$, wherein $R^g$ is selected from oxo, OH and the following groups which are unsubstituted or optionally substituted by one, two or more $R^h$: $C_{1-6}$ alkyl, $C_{1-6}$ alkoxy, $C_{3-10}$ cycloalkyl, $C_{6-14}$ aryl, 5-14 membered heteroaryl, -$SO_2$-$C_{6-14}$ aryl;
each $R^4$ is the same or different, and is independently selected from H, $C_{1-6}$ alkyl, $C_{2-6}$ alkenyl, $C_{2-6}$ alkynyl, $C_{3-10}$ cycloalkyl, $C_{3-10}$ cycloalkenyl, $C_{3-10}$ cycloalkynyl, $C_{6-14}$ aryl, 5-14 membered heteroaryl, 3-10 membered heterocyclyl, $C_{1-6}$ alkyloxy, $C_{2-6}$ alkenyloxy, $C_{2-6}$ alkynyloxy, $C_{3-10}$ cycloalkyloxy, $C_{3-10}$ cycloalkenyloxy, $C_{3-10}$ cycloalkynyloxy, $C_{6-14}$ aryloxy, 5-14 membered heteroaryloxy , 3-10 membered heterocyclyloxy, -N($C_{1-6}$ alkyl)$_2$;
$R^h$ is selected from halogen, $C_{1-6}$ alkoxy, $NH_2$, -N($C_{1-6}$ alkyl)$_2$,-NH$C_{6-14}$ aryl, -NH$C_{1-6}$ alkyl;
or, where an atom (such as a carbon atom) in K is substituted by two or more $R^g$, the two $R^g$ can also form a ring (including but not limited to $C_{3-10}$ cycloalkyl, $C_{3-10}$ cycloalkenyl, $C_{3-10}$ cycloalkynyl, 3-10 membered heterocyclyl, etc.) together with the atom to which they are attached.

**4.** The compound according to the compound of any one of claims 1-3, wherein:
the compound of formula I is selected from the following compounds:

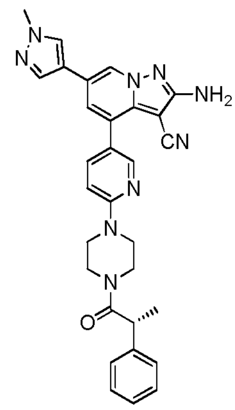

APS007

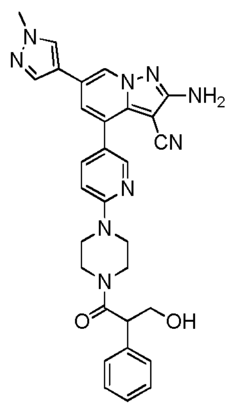

APS008

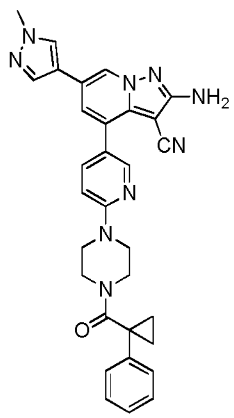

APS009

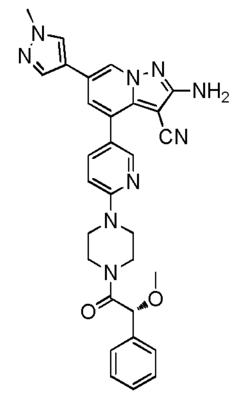

APS010

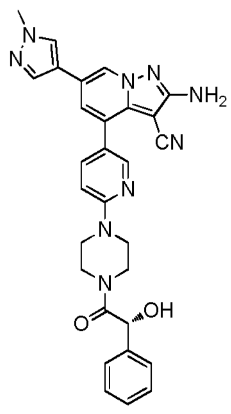

APS011

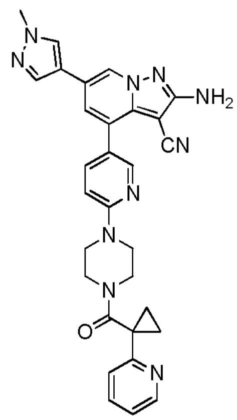

APS012

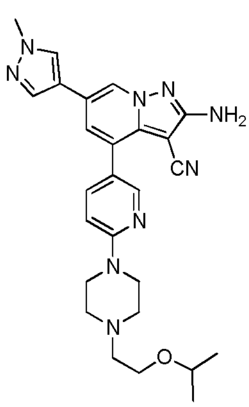

APS013

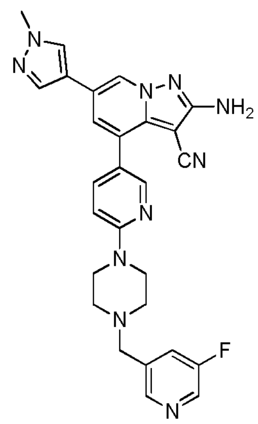

APS014

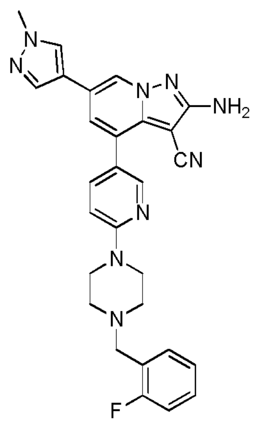

APS015

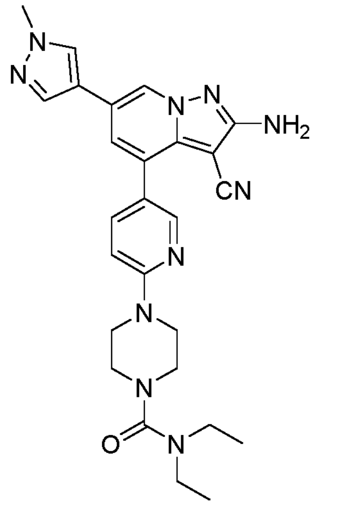

APS016

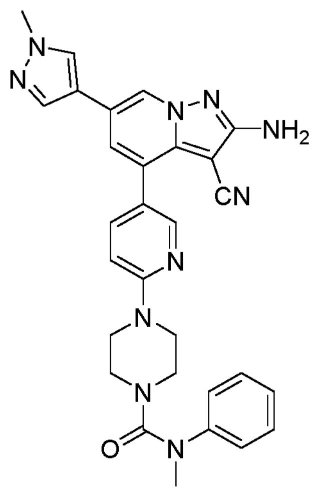

APS017

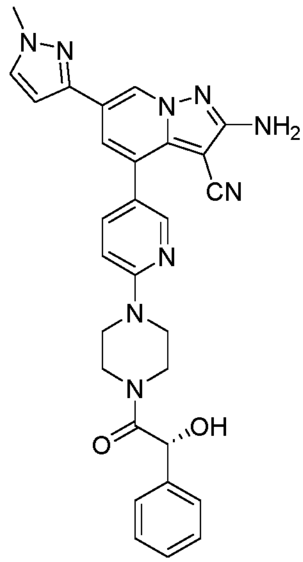

APS018

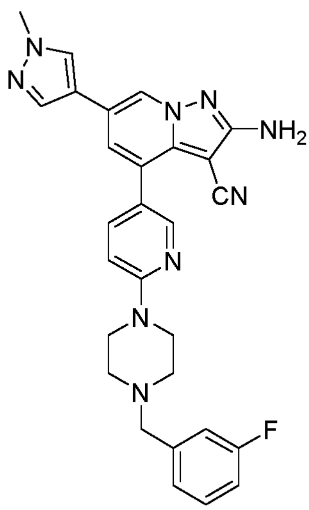

APS019

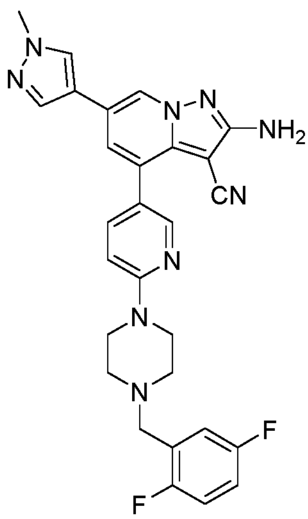

APS020

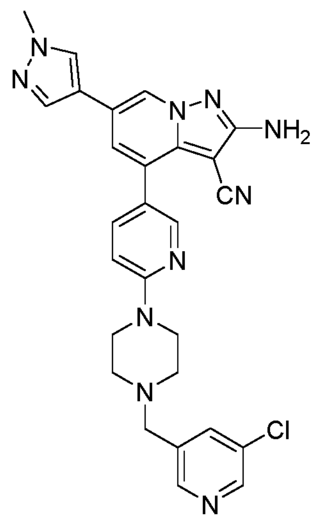

APS021

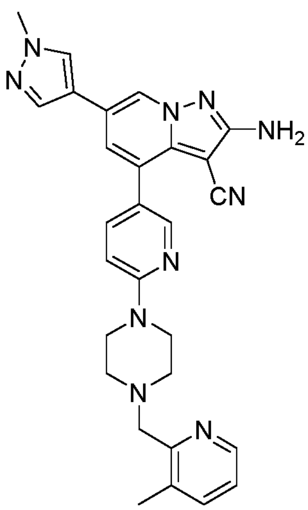

APS022

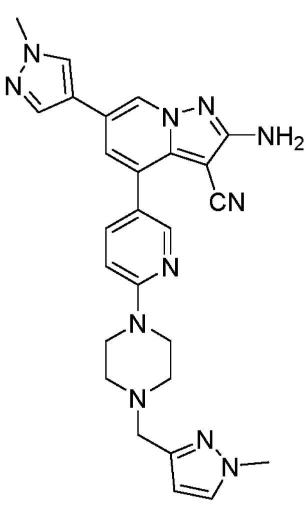

APS023

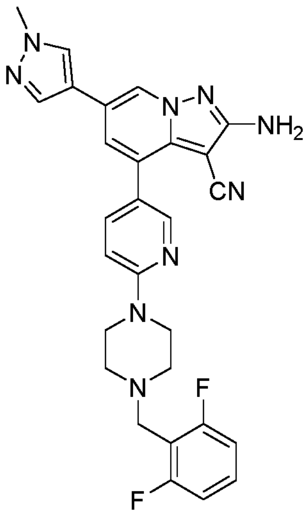

APS024

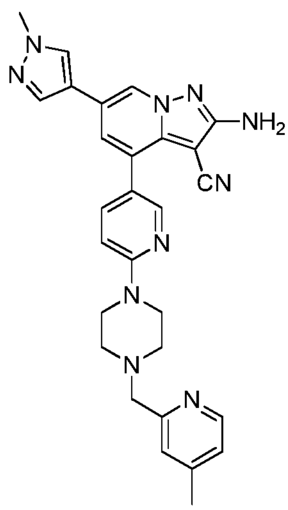

APS025

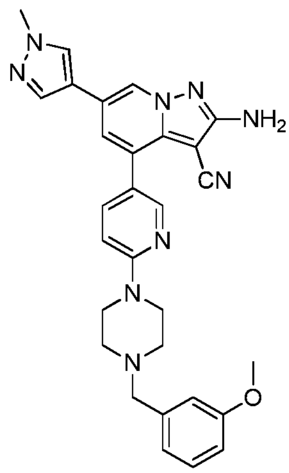

APS026

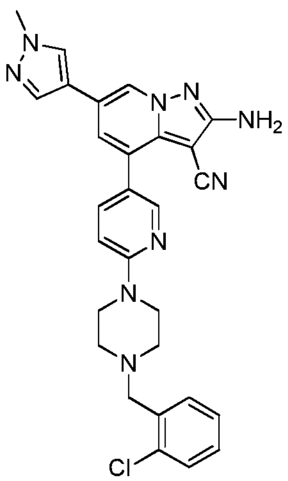

APS027

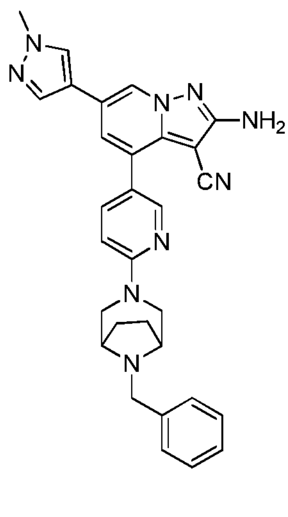

APS028

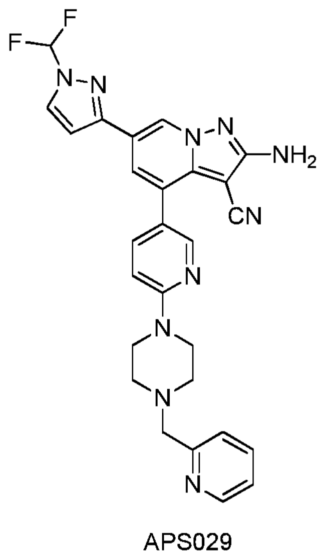

APS029

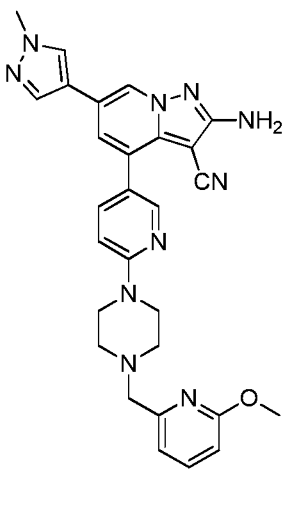

APS030

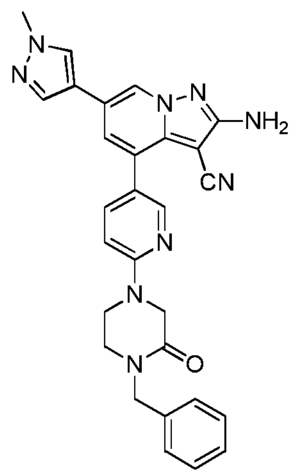

APS031

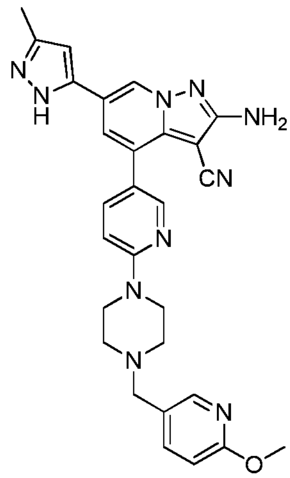

APS032

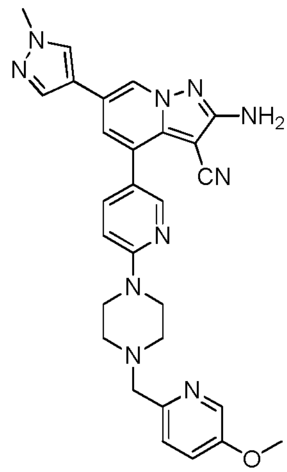

APS033

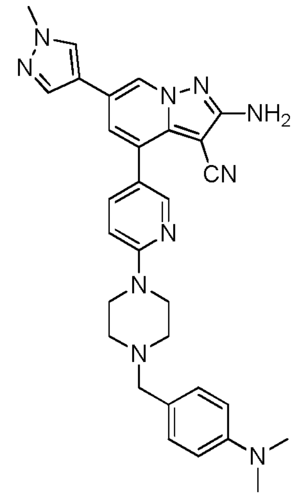

APS034

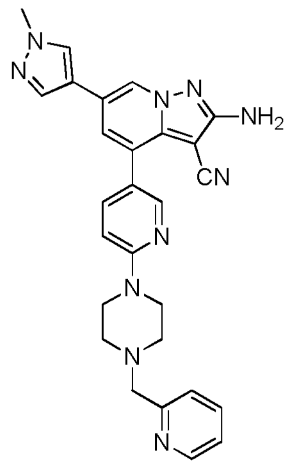

APS035

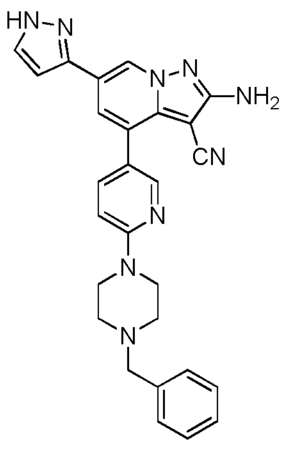

APS036

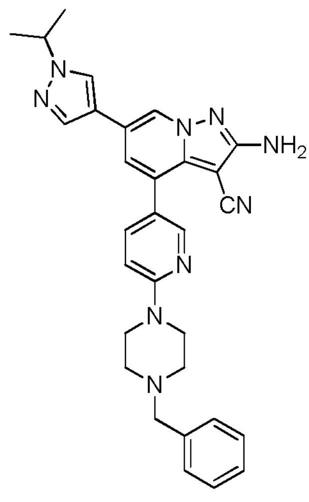

APS037

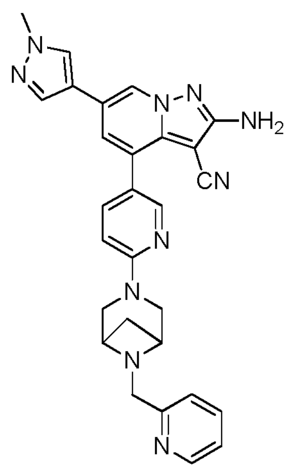

APS038

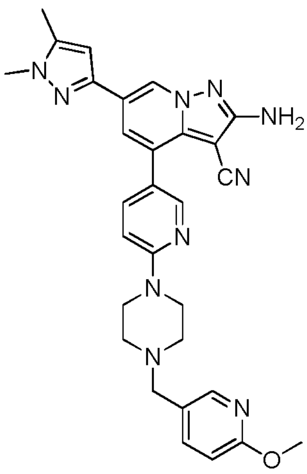

APS039

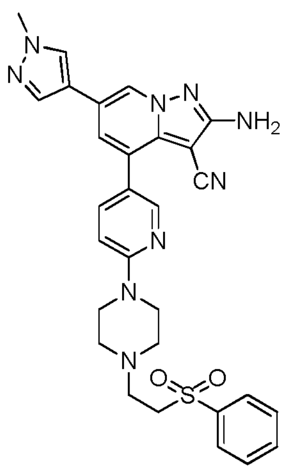

APS040

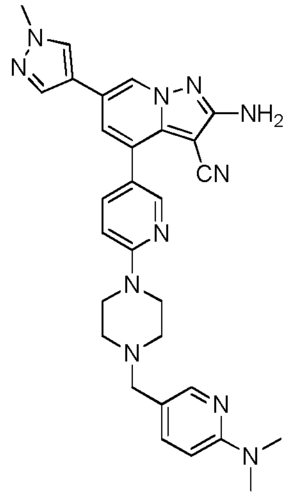

APS041

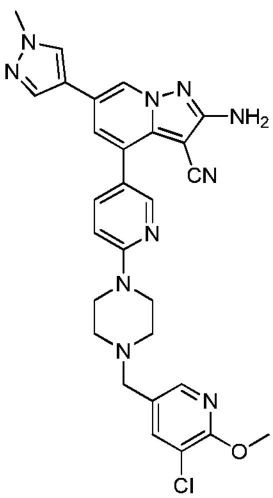

APS042

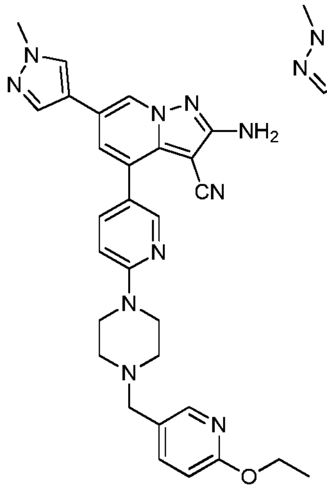

APS043

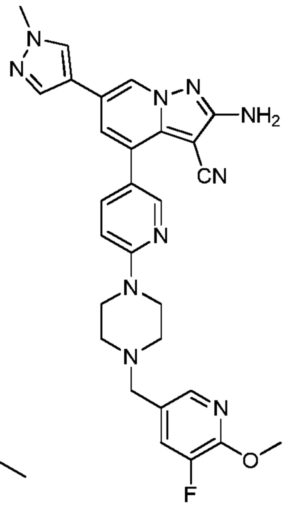

APS044

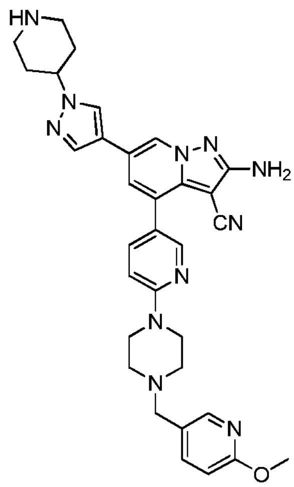

APS045

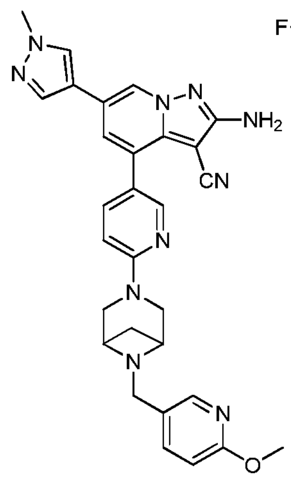

APS046

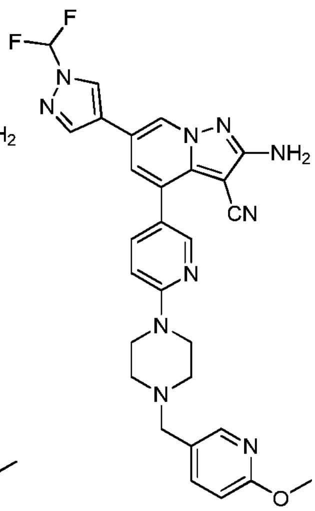

APS047

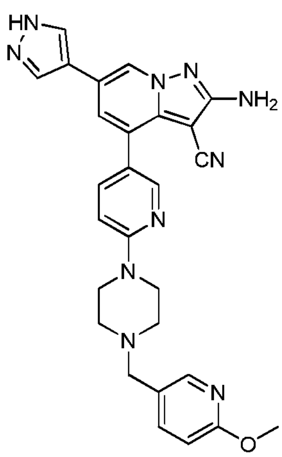

APS048

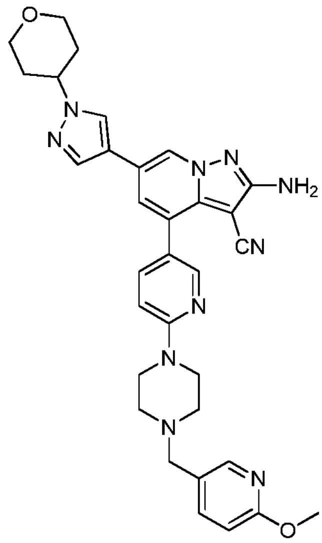

APS049

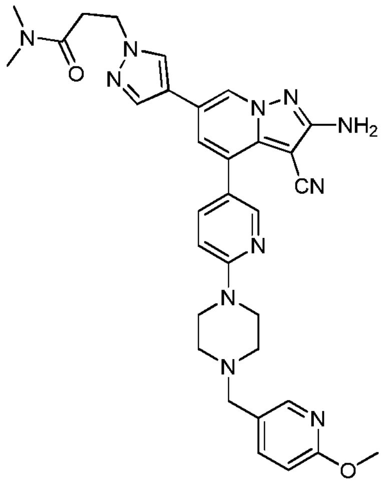

APS050

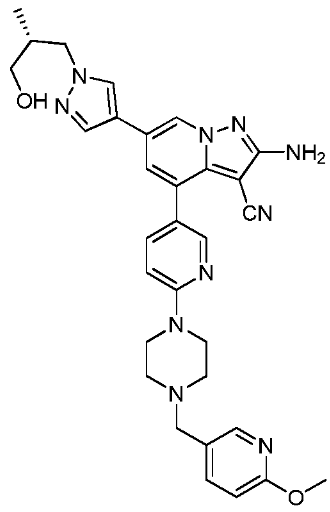

APS051

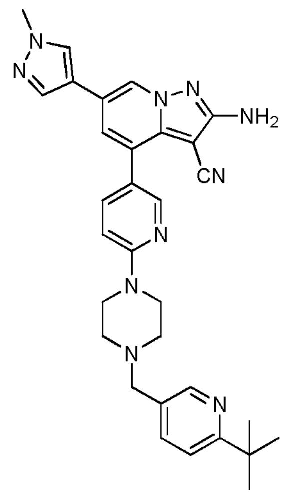

APS052

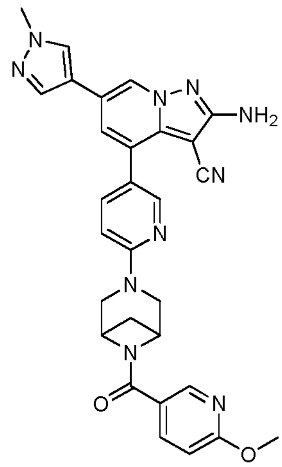

APS053

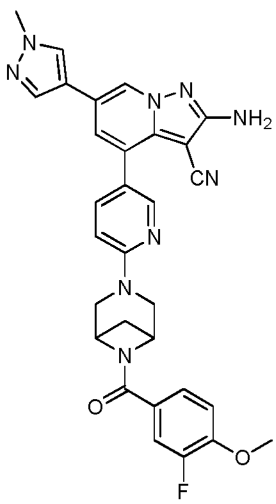

APS054

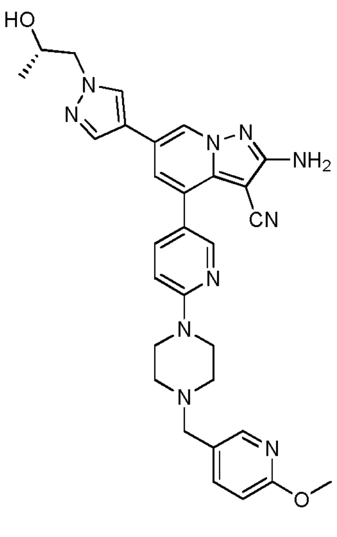

APS055

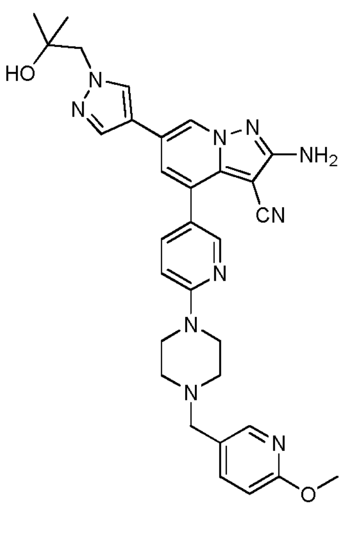

APS056

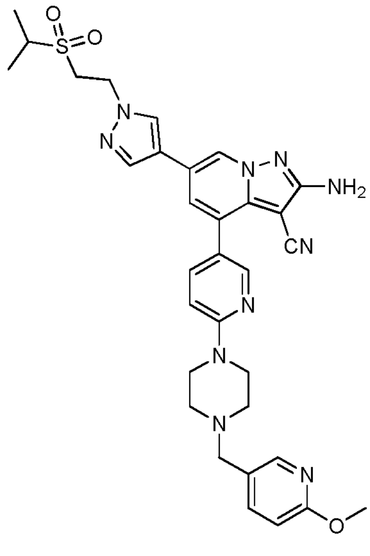

APS057

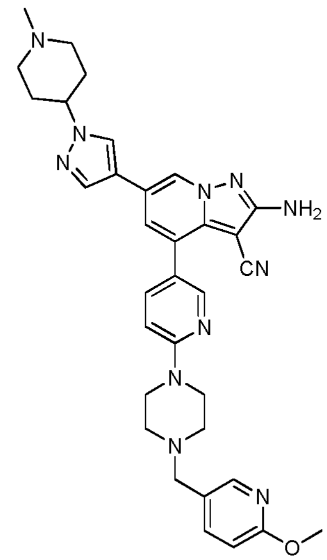

APS058

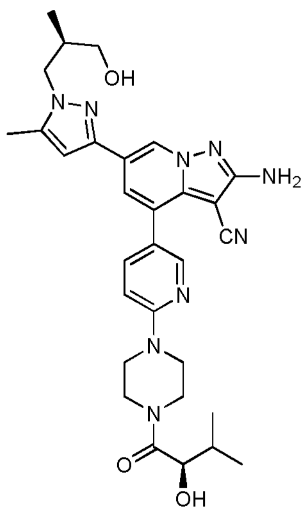

APS059

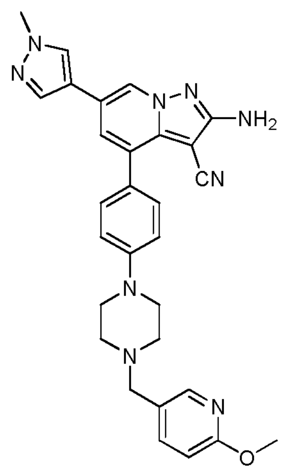

APS060

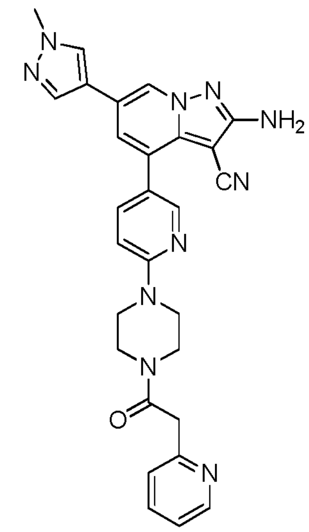

APS061

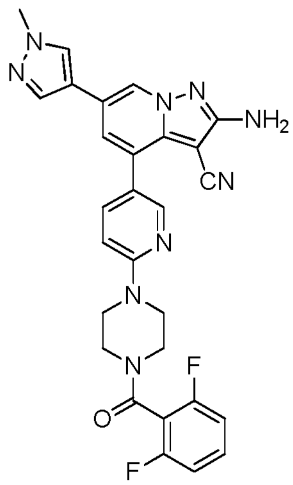

APS062

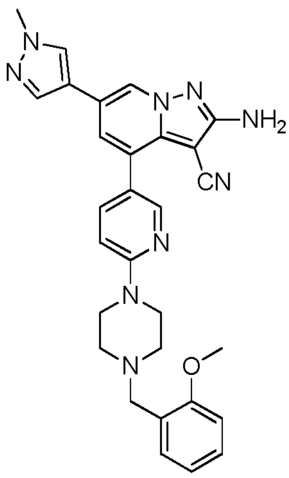

APS063

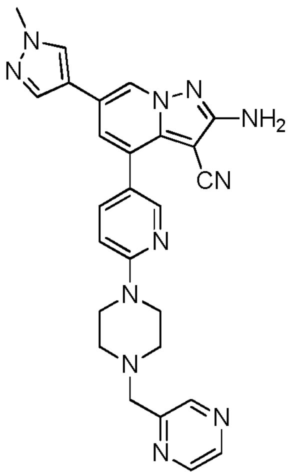

APS064

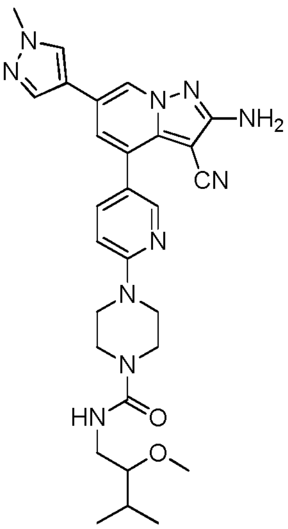

APS065

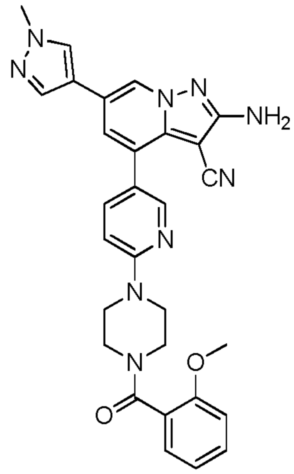

APS066

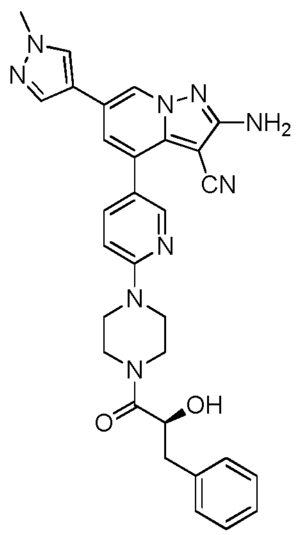

APS067

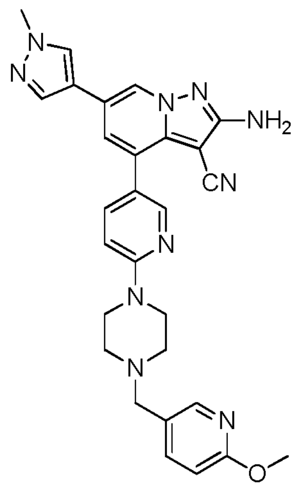

APS068

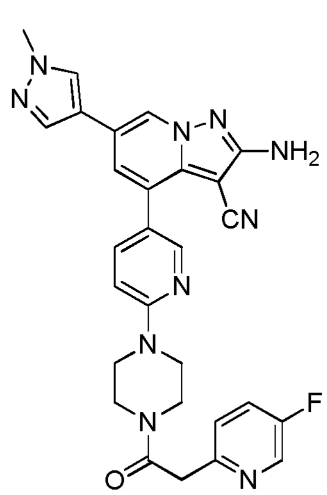

APS069

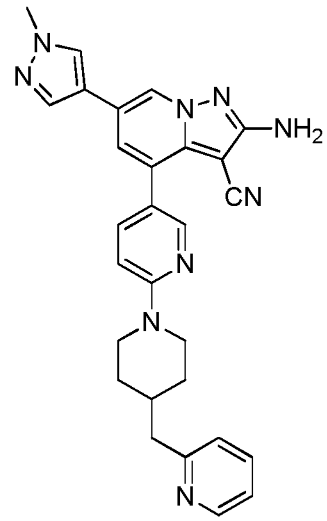

APS070

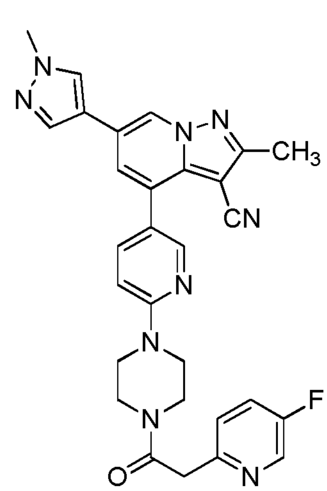

APS071

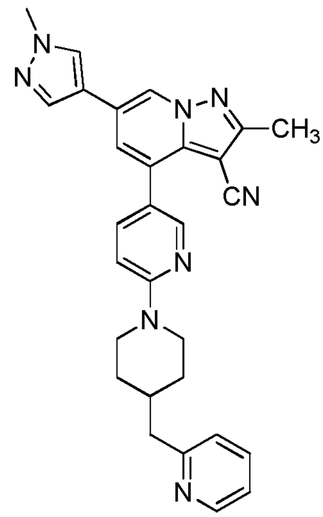

APS072

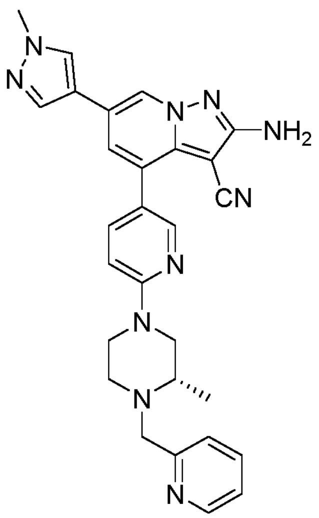

APS073

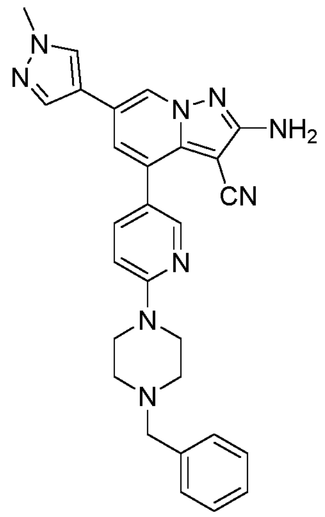

APS074

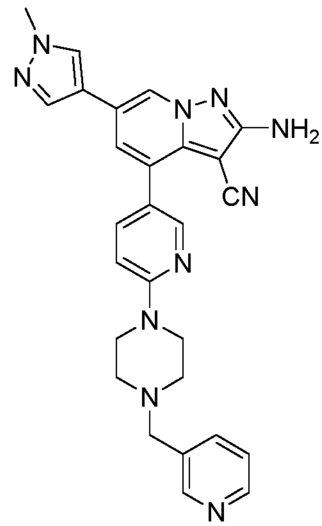

APS075

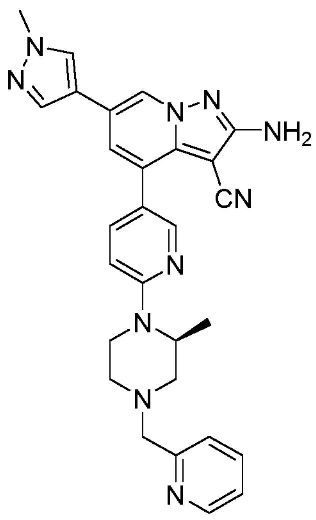

APS076

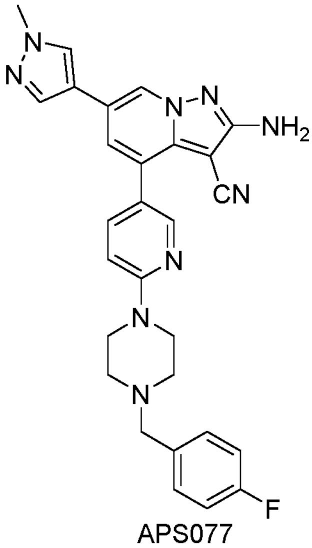

APS077

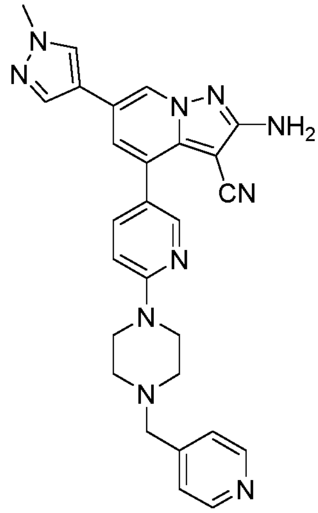

APS078

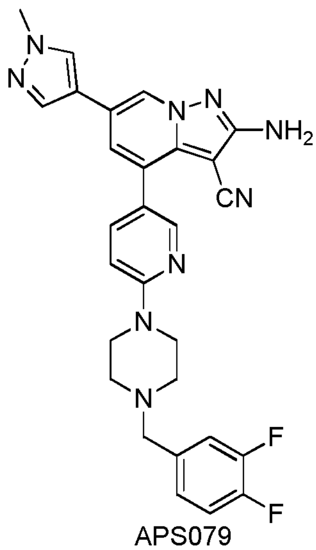

APS079

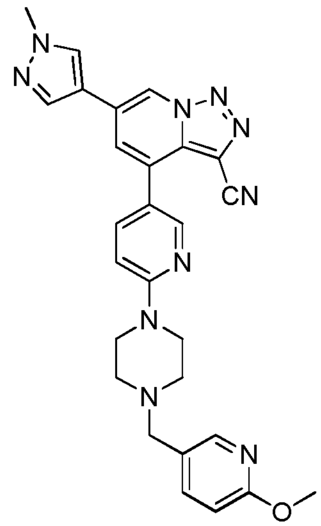

APS081

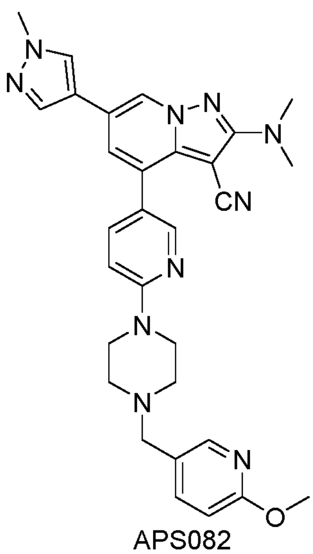

APS082

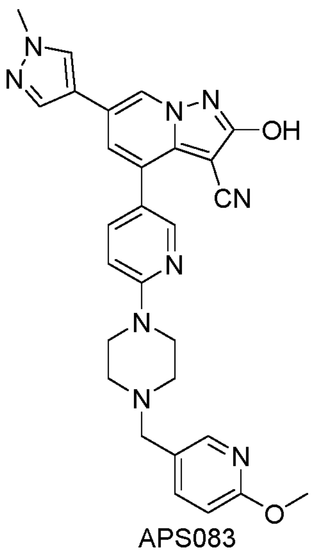

APS083

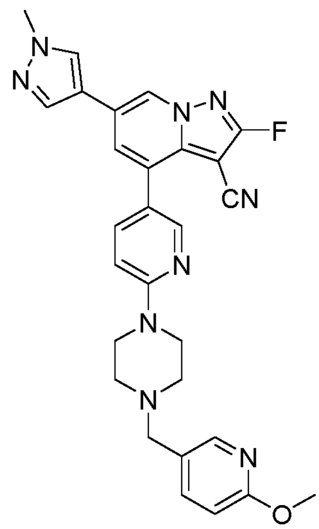

APS084

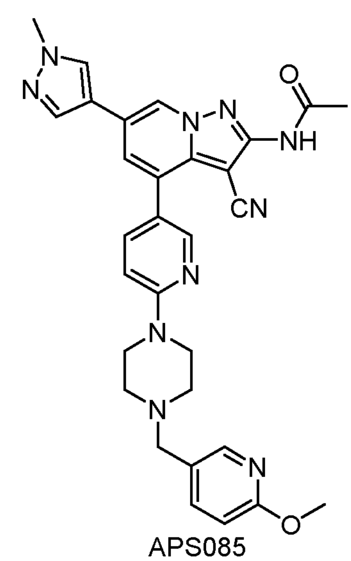

APS085

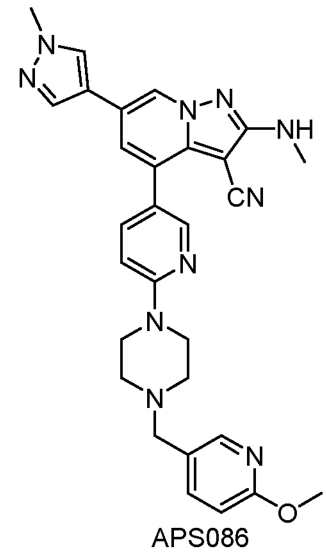

APS086

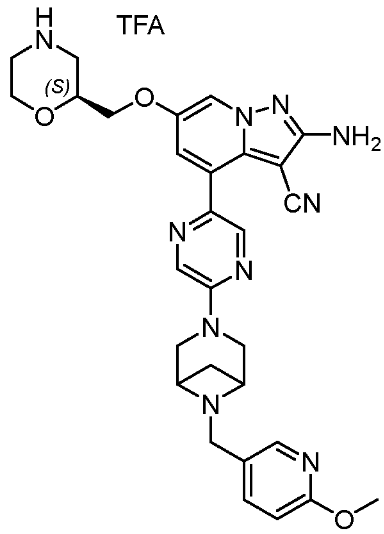

APS087

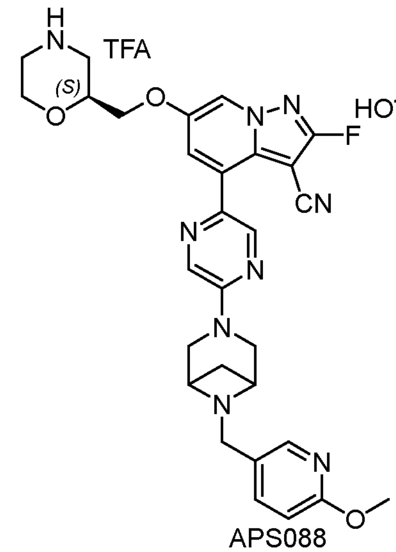

APS088

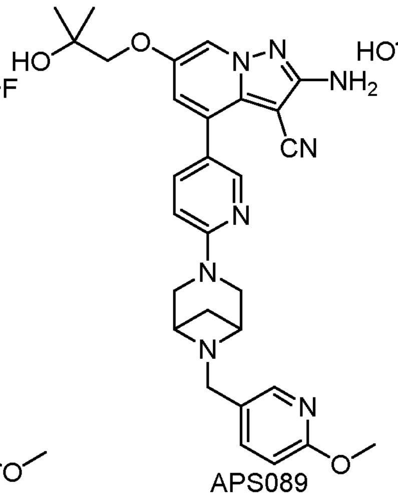

APS089

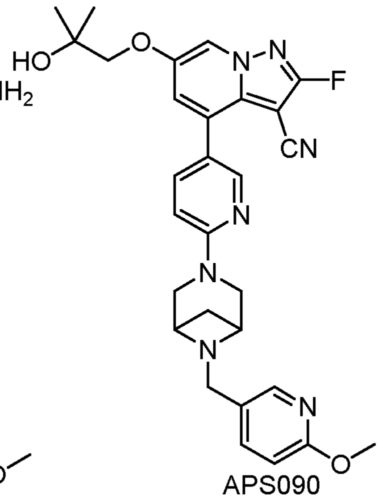

APS090

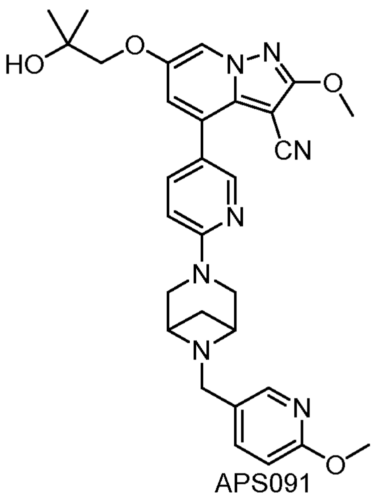

APS091

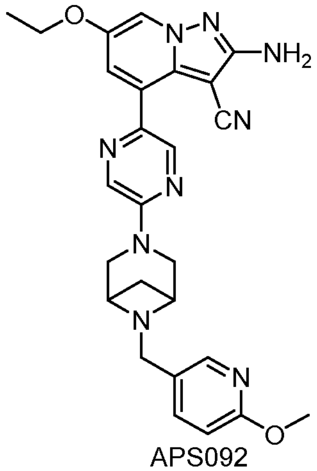

APS092

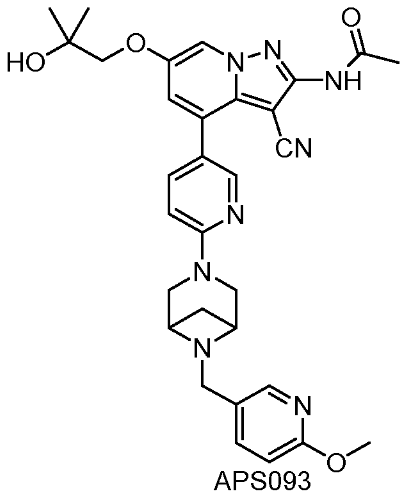

APS093

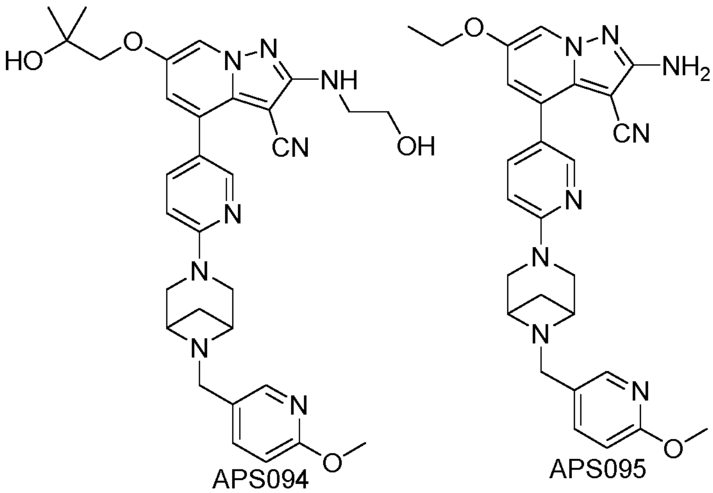

APS094

APS095

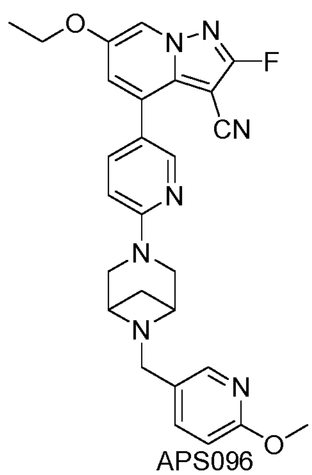

APS096

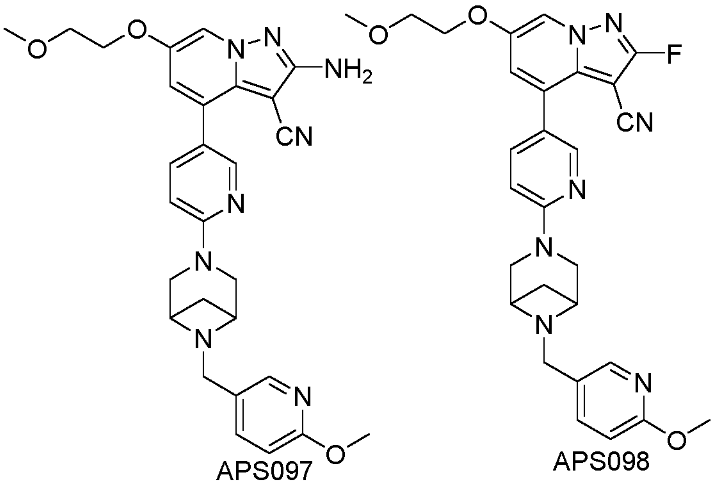

APS097

APS098

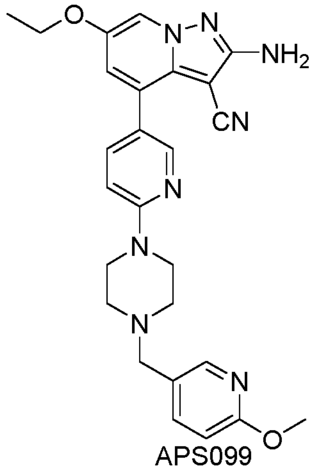

APS099

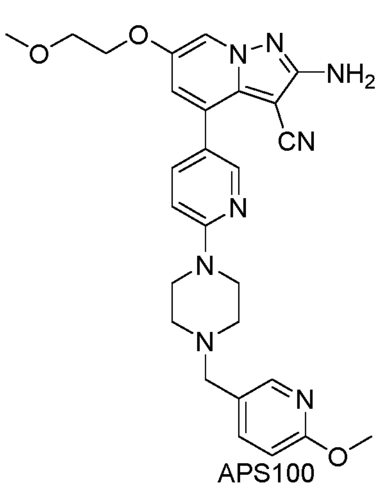

APS100

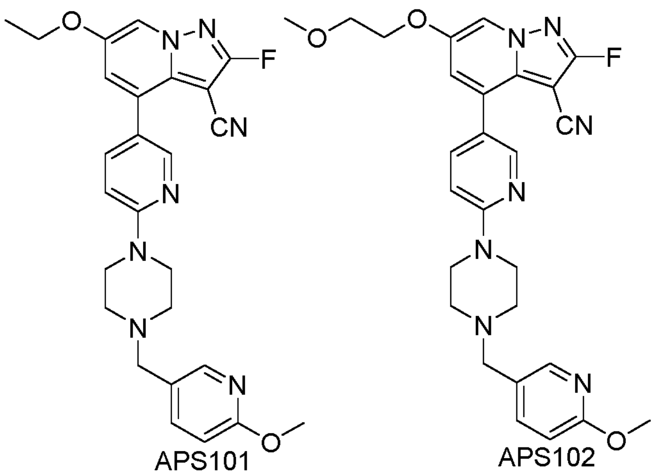

APS101

APS102

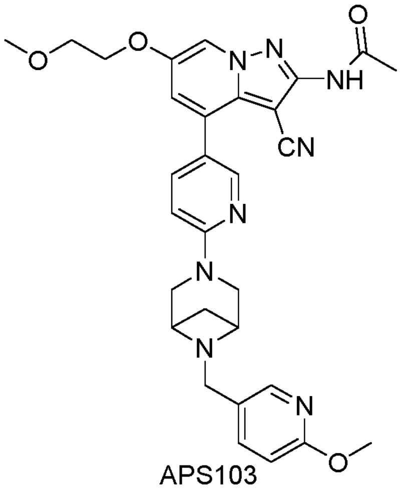

APS103

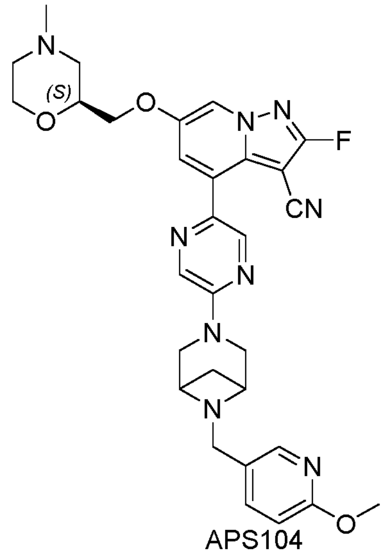

APS104

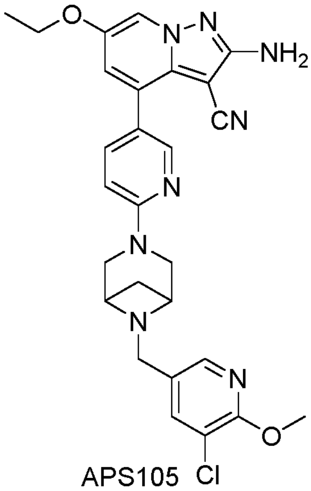

APS105

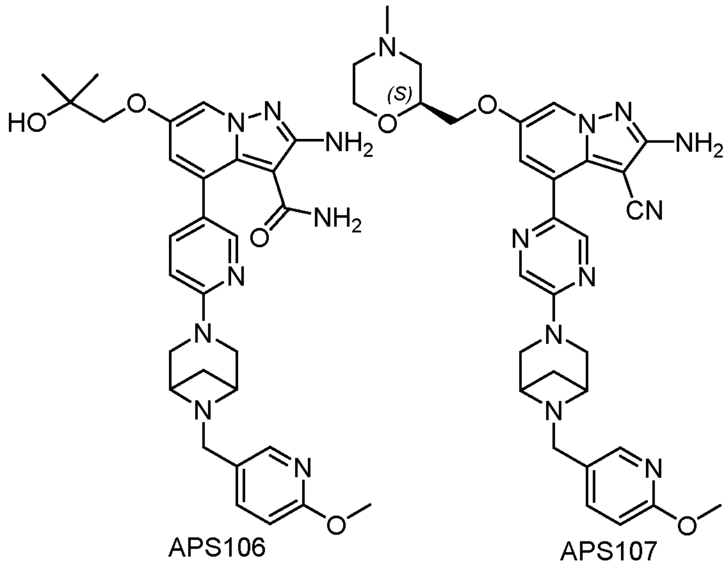

APS106

APS107

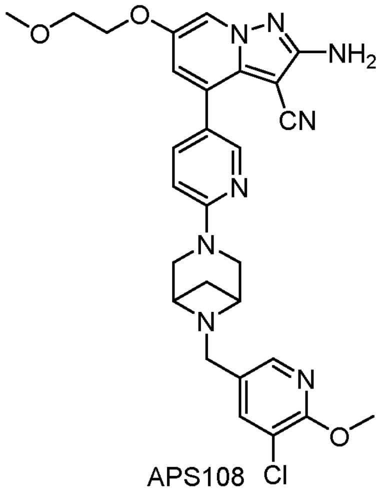

APS108

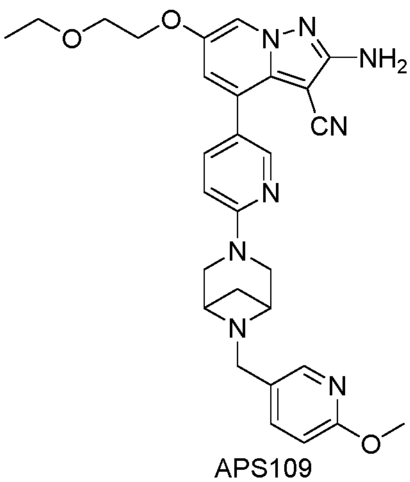

APS109

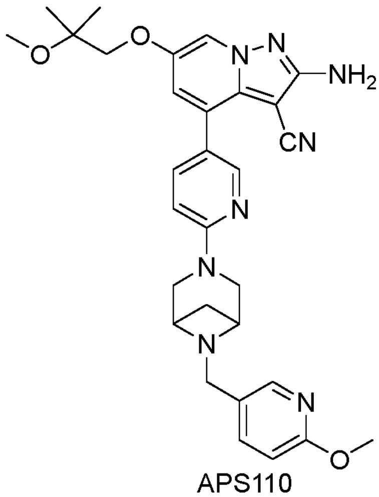

APS110

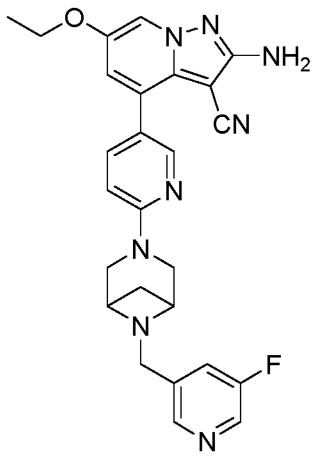

APS111

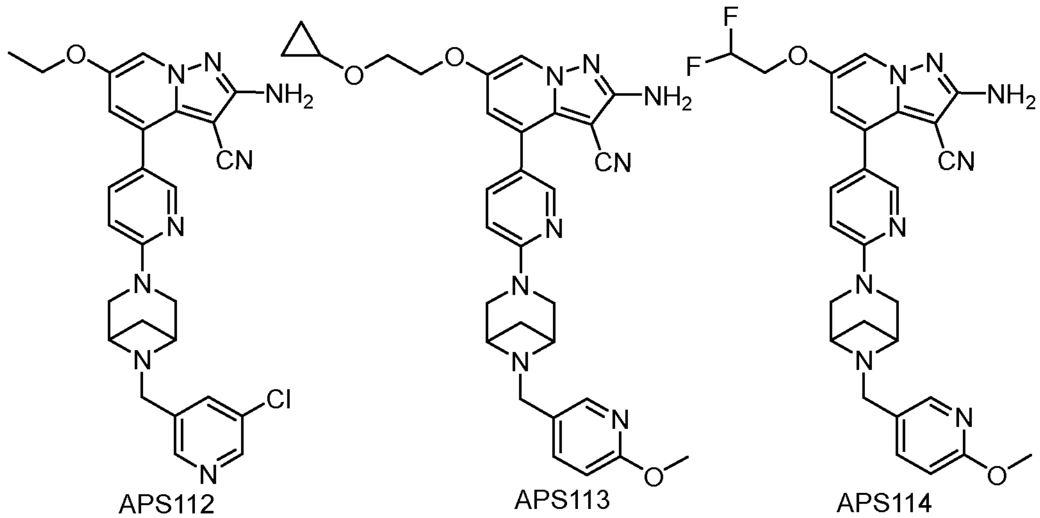

APS112 APS113 APS114

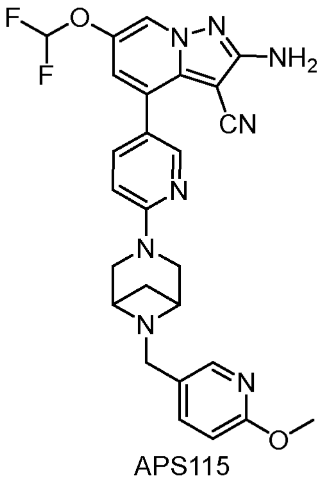

APS115

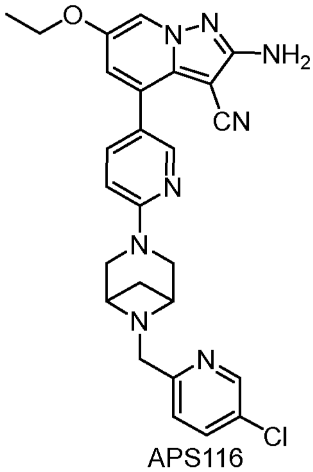

APS116

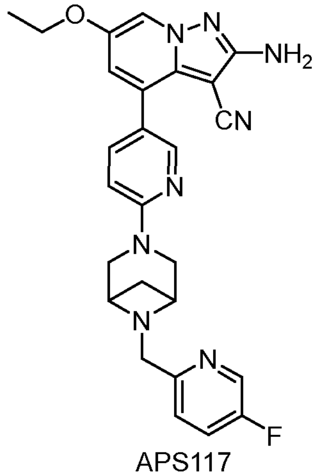

APS117

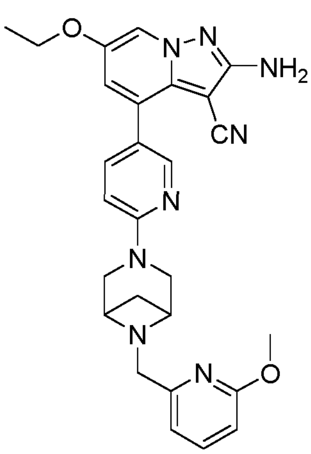

APS118

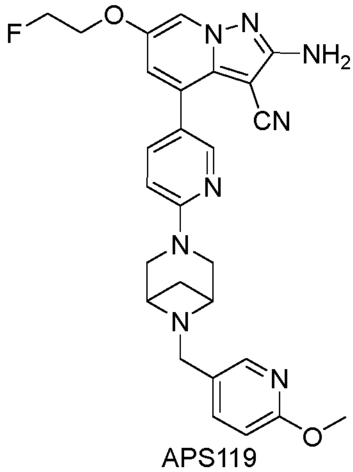

APS119

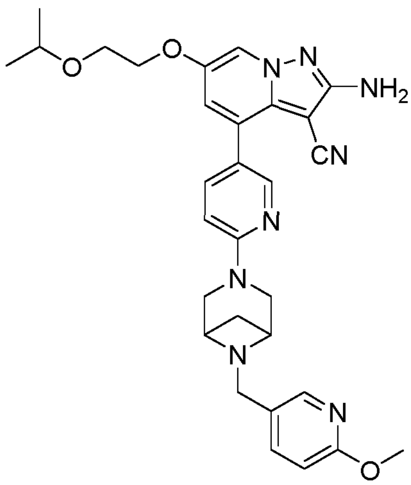

APS120

**5.** A preparation method of the compound according to any one of claims 1 to 4, wherein the preparation method comprises:

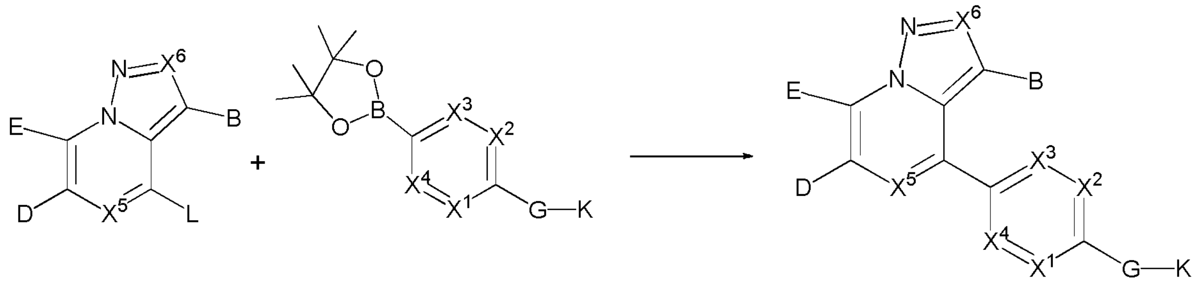

II III I

the compound II undergoes Suzuki reaction with the compound III to obtain the compound I;
wherein, B, D, E, G, K, $X^1$, $X^2$, $X^3$, $X^4$, $X^5$ and $X^6$ are as defined in any one of claims 1-4 respectively, provided that B in the formula III represents boron; L is selected from a leaving group such as halogen and OTf.

**6.** A pharmaceutical composition, comprising a therapeutically effective amount of at least one of the compound, or the stereoisomer, racemate, tautomer, isotope labelled derivative, nitrogen oxide, pharmaceutically acceptable salt or solvate thereof according to any one of claims 1 to 4.

**7.** The pharmaceutical composition according to claim 8, wherein the pharmaceutical composition further comprises one or more pharmaceutically acceptable carriers or excipients.

**8.** The pharmaceutical composition according to claim 6 or 7, wherein the pharmaceutical composition further comprises one or more additional therapeutic agents.

**9.** Use of at least one of the compound, or the stereoisomer, racemate, tautomer, isotope labelled derivative, nitrogen oxide, pharmaceutically acceptable salt and solvate thereof according to any one of claims 1 to 4 in the preparation of a medicament for treating a RET kinase-mediated disease; or,for inhibiting RET kinase activity; orfor treating a RET-related disease or conditions; or
use of the same in the preparation of a medicament for treating cancer and/or inhibiting metastasis associated with a specific cancer; or
use of the same in the preparation of a medicament for treating irritable bowel syndrome (IBS) or pain associated with IBS; or
use of the same in the preparation of a medicament for providing supportive care to a cancer patient; or
use of the same in the preparation of a medicament for inhibiting the activity of RET kinase; or
use of the same in the preparation of a medicament for treating a RET-related disease or disorder.

**10.** The use according to claim 9, wherein the RET kinase-mediated disease comprises cancer, irritable bowel syndrome or their related pain.

**INTERNATIONAL SEARCH REPORT**

International application No.
**PCT/CN2019/109502**

**A. CLASSIFICATION OF SUBJECT MATTER**

C07D 471/04(2006.01)i； C07D 487/04(2006.01)i； A61K 31/4985(2006.01)i； A61K 31/4162(2006.01)i； A61P 35/00(2006.01)i； A61P 1/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

CNABS, DWPI, CNTXT, USTXT, EPTXT, CNKI, STN: 王志健, 志健金瑞, 吡唑, 吡啶, 吡唑并吡啶, 吡嗪, 二唑, 吡唑并吡嗪, 吡嗪并二唑, RET激酶, 抑制剂, 癌症, 肿瘤, 肠易激综合征, 铃木反应, 偶联, pyrazolo+, pyrazole, pyrazine, pyridine, pyrido+, RET kinase inhibitor, cancer, tumor, irritable bowel syndrome, suzuki, coupling

**C. DOCUMENTS CONSIDERED TO BE RELEVANT**

| Category* | Citation of document, with indication, where appropriate, of the relevant passages | Relevant to claim No. |
|---|---|---|
| X | CN 108349969 A (ARRAY BIOPHARMA INC.) 31 July 2018 (2018-07-31)<br>description, paragraphs [0008]-[0016], [0024], [0025], [0030], [0723], and [0726], and embodiments | 1-10 |
| X | WO 2018071447 A1 (ANDREWS, S. W. et al.) 19 April 2018 (2018-04-19)<br>description, paragraphs [0007]-[0010], [0013], [0014], [0028], and [00497]-[00500], and embodiments | 1-10 |
| X | WO 2018136661 A1 (ANDREWS, S. W. et al.) 26 July 2018 (2018-07-26)<br>description, paragraphs [0007]-[0010], [0013], [0014], [0028], and [00497]-[00500], and embodiments | 1-10 |
| A | CN 103492386 A (GALAPAGOS NV) 01 January 2014 (2014-01-01)<br>entire description | 1-10 |
| A | CN 102827073 A (AGIOS PHARMACEUTICALS, INC.) 19 December 2012 (2012-12-19)<br>entire description | 1-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 December 2019** | **30 December 2019** |

Name and mailing address of the ISA/CN

**China National Intellectual Property Administration (ISA/CN)**
**No. 6, Xitucheng Road, Jimenqiao Haidian District, Beijing 100088**
**China**

Facsimile No. **(86-10)62019451**

Authorized officer

Telephone No.

## INTERNATIONAL SEARCH REPORT

International application No.
**PCT/CN2019/109502**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9**（部分）
   because they relate to subject matter not required to be searched by this Authority, namely:

   [1] The subject matter of claim 9 (partially) belongs to a method for treatment of a disease of the human body stated in PCT Rule 39.1(IV). The present search report is performed based on the use of the compound or pharmaceutical composition in preparation of a drug for treating corresponding diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2019/109502**

| Patent document cited in search report | Publication date (day/month/year) | Patent family member(s) | Publication date (day/month/year) |
|---|---|---|---|
| CN 108349969 A | 31 July 2018 | US 2017096425 A1 | 06 April 2017 |
| | | US 10174027 B2 | 08 January 2019 |
| | | BR 112018000808 A2 | 04 September 2018 |
| | | IL 256898 D0 | 29 March 2018 |
| | | PH 12018500121 A1 | 23 July 2018 |
| | | MX 2018000577 A | 05 September 2018 |
| | | US 10138243 B2 | 27 November 2018 |
| | | US 2019127373 A1 | 02 May 2019 |
| | | US 10023570 B2 | 17 July 2018 |
| | | US 2019127374 A1 | 02 May 2019 |
| | | EP 3322706 A1 | 23 May 2018 |
| | | EA 201890318 A1 | 31 August 2018 |
| | | US 2019127375 A1 | 02 May 2019 |
| | | US 2018179203 A1 | 28 June 2018 |
| | | CL 2018000119 A1 | 29 June 2018 |
| | | US 2018186791 A1 | 05 July 2018 |
| | | KR 20180041135 A | 23 April 2018 |
| | | CA 2992586 A1 | 19 January 2017 |
| | | US 2018186790 A1 | 05 July 2018 |
| | | AU 2016291676 A1 | 08 February 2018 |
| | | WO 2017011776 A1 | 19 January 2017 |
| | | US 10174028 B2 | 08 January 2019 |
| | | JP 2018532690 A | 08 November 2018 |
| WO 2018071447 A1 | 19 April 2018 | IL 265916 D0 | 30 June 2019 |
| | | CO 2019004650 A2 | 21 May 2019 |
| | | CA 3039760 A1 | 19 April 2018 |
| | | US 2018134702 A1 | 17 May 2018 |
| | | EP 3523301 A1 | 14 August 2019 |
| | | KR 20190076976 A | 02 July 2019 |
| | | US 10172851 B2 | 08 January 2019 |
| | | US 2018133200 A1 | 17 May 2018 |
| | | DO P2019000090 A | 30 June 2019 |
| | | US 2018133213 A1 | 17 May 2018 |
| | | US 2019183886 A1 | 20 June 2019 |
| | | PH 12019500775 A1 | 01 July 2019 |
| | | BR 112019007144 A2 | 02 July 2019 |
| | | US 10137124 B2 | 27 November 2018 |
| | | TW 201825488 A | 16 July 2018 |
| | | CN 110382494 A | 25 October 2019 |
| | | US 10112942 B2 | 30 October 2018 |
| | | AU 2017342022 A1 | 23 May 2019 |
| | | SG 11201903144P A | 30 May 2019 |
| WO 2018136661 A1 | 26 July 2018 | CN 110267960 A | 20 September 2019 |
| | | CA 3049136 A1 | 26 July 2018 |
| CN 103492386 A | 01 January 2014 | MX 360697 B | 14 November 2018 |
| | | TW I536990 B | 11 June 2016 |
| | | MX 2013012281 A | 21 November 2013 |
| | | US 9987272 B2 | 05 June 2018 |
| | | BR 112013027338 A2 | 08 August 2017 |
| | | HK 1195311 A1 | 24 June 2016 |

Form PCT/ISA/210 (patent family annex) (January 2015)

**INTERNATIONAL SEARCH REPORT**
**Information on patent family members**

International application No.
**PCT/CN2019/109502**

| Patent document cited in search report | Publication date (day/month/year) | Patent family member(s) | Publication date (day/month/year) |
|---|---|---|---|
| | | SG 194508 A1 | 30 December 2013 |
| | | MX 355653 B | 26 April 2018 |
| | | UY 34037 A | 30 November 2012 |
| | | JP 2014512405 A | 22 May 2014 |
| | | CN 103492386 B | 09 March 2016 |
| | | US 2012277247 A1 | 01 November 2012 |
| | | EP 2702058 B1 | 19 August 2015 |
| | | AU 2012247482 B2 | 05 March 2015 |
| | | ZA 201307793 B | 29 April 2015 |
| | | KR 20140025430 A | 04 March 2014 |
| | | US 2016213666 A1 | 28 July 2016 |
| | | MX 2013012282 A | 21 November 2013 |
| | | EP 2702059 B1 | 19 August 2015 |
| | | US 2018078547 A1 | 22 March 2018 |
| | | TW 201247202 A | 01 December 2012 |
| | | KR 101934707 B1 | 03 January 2019 |
| | | CA 2833963 A1 | 01 November 2012 |
| | | AU 2012247484 B2 | 19 February 2015 |
| | | US 9241931 B2 | 26 January 2016 |
| | | US 8975406 B2 | 10 March 2015 |
| | | US 8802682 B2 | 12 August 2014 |
| | | JP 2014512404 A | 22 May 2014 |
| | | WO 2012146657 A1 | 01 November 2012 |
| | | JP 5947371 B2 | 06 July 2016 |
| | | IL 228786 D0 | 31 December 2013 |
| | | AU 2012247482 A1 | 28 March 2013 |
| | | US 2015031706 A1 | 29 January 2015 |
| | | US 2014051677 A1 | 20 February 2014 |
| | | CA 2833942 A1 | 01 November 2012 |
| | | EP 2702058 A1 | 05 March 2014 |
| | | EA 201391584 A1 | 30 April 2014 |
| | | JP 5947372 B2 | 06 July 2016 |
| | | EA 024743 B1 | 31 October 2016 |
| | | WO 2012146659 A1 | 01 November 2012 |
| | | NZ 617528 A | 25 September 2015 |
| | | EP 2702059 A1 | 05 March 2014 |
| | | CN 103492385 B | 11 May 2016 |
| | | AU 2012247484 A1 | 28 March 2013 |
| | | HK 1195312 A1 | 24 June 2016 |
| | | CA 2833963 C | 17 September 2019 |
| | | AR 086042 A1 | 13 November 2013 |
| | | CN 103492385 A | 01 January 2014 |
| | | IL 228786 A | 30 April 2017 |
| CN 102827073 A | 19 December 2012 | AR 086977 A1 | 05 February 2014 |
| | | AU 2012269648 A1 | 16 January 2014 |
| | | MX 345928 B | 23 February 2017 |
| | | US 2016229876 A1 | 11 August 2016 |
| | | EP 2721019 A1 | 23 April 2014 |
| | | JP 2014519518 A | 14 August 2014 |
| | | WO 2012171337 A1 | 20 December 2012 |

Form PCT/ISA/210 (patent family annex) (January 2015)

**INTERNATIONAL SEARCH REPORT**
**Information on patent family members**

International application No.
**PCT/CN2019/109502**

| Patent document cited in search report | Publication date (day/month/year) | Patent family member(s) | | | Publication date (day/month/year) |
|---|---|---|---|---|---|
| | | EP | 2721019 | A4 | 19 November 2014 |
| | | AU | 2012269648 | B2 | 20 April 2017 |
| | | CA | 2839675 | A1 | 20 December 2012 |
| | | US | 9856279 | B2 | 02 January 2018 |
| | | JP | 6152098 | B2 | 21 June 2017 |
| | | EP | 2721019 | B1 | 08 August 2018 |
| | | TW | I628165 | B | 01 July 2018 |
| | | TW | 201317215 | A | 01 May 2013 |
| | | US | 2014213580 | A1 | 31 July 2014 |
| | | MX | 2013014922 | A | 11 February 2014 |
| | | ES | 2694160 | T3 | 18 December 2018 |
| | | CA | 2839675 | C | 11 June 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201811162497
- CN 108349969 A


### Non-patent literature cited in the description


- *Cell,* 1985, vol. 42 (2), 581-588
- *Mol Cell Endocrinol,* 2010, vol. 322 (1-2), 2-7
- *J. Clin. Oncol.,* 2012, vol. 30 (2), 200-202
- *Cell,* 1990, vol. 60 (4), 557-563
- *hyroid,* 2009, vol. 19 (6), 565-612
- *Endocr Rev,* 2006, vol. 27 (5), 535-560
- *Proc Natl Acad Sci USA,* 2000, vol. 97 (1), 268-273
- *Nat Med,* 2012, vol. 18 (3), 375-377
- *Cancer,* 2013, vol. 119 (8), 1486-1494
- *Genome Res,* 2012, vol. 22 (3), 436-445
- *Cancer,* 2011, vol. 117 (12), 2709-2718
- **QIAN et al.** *Mol cancer,* 2014, vol. 13, 176
- *Nature Reviews cancer,* 2014, vol. 14, 173-186